# EUROPEAN PATENT APPLICATION

(11) **EP 1 810 677 A1**
(43) Date of publication of application: **25.07.2007**
(21) Application number: 05793826.8
(22) Date of filing: 07.10.2005
(51) Int. Cl.: A61K 31/4184, A61K 31/437, A61K 31/4439, A61K 31/454, A61P 1/18, A61P 3/00, A61P 3/04, A61P 3/06, A61P 3/10, A61P 5/50

(54) **RECEPTOR FUNCTION REGULATING AGENT**

(30) Priority: 08.10.2004 JP 2004296963
(71) Applicant: Takeda Pharmaceutical Company Limited, Osaka-shi, Osaka 541-0045 (JP)
(72) Inventor: ITOH, Fumio, c/o TAKEDA PHARMACEUTICAL COMPANY LTD, Osaka-shi, Osaka 532-8686 (JP); SASAKI, Satoshi, c/o TAKEDA PHARMACEUTICAL CO LTD, Tsukuba-shi, Ibaraki 3004293 (JP); MIYAZAKI, Junichi, c/o TAKEDA PHARMA. CO. LTD., Osaka-shi, Osaka 5328686 (JP); SUZUKI, Nobuhiro, c/o TAKEDA PHARMA. COMPANY LTD., Tsukuba-shi, Ibaraki 3004293 (JP)
(74) Representative: Rickard, Timothy Mark Adrian
(86) International application number: PCT/JP2005/018908
(87) International publication number: WO 2006/038738

(57) **Abstract**

The present invention relates to a GPR40 receptor function regulator comprising a fused imidazole compound represented by the formula: 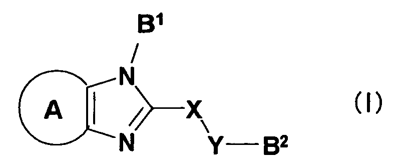 wherein each symbol is as defined in the specification, or a salt thereof or a prodrug thereof. The GPR40 receptor function regulator is useful as an agent for the prophylaxis or treatment of obesity, hyperinsulinemia, type 2 diabetes and the like.

## Description

### Technical Field

The present invention relates to a GPR40 receptor function regulator useful as an agent for the prophylaxis or treatment of obesity, hyperinsulinemia, type 2 diabetes and the like.

### Background Art

It has been reported that GPR40 is one of the G Protein-coupled Receptor (GPCR), and GPR40 agonist and antagonist are useful as a therapeutic drug for type 2 diabetes, obesity, impaired glucose tolerance, insulin resistance, neurodegenerative disease (e.g., Alzheimer's disease) and the like (see WO02/057783).

In addition, it has been reported that a "compound having an aromatic ring and a group capable of releasing cation" is useful as a GPR40 receptor function regulator, GPR40 agonist is useful as an agent for the prophylaxis or treatment of diseases such as diabetes, impaired glucose tolerance, ketosis, acidosis, diabetic neuropathy, diabetic nephropathy, diabetic retinopathy, hyperlipidemia, sexual dysfunction, dermatic diseases, arthropathy, osteopenia, arteriosclerosis, thrombotic disease, dyspepsia, deficits in memory and learning and the like, and GPR40 antagonist is useful as an agent for the prophylaxis or treatment of diseases such as obesity, hyperlipidemia, type 2 diabetes, hypoglycemia, hypertension, diabetic neuropathy, diabetic nephropathy, diabetic retinopathy, edema, insulin resistance syndrome, unstable diabetes, fatty atrophy, insulin allergy, insulinoma, arteriosclerosis, thrombotic disease, lipotoxicity, hyperinsulinemia, cancer and the like (see WO04/041266).

As fused imidazole compounds, the following compounds have been reported.

For example, it has been reported that a compound represented by the formula: 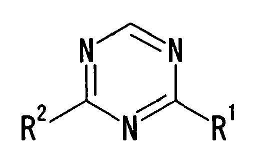 wherein R¹ and R² are each independently R³; R⁸; NHR³; NHR⁵; NHR⁶; NR⁵R⁵; NR⁵R⁶; SR⁵; SR⁶; SR³; OR⁵; OR⁶; OR³; C(O)R³; heterocyclyl optionally substituted by 1 to 4 independent R⁴; or C₁₋₁₀ alkyl optionally substituted by 1 to 4 independent R⁴; and R³ is independently aryl; phenyl optionally substituted by 1 to 5 independent R⁴; or heteroaryl optionally substituted by 1 to 4 independent R⁴,
has kinase activity inhibitory action, and is useful for the treatment of diseases influenced by the kinase activity (see US2004/0116388).

It has been reported that a compound represented by the formula: 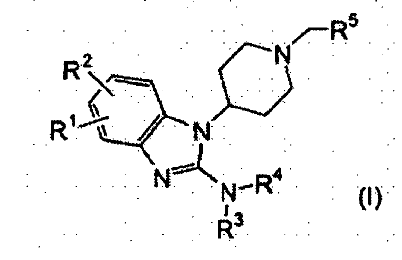 wherein
R¹ and R² are each independently a hydrogen atom, halo, hydroxy, C₁₋₄ alkyl, halo C₁₋₄ alkyl and the like; R³ and R⁴ are each independently a hydrogen atom, halo C₁₋₁₀ alkyl, optionally substituted C₁₋₆ alkyl and the like; or R³ and R⁴ form, together with the nitrogen atom they are bonded to, fully saturated, partially saturated or fully unsaturated heterocycle optionally substituted; and R⁵ is C₄₋₁₁ cycloalkyl and the like, has ORL-1 receptor agonist activity, and is useful as an analgesic drug (see EP1069124).

It has been reported that a compound represented by the formula: 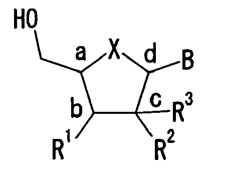 wherein
R¹ is a hydrogen atom, hydroxy, alkyl, hydroxy alkyl, alkoxy, halogen, cyano, isocyano or azido; R² is a hydrogen atom, hydroxy, alkoxy, chlorine, bromine or iodine; R³ is a hydrogen atom; or R² and R³ in combination form =CH₂; or R² and R³ are fluorines; X is O, S or CH₂; a, b, c and d are asymmetric carbon atoms substituted by 4 different substituents; and B is a purine base, an oxidized purine base or a pyrimidine base, is useful as a hepatitis C virus RNA replication inhibitor (see WO02/18404).

It has been reported that a compound represented by the formula: 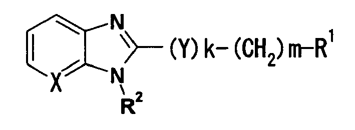 wherein
X is N or CH; Y is O, S, SO, SO₂ or NH; k is 0 or 1; m is 0, 1 or 2; R¹ is a phenyl group, a pyridyl group, a dihydropyridyl-4-one group; and R² is a phenyl group, a pyridyl group, a substituted phenyl group and the like,
is useful as a therapeutic agent for hepatic diseases (see WO99/57103).

It has been reported that a compound represented by the formula: 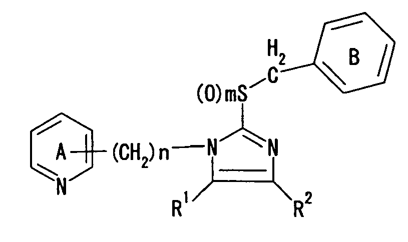 wherein
ring A is a pyridyl group or a substituted pyridyl group; ring B is a phenyl group or a substituted phenyl group; R¹ and R² are hydrogen atoms, or R¹ and R² in combination form -(CH₂)q-; m is 1 or 2; n is 0, 1 or 2; and q is 3 or 4,
is useful as an antiulcer agent (see USP4996217).

It has been reported that a compound represented by the formula: 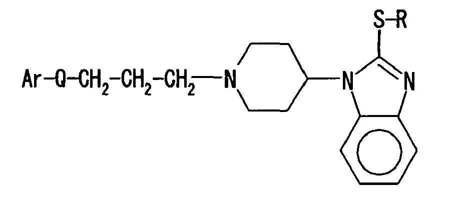 wherein
Q is a carbonyl group or a -CH(Ar)- group; Ar is a phenyl group or a halophenyl group; and R is a linear or branched lower alkyl group, an aralkyl group, a benzoyl lower alkyl group and the like,
is useful as a psychotropic drug (see JP-A-51-70766).

It has been reported that a compound represented by the formula: 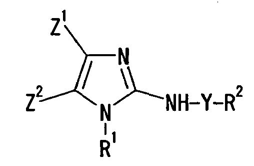 wherein
R¹ is a hydrogen atom, C₁₋₈ alkyl, hetero C₁₋₈ alkyl, fluoro C₁₋₄ alkyl, cycloalkyl C₁₋₈ alkyl, heterocyclo C₁₋₈ alkyl, aryl, aryl C₁₋₈ alkyl, cyclo C₃₋₈ alkyl-C₁₋₈ alkyl, cycle C₃₋₈ alkylhetero C₁₋₈ alkyl, heterocyclo C₁₋₈ alkyl, arylhetero C₁₋₈ alkyl or heteroaryl; R² is C₁₋₈ alkyl, hetero C₁₋₈ alkyl, perfluoro C₁₋₄ alkyl, aryl or heteroaryl; Y is C(O), S(O)m, S(O)₂NR', C(O)NR', CR³R⁴, C(NR'), C(=CR³R⁴), CR³(OR') or CR³(NR'R"); m is 1 or 2; Z¹ and Z² are each independently H, halogen, CN, CO₂R', CONR'R", C₁₋₄ alkyl, C₁₋₄ heteroalkyl, perfluoro C₁₋₄ alkyl, aryl, heteroaryl, NR'R" or OR"; Z¹ and Z² in combination form fused 5-, 6-, 7- or 8-membered cycloalkane, heterocycloalkane, or an aromatic or heteroaromatic ring; R³ and R⁴ are each independently H and the like; and R' and R" are each independently H and the like,
regulates kinases relating to interleukin-1 receptors, and is useful for the prophylaxis or treatment of inflammation (see WO03/030902).

However, there is no report documenting that these known fused imidazole compounds have a GPR40 receptor function regulating action.

### Disclosure of the Invention

The present invention aims at providing a GPR40 receptor function regulator useful as an agent for the prophylaxis or treatment obesity, hyperinsulinemia, type 2 diabetes and the like.

The present inventors have intensively conducted various studies and found that the fused imidazole compound represented by the following formula (I) unexpectedly has a superior GPR40 receptor function regulating action, and is useful as an agent for the prophylaxis or treatment of pathology or disease involving GPR40, and completed the present invention.

Accordingly, the present invention provides the following.
(1) A GPR40 receptor function regulator comprising a fused imidazole compound represented by the formula: 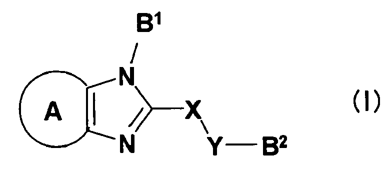 wherein
   ring A is an optionally substituted ring,
   B¹ and B² are each independently an optionally substituted cyclic group,
   X is an optionally substituted C₁₋₃ alkylene group, -O-, -NR^{X}-or -S(O)n^{X}- wherein R^{X} is a hydrogen atom or a substituent, and
   n^{X} is an integer of 0 to 2, and
   Y is a bond, an optionally substituted C₁₋₃ alkylene group; -0-, -NR^{Y}- or -S(O)n^{Y}- wherein R^{Y} is a hydrogen atom or a substituent, and n^{Y} is an integer of 0 to 2,
   or a salt thereof [hereinafter sometimes to be abbreviated as compound (I)] or a prodrug thereof.
(2) The regulator of the aforementioned (1), which is a regulator of a physiological function involving a GPR40 receptor or an agent for the prophylaxis or treatment of a pathology or disease involving GPR40 receptor.
(3) The regulator of the aforementioned (1), which is an insulin secretion regulator, a pancreas protector or an insulin sensitizer.
(4) The regulator of the aforementioned (1), which is an agent for the prophylaxis or treatment of diabetes, diabetic neuropathy, diabetic nephropathy, retinopathy, obesity, metabolic syndrome, insulin resistance, impaired glucose tolerance, hyperinsulinemia, hypertension, hyperlipidemia, arteriosclerosis, cardiac failure, cardiac infarction, thrombotic disease, deficits in memory and learning, depression and mania, visual disorder, appestat disorder, lipotoxicity, pancreatic fatigue, immune disease, inflammatory disease or cancer.
(5) A method of regulating a GPR40 receptor function, which comprises administering an effective amount of the fused imidazole compound or a salt thereof or a prodrug thereof of the aforementioned (1) to a mammal.
(6) Use of the fused imidazole compound or a salt thereof or a prodrug thereof of the aforementioned (1) for the production of a GPR40 receptor function regulator.
(7) A compound represented by the formula: 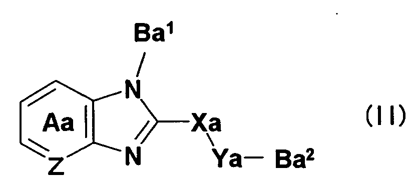 wherein
   ring Aa is a benzene ring substituted by substituent(s) other than a nitro group and a diethylsulfamoyl group, or an optionally substituted pyridine ring,
   Z is CH or N,
   Ba¹ is an optionally substituted 5-membered aromatic group or an optionally substituted 6-membered cyclic group,
   Ba² is an optionally substituted 5- or 6-membered aromatic group,
   Xa is -O-, -NRa- wherein Ra is a hydrogen atom or an optionally substituted C₁₋₆ alkyl group, or -S-, and
   Ya is an optionally substituted C₁₋₃ alkylene group,
   provided that Xa-Ya should not be -NHCO-, and Ba¹ should not be a substituted triazinyl group,
   or a salt thereof,
   with the proviso that 2-(benzylthio)-5-chloro-1-phenyl-1H-benzimidazole and 2-(2-chlorobenzylthio)-5-chloro-1-phenyl-1H-benzimidazole are excluded [hereinafter sometimes to be abbreviated as compound (II)].
(8) The compound of the aforementioned (7), wherein ring Aa is a benzene ring substituted by 1 to 3 halogen atoms.
(9) The compound of the aforementioned (7), wherein Ba¹ is an optionally substituted phenyl group.
(10) The compound of the aforementioned (7), wherein Ba¹ is a phenyl group having a substituent at the para-position.
(11) The compound of the aforementioned (10), wherein the substituent is selected from an optionally substituted C₁₋₆ alkyl group, an optionally substituted C₁₋₆ alkoxy group, a C₁₋₆ alkoxy-carbonyl group and a C₁₋₆ alkyl-carbonyl group.
(12) The compound of the aforementioned (7), wherein Ba² is an optionally substituted phenyl group or an optionally substituted pyridyl group.
(13) The compound of the aforementioned (7), wherein Ba² is a phenyl group having a substituent at the para-position or a 3-pyridyl group having a substituent at the 6-position.
(14) The compound of the aforementioned (13), wherein the substituent is selected from a halogen atom and an optionally halogenated C₁₋₆ alkyl group.
(15) The compound of the aforementioned (7), wherein Xa is -S-or -NH-.
(16) The compound of the aforementioned (7), wherein Ya is a methylene group.
(17) The compound of the aforementioned (7), which is 6-chloro-2-[(4-chlorobenzyl)thio]-1-(4-ethoxyphenyl)-1H-benzimidazole, 6-chloro-1-(4-ethoxyphenyl)-2-({[6-(trifluoromethyl)pyridin-3-yl]methyl}thio)-1H-benzimidazole, 2-[(4-tert-butylbenzyl)thio]-6-chloro-1-(4-ethoxyphenyl)-1H-benzimidazole, 2-[(4-chlorobenzyl)thio]-1-(4-methoxyphenyl)-1H-imidazo[4,5-b]pyridine, 5-chloro-2-[(4-chlorobenzyl)thio]-1-[4-(2,2,2-trifluoroethoxy)phenyl]-1H-benzimidazole, 1-(4-butoxyphenyl)-N-(4-tert-butylbenzyl)-5,6-dichloro-1H-benzimidazol-2-amine, or N-(4-tert-butylbenzyl)-5,6-dichloro-1-[4-(trifluoromethoxy)phenyl]-1H-benzimidazol-2-amine.
(18) A prodrug of the compound of the aforementioned (7).
(19) A pharmaceutical agent comprising the compound of the aforementioned (7) or a prodrug thereof.
(20) A method of antagonizing a GPR40 receptor, which comprises administering an effective amount of a non-peptidic nitrogen-containing heterocyclic compound to a mammal.

The GPR40 receptor function regulator of the present invention is useful as an agent for the prophylaxis or treatment of obesity, hyperinsulinemia, type 2 diabetes and the like.

### Best Mode for Embodying the Invention

Unless otherwise specified, as the "halogen atom" in the present specification, fluorine atom, chlorine atom, bromine atom, iodine atom can be mentioned.

Unless otherwise specified, as the "optionally substituted hydrocarbon group" in the present specification, for example, an "optionally substituted C₁₋₆ alkyl group", an "optionally substituted C₂₋₆ alkenyl group", an "optionally substituted C₂₋₆ alkynyl group", an "optionally substituted C₃₋₈ cycloalkyl group", an "optionally substituted C₆₋₁₄ aryl group", an "optionally substituted C₇₋₁₆ aralkyl group" and the like can be mentioned.

Unless otherwise specified, as the "C₁₋₆ alkyl group" in the present specification, for example, methyl, ethyl, propyl, isopropyl, butyl, isobutyl, sec-butyl, tert-butyl, pentyl, isopentyl, neopentyl, hexyl and the like can be mentioned.

Unless otherwise specified, as the "C₂₋₆ alkenyl group" in the present specification, for example, vinyl, 1-propenyl, allyl, isopropenyl, 2-buten-1-yl, 4-penten-1-yl, 5-hexen-1-yl and the like can be mentioned.

Unless otherwise specified, as the "C₂₋₆ alkynyl group" in the present specification, for example, ethynyl, propargyl, 1-propynyl, 2-butyn-1-yl, 4-pentyn-1-yl, 5-hexyn-1-yl and the like can be mentioned.

Unless otherwise specified, as the "C₃₋₈ cycloalkyl group" in the present specification, for example, cyclopropyl, cyclobutyl, cyclopentyl, cyclohexyl and the like can be mentioned.

Unless otherwise specified, as the "C₆₋₁₄ aryl group" in the present specification, for example, phenyl, 1-naphthyl, 2-naphthyl, 2-biphenylyl, 3-biphenylyl, 4-biphenylyl, 2-anthryl and the like can be mentioned. The C₆₋₁₄ aryl may be optionally saturated partially, and as the partially saturated C₆₋₁₄ aryl, for example, tetrahydronaphthyl and the like can be mentioned. In addition, as the C₆₋₁₄ aryl group, a group derived form a fused ring wherein the below-mentioned "aromatic hydrocarbon" and the below-mentioned "alicyclic hydrocarbon" are condensed, for example, indanyl, indenyl, fluorenyl and the like can be mentioned.

Unless otherwise specified, as the "C₇₋₁₆ aralkyl group" in the present specification, for example, benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, 4-biphenylylmethyl, 9-fluorenylmethyl and the like can be mentioned.

Unless otherwise specified, as the "optionally substituted hydroxy group" in the present specification, for example, a "hydroxy group", an "optionally substituted C₁₋₆ alkoxy group", an "optionally substituted C₃₋₆ alkenyloxy group", an "optionally substituted C₃₋₆ alkynyloxy group", an "optionally substituted C₃₋₈ cycloalkyloxy group", an "optionally substituted heterocyclyloxy group", an "optionally substituted C₆₋₁₄ aryloxy group", an "optionally substituted C₇₋₁₆ aralkyloxy group", an "C₁₋₆ alkyl-carbonyloxy group", an "optionally substituted C₁₋₆ alkylsulfonyloxy group", an "optionally substituted heterocyclylsulfonyloxy group" and the like can be mentioned.

Unless otherwise specified, as the "C₁₋₆ alkoxy group" in the present specification, for example, methoxy, ethoxy, propoxy, isopropoxy, butoxy, isobutoxy, sec-butoxy, tert-butoxy, pentyloxy, hexyloxy and the like can be mentioned.

Unless otherwise specified, as the "C₃₋₆ alkenyloxy group" in the present specification, for example, allyloxy, 2-buten-1-yloxy, 4-penten-1-yloxy, 5-hexen-1-yloxy and the like can be mentioned.

Unless otherwise specified, as the "C₃₋₆ alkynyloxy group" in the present specification, for example, propargyloxy, 2-butyn-1-yloxy, 4-pentyn-1-yloxy, 5-hexyn-1-yloxy and the like can be mentioned.

Unless otherwise specified, as the "C₃₋₈ cycloalkyloxy group" in the present specification, for example, cyclopropyloxy, cyclobutyloxy, cyclopentyloxy, cyclohexyloxy and the like can be mentioned.

As the "heterocyclyloxy group" in the present specification, a hydroxy group substituted by the below-mentioned "heterocyclic group" can be mentioned. As preferable examples of the heterocyclyloxy group, tetrahydropyranyloxy, thiazolyloxy, pyridyloxy, pyrazolyloxy, oxazolyloxy, thienyloxy, furyloxy and the like can be mentioned.

Unless otherwise specified, as the "C₆₋₁₄ aryloxy group" in the present specification, for example, phenoxy, 1-naphthyloxy, 2-naphthyloxy and the like can be mentioned.

Unless otherwise specified, as the "C₇₋₁₆ aralkyloxy group" in the present specification, for example, benzyloxy, phenethyloxy, 9-fluorenylmethyloxy and the like can be mentioned.

Unless otherwise specified, as the "C₁₋₆ alkyl-carbonyloxy group" in the present specification, for example, acetyloxy and the like can be mentioned.

Unless otherwise specified, as the "C₁₋₆ alkylsulfonyloxy group" in the present specification, for example, methylsulfonyloxy, ethylsulfonyloxy and the like can be mentioned.

As the "heterocyclylsulfonyloxy group" in the present specification, a sulfonyloxy group substituted by the below-mentioned "heterocyclic group" can be mentioned. As preferable examples of the heterocyclylsulfonyloxy group, thienylsulfonyloxy, furylsulfonyloxy and the like can be mentioned.

Unless otherwise specified, as the "optionally substituted mercapto group" in the present specification, for example, a "mercapto group", an "optionally substituted C₁₋₆ alkylthio group", an "optionally substituted C₃₋₆ alkenylthio group", an "optionally substituted C₃₋₆ alkynylthio group", an "optionally substituted C₃₋₈ cycloalkylthio group", an "optionally substituted heterocyclylthio group", an "optionally substituted C₆₋₁₄ arylthio group", an "optionally substituted C₇₋₁₆ aralkylthio group" and the like can be mentioned.

Unless otherwise specified, as the "C₁₋₆ alkylthio group" in the present specification, for example, methylthio, ethylthio, propylthio, isopropylthio, butylthio, isobutylthio, sec-butylthio, tert-butylthio and the like can be mentioned.

Unless otherwise specified, as the "C₃₋₆ alkenylthio group" in the present specification, for example, allylthio, 2-buten-1-ylthio, 4-penten-1-ylthio, 5-hexen-1-ylthio and the like can be mentioned.

Unless otherwise specified, as the "C₃₋₆ alkynylthio group" in the present specification, for example, propargylthio, 2-butyn-1-ylthio, 4-pentyn-1-ylthio, 5-hexyn-1-ylthio and the like can be mentioned.

Unless otherwise specified, as the "C₃₋₈ cycloalkylthio group" in the present specification, for example, cyclopropylthio, cyclobutylthio, cyclopentylthio, cyclohexylthio and the like can be mentioned.

As the "heterocyclylthio group" in the present specification, a mercapto group substituted by the below-mentioned "heterocyclic group" can be mentioned. As preferable examples of the heterocyclylthio group, tetrahydropyranylthio, thiazolylthio, pyridylthio, pyrazolylthio, oxazolylthio, thienylthio, furylthio and the like can be mentioned.

Unless otherwise specified, as the "C₆₋₁₄ arylthio group" in the present specification, for example, phenylthio, 1-naphthylthio, 2-naphthylthio and the like can be mentioned.

Unless otherwise specified, as the "C₇₋₁₆ aralkylthio group" in the present specification, for example, benzylthio, phenethylthio and the like can be mentioned.

Unless otherwise specified, as the "heterocyclic group" in the present specification, for example, a 3- to 14-membered (monocyclic, bicyclic or tricyclic) heterocyclic group containing, as a ring-constituting atom besides carbon atoms, one or two kinds of 1 to 4 heteroatoms selected from a nitrogen atom, a sulfur atom and an oxygen atom, preferably (i) a 5- to 14-membered (preferably 5- to 10-membered) aromatic heterocyclic group, (ii) a 3- to 10-membered non-aromatic heterocyclic group and the like can be mentioned. Of these, a 5 or 6-membered aromatic heterocyclic group is preferable. The above-mentioned nitrogen atom and the sulfur atom as the ring-constituting atom may be oxidized into N-oxide, S-oxide or S,S-dioxide.

Specifically, as the "heterocyclic group", for example, 5-membered aromatic heterocyclic groups such as thienyl (e.g., 2-thienyl, 3-thienyl), furyl (e.g., 2-furyl, 3-furyl), thiazolyl (e.g., 2-thiazolyl, 4-thiazolyl, 5-thiazolyl), oxazolyl (e.g., 2-oxazolyl, 4-oxazolyl, 5-oxazolyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), isothiazolyl (e.g., 3-isothiazolyl, 4-isothiazolyl, 5-isothiazolyl), isoxazolyl (e.g., 3-isoxazolyl, 4-isoxazolyl, 5-isoxazolyl) and the like; 6-membered aromatic heterocyclic groups such as pyridyl (e.g., 2-pyridyl, 3-pyridyl, 4-pyridyl), pyrazinyl, pyrimidinyl (e.g., 2-pyrimidinyl, 4-pyrimidinyl), pyridazinyl (e.g., 3-pyridazinyl, 4-pyridazinyl) and the like;
bicyclic aromatic heterocyclic groups such as quinolyl (e.g., 2-quinolyl, 3-quinolyl, 4-quinolyl, 5-quinolyl, 8-quinolyl), isoquinolyl (e.g., 1-isoquinolyl, 3-isoquinolyl, 4-isoquinolyl, 5-isoquinolyl), quinazolinyl (e.g., 2-quinazolinyl, 4-quinazolinyl, 5-quinazolinyl, 6-quinazolinyl), quinoxalinyl (e.g., 2-quinoxalinyl, 5-quinoxalinyl, 6-quinoxalinyl), cinnolinyl (e.g., 3-cinnolinyl, 4-cinnolinyl, 5-cinnolinyl, 6-cinnolinyl), phthalazinyl (e.g., 1-phthalazinyl, 5-phthalazinyl, 6-phthalazinyl), indolyl (e.g., 1-indolyl, 2-indolyl, 3-indolyl), benzothiazolyl (e.g., 2-benzothiazolyl), benzoxazolyl (e.g., 2-benzoxazolyl), benzimidazolyl (e.g., 1-benzimidazolyl, 2-benzimidazolyl), benzo[b]furanyl (e.g., 2-benzo[b]furanyl, 3-benzo[b]furanyl), benzo[c]furanyl (e.g., 1-benzo[c]furanyl), benzo[b]thienyl (e.g., 2-benzo[b]thienyl, 3-benzo[b]thienyl), benzo[c]thienyl (e.g., 1-benzo[c]thienyl) and the like;
3-membered non-aromatic heterocyclic groups such as oxiranyl (e.g., 2-oxiranyl), thiiranyl (e.g., 2-thiiranyl), aziridinyl (e.g., 1-aziridinyl, 2-aziridinyl) and the like; 4-membered non-aromatic heterocyclic groups such as oxetanyl (e.g., 2-oxetanyl), thietanyl (e.g., 2-thietanyl), azetidinyl (e.g., 1-azetidinyl, 2-azetidinyl, 3-azetidinyl) and the like; 5-membered non-aromatic heterocyclic groups such as pyrrolidinyl (e.g., 1-pyrrolidinyl, 2-pyrrolidinyl, 3-pyrrolidinyl), oxazolidinyl (e.g., 2-oxazolidinyl), thiazolidinyl (e.g., 2-thiazolidinyl), imidazolinyl (e.g., 1-imidazolinyl, 2-imidazolinyl, 4-imidazolinyl), dioxolanyl (e.g., 2-dioxolanyl), oxazolinyl (e.g., 2-oxazolinyl), thiazolinyl (e.g., 2-thiazolinyl), tetrahydrofuranyl (e.g., 2-tetrahydrofuranyl), tetrahydrothienyl (e.g., 2-tetrahydrothienyl) and the like;
6-membered non-aromatic heterocyclic groups such as piperidinyl (e.g., 1-piperidinyl, 2-piperidinyl, 3-piperidinyl, 4-piperidinyl), piperazinyl (e.g., 1-piperazinyl, 2-piperazinyl), morpholinyl (e.g., 2-morpholinyl, 3-morpholinyl, 4-morpholinyl), thiomorpholinyl (e.g., 2-thiomorpholinyl, 3-thiomorpholinyl, 4-thiomorpholinyl), tetrahydropyranyl (e.g., 2-tetrahydropyranyl), tetrahydrothiopyranyl (e.g., 2-tetrahydrothiopyranyl), dioxanyl (e.g., 1,4-dioxan-2-yl) and the like;
7-membered non-aromatic heterocyclic groups such as azepanyl (e.g., 1-azepanyl, 2-azepanyl, 3-azepanyl), homopiperazinyl (e.g., 1-homopiperazinyl, 2-homopiperazinyl, 5-homopiperazinyl), 1,4-oxazepanyl (e.g., 1,4-oxazepan-4-yl), 1,4-thiazepanyl (e.g., 1,4-thiazepanyl-4-yl) and the like; bicyclic non-aromatic heterocyclic groups such as 2,3-dihydrobenzo[b]furanyl (e.g., 2,3-dihydro-2-benzo[b]furanyl, 2,3-dihydro-3-benzo[b]furanyl, 2,3-dihydro-4-benzo[b]furanyl, 2,3-dihydro-5-benzo[b]furanyl), 1,3-dihydrobenzo[c]furanyl (e.g., 1,3-dihydro-1-benzo[c]furanyl, 1,3-dihydro-4-benzo[c]furanyl, 1,3-dihydro-5-benzo[c]furanyl), 2,3-dihydrobenzo[b]thienyl (e.g., 2,3-dihydro-2-benzo[b]thienyl, 2,3-dihydro-3-benzo[b]thienyl, 2,3-dihydro-4-benzo[b]thienyl, 2,3-dihydro-5-benzo[b]thienyl), 1,3-dihydrobenzo[c]thienyl (e.g., 1,3-dihydro-1-benzo[c]thienyl, 1,3-dihydro-4-benzo[c]thienyl, 1,3-dihydro-5-benzo[c]thienyl), 1,3-benzodioxol-5-yl, tetrahydroquinolyl (e.g., 1-tetrahydroquinolyl, 2-tetrahydroquinolyl, 3-tetrahydroquinolyl, 4-tetrahydroquinolyl, 5-tetrahydroquinolyl, 8-tetrahydroquinolyl), tetrahydroisoquinolyl (e.g., 1-tetrahydroisoquinolyl, 2-tetrahydroisoquinolyl, 3-tetrahydroisoquinolyl, 4-tetrahydroisoquinolyl, 5-tetrahydroisoquinolyl), indolinyl (e.g., 1-indolinyl, 2-indolinyl, 3-indolinyl) and the like; and the like can be mentioned.

As the "heterocyclylcarbonyl group" in the present specification, a carbonyl group substituted by the above-mentioned "heterocyclic group" can be mentioned. As preferable examples of the heterocyclylcarbonyl group, furoyl, thenoyl, pyrazolylcarbonyl, thiazolylcarbonyl, oxazolylcarbonyl, pyridylcarbonyl, indolylcarbonyl, benzofuranylcarbonyl, benzothienylcarbonyl, quinolylcarbonyl, isoquinolylcarbonyl, pyrrolidinylcarbonyl, piperidinylcarbonyl, tetrahydropyranylcarbonyl and the like can be mentioned.

As the "aromatic heterocyclylcarbonyl group" in the present specification, a carbonyl group substituted by an "aromatic heterocyclic group" from among those exemplified as the above-mentioned "heterocyclic group" can be mentioned. As preferable examples of the aromatic heterocyclylcarbonyl group, furoyl, thenoyl, pyrazolylcarbonyl, thiazolylcarbonyl, oxazolylcarbonyl, pyridylcarbonyl, indolylcarbonyl, benzofuranylcarbonyl, benzothienylcarbonyl, quinolylcarbonyl, isoquinolylcarbonyl and the like can be mentioned.

As the "nitrogen-containing non-aromatic heterocyclylcarbonyl group" in the present specification, a carbonyl group substituted by a "nitrogen-containing non-aromatic heterocyclic group" from among those exemplified as the above-mentioned "heterocyclic group" can be mentioned. As preferable examples of the nitrogen-containing non-aromatic heterocyclylcarbonyl group, pyrrolidinylcarbonyl, piperidinylcarbonyl and the like can be mentioned.

Unless otherwise specified, as the "optionally halogenated C₁₋₆ alkyl group" in the present specification, the above-mentioned "C₁₋₆ alkyl group" optionally substituted by 1 to 5 the above-mentioned "halogen atoms" can be mentioned. For example, methyl, ethyl, propyl, isopropyl, butyl, tert-butyl, isobutyl, trifluoromethyl and the like can be mentioned.

Unless otherwise specified, as the "optionally halogenated C₁₋₆ alkoxy group" in the present specification, the above-mentioned "C₁₋₆ alkoxy group" optionally substituted by 1 to 5 the above-mentioned "halogen atoms" can be mentioned. For example, methoxy, ethoxy, isopropoxy, tert-butoxy, trifluoromethoxy, 2,2,2-trifluoroethoxy, 1,1,2,2-tetrafluoroethoxy and the like can be mentioned.

Unless otherwise specified, as the "C₁₋₆ alkyl-carbonyl group" in the present specification, for example, acetyl, isobutanoyl, isopentanoyl and the like can be mentioned.

Unless otherwise specified, as the "C₃₋₈ cycloalkyl-carbonyl group" in the present specification, for example, cyclopropylcarbonyl, cyclobutylcarbonyl, cyclopentylcarbonyl, cyclohexylcarbonyl and the like can be mentioned.

Unless otherwise specified, as the "C₆₋₁₄ aryl-carbonyl group" in the present specification, for example, benzoyl, 1-naphthoyl, 2-naphthoyl and the like can be mentioned.

Unless otherwise specified, as the "C₇₋₁₆ aralkyl-carbonyl group" in the present specification, for example, phenylacetyl, 2-phenylpropanoyl and the like can be mentioned.

Unless otherwise specified, as the "C₁₋₆ alkoxy-carbonyl group" in the present specification, for example, methoxycarbonyl, ethoxycarbonyl, propoxycarbonyl, tert-butoxycarbonyl and the like can be mentioned.

Unless otherwise specified, as the "C₆₋₁₄ aryloxy-carbonyl group" in the present specification, for example, phenyloxycarbonyl, naphthyloxycarbonyl and the like can be mentioned.

Unless otherwise specified, as the "C₇₋₁₆ aralkyloxy-carbonyl group" in the present specification, for example, benzyloxycarbonyl, phenethyloxycarbonyl, 9-fluorenylmethyloxycarbonyl and the like can be mentioned.

Unless otherwise specified, as the "C₁₋₆ alkylsulfonyl group" in the present specification, for example, methylsulfonyl, ethylsulfonyl and the like can be mentioned.

Unless otherwise specified, as the "C₆₋₁₄ arylsulfonyl group" in the present specification, for example, phenylsulfonyl, 1-naphthylsulfonyl, 2-naphthylsulfonyl and the like can be mentioned.

Unless otherwise specified, as the "C₁₋₆ alkylsulfinyl group" in the present specification, for example, methylsulfinyl, ethylsulfinyl and the like can be mentioned.

Unless otherwise specified, as the "C₆₋₁₄ arylsulfinyl group" in the present specification, for example, phenylsulfinyl, 1-naphthylsulfinyl, 2-naphthylsulfinyl and the like can be mentioned.

Unless otherwise specified, as the "mono- or di-C₁₋₆ alkyl-carbamoyl group" in the present specification, a carbamoyl group mono- or di-substituted by the above-mentioned "C₁₋₆ alkyl group" can be mentioned. For example, methylcarbamoyl, ethylcarbamoyl, dimethylcarbamoyl, diethylcarbamoyl, ethylmethylcarbamoyl and the like can be mentioned.

Unless otherwise specified, as the "N-C₁₋₆ alkyl-N-C₁₋₆ alkoxy-carbamoyl group" in the present specification, a carbamoyl group di-substituted by the above-mentioned "C₁₋₆ alkyl group" and the above-mentioned "C₁₋₆ alkoxy group" can be mentioned. For example, N-methyl-N-methoxycarbamoyl and the like can be mentioned.

Unless otherwise specified, as the "mono- or di-C₆₋₁₄ aryl-carbamoyl group" in the present specification, a carbamoyl group mono- or di-substituted by the above-mentioned "C₆₋₁₄ aryl group" can be mentioned. For example, phenylcarbamoyl, 1-naphthylcarbamoyl, 2-naphthylcarbamoyl and the like can be mentioned.

Unless otherwise specified, as the "C₃₋₈ cycloalkyl-carbamoyl group" in the present specification, a carbamoyl group mono-substituted by the above-mentioned "C₃₋₈ cycloalkyl" can be mentioned. For example, cyclopropylcarbamoyl, cyclopentylcarbamoyl, cyclohexylcarbamoyl and the like can be mentioned.

Unless otherwise specified, as the "C₇₋₁₆ aralkyl-carbamoyl group" in the present specification, a carbamoyl group mono-substituted by the above-mentioned "C₇₋₁₆ aralkyl" can be mentioned. For example, benzylcarbamoyl and the like can be mentioned.

Unless otherwise specified, as the "mono- or di-C₁₋₆ alkylsulfamoyl group" in the present specification, a sulfamoyl group mono- or di-substituted by the above-mentioned "C₁₋₆ alkyl group" can be mentioned, for example, methylsulfamoyl, ethylsulfamoyl, dimethylsulfamoyl, diethylsulfamoyl and the like can be mentioned.

Unless otherwise specified, as the "mono- or di-C₆₋₁₄ arylsulfamoyl group" in the present specification, a sulfamoyl group mono- or di-substituted by the above-mentioned "C₆₋₁₄ aryl group" can be mentioned, for example, phenylsulfamoyl, diphenylsulfamoyl, 1-naphthylsulfamoyl, 2-naphthylsulfamoyl and the like can be mentioned.

Unless otherwise specified, as the "mono- or di-C₁₋₆ alkyl-amino group" in the present specification, an amino group mono- or di-substituted by the above-mentioned "C₁₋₆ alkyl group" can be mentioned. For example, methylamino, ethylamino, propylamino, dimethylamino, diethylamino and the like can be mentioned.

Unless otherwise specified, as the "mono- or di-C₆₋₁₄ aryl-amino group" in the present specification, an amino group mono- or di-substituted by the above-mentioned "C₆₋₁₄ aryl group" can be mentioned. For example, phenylamino, diphenylamino, 1-naphthylamino, 2-naphthylamino and the like can be mentioned.

Unless otherwise specified, as the "mono- or di-C₇₋₁₆ aralkyl-amino group", an amino group mono- or di-substituted by the above-mentioned "C₇₋₁₆ aralkyl group" can be mentioned. For example, benzylamino, phenethylamino and the like can be mentioned.

Unless otherwise specified, as the "N-C₁₋₆ alkyl-N-C₆₋₁₄ aryl-amino group" in the present specification, an amino group di-substituted by the above-mentioned "C₁₋₆ alkyl group" and the above-mentioned "C₆₋₁₄ aryl" can be mentioned. For example, N-methyl-N-phenylamino, N-ethyl-N-phenylamino and the like can be mentioned.

Unless otherwise specified, as the "N-C₁₋₆ alkyl-N-C₇₋₁₆ aralkyl-amino group" in the present specification, an amino group di-substituted by the above-mentioned "C₁₋₆ alkyl group" and the above-mentioned "C₇₋₁₆ aralkyl group" can be mentioned. For example, N-methyl-N-benzylamino, N-ethyl-N-benzylamino and the like can be mentioned.

Unless otherwise specified, as the "N-C₁₋₆ alkyl-N-C₁₋₆ alkyl-carbonyl-amino group" in the present specification, an amino group di-substituted by the above-mentioned "C₁₋₆ alkyl group" and the above-mentioned "C₁₋₆ alkyl-carbonyl group" can be mentioned. For example, N-methyl-N-acetylamino, N-ethyl-N-acetylamino and the like can be mentioned.

Unless otherwise specified, as the "C₁₋₆ alkoxy-carbonyl-amino group" in the present specification, an amino group substituted by the above-mentioned "C₁₋₆ alkoxy-carbonyl group" can be mentioned. For example, methoxycarbonylamino, ethoxycarbonylamino, tert-butoxycarbonylamino and the like can be mentioned.

As the "optionally substituted C₁₋₆ alkyl group", "optionally substituted C₂₋₆ alkenyl group", "optionally substituted C₂₋₆ alkynyl group", "optionally substituted C₁₋₆ alkoxy group", "optionally substituted C₃₋₆ alkenyloxy group", "optionally substituted C₃₋₆ alkynyloxy group", "optionally substituted C₁₋₆ alkylsulfonyloxy group", "optionally substituted C₁₋₆ alkylthio group", "optionally substituted C₃₋₆ alkenylthio group" and "optionally substituted C₃₋₆ alkynylthio group" in the present specification, for example, a "C₁₋₆ alkyl group", a "C₂₋₆ alkenyl group", a "C₂₋₆ alkynyl group", a "C₁₋₆ alkoxy group", a "C₃₋₆ alkenyloxy group", a "C₃₋₆ alkynyloxy group", a "C₁₋₆ alkylsulfonyloxy group", a "C₁₋₆ alkylthio group", a "C₃₋₆ alkenylthio group" and a "C₃₋₆ alkynylthio group", each of which optionally has, at substitutable positions, 1 to 5 substituents selected from
(1) a halogen atom;
(2) a hydroxy group;
(3) an amino group;
(4) a nitro group;
(5) a cyano group;
(6) a C₃₋₈ cycloalkyl group;
(7) a C₆₋₁₄ aryl group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(8) a heterocyclic group (preferably furyl, thienyl, pyrazolyl, thiazolyl, oxazolyl, pyridyl) optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(9) an optionally halogenated C₁₋₆ alkoxy group;
(10) a C₆₋₁₄ aryloxy group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(11) a C₇₋₁₆ aralkyloxy group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(12) a Cₗ-₆ alkyl-carbonyloxy group;
(13) a C₁₋₆ alkylthio group;
(14) a carboxyl group;
(15) a C₁₋₆ alkoxy-carbonyl group;
(16) a C₇₋₁₆ aralkyloxy-carbonyl group;
(17) a C₁₋₆ alkylsulfonyl group;
(18) a carbamoyl group;
(19) a thiocarbamoyl group;
(20) a mono- or di-C₁₋₆ alkyl-carbamoyl group;
(21) a sulfamoyl group;
(22) a mono- or di-C₁₋₆ alkyl-sulfamoyl group;
(23) a mono- or di-C₁₋₆ alkyl-amino group;
(24) a C₁₋₆ alkyl-carbonyl group;
(25) a heterocyclylcarbonyl group (preferably furoyl, thenoyl, pyrazolylcarbonyl, thiazolylcarbonyl, oxazolylcarbonyl, pyridylcarbonyl) optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(26) a formyl group; and the like can be mentioned.

As the "optionally substituted C₃₋₈ cycloalkyl group", "optionally substituted C₆₋₁₄ aryl group", "optionally substituted C₇₋₁₆ aralkyl group", "optionally substituted C₃₋₈ cycloalkyloxy group", "optionally substituted heterocyclyloxy group", "optionally substituted C₆₋₁₄ aryloxy group", "optionally substituted C₇₋₁₆ aralkyloxy group", "optionally substituted heterocyclylsulfonyloxy group", "optionally substituted C₃₋₈ cycloalkylthio group", "optionally substituted heterocyclylthio group", "optionally substituted C₆₋₁₄ arylthio group", "optionally substituted C₇₋₁₆ aralkylthio group" and "optionally substituted heterocyclic group" in the present specification, for example, a "C₃₋₈ cycloalkyl group", a "C₆₋₁₄ aryl group", a "C₇₋₁₆ aralkyl group", a "C₃₋₈ cycloalkyloxy group", a "heterocyclyloxy group", a "C₆₋₁₄ aryloxy group", a "C₇₋₁₆ aralkyloxy group", a "heterocyclylsulfonyloxy group", a "C₃₋₈ cycloalkylthio group", a "heterocyclylthio group", a "C₆₋₁₄ arylthio group", a "C₇₋₁₆ aralkylthio group" and a "heterocyclic group", each of which optionally has, at substitutable positions, 1 to 5 substituents selected from
(1) a halogen atom;
(2) a hydroxy group;
(3) an amino group;
(4) a nitro group;
(5) a cyano group;
(6) an optionally substituted C₁₋₆ alkyl group;
(7) a C₃₋₈ cycloalkyl group;
(8) a C₆₋₁₄ aryl group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(9) a heterocyclic group (preferably furyl, thienyl, pyrazolyl, thiazolyl, oxazolyl, pyridyl) optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(10) an optionally halogenated C₁₋₆ alkoxy group;
(11) a C₆₋₁₄ aryloxy group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(12) a C₇₋₁₆ aralkyloxy group optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(13) a C₁₋₆ alkyl-carbonyloxy group;
(14) a C₁₋₆ alkylthio group;
(15) a carboxyl group;
(16) a C₁₋₆ alkoxy-carbonyl group;
(17) a C₇₋₁₆ aralkyloxy-carbonyl group;
(18) a C₁₋₆ alkylsulfonyl group;
(19) a carbamoyl group;
(20) a thiocarbamoyl group;
(21) a mono- or di-C₁₋₆ alkyl-carbamoyl group;
(22) a sulfamoyl group;
(23) a mono- or di-C₁₋₆ alkyl-sulfamoyl group;
(24) a mono- or di-C₁₋₆ alkyl-amino group;
(25) a C₁₋₆ alkyl-carbonyl group;
(26) a heterocyclylcarbonyl group (preferably furoyl, thenoyl, pyrazolylcarbonyl, thiazolylcarbonyl, oxazolylcarbonyl, pyridylcarbonyl) optionally substituted by 1 to 3 substituents selected from a halogen atom, a hydroxy group, an amino group, a nitro group, a cyano group, an optionally halogenated C₁₋₆ alkyl group, an optionally halogenated C₁₋₆ alkoxy group, a C₁₋₆ alkylthio group and a carboxyl group;
(27) a formyl group; and the like can be mentioned.

Unless otherwise specified, as the "optionally substituted acyl group" in the present specification, a group represented by the formula: -COR¹, -CO-OR¹, -SO₂R¹, -SOR¹, - PO(OR¹)(OR²), -CO-NR^{1a}R^{2a}, -CS-NR^{1a}R^{2a} or -SO₂-NR^{1a}R^{2a} wherein R¹ and R² are the same or different and each is a hydrogen atom, an optionally substituted hydrocarbon group or an optionally substituted heterocyclic group, and R^{1a} and R^{2a} are the same or different and each is a hydrogen atom, an optionally substituted hydrocarbon group, an optionally substituted heterocyclic group or an optionally substituted hydroxy group, or R^{1a} and R^{2a} optionally form, together with the adjacent nitrogen atom, an optionally substituted nitrogen-containing heterocycle, and the like can be mentioned.

As the "nitrogen-containing heterocycle" of the "optionally substituted nitrogen-containing heterocycle" formed by R^{1a} and R^{2a} together with the adjacent nitrogen atom, for example, a 5- to 7-membered nitrogen-containing heterocycle containing, as a ring-constituting atom besides carbon atoms, at least one nitrogen atom and optionally further containing one or two heteroatoms selected from an oxygen atom, a sulfur atom and a nitrogen atom can be mentioned. As preferable examples of the nitrogen-containing heterocycle, pyrrolidine, imidazolidine, pyrazolidine, thiazolidine, oxazolidine, piperidine, piperazine, morpholine, thiomorpholine and the like can be mentioned.

The nitrogen-containing heterocycle optionally has 1 or 2 substituents at substitutable positions. As such substituents, a hydroxy group, an optionally halogenated C₁₋₆ alkyl group, an optionally halogenated C₁₋₆ alkoxy group, a C₆₋₁₄ aryl group, a C₇₋₁₆ aralkyl group and the like can be mentioned.

As preferable examples of the "optionally substituted acyl group",
a formyl group;
a carboxyl group;
a carbamoyl group;
a sulfamoyl group;
a C₁₋₆ alkyl-carbonyl group;
a C₁₋₆ alkoxy-carbonyl group;
a C₃₋₈ cycloalkyl-carbonyl group;
a C₆₋₁₄ aryl-carbonyl group;
a C₇₋₁₆ aralkyl-carbonyl group;
a C₆₋₁₄ aryloxy-carbonyl group;
a C₇₋₁₆ aralkyloxy-carbonyl group;
a mono- or di-C₁₋₆ alkyl-carbamoyl group optionally substituted by 1 or 2 substituents selected from a hydroxy group and a C₁₋₆ alkoxy group (including a N-C₁₋₆ alkyl-N-C₁₋₆ alkoxy-carbamoyl group);
a mono- or di-C₆₋₁₄ aryl-carbamoyl group;
a C₃₋₈ cycloalkyl-carbamoyl group;
a C₇₋₁₆ aralkyl-carbamoyl group;
a C₁₋₆ alkylsulfonyl group;
a C₆₋₁₄ arylsulfonyl group;
a nitrogen-containing non-aromatic heterocyclylcarbonyl group; an aromatic heterocyclylcarbonyl group;
a C₁₋₆ alkylsulfinyl group;
a C₆₋₁₄ arylsulfinyl group;
a thiocarbamoyl group;
a mono- or di-C₁₋₆ alkylsulfamoyl group;
a mono- or di-C₆₋₁₄ arylsulfamoyl group;
and the like can be mentioned.

Unless otherwise specified, as the "optionally substituted amino group" in the present specification, an amino group optionally substituted by 1 or 2 substituents selected from
(1) an optionally substituted C₁₋₆ alkyl group;
(2) an optionally substituted C₂₋₆ alkenyl group;
(3) an optionally substituted C₂₋₆ alkynyl group;
(4) an optionally substituted C₃₋₈ cycloalkyl group;
(5) an optionally substituted C₆₋₁₄ aryl group;
(6) an optionally substituted C₇₋₁₆ aralkyl group;
(7) an optionally substituted C₁₋₆ alkoxy group;
(8) an optionally substituted acyl group;
(9) an optionally substituted heterocyclic group (preferably furyl, pyridyl, thienyl, pyrazolyl, thiazolyl, oxazolyl); and the like can be mentioned. In addition, when the "optionally substituted amino group" is an amino group substituted by 2 substituents, these substituents optionally form, together with the adjacent nitrogen atom, a nitrogen-containing heterocycle. As the "nitrogen-containing heterocycle", for example, a 5- to 7-membered nitrogen-containing heterocycle containing, as a ring-constituting atom besides carbon atoms, at least one nitrogen atom and optionally further containing one or two heteroatoms selected from an oxygen atom, a sulfur atom and a nitrogen atom can be mentioned. As preferable examples of the nitrogen-containing heterocycle, pyrrolidine, imidazolidine, pyrazolidine, thiazolidine, oxazolidine, piperidine, piperazine, morpholine, thiomorpholine and the like can be mentioned.

As preferable examples of the "optionally substituted amino group",
a mono- or di-C₁₋₆ alkyl-amino group;
a mono- or di-C₆₋₁₄ aryl-amino group;
a mono- or di-C₇₋₁₆ aralkyl-amino group;
a N-C₁₋₆ alkyl-N-C₆₋₁₄ aryl-amino group;
a N-C₁₋₆ alkyl-N-C₇₋₁₆ aralkyl-amino group;
a N-C₁₋₆ alkyl-N-C₁₋₆ alkyl-carbonyl-amino group;
a C₁₋₆ alkoxy-carbonyl-amino group;
and the like can be mentioned.

The "C₁₋₃ alkylene group" of the "optionally substituted C₁₋₃ alkylene group" in the present specification may be linear or branched, for example, methylene, methylmethylene, dimethylmethylene, ethylene, 1-methylethylene, 2-methylethylene and the like can be mentioned. The C₁₋₃ alkylene group optionally has 1 to 3 substituents at substitutable positions. As such substituents, for example,
(1) a hydroxy group;
(2) an oxo group;
(3) a C₆₋₁₄ aryl group;
(4) a mono- or di-C₆₋₁₄ aryl-carbamoyl group optionally substituted by 1 to 3 C₁₋₆ alkyl groups;
(5) an aromatic heterocyclylcarbonyl group (e.g., pyridylcarbonyl, furoyl, thenoyl, indolylcarbonyl);
(6) a halogen atom;
(7) a cyano group;
(8) a C₁₋₆ alkoxy group;
(9) a C₁₋₆ alkylthio group;
(10) a C₁₋₆ alkyl group;
   and the like can be mentioned.

The definition of each symbol in the formula (I) is explained in detail in the following.

In the formula (I), ring A is an optionally substituted ring.

As used herein, as the "ring" of the "optionally substituted ring", for example, aromatic rings such as an aromatic hydrocarbon, an aromatic heterocycle and the like; non-aromatic rings such as an alicyclic hydrocarbon, a non-aromatic heterocycle and the like can be mentioned.

As the aromatic hydrocarbon, for example, an aromatic hydrocarbon having 6 to 14 carbon atoms can be mentioned. As preferable examples of the aromatic hydrocarbon, benzene, naphthalene, anthracene, phenanthrene, acenaphthylene and the like can be mentioned.

As the aromatic heterocycle, for example, a 5- to 7-membered monocyclic aromatic heterocycle containing, as a ring-constituting atom besides carbon atoms, 1 to 4 heteroatoms selected from an oxygen atom, a sulfur atom and a nitrogen atom and or a fused aromatic heterocycle can be mentioned. As the fused aromatic heterocycle, for example, a ring wherein such 5- to 7-membered monocyclic aromatic heterocycle, and a 6-membered ring containing 1 or 2 nitrogen atoms, a benzene ring or a 5-membered ring containing one sulfur atom are condensed, and the like can be mentioned.

As preferable examples of the aromatic heterocycle, monocyclic aromatic heterocycles such as furan, thiophene, pyrrole, imidazole, pyrazole, isoxazole, isothiazole, oxazole, thiazole, 1,2,3-oxadiazole, 1,2,3-thiadiazole, 1,2,3-triazole, pyridine, pyrimidine, pyridazine, pyrazine, triazine and the like;
fused aromatic heterocycles such as quinoline, quinazoline, quinoxaline, benzofuran, benzothiophene, benzoxazole, benzothiazole, benzimidazole, indole, 1H-indazole, 1H-pyrrolo[2,3-b]pyrazine, 1H-pyrrolopyridine, 1H-imidazopyridine, 1H-imidazopyrazine, isoquinoline, benzothiadiazole and the like;
and the like can be mentioned.

As the alicyclic hydrocarbon, a saturated or unsaturated alicyclic hydrocarbon having 3 to 12 carbon atoms, for example, a cycloalkane, a cycloalkene, a cycloalkadiene and the like can be mentioned.

As preferable examples of the cycloalkane, a cycloalkane having 3 to 10 carbon atoms, for example, cyclopropane, cyclobutane, cyclopentane, cyclohexane, cycloheptane, cyclooctane, bicyclo[2.2.1]heptane, bicyclo[2.2.2]octane, bicyclo[3.2.1]octane, bicyclo[3.2.2]nonane, bicyclo[3.3.1]nonane, bicyclo[4.2.1]nonane, bicyclo[4.3.1]decane and the like can be mentioned.

As preferable examples of the cycloalkene, a cycloalkene having 4 to 10 carbon atoms, for example, cyclobutene, cyclopentene, cyclohexene and the like can be mentioned.

As preferable examples of the cycloalkadiene, a cycloalkadiene having 4 to 10 carbon atoms, for example, 2,4-cyclopentadiene, 2,4-cyclohexadiene, 2,5-cyclohexadiene and the like can be mentioned.

As the non-aromatic heterocycle, for example, a 5- to 7-membered monocyclic non-aromatic heterocycle containing, as a ring-constituting atom besides carbon atoms, 1 to 4 heteroatoms selected from an oxygen atom, a sulfur atom and a nitrogen atom or a fused non-aromatic heterocycle can be mentioned. As the fused non-aromatic heterocycle, for example, a ring wherein such 5- to 7-membered monocyclic non-aromatic heterocycle, and a 6-membered ring containing 1 or 2 nitrogen atoms, a benzene ring or a 5-membered ring containing one sulfur atom are condensed, and the like can be mentioned.

As preferable examples of the non-aromatic heterocycle, monocyclic non-aromatic heterocycles such as pyrrolidine, pyrroline, pyrazolidine, oxazolidine, thiazolidine, imidazolidine, imidazoline, tetrahydrofuran, piperidine, piperazine, morpholine, thiomorpholine, hexamethylenimine (azepane), tetrahydropyridine and the like; benzene ring-fused non-aromatic heterocycles such as dihydrobenzofuran and the like and the like can be mentioned.

Of the above-mentioned ring, an aromatic ring is preferable, and a benzene ring and a pyridine ring are preferable.

The "ring" of the "optionally substituted ring" for ring A optionally has, for example, 1 to 5, preferably 1 to 3, substituents at substitutable positions. As such "substituents", for example, a halogen atom, a nitro group, a cyano group, an optionally substituted hydrocarbon group, an optionally substituted hydroxy group, an optionally substituted mercapto group, an optionally substituted heterocyclic group, an optionally substituted acyl group, an optionally substituted amino group and the like can be mentioned. When the ring has 2 or more substituents, respective substituents may be the same or different.

As preferable examples of the substituents of ring A,
(1) a halogen atom;
(2) a nitro group;
(3) a C₁₋₆ alkoxy group;
(4) a mono- or di-C₁₋₆ alkyl-sulfamoyl group;
(5) a C₁₋₆ alkoxy-carbonyl group;
(6) a C₁₋₆ alkyl group;
(7) a cyano group;
   and the like can be mentioned.

Ring A is preferably a benzene ring substituted by the above-mentioned substituent(s), or a pyridine ring, more preferably a benzene ring substituted by 1 to 3 halogen atoms.

In the formula (I), B¹ and B² are each independently optionally substituted cyclic group.

As used herein, as the "cyclic group" of the "optionally substituted cyclic group", for example, a C₃₋₈ cycloalkyl group, a C₆₋₁₄ aryl group, a heterocyclic group and the like can be mentioned. Of these, a cyclohexyl group, a phenyl group, a pyridyl group, a piperidinyl group, a dihydrobenzo[b]furanyl group and the like are preferable.

B¹ is preferably an optionally substituted phenyl group, more preferably a phenyl group having a substituent at the para-position.

B² is preferably an optionally substituted phenyl group or an optionally substituted pyridyl group, more preferably a phenyl group having a substituent at the para-position or a 3-pyridyl group having a substituent at the 6-position.

The "cyclic group" of the "optionally substituted cyclic group" for B¹ or B² optionally has, for example, 1 to 5, preferably 1 to 3, substituents at substitutable positions. As such "substituents", those exemplified as the substituents which the aforementioned "ring" of the "optionally substituted ring" for ring A optionally has, can be mentioned. When the cyclic group has 2 or more substituents, respective substituents may be the same or different.

As the preferable examples of the substituents of B¹ or B²,
(1) a halogen atom;
(2) a hydroxy group;
(3) a cyano group;
(4) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(5) a C₆₋₁₄ aryl group;
(6) a C₇₋₁₆ aralkyl group;
(7) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(8) a C₁₋₆ alkylsulfonyloxy group;
(9) a C₆₋₁₄ aryloxy group;
(10) a C₇₋₁₆ aralkyloxy group;
(11) a carboxyl group;
(12) a C₁₋₆ alkoxy-carbonyl group;
(13) a C₁₋₆ alkyl-carbonyl group;
(14) a mono- or di-C₁₋₆ alkyl-carbamoyl group optionally substituted by 1 or 2 substituents selected from a hydroxy group and a C₁₋₆ alkoxy group (including a N-C₁₋₆ alkyl-N-C₁₋₆ alkoxy-carbamoyl group);
(15) a C₁₋₆ alkylthio group;
(16) a C₁₋₆ alkylsulfonyl group;
(17) a heterocyclic group (preferably oxazolinyl, oxazolyl, dioxolanyl);
(18) a C₁₋₆ alkoxy-carbonyl-amino group;
   and the like can be mentioned. Of these, the substituent of B¹ is preferably selected from an optionally substituted C₁₋₆ alkyl group, an optionally substituted C₁₋₆ alkoxy group, a C₁₋₆ alkoxy-carbonyl group and a C₁₋₆ alkyl-carbonyl group, and the substituent of B² is preferably selected from a halogen atom and an optionally halogenated C₁₋₆ alkyl group.

In the formula (I), X is an optionally substituted C₁₋₃ alkylene group, -O-, -NR^{X}- or -S(O)n^{X}- wherein R^{X} is a hydrogen atom or a substituent, and n^{X} is an integer of 0 to 2.

The "optionally substituted C₁₋₃ alkylene group" for X is preferably a methylene group.

As the "substituent" for R^{X}, for example, an optionally substituted C₁₋₆ alkyl group, a formyl group, a C₁₋₆ alkyl-carbonyl group, a C₁₋₆ alkoxy-carbonyl group, a C₇₋₁₆ aralkyl-carbonyl group, a C₆₋₁₄ aryloxy-carbonyl group, a C₇₋₁₆ aralkyloxy-carbonyl group and the like can be mentioned. R^{X} is preferably a hydrogen atom.

n^{X} is preferably 0.

X is preferably -S- or -NH-.

In the formula (I), Y is a bond, an optionally substituted C₁₋₃ alkylene group, -O-, -NR^{Y}- or -S(O)n^{Y}- wherein R^{Y} is a hydrogen atom or a substituent, and n^{Y} is an integer of 0 to 2.

The "optionally substituted C₁₋₃ alkylene group" for Y is preferably a methylene group, an ethylene group, a methylmethylene group and the like, each of which optionally has 1 or 2 substituents selected from
(1) a hydroxy group;
(2) an oxo group;
(3) a C₆₋₁₄ aryl group;
(4) a mono- or di-C₆₋₁₄ aryl-carbamoyl group optionally substituted by 1 to 3 C₁₋₆ alkyl groups;
(5) an aromatic heterocyclylcarbonyl group (preferably indolylcarbonyl)
   and the like.

As the substituent for R^{Y}, those exemplified as the aforementioned R^{X} can be mentioned. R^{Y} is preferably a hydrogen atom.

n^{Y} is preferably 0.

Y is preferably an optionally substituted C₁₋₃ alkylene group or -O-, more preferably a methylene group.

Compound (I) is preferably compound (II).

In the formula (II), ring Aa is a substituted benzene ring (provided that the substituent(s) that the benzene ring has should not be a nitro group and a diethylsulfamoyl group), or an optionally substituted pyridine ring.

As used herein, as the substituents of benzene ring or pyridine ring, those exemplified as the substituents which the aforementioned "ring" of the "optionally substituted ring" for ring A in the formula (I) optionally has, can be mentioned. The number of the substituents is, for example, 1 to 5, preferably 1 to 3.

Ring Aa is preferably a benzene ring substituted by 1 to 3 substituents selected from
(1) a halogen atom;
(2) a C₁₋₆ alkoxy group;
(3) a C₁₋₆ alkoxy-carbonyl group;
   and the like, or a pyridine ring, more preferably a benzene ring substituted by 1 to 3 halogen atoms.

In the formula (II), Z is CH or N, preferably CH.

In the formula (II), Ba¹ is an optionally substituted 5-membered aromatic group or an optionally substituted 6-membered cyclic group.

As used herein, as the "5-membered aromatic group" of the "optionally substituted 5-membered aromatic group", a 5-membered aromatic heterocyclic group exemplified as the aforementioned "heterocyclic group" and the like can be mentioned.

As the "6-membered cyclic group" of the "optionally substituted 6-membered cyclic group", a cyclohexyl group, a phenyl group, a 6-membered aromatic heterocyclic group exemplified as the aforementioned "heterocyclic group", a 6-membered non-aromatic heterocyclic group exemplified as the aforementioned "heterocyclic group" and the like can be mentioned. Of these, a cyclohexyl group, a phenyl group, a pyridyl group, a piperidinyl group and the like are preferable.

Ba¹ is preferably an optionally substituted phenyl group, more preferably a phenyl group having a substituent at the para-position.

The "5-membered aromatic group" of the "optionally substituted 5-membered aromatic group" and the "6-membered cyclic group" of the "optionally substituted 6-membered cyclic group" optionally have, for example, 1 to 5, preferably 1 to 3, substituents at substitutable positions. As such "substituents", those exemplified as the substituents which the aforementioned "ring" of the "optionally substituted ring" for ring A in the formula (I) optionally has, can be mentioned.

As preferable examples of the substituents of Ba¹,
(1) a halogen atom;
(2) a hydroxy group;
(3) a cyano group;
(4) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(5) a C₆₋₁₄ aryl group;
(6) a C₇₋₁₆ aralkyl group;
(7) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(8) a C₁₋₆ alkylsulfonyloxy group;
(9) a C₆₋₁₄ aryloxy group;
(10) a C₇₋₁₆ aralkyloxy group;
(11) a carboxyl group;
(12) a C₁₋₆ alkoxy-carbonyl group;
(13) a C₁₋₆ alkyl-carbonyl group;
(14) a mono- or di-C₁₋₆ alkyl-carbamoyl group optionally substituted by 1 or 2 substituents selected from a hydroxy group and a C₁₋₆ alkoxy group (including a N-C₁₋₆ alkyl-N-C₁₋₆ alkoxy-carbamoyl group);
(15) a C₁₋₆ alkylthio group;
(16) a C₁₋₆ alkylsulfonyl group;
(17) a heterocyclic group (preferably oxazolinyl, oxazolyl, dioxolanyl);
(18) a C₁₋₆ alkoxy-carbonyl-amino group;
   and the like can be mentioned. The substituents are preferably selected from an optionally substituted C₁₋₆ alkyl group, an optionally substituted C₁₋₆ alkoxy group, a C₁₋₆ alkoxy-carbonyl group and a C₁₋₆ alkyl-carbonyl group.

In the formula (II), Ba² is an optionally substituted 5-or 6-membered aromatic group. As used herein, as the "5-membered aromatic group" of the "optionally substituted 5- or 6-membered aromatic group", a 5-membered aromatic heterocyclic group exemplified as the aforementioned "heterocyclic group" and the like can be mentioned. As the "6-membered aromatic group" of the "optionally substituted 5- or 6-membered aromatic group", a phenyl group, a 6-membered aromatic heterocyclic group exemplified as the aforementioned "heterocyclic group" and the like can be mentioned. Of these, a phenyl group, a 6-membered aromatic heterocyclic group (preferably a pyridyl group) and the like are preferable.

Ba² is preferably an optionally substituted phenyl group or an optionally substituted pyridyl group, more preferably a phenyl group having a substituent at the para-position or a 3-pyridyl group having a substituent at the 6-position.

The "5- or 6-membered aromatic group" of the "optionally substituted 5- or 6-membered aromatic group" optionally has, for example, 1 to 5, preferably 1 to 3, substituents at substitutable positions. As such "substituents", those exemplified as the substituents which the aforementioned "ring" of the "optionally substituted ring" for ring A in the formula (I) optionally has, can be mentioned.

As preferable examples of the substituents of the Ba²,
(1) a halogen atom;
(2) a cyano group;
(3) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(4) a C₆₋₁₄ aryl group;
(5) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group, a C₃₋₈ cycloalkyl group and the like);
(6) a C₆₋₁₄ aryloxy group;
(7) a C₁₋₆ alkoxy-carbonyl group;
(8) a C₁₋₆ alkyl-carbonyl group;
   and the like can be mentioned. The substituents are preferably selected from a halogen atom and an optionally halogenated C₁₋₆ alkyl group.

In the formula (II), Xa is -O-, -NRa- wherein Ra is a hydrogen atom or an optionally substituted C₁₋₆ alkyl group, or -S-.

Ra is preferably a hydrogen atom.

Xa is preferably -S- or -NH-.

In the formula (II), Ya is an optionally substituted C₁₋₃ alkylene group. Ya is preferably a methylene group, an ethylene group or a methylmethylene group, each of which optionally has 1 or 2 substituents selected from
(1) a hydroxy group;
(2) an oxo group;
(3) a C₆₋₁₄ aryl group;
(4) a mono- or di-C₆₋₁₄ aryl-carbamoyl group optionally substituted 1 to 3 C₁₋₆ alkyl groups;
(5) an aromatic heterocyclylcarbonyl group (preferably indolylcarbonyl);
(6) a C₁₋₆ alkyl group;
   and the like, more preferably a methylene group.

In the formula (II), Xa-Ya should not be -NHCO-, and Ba¹ should not be a substituted triazinyl group.

In addition, compound (II) should not comprise 2-(benzylthio)-5-chloro-1-phenyl-1H-benzimidazole and 2-(2-chlorobenzylthio)-5-chloro-1-phenyl-1H-benzimidazole.

Moreover, compound (II) is preferably not 1-(4-tert-butylphenyl)-5-methoxycarbonyl-2-[(4-methoxycarbonylbenzyl)thio]-1H-benzimidazole, 2-[2-(1,4-benzodioxan-6-yl)-2-oxoethylthio]-1-(4-tert-butylphenyl)-5-methoxycarbonyl-1H-benzimidazole, 1-(4-tert-butylphenyl)-2-[(3,5-dimethoxybenzyl)thio]-5-methoxycarbonyl-1H-benzimidazole, 1-(4-tert-butylphenyl)-5-methoxycarbonyl-2-[(5-methyl-3-isoxazolylmethyl)thio]-1H-benzimidazole, 1-(4-tert-butylphenyl)-5-methoxycarbonyl-2-[2-(2-thienyl)-2-oxoethylthio]-1H-benzimidazole, and 2-benzylthio-1-(4-tert-butylphenyl)-5-methoxycarbonyl-1H-benzimidazole.

As preferable examples of compound (II), the following compounds can be mentioned.

### [Compound A]

A compound wherein
ring Aa is a benzene ring substituted by 1 to 3 halogen atoms; Z is CH;
Ba¹ is a cyclohexyl group, a phenyl group, a pyridyl group or a piperidinyl group, each of which optionally has 1 to 3 substituents selected from
(1) a halogen atom;
(2) a hydroxy group;
(3) a C₁₋₆ alkyl group;
(4) a C₇₋₁₆ aralkyl group (preferably benzyl);
(5) a C₁₋₆ alkoxy group optionally substituted by 1 to 3 substituents selected from a halogen atom, a hydroxy group, a C₁₋₆ alkoxy group, a carboxyl group, a C₁₋₆ alkoxy-carbonyl group, a C₁₋₆ alkyl-carbonyloxy group and C₃₋₈ cycloalkyl group;
(6) a C₁₋₆ alkylsulfonyloxy group;
(7) a carboxyl group;
(8) a C₁₋₆ alkoxy-carbonyl group;
(9) a C₁₋₆ alkyl-carbonyl group;
(10) a mono- or di-C₁₋₆ alkyl-carbamoyl group optionally substituted by 1 or 2 substituents selected from a hydroxy group and a C₁₋₆ alkoxy group;
   and the like;
   Ba² is a phenyl group or a pyridyl group, each of which optionally has 1 to 3 substituents selected from

(1) a halogen atom;
(2) a cyano group;
(3) a C₁₋₆ alkyl group optionally substituted by 1 to 3 halogen atoms;
(4) a C₆₋₁₄ aryl group (preferably phenyl);
(5) a C₁₋₆ alkoxy group optionally substituted by 1 to 3 halogen atoms;
(6) a C₆₋₁₄ aryloxy group;
(7) a C₁₋₆ alkoxy-carbonyl group;
(8) a C₁₋₆ alkyl-carbonyl group;
   and the like;
   Xa is -S- or -NH-; and
   Ya is a methylene group or an ethylene group, each of which optionally has 1 or 2 substituents selected from

(1) a hydroxy group;
(2) an oxo group;
(3) a C₆₋₁₄ aryl group;
(4) a mono- or di-C₆₋₁₄ aryl-carbamoyl group optionally substituted by 1 to 3 C₁₋₆ alkyl groups;
(5) an aromatic heterocyclylcarbonyl group (preferably indolylcarbonyl);
   and the like
   (Ya is preferably a methylene group).

### [Compound B]

A compound wherein
ring Aa is a benzene ring substituted by 1 to 3 substituents selected from
(1) a halogen atom;
(2) a C₁₋₆ alkoxy group; and
(3) a C₁₋₆ alkoxy-carbonyl group,
   or pyridine ring;
   Z is CH or N;
   Ba¹ is a cyclohexyl group, a phenyl group, a pyridyl group or a piperidinyl group, each of which is optionally substituted by 1 to 3 substituents selected from

(1) a halogen atom;
(2) a hydroxy group;
(3) a cyano group;
(4) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(5) a C₆₋₁₄ aryl group;
(6) a C₇₋₁₆ aralkyl group (preferably benzyl);
(7) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(8) a C₁₋₆ alkylsulfonyloxy group;
(9) a C₆₋₁₄ aryloxy group;
(10) a C₇₋₁₆ aralkyloxy group;
(11) a carboxyl group;
(12) a C₁₋₆ alkoxy-carbonyl group;
(13) a C₁₋₆ alkyl-carbonyl group;
(14) a mono- or di-C₁₋₆ alkyl-carbamoyl group optionally substituted by 1 or 2 substituents selected from a hydroxy group and a C₁₋₆ alkoxy group (including a N-C₁₋₆ alkyl-N-C₁₋₆ alkoxy-carbamoyl group);
(15) a C₁₋₆ alkylthio group;
(16) a C₁₋₆ alkylsulfonyl group;
(17) a heterocyclic group (preferably oxazolinyl, oxazolyl, dioxolanyl); and
(18) a C₁₋₆ alkoxy-carbonyl-amino group;
   Ba² is a phenyl group or a pyridyl group, each of which is optionally substituted by 1 to 3 substituents selected from

(1) a halogen atom;
(2) a cyano group;
(3) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(4) a C₆₋₁₄ aryl group (preferably phenyl);
(5) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(6) a C₆₋₁₄ aryloxy group;
(7) a C₁₋₆ alkoxy-carbonyl group; and
(8) a C₁₋₆ alkyl-carbonyl group;
   Xa is -S- or -NH-; and
   Ya is a methylene group, an ethylene group or a methylmethylene group, each of which is optionally substituted by 1 or 2 substituents selected from

(1) a hydroxy group;
(2) an oxo group;
(3) a C₆₋₁₄ aryl group;
(4) a mono- or di-C₆₋₁₄ aryl-carbamoyl group optionally substituted by 1 to 3 C₁₋₆ alkyl groups;
(5) an aromatic heterocyclylcarbonyl group (preferably indolylcarbonyl); and
(6) a C₁₋₆ alkyl group;
   (Ya is preferably a methylene group).

### [Compound C]

A compound wherein
ring Aa is a benzene ring substituted by 1 to 3 halogen atoms; Z is CH;
Ba¹ is a phenyl group having, at the para-position, 1 to 3 substituents selected from
(1) an optionally substituted C₁₋₆ alkyl group (preferably a C₁₋₆ alkyl group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(2) an optionally substituted C₁₋₆ alkoxy group (preferably a C₁₋₆ alkoxy group optionally substituted by 1 to 4 substituents selected from a halogen atom, a hydroxy group, a C₁₋₆ alkoxy group, a C₆₋₁₄ aryloxy group, a carboxyl group, a C₁₋₆ alkoxy-carbonyl group, a C₁₋₆ alkyl-carbonyloxy group and a C₃₋₈ cycloalkyl group);
(3) a C₁₋₆ alkoxy-carbonyl group; and
(4) a C₁₋₆ alkyl-carbonyl group;
Ba² is
(1) a phenyl group having, at the para-position, 1 to 3 substituents selected from an halogen atom and an optionally halogenated C₁₋₆ alkyl group; or
(2) a 3-pyridyl group having, at the 6-position, 1 to 3 substituents selected from a halogen atom and an optionally
   halogenated C₁₋₆ alkyl group;
   Xa is -S- or -NH-; and
   Ya is a methylene group.

### [Compound D]

6-chloro-2-[(4-chlorobenzyl)thio]-1-(4-ethoxyphenyl)-1H-benzimidazole (Example 10), 6-chloro-1-(4-ethoxyphenyl)-2-({[6-(trifluoromethyl)pyridin-3-yl]methyl}thio)-1H-benzimidazole (Example 36), 2-[(4-tert-butylbenzyl)thio]-6-chloro-1-(4-ethoxyphenyl)-1H-benzimidazole (Example 38), 2-[(4-chlorobenzyl)thio]-1-(4-methoxyphenyl)-1H-imidazo[4,5-b]pyridine (Example 63), 5-chloro-2-[(4-chlorobenzyl)thio]-1-[4-(2,2,2-trifluoroethoxy)phenyl]-1H-benzimidazole (Example 67), 1-(4-butoxyphenyl)-N-(4-tert-butylbenzyl)-5,6-dichloro-1H-benzimidazol-2-amine (Example 99), and N-(4-tert-butylbenzyl)-5,6-dichloro-1-[4-(trifluoromethoxy)phenyl]-1H-benzimidazol-2-amine (Example 105).

As a salt of compound (I) [hereinafter including compound (II)], for example, metal salts, ammonium salts, salts with organic bases, salts with inorganic acids, salts with organic acids, salts with basic or acidic amino acids and the like can be mentioned.

Preferable examples of the metal salt include alkali metal salts such as sodium salt, potassium salt and the like; alkaline earth metal salts such as calcium salt, magnesium salt, barium salt and the like; aluminum salt and the like.

Preferable examples of the salt with organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like.

Preferable examples of the salt with inorganic acid include a salt with hydrochloric acid, hydrobromic acid, nitric acid, sulfuric acid, phosphoric acid and the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include a salt with arginine, lysine, ornithine and the like. Preferable examples of the salt with acidic amino acid include a salt with aspartic acid, glutamic acid and the like.

Of the above-mentioned salts, a pharmacologically acceptable salt is preferable.

The prodrug of compound (I) means a compound which is converted to compound (I) with a reaction due to an enzyme, gastric acid, etc. under the physiological condition in the living body, that is, a compound which is converted to compound (I) by enzymatic oxidation, reduction, hydrolysis, etc.; a compound which is converted to compound (I) by hydrolysis etc. due to gastric acid, and the like.

Examples of a prodrug of compound (I) include a compound wherein an amino group of compound (I) is acylated, alkylated or phosphorylated (e.g., a compound wherein an amino group of compound (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated or tert-butylated, and the like); a compound wherein a hydroxy group of compound (I) is acylated, alkylated, phosphorylated or borated (e.g., a compound wherein a hydroxy group of compound (I) is acetylated, palmitoylated, propanoylated, pivaloylated, succinylated, fumarylated, alanylated or dimethylaminomethylcarbonylated, and the like); a compound wherein a carboxyl group of compound (I) is esterified or amidated (e.g., a compound wherein a carboxyl group of compound (I) is C₁₋₆ alkyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified or methylamidated, and the like) and the like. Of these, a compound wherein a carboxyl group of compound (I) is esterified by C₁₋₆ alkyl group such as methyl, ethyl, tert-butyl and the like is preferable. These compounds can be produced from compound (I) according to a method known per se.

A prodrug of compound (I) may be a compound which is converted to compound (I) under physiological conditions as described in Development of Pharmaceutical Products, vol. 7, Molecule Design, 163-198, Hirokawa Shoten (1990).

Hereinafter the production methods of compound (II) are explained.

Compound (I) can be produced according to the production methods of compound (II), which are explained in detail in the following, or a method analogous thereto.

Each symbol in the schematic drawings of the following reaction schemes is as defined above unless particularly described. Each compound described in the reaction schemes may form a salt as long as it does not inhibit the reaction, and as such salt, those similar to the salts of compound (I) can be mentioned.

Compound (II) can be produced, for example, according to the method as shown in the following Schemes.

When amination reaction, halogenation reaction, reduction reaction, oxidation reaction and the like are conducted in the following production methods, these reactions are carried out according to methods known *per se*. As such methods, for example, the methods described in ORGANIC FUNCTIONAL GROUP PREPARATIONS) second ed., ACADEMIC PRESS, INC. 1989; Comprehensive Organic Transformations, VCH Publishers Inc., 1989, and the like, and the like can be mentioned.

The object products obtained by the following production methods can be isolated and purified by known separation and purification means, such as concentration, concentration under reduced pressure, solvent extraction, crystallization, recrystallization, phase transfer, chromatography and the like. When the production methods contain plural steps, the synthetic intermediate may be isolated and purified by known separation and purification means, or used for the next step in the form of a reaction mixture without isolation and purification.

Compound (II) can be produced, for example, according to [Method A] to [Method C], or a method analogous thereto.

### [Method A]

Compound (II) can be produced, for example, by reacting compound (2a-1) with compound (2a-2). 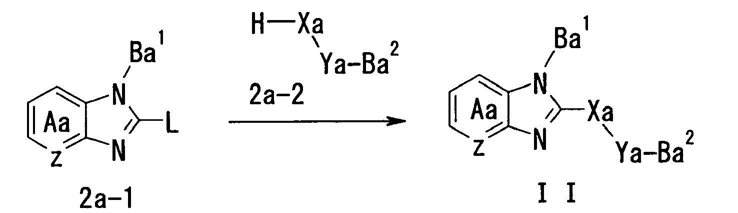 wherein L is a leaving group, and the other symbols are as defined above.

As the leaving group for L, for example, a halogen atom (e.g., chlorine, bromine, iodine), an optionally halogenated C₁₋₆ alkylsulfonyloxy group (e.g., methylsulfonyloxy, ethylsulfonyloxy, trifluoromethylsulfonyloxy), a C₆₋₁₀ arylsulfonyloxy group optionally substituted by a C₁₋₆ alkyl group (e.g., benzenesulfonyloxy, 4-toluenesulfonyloxy), a methylthio group, a methanesulfonyl group and the like can be mentioned.

This reaction can be carried out in a solvent that does not adversely influence the reaction and, where necessary, in the presence of a base.

As the solvent that does not adversely influence the reaction, for example, alcohol solvents (e.g., methanol, ethanol, isopropanol), ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), ketone solvents (e.g., acetone, 2-butanone), nitrile solvents (e.g., acetonitrile), amide solvents (e.g., dimethylformamide), ester solvents (e.g., methyl acetate, ethyl acetate) and the like can be mentioned. Of these, alcohol solvents, ether solvents, hydrocarbon solvents, halogenated hydrocarbon solvents, amide solvents are preferable. These solvents may be used in a mixture of two or more kinds thereof mixed at an appropriate ratio.

The amount of compound (2a-2) to be used is generally 1 to 20 molar equivalents, preferably 1 to 10 molar equivalents, per 1 mol of compound (2a-1).

As the base, for example, alkali metal hydrides such as sodium hydride and the like; alkali metal carbonates such as potassium carbonate, sodium carbonate and the like; alkali metal hydroxides such as potassium hydroxide, sodium hydroxide and the like; tertiary amines such as triethylamine and the like, and the like can be used. The amount of the base to be used is generally 1 to 10 molar equivalents, preferably 1 to 5 molar equivalents, per 1 mol of compound (2a-1).

The reaction temperature is generally -10 to 180°C, preferably 0 to 140°C.

The reaction time is generally 0.5 to 100 hr, preferably 1 to 48 hr.

Compound (2a-1) used as a starting compound can be produced according to the below-mentioned [Method D], [Method E] or [Method F]. Compound (2a-2) used as a starting compound can be produced according to a method known per se.

### [Method B]

Compound (IIa), which is compound (II) wherein Xa is S, can be produced, for example, by reacting compound (2b-1) with compound (2b-2). 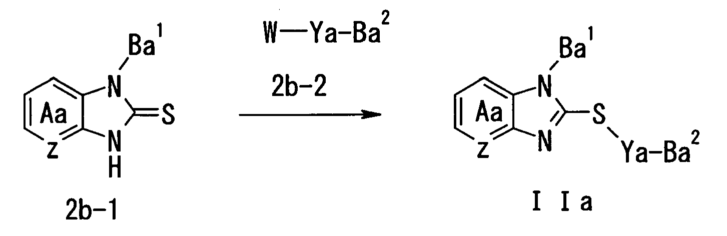 wherein W is a leaving group, and the other symbols are as defined above.

As the leaving group for W, for example, a halogen atom (e.g., chlorine, bromine, iodine), an optionally halogenated C₁₋₆ alkylsulfonyloxy group (e.g., methylsulfonyloxy, ethylsulfonyloxy, trifluoromethylsulfonyloxy), a C₆₋₁₀ arylsulfonyloxy group optionally substituted by a C₁₋₆ alkyl group (e.g., benzenesulfonyloxy, 4-toluenesulfonyloxy), a hydroxy group and the like can be mentioned. Of these, a halogen atom, an optionally halogenated C₁₋₆ alkylsulfonyloxy group and the like are preferable.

This reaction can be carried out in a solvent that does not adversely influence the reaction and, where necessary, in the presence of a base.

As the solvent that does not adversely influence the reaction, those exemplified in the aforementioned [Method A], water and the like can be mentioned. These solvents may be used in a mixture of two or more kinds thereof mixed at an appropriate ratio.

The amount of compound (2b-2) to be used is generally 1 to 10 molar equivalents, preferably 1 to 5 molar equivalents, per 1 mol of compound (2b-1).

As the base, for example, those exemplified in the aforementioned [Method A] can be used. The amount of the base to be used is generally 1 to 10 molar equivalents, preferably 1 to 5 molar equivalents, per 1 mol of compound (2b-1).

The reaction temperature is generally -10 to 100°C, preferably 0 to 60°C.

The reaction time is generally 0.5 to 100 hr, preferably 1 to 48 hr.

Compound (2b-1) used as a starting compound can be produced according to the below-mentioned [Method G]. Compound (2b-2) used as a starting compound can be produced according to a method known per se.

### [Method C]

Compound (IIb), which is compound (II) wherein Xa is NH, can be produced, for example, by subjecting compound (2c) to a S-methylation reaction and subjecting the resulting compound to a cyclization reaction. 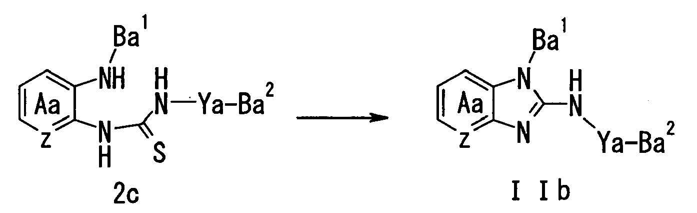 wherein the symbols are as defined above.

The S-methylation reaction and cyclization reaction can be carried out in a solvent that does not adversely influence the reaction and, where necessary, in the presence of a base.

As the solvent that does not adversely influence the reaction, for example, those exemplified in the aforementioned [Method A] can be used.

The S-methylation reaction is carried out using a methylating reagent according to a method known per se. As the reagent, for example, methyl iodide, dimethylsulfuric acid and the like can be mentioned. The amount of the reagent to be used is generally 1 to 10 molar equivalents, preferably 1 to 5 molar equivalents, per 1 mol of compound (2c).

As the base, for example, alkali metal carbonates such as potassium carbonate, sodium carbonate and the like; alkali metal hydroxides such as potassium hydroxide, sodium hydroxide and the like; tertiary amines such as triethylamine and the like; cyclic amines such as pyridine and the like, and the like can be mentioned. The amount of the base to be used is generally 1 to 10 molar equivalents, preferably 1 to 5 molar equivalents, per 1 mol of compound (2c).

The reaction temperature of the S-methylation reaction is generally -10 to 100°C, preferably 0 to 40°C.

The reaction time of the S-methylation reaction is generally 0.5 to 100 hr, preferably 1 to 48 hr.

The cyclization reaction is carried out in the presence of a base, according to a method known per se. The cyclization reaction sometimes proceeds under the conditions of the aforementioned S-methylation reaction. Alternatively, the cyclization reaction proceeds by increasing the reaction temperature after the production of the S-methyl compound.

As the base, for example, alkali metal carbonates such as potassium carbonate, sodium carbonate and the like; alkali metal hydroxides such as potassium hydroxide, sodium hydroxide and the like; tertiary amines such as triethylamine and the like; cyclic amines such as pyridine and the like, and the like can be mentioned. The amount of the base to be used is generally 1 to 10 molar equivalents, preferably 1 to 5 molar equivalents, per 1 mol of the S-methyl compound obtained by the aforementioned S-methylation reaction.

The reaction temperature of the cyclization reaction is generally 20 to 150°C, preferably 50 to 100°C.

The reaction time of the cyclization reaction is generally 0.5 to 100 hr, preferably 6 to 48 hr.

The starting compound (2c) can be produced according to the below-mentioned [Method H].

Compound (2a-1) used as a starting compound in the aforementioned [Method A] can be produced according to a method known per se.

For example, compound (2a-1a), which is compound (2a-1) wherein L is a halogen atom, can be produced according to the following [Method D] or [Method E].

### [Method D]

Compound (2a-1a) can be produced, for example, by reacting compound (2d-1) with compound (2d-2) to give compound (2e), subjecting compound (2e) to a reduction reaction to give compound (2f), subjecting compound (2f) to a cyclization reaction to give compound (2g), and subjecting compound (2g) to a halogenation reaction. 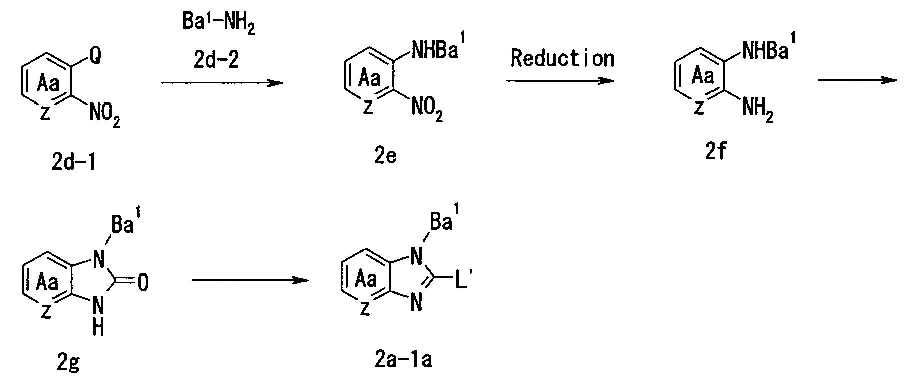 wherein Q is a leaving group, L' is a halogen atom, and the other symbols are as defined above.

As the leaving group for Q, for example, a halogen atom (e.g., fluorine, chlorine, bromine, iodine), an optionally halogenated C₁₋₆ alkylsulfonyloxy group (e.g., methylsulfonyloxy, ethylsulfonyloxy, trifluoromethylsulfonyloxy), a C₆₋₁₀ arylsulfonyloxy group optionally substituted by a C₁₋₆ alkyl group (e.g., benzenesulfonyloxy, 4-toluenesulfonyloxy) and the like can be mentioned. Of these, a halogen atom, an optionally halogenated C₁₋₆ alkylsulfonyloxy group and the like are preferable.

As the halogen atom for L', chlorine, bromine or iodine can be used.

The reaction of compound (2d-1) with compound (2d-2) can be carried out, if necessary, in a solvent that does not adversely influence the reaction, in the presence of a base as necessary.

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), nitrile solvents (e.g., acetonitrile), amide solvents (e.g., dimethylformamide), water and the like can be mentioned. These solvents may be used in a mixture of two or more kinds thereof mixed at an appropriate ratio.

As the base, for example, amines such as triethylamine, diisopropylethylamine, 4-dimethylaminopyridine, triethylenediamine, tetramethylethylenediamine, N-methylmorpholine and the like; alkali metal carbonates such as potassium carbonate, sodium carbonate and the like; alkali metal hydrogencarbonates such as potassium hydrogencarbonate, sodium hydrogencarbonate and the like, and the like can be mentioned.

The amount of the base to be used is generally 1 to 10 molar equivalents, preferably 1 to 5 molar equivalents, per 1 mol of compound (2d-1).

The amount of compound (2d-2) to be used is generally 1 to 5 molar equivalents, preferably 1 to 1.5 molar equivalents, per 1 mol of compound (2d-1).

When Q is not a fluorine atom, this reaction may be carried out in the presence of an alkali metal fluoride (e.g., potassium fluoride, sodium fluoride and the like) (SYNTHESIS, 1990,(5), p430). The amount of the alkali metal fluoride to be used is generally 1 to 5 molar equivalents, preferably 1 to 1.5 molar equivalents, per 1 mol of compound (2d-1).

The reaction temperature is generally 0 to 200°C, preferably 20 to 170°C.

The reaction time is generally 0.5 to 100 hr, preferably 1 to 48 hr.

The reduction reaction of compound (2e) is carried out by a catalytic hydrogenation reaction, when ring Aa and Ba¹ do not have a functional group (e.g., a halogen atom, a benzylamino group, a benzyloxy group) capable of a chemical reaction due to the catalytic hydrogenation reaction.

The catalytic hydrogenation reaction is carried out, for example, in the presence of a catalyst (e.g., platinum oxide; palladium, ruthenium, rhodium or iridium attached onto activated carbon, barium sulfate, calcium carbonate and the like; Raney-nickel) and a hydrogen source (e.g., hydrogen, cyclohexene, hydrazine, ammonium formate).

The amount of the catalyst to be used is generally 0.01 to 5 gram, preferably 0.1 to 0.5 gram, per 1 gram of compound (2e).

This reaction can be carried out in a solvent that does not adversely influence the reaction and, where necessary, in the presence of a base.

As the solvent that does not adversely influence the reaction, for example, those exemplified in the aforementioned [Method B] can be used. These solvents may be used in a mixture of two or more kinds thereof mixed at an appropriate ratio.

As the acid, for example, formic acid, acetic acid, hydrochloric acid, methanesulfonic acid, 4-toluenesulfonic acid and the like can be mentioned.

When hydrogen is used in this reaction, the pressure is generally 1 to 10 atm, preferably 1 to 2 atm.

The reaction temperature is generally 0 to 100°C, preferably 20 to 60°C.

The reaction time is generally 0.5 to 100 hr, preferably 1 to 48 hr.

The reduction reaction of compound (2e) is carried out using a reducing agent such as 1) sodium hydrosulfite; 2) a combination of a metal (e.g., iron, zinc) and a acidic compound (e.g., hydrochloric acid, sulfuric acid, methanesulfonic acid, ammonium chloride) ; 3) a metal hydride (e.g., nickel borohydride); and the like, when ring Aa or Ba¹ have a functional group (e.g., a halogen atom, a benzylamino group, a benzyloxy group) capable of a chemical reaction due to the catalytic hydrogenation reaction.

This reaction can be carried out in a solvent that does not adversely influence the reaction.

As the solvent that does not adversely influence the reaction,
1) when sodium hydrosulfite or a combination of a metal and an acidic compound is used as a reducing agent, for example, alcohol solvents (e.g., methanol, ethanol, propanol), ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), nitrile solvents (e.g., acetonitrile), amide solvents (e.g., dimethylformamide), water and the like;
2) when a metal hydride is used as a reducing agent, for example, alcohol solvents (e.g., methanol, ethanol, propanol), ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), nitrile solvents (e.g., acetonitrile), amide solvents (e.g., dimethylformamide) and the like;
   can be mentioned. These solvents may be used in a mixture of two or more kinds thereof mixed at an appropriate ratio.

The amount of the reducing agent to be used is generally 1 to 100 molar equivalents, preferably 1 to 50 molar equivalents, per 1 mol of compound (2e).

The reaction temperature is generally -10 to 150°C, preferably 0 to 110°C.

The reaction time is generally 0.5 to 100 hr, preferably 1 to 48 hr.

The cyclization reaction of compound (2f) can be carried out using a reagent for synthesizing a cyclic urea in a solvent that does not adversely influence the reaction and, where necessary, in the presence of a base.

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), ketone solvents (e.g., acetone, 2-butanone), nitrile solvents (e.g., acetonitrile), amide solvents (e.g., dimethylformamide), ester solvents (e.g., methyl acetate, ethyl acetate) and the like can be mentioned. These solvents may be used in a mixture of two or more kinds thereof mixed at an appropriate ratio.

As the reagent for synthesizing a cyclic urea, for example, phosgene or compounds analogous thereof (e.g., triphosgene), 1,1-carbonyldiimidazole, carbonate compounds (e.g., diphenyl carbonate, dimethyl carbonate, diethyl carbonate, 2-oxo-1,3-dioxolane), halogenated formates (e.g., methyl chloroformate, ethyl chloroformate, phenyl chloroformate, 4-nitrophenyl chloroformate) and the like can be used.

The amount of the reagent to be used is generally 1 to 10 molar equivalents, preferably 1 to 5 molar equivalents, per 1 mol of compound (2f).

As the base, for example, those exemplified in the aforementioned [Method A] can be used. The amount of the base to be used is generally 1 to 10 molar equivalents, preferably 1 to 5 molar equivalents, per 1 mol of compound (2f).

The reaction temperature is generally -10 to 100°C, preferably 0 to 30°C.

The reaction time is generally 0.5 to 100 hr, preferably 1 to 48 hr.

The halogenation reaction of compound (2g) can be carried out using a halogenating reagent and, where necessary, in a solvent that does not adversely influence the reaction.

As the halogenating reagent, for example, thionyl chloride, phosphoryl chloride, phosphorus pentachloride, phosphorus tribromide and the like can be mentioned.

The amount of the halogenating reagent to be used is generally 1 to 20 molar equivalents, preferably 2 to 10 molar equivalents, per 1 mol of compound (2g).

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), ester solvents (e.g., methyl acetate, ethyl acetate) and the like can be mentioned. These solvents may be used in a mixture of two or more kinds thereof mixed at an appropriate ratio.

The reaction temperature is generally 0 to 120°C, preferably 20 to 100°C.

The reaction time is generally 0.5 to 100 hr, preferably 1 to 48 hr.

Compound (2d-1) and compound (2d-2) used as starting compounds can be produced according to a method known per se.

### [Method E]

Compound (2a-1a) can also be produced by subjecting compound (2f) to a cyclization reaction to give compound (2h), and subjecting compound (2h) to a halogenation reaction. 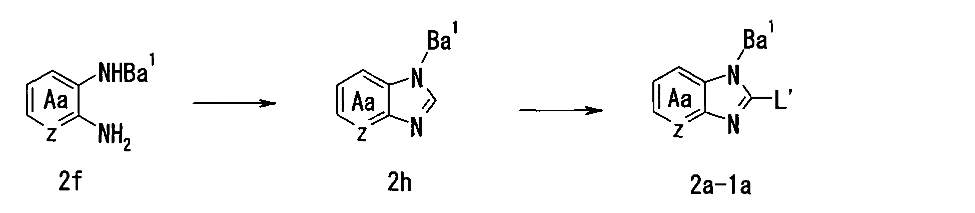 wherein the symbols are as defined above.

The cyclization reaction of compound (2f), for example, can be carried out using an orthoformate, if necessary, in a solvent that does not adversely influence the reaction, in the presence of an acid as necessary.

As the orthoformate, for example, methyl orthoformate, ethyl orthoformate and the like can be mentioned.

The amount of the orthoformate to be used is generally 1 to 20 molar equivalents, preferably 1 to 10 molar equivalents, per 1 mol of compound (2f).

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), amide solvents (e.g., dimethylformamide), ester solvents (e.g., methyl acetate, ethyl acetate) and the like can be mentioned. These solvents may be used in a mixture of two or more kinds thereof mixed at an appropriate ratio.

As the acid, for example, hydrochloric acid, sulfuric acid, methanesulfonic acid, Lewis acids (e.g., zinc chloride, iron chloride, titanium chloride) and the like can be mentioned. The amount of the acid to be used is generally 0.05 to 5 molar equivalents, preferably 0.1 to 1 molar equivalent, per 1 mol of compound (2f).

The reaction temperature is generally 0 to 100°C, preferably 20 to 60°C.

The reaction time is generally 0.5 to 100 hr, preferably 1 to 48 hr.

The halogenation reaction of compound (2h) carried out using a halogenating reagent in a solvent that does not adversely influence the reaction.

As the halogenating reagent, for example, N-bromosuccinimide, N-chlorosuccinimide, bromine, chlorine and the like can be mentioned. The amount of the halogenating reagent to be used is generally 1 to 10 molar equivalents, preferably 1 to 3 molar equivalents, per 1 mol of compound (2h).

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride) and the like can be mentioned.

The reaction temperature is generally -20 to 100°C, preferably 0 to 60°C.

The reaction time is generally 0.5 to 100 hr, preferably 1 to 48 hr.

Compound (2f) used as a starting compound can be produced according to the aforementioned [Method D] or a method known per se.

Compound (2a-1b) and compound (2a-1c), which are compounds (2a-1), used as a starting compound in the aforementioned [Method A], wherein L is a methylthio group and a methanesulfonyl group, respectively, can be produced, for example, according to the following [Method F].

### [Method F]

Compound (2a-1b) can be produced by reacting compound (2b-1) with a methylating reagent. Compound (2a-1c) can be produced by subjecting compound (2a-1b) to an oxidization reaction. 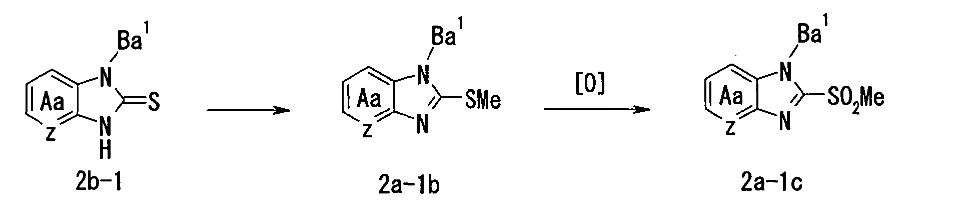 wherein the symbols are as defined above.

As the methylating reagent used for the production of compound (2a-1b), for example, those exemplified in the aforementioned [Method C] can be used. The amount of the reagent to be used is generally 1 to 10 molar equivalents, preferably 2 to 5 molar equivalents, per 1 mol of compound (2b-1).

This reaction can be carried out in a solvent that does not adversely influence the reaction.

As the solvent that does not adversely influence the reaction, for example, ether solvents (e.g., diethyl ether, tetrahydrofuran, dioxane), hydrocarbon solvents (e.g., benzene, toluene, hexane, heptane), halogenated hydrocarbon solvents (e.g., dichloromethane, dichloroethane, chloroform, carbon tetrachloride), ketone solvents (e.g., acetone, 2-butanone), nitrile solvents (e.g., acetonitrile), amide solvents (e.g., dimethylformamide), ester solvents (e.g., methyl acetate, ethyl acetate), water and the like can be mentioned.

The reaction temperature is generally -10 to 100°C, preferably 0 to 30°C.

The reaction time is generally 0.5 to 100 hr, preferably 1 to 48 hr.

The oxidization reaction of compound (2a-1b) is carried out using an oxidizing agent in a solvent that does not adversely influence the reaction.

As the solvent that does not adversely influence the reaction, for example, hydrocarbon solvents (e.g., benzene, toluene, hexane, heptane), halogenated hydrocarbon solvents (e.g., dichloromethane, dichloroethane, chloroform, carbon tetrachloride), amide solvents (e.g., dimethylformamide), ester solvents (e.g., methyl acetate, ethyl acetate), acetic acid, water and the like can be mentioned.

As the oxidizing agent, for example, m-chloroperbenzoic acid, OXONE, aqueous hydrogen peroxide solution and the like can be mentioned. The amount of the oxidizing agent to be used is generally 2 to 5 molar equivalents, preferably 2 to 4 molar equivalents, per 1 mol of compound (2a-1b).

The reaction temperature is generally -10 to 100°C, preferably 0 to 40°C.

The reaction time is generally 0.5 to 100 hr, preferably 1 to 48 hr.

Compound (2b-1) used as a starting compound in the aforementioned [Method B] and [Method F] can be produced, for example, according to the following [Method G].

### [Method G]

Compound (2b-1) can be produced, for example, by reacting compound (2f) with a thiocarbonylating reagent. 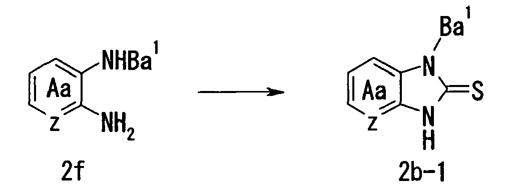 wherein the symbols are as defined above.

This reaction can be carried out in a solvent that does not adversely influence the reaction and, where necessary, in the presence of a base.

As the solvent that does not adversely influence the reaction, those exemplified in the cyclization reaction of compound (2f) in the aforementioned [Method D] can be mentioned. These solvents may be used in a mixture of two or more kinds thereof mixed at an appropriate ratio.

As the thiocarbonylating reagent, for example, thiophosgene, 1,1'-thiocarbonyldiimidazole, carbon disulfide and the like can be mentioned. The amount of the reagent to be used is generally 1 to 10 molar equivalents, preferably 1 to 5 molar equivalents, per 1 mol of compound (2f).

As the base, for example, amines such as triethylamine, pyridine and the like can be mentioned. The amount of the base to be used is generally 1 to 10 molar equivalents, preferably 1 to 5 molar equivalents, per 1 mol of compound (2f).

The reaction temperature is generally -10 to 100°C, preferably 0 to 50°C.

The reaction time is generally 0.5 to 100 hr, preferably 1 to 48 hr.

Compound (2c) used as a starting compound can be produced, for example, according to the following [Method H].

### [Method H]

Compound (2c) can be produced, for example, by reacting compound (2f) with compound (2i). 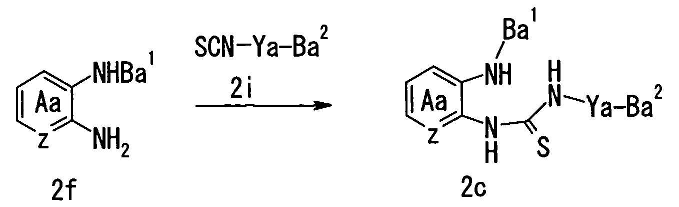 wherein the symbols are as defined above.

This reaction can be carried out in a solvent that does not adversely influence the reaction and, where necessary, in the presence of a base.

As the solvent that does not adversely influence the reaction, those exemplified in the cyclization reaction of compound (2f) in the aforementioned [Method D] can be mentioned. These solvents may be used in a mixture of two or more kinds thereof mixed at an appropriate ratio.

The amount of compound (2i) to be used is generally 1 to 5 molar equivalents, preferably 1 to 3 molar equivalents, per 1 mol of compound (2f).

As the base, for example, those exemplified in the aforementioned [Method G] can be used. The amount of the base to be used is generally 0.5 to 10 molar equivalents, preferably 1 to 5 molar equivalents, per 1 mol of compound (2i).

The reaction temperature is generally -10 to 10°C, preferably 0 to 40°C.

The reaction time is generally 0.5 to 100 hr, preferably 1 to 48 hr.

Compound (2i) used as a starting compound can be produced according to a method known per se.

When ring Aa, Ba¹ or Ba² in compound (II) has a substituent containing a convertible functional group (e.g., a carboxyl group, an amino group, a hydroxy group, a carbonyl group, a mercapto group, an ester group, a sulfo group, a halogen atom), various compounds can be produced by converting the functional group according to a method known per se or a method analogous thereto.

The carboxyl group can be converted, for example, by reactions such as esterification, reduction, amidation, conversion to an optionally protected amino group, and the like.

The amino group can be converted, for example, by a reaction such as amidation, sulfonylation, nitrosation, alkylation, arylation, imidation and the like.

The hydroxy group can be converted, for example, by a reaction such as esterification, carbamoylation, sulfonylation, alkylation, arylation, oxidation, halogenation and the like.

The carbonyl group can be converted, for example, by a reaction such as reduction, oxidation, imination (including oximation, hydrazonation), (thio)ketalization, alkylidenation, thiocarbonylation and the like.

The mercapto group can be converted, for example, by a reaction such as alkylation, oxidation and the like.

The ester group can be converted, for example, by a reaction such as reduction, hydrolysis and the like.

The sulfo group can be converted, for example, by a reaction such as sulfonamidation, reduction and the like.

The halogen atom can be converted, for example, by various nucleophilic displacement reactions, various coupling reactions and the like.

In each of the above-mentioned reactions, when the compound is obtained as a free compound, it can be converted to a salt according to a conventional method, and when the compound is obtained as a salt, it can be converted to a free form or other salt according to a conventional method.

In addition, in each of the aforementioned reactions, when the starting compound has an amino group, a carboxyl group, a hydroxy group or a mercapto group as a substituent, a protecting group generally used in peptide chemistry and the like may be introduced into these groups. By removing the protecting group as necessary after the reaction, the objective compound can be obtained.

As the amino-protecting group, for example, formyl group; C₁₋₆ alkyl-carbonyl group, phenylcarbonyl group, C₁₋₆ alkoxy-carbonyl group, allyloxycarbonyl (Alloc) group, phenyloxycarbonyl group, fluorenylmethyloxycarbonyl (Fmoc) group, C₇₋₁₀ aralkyl-carbonyl group (e.g., benzylcarbonyl), C₇₋₁₀ aralkyl-oxycarbonyl group (e.g., benzyloxycarbonyl (Z)), C₇₋₂₀ aralkyl group (e.g., benzyl, trityl), phthaloyl group, dithiasuccinoyl group and N,N-dimethylaminomethylene group, each of which optionally has substituent(s), and the like can be mentioned. As the substituent(s), for example, phenyl group, halogen atom, C₁₋₆ alkyl-carbonyl group, optionally halogenated C₁₋₆ alkoxy group, nitro group and the like are used. The number of the substituent(s) is 1 to 3.

As the carboxyl-protecting group, for example, C₁₋₆ alkyl group, allyl group, C₇₋₂₀ aralkyl group (e.g., benzyl, trityl), phenyl group and trialkylsilyl group (e.g., trimethylsilyl, tert-butyldimethylsilyl, diisopropylethylsilyl), each of which optionally has substituent(s), and the like can be mentioned. As the substituent(s), for example, halogen atom, formyl group, C₁₋₆ alkyl-carbonyl group, optionally halogenated C₁₋₆ alkoxy group, nitro group and the like are used. The number of the substituent(s) is 1 to 3.

As the hydroxy-protecting group, for example, C₁₋₆ alkyl group, C₇₋₂₀ aralkyl group (e.g., benzyl, trityl), formyl group, C₁₋₆ alkyl-carbonyl group, benzoyl group, C₇₋₁₀ aralkyl-carbonyl group (e.g., benzylcarbonyl), tetrahydropyranyl group, furanyl group and trialkylsilyl group (e.g., trimethylsilyl, tert-butyldimethylsilyl, diisopropylethylsilyl), each of which optionally has substituent(s), and the like can be mentioned. As the substituent(s), for example, halogen atom, C₁₋₆ alkyl group, phenyl group, C₇₋₁₆ aralkyl group, C₁₋₆ alkoxy group, nitro group and the like are used. The number of the substituent(s) is 1 to 4.

As the mercapto-protecting group, for example, C₁₋₆ alkyl group, C₁₋₆ alkyl-carbonyl group and C₇₋₂₀ aralkyl group (e.g., benzyl, trityl), each of which optionally has substituent(s), and the like can be mentioned. As the substituent(s), for example, halogen atom, C₁₋₆ alkyl group, phenyl group, C₇₋₁₆ aralkyl group, C₁₋₆ alkoxy group, nitro group and the like are used. The number of the substituent(s) is 1 to 4.

For elimination of the protecting group, a method known per se or a method analogous thereto is used. For example, treatments with acid, base, reduction, ultraviolet rays, hydrazine, phenylhydrazine, sodium N-methyldithiocarbamate, tetrabutylammonium fluoride, palladium acetate and the like are used.

Compound (I) thus obtained, other reaction intermediates and starting compounds thereof can be isolated or purified from the reaction mixture according to a method known per se, for example, extraction, concentration, neutralization, filtration, distillation, recrystallization, column chromatography, thin layer chromatography, preparative high pressure liquid chromatography (preparative HPLC), intermediate pressure preparative liquid chromatography (intermediate pressure preparative LC) and the like.

When compound (I) has optical isomers, these respective optical isomers and mixtures thereof are naturally encompassed in the scope of the present invention, and where desired, these isomers can be also subjected to optical resolution according to a method known per se or individually produced.

When the compound (I) is present as a configurational isomer, diastereomer, conformer or the like, each can be isolated by the above separation and purification methods on demand. In addition, when the compound (I) is in the form of a racemate, it can be separated into S- and R-forms by conventional optical resolution.

When the compound (I) includes stereoisomers, both the isomers alone and mixtures of each isomers are included in the scope of the present invention.

In addition, the compound (I) may be a hydrate or non-hydrate.

The compound (I) may be labeled with an isotope (e.g., ³H, ¹⁴C, ³⁵_{S}) or the like.

Compound (I), compound (II) and prodrugs thereof (hereinafter sometimes to be abbreviated as the compound of the present invention) have a GPR40 receptor function regulating action, particularly a GPR40 receptor antagonist activity, and show low toxicity (e.g., acute toxicity, chronic toxicity, genetic toxicity, reproductive toxicity, cardiotoxicity, drug interaction, carcinogenicity), they are useful as safe GPR40 receptor function regulators, preferably GPR40 antagonists.

Accordingly, the compound of the present invention is useful as a regulator of a physiological function involving a GPR40 receptor or an agent for the prophylaxis or treatment of a pathology or disease involving a GPR40 receptor.

To be specific, the compound of the present invention is useful as an insulin secretion regulator (preferably an insulin secretion inhibitor), an obesity improving agent, a pancreas protector (a pancreatic β cell protector) or an insulin sensitizer.

For example, while fatty acid promotes secretion of insulin from pancreatic β cells, excessive insulin secretion is considered to encourage obesity and promote insulin resistance. In addition, excessive stimulation of insulin secretion from pancreatic β cells by fatty acid is considered to cause pancreatic fatigue. Therefore, a pharmaceutical agent capable of suppressing excessive insulin secretion from pancreatic β cells is useful as an agent for the prophylaxis or treatment or improvement of obesity, insulin resistance, pancreatic fatigue and the like. In addition, the agent of the present invention is useful as an agent for the prophylaxis or treatment of type 2 diabetes, since it suppresses pancreatic fatigue to maintain or recover the glucose-dependent insulin secretion capability, which is an important function of pancreatic β cells.

The compound of the present invention is useful as an agent for the prophylaxis or treatment of diseases such as diabetes (e.g., type 1 diabetes, type 2 diabetes, gestational diabetes, unstable diabetes, obese diabetes), impaired glucose tolerance, ketosis, acidosis, diabetic neuropathy, diabetic nephropathy, retinopathy (e.g., diabetic retinopathy), obesity, metabolic syndrome, insulin resistance, hyperinsulinemia, hypertension, hyperlipidemia (e.g., hypertriglyceridemia, hypercholesterolemia, hypoHDL-emia, postprandial hyperlipemia), arteriosclerosis, cardiac failure, cardiac infarction, sexual dysfunction, dermatic diseases, arthropathy, osteopenia, thrombotic diseases (e.g., foot ulcer), deficits in memory and learning, depression, depression and mania, schizophrenia, attention deficit hyperactivity disorder, visual disorder, appestat disorders (e.g., hyperorexia), hypoglycemia, edema, fatty atrophy, lipotoxicity, pancreatic fatigue, cancer (e.g., insulinoma, breast cancer), immune diseases (e.g., immunodeficiency), inflammatory diseases (e.g., enteritis, arthritis, allergy), multiple sclerosis, dyspepsia, acute renal failure and the like.

Based on its superior GPR40 receptor antagonist activity, the compound of the present invention is useful as an agent for the prophylaxis or treatment of diabetes, diabetic neuropathy, diabetic nephropathy, retinopathy (e.g., diabetic retinopathy), obesity, metabolic syndrome, insulin resistance, impaired glucose tolerance, hyperinsulinemia, hypertension, hyperlipidemia, arteriosclerosis, cardiac failure, cardiac infarction, thrombotic disease (e.g., foot ulcer), deficits in memory and learning, depression and mania, visual disorder, appestat disorder, lipotoxicity, pancreatic fatigue, immune disease (e.g., immunodeficiency), inflammatory disease (e.g., enteritis, arthritis, allergy) or cancer (e.g., breast cancer).

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing any of a fasting blood glucose level (glucose concentration of intravenous plasma) of not less than 126 mg/dl, a 75 g oral glucose tolerance test 2 h level (glucose concentration of intravenous plasma) of not less than 200 mg/dl, and a non-fasting blood glucose level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 h level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes; and progress of obesity into diabetes.

Since the compound of the present invention has a superior GPR40 receptor antagonist activity, it can afford a remarkable effect (e.g., treatment effect on obesity, diabetes and the like) for patients showing a high blood free fatty acid value.

The compound of the present invention can be safely administered orally or parenterally (e.g., topical, rectal, intravenous administration etc.) to a mammal (e.g., mouse, rat, hamster, rabbit, cat, dog, bovine, sheep, monkey, human etc.) after, where necessary, admixing with a pharmacologically acceptable carrier to give a pharmaceutical preparation according to a method known *per se* used for the general production method for pharmaceutical preparations.

The dosage form of the aforementioned pharmaceutical preparation (hereinafter including "GPR40 receptor function regulator comprising compound (I) or a prodrug thereof" and "pharmaceutical agent comprising compound (II) or a prodrug thereof") is, for example, an oral agent such as tablets (inclusive of sublingual tablets and orally disintegrable tablets), capsules (inclusive of soft capsules and micro capsules), granules, powders, troches, syrups, emulsions, suspensions and the like; or a parenteral agent such as injections (e.g., subcutaneous injections, intravenous injections, intramuscular injections, intraperitoneal injections, drip infusions etc.), external agents (e.g., transdermal preparations, ointments etc.), suppositories (e.g., rectal suppositories, vaginal suppositories etc.), pellets, nasal preparations, pulmonary preparations (inhalations), ophthalmic preparations and the like.

These preparations may be controlled-release preparations such as rapid-release preparations and sustained-release preparations (e.g., sustained-release microcapsules).

The content of the compound of the present invention in a pharmaceutical preparation of the present invention is about 0.01 to about 100% by weight relative to the whole preparation. The dose of the compound of the present invention varies depending on administration subjects, administration route, diseases, condition and the like. When the compound is orally administered to an adult patient with diabetes (body weight about 60 kg), the dose is about 0.01 to about 30 mg/kg body weight per day, preferably about 0.1 to about 20 mg/kg body weight per day, more preferably about 1 to about 20 mg/kg body weight per day, which may be given at once or several portions a day.

Various organic or inorganic carriers conventionally used as materials for pharmaceutical preparations are used as a pharmacologically acceptable carrier, which are added as excipient, lubricant, binder and disintegrant for solid preparations; and solvent, dissolution aids, suspending agent, isotonicity agent, buffer and soothing agent and the like for liquid preparations. Where necessary, additive such as preservative, antioxidant, coloring agent, sweetening agent, adsorbing agent, wetting agent and the like can be used.

As the excipient, for example, lactose, sucrose, D-mannitol, starch, corn starch, crystalline cellulose, light silicic anhydride and the like can be mentioned.

As the lubricant, for example, magnesium stearate, calcium stearate, talc, colloidal silica and the like can be mentioned.

As the binder, for example, crystalline cellulose, sucrose, D-mannitol, dextrin, hydroxypropylcellulose, hydroxypropylmethylcellulose, polyvinylpyrrolidone, starch, sucrose, gelatin, methylcellulose, carboxymethylcellulose sodium and the like can be mentioned.

As the disintegrant, for example, starch, carboxymethylcellulose, carboxymethylcellulose calcium, carboxymethylstarch sodium, L-hydroxypropylcellulose and the like can be mentioned.

As the solvent, for example, water for injection, alcohol, propylene glycol, macrogol, sesame oil, corn oil, olive oil and the like can be mentioned.

As the dissolution aids, for example, polyethylene glycol, propylene glycol, D-mannitol, benzyl benzoate, ethanol, trisaminomethane, cholesterol, triethanolamine, sodium carbonate, sodium citrate and the like can be mentioned.

As the suspending agent, for example, surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, and the like can be mentioned.

As an isotonicity agent, for example, glucose, D-sorbitol, sodium chloride, glycerin, D-mannitol and the like can be mentioned.

As the buffer, for example, buffers such as phosphate, acetate, carbonate, citrate and the like, and the like can be mentioned.

As the soothing agent, for example, benzyl alcohol and the like can be mentioned.

As the preservative, for example, p-hydroxybenzoates, chlorobutanol, benzyl alcohol, phenethyl alcohol, dehydroacetic acid, sorbic acid and the like can be mentioned.

As the antioxidant, for example, sulfite, ascorbic acid, α-tocopherol and the like can be mentioned.

As the coloring agent, for example, water-soluble edible tar pigments (e.g., foodcolors such as Food Color Red Nos. 2 and 3, Food Color Yellow Nos. 4 and 5, Food Color Blue Nos. 1 and 2), water insoluble lake pigments (e.g., aluminum salt of the aforementioned water-soluble edible tar pigment), natural pigments (e.g., β-carotene, chlorophil, red iron oxide) and the like can be mentioned.

As the sweetening agent, for example, saccharin sodium, dipotassium glycyrrhizinate, aspartame, stevia and the like can be mentioned.

The compound of the present invention can be used in combination with drugs such as a therapeutic agent for diabetes, a therapeutic agent for diabetic complications, a therapeutic agent for hyperlipidemia, an antihypertensive agent, an antiobestic agent, a diuretic, a chemotherapeutic agent, an immunotherapeutic agent, an antiinflammatory drug, an antithrombotic agent, a therapeutic agent for osteoporosis, a vitamin, an antidementia agent, a therapeutic agent for incontinentia or pollakiuria, a therapeutic agent for dysurea and the like (hereinafter to be referred to as drug X).

Examples of the therapeutic agent for diabetes include insulin preparations (e.g., animal insulin preparations extracted from the pancreas of bovine and pig; human insulin preparations genetically synthesized using *Escherichia coli,* yeast; zinc insulin; protamine zinc insulin; fragment or derivative of insulin (e.g., INS-1), oral insulin preparation), insulin sensitizers (e.g., Pioglitazone or a salt thereof (preferably hydrochloride), Rosiglitazone or a salt thereof (preferably maleate), Reglixane (JTT-501), Netoglitazone (MCC-555), FK-614, Rivoglitazone (CS-011), Muraglitazar (BMS-298585), compounds described in WO99/58510 (e.g., (E)-4-[4-(5-methyl-2-phenyl-4-oxazolylmethoxy)benzyloxyimino]-4-phenylbutyric acid), compounds described in WO01/38325, Tesaglitazar (AZ-242), Edaglitazone (BM-13-1258), LM-4156, Metaglidasen (MBX-102), LY-519818, MX-6054, LY-510929, Balaglitazone (NN-2344), T-131 or a salt thereof, THR-0921), α-glucosidase inhibitors (e.g., voglibose, acarbose, miglitol, emiglitate), biguanides (e.g., phenformin, metformin, buformin or salts thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride), repaglinide, senaglinide, mitiglinide or calcium salt hydrate thereof, nateglinide], GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], dipeptidyl peptidase IV inhibitors (e.g., NVP-DPP-278, PT-100, P32/98, P93/01, NVP-DPP-728, Vildagliptin (LAF237), TS-021, Sitagliptin phosphate (MK-0431), Saxagliptin (BMS-477118), E-3024, T-6666(TA-6666), 823093, 825964, 815541), β3 agonist (e.g., CL-316243, SR-58611-A, UL-TG-307, AJ-9677, AZ40140), amylin agonists (e.g., pramlintide), phosphotyrosine phosphatase inhibitors (e.g., sodium vanadate), gluconeogenesis inhibitors (e.g., glycogen phosphorylase inhibitor, glucose-6-phosphatase inhibitor, glucagon antagonist), SGLT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists (e.g., compounds described in WO01/25228, WO03/42204, WO98/44921, WO98/45285, WO99/22735), glucokinase activators (e.g., Ro-28-1675) and the like.

Examples of the therapeutic agent for diabetic complications include aldose reductase inhibitors (e.g., Tolrestat, Epalrestat, Zenarestat, Zopolrestat, Fidarestat, Ranirestat (AS-3201), Minalrestat, CT-112), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), protein kinase C (PKC) inhibitors (e.g., ruboxistaurin mesylate; LY-333531), AGE inhibitors (e.g., ALT-945, pimagedine, N-phenacylthiazolium bromide (ALT-766), EXO-226, ALT-711, Pyridorin, Pyridoxamine), active oxygen scavengers (e.g., thioctic acid), cerebral vasodilators (e.g., tiapride), somatostatin receptor agonists (e.g., BIM23190), apoptosis signal regulating kinase-1 (ASK-1) inhibitors and the like.

Examples of the therapeutic agent for hyperlipidemia include HMG-CoA reductase inhibitors (e.g., pravastatin, simvastatin, lovastatin, atorvastatin, fluvastatin, pitavastatin, rosuvastatin and salts thereof (e.g., sodium salt, calcium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), antioxidants (e.g., lipoic acid, probucol), ACAT inhibitors (e.g., Avasimibe, Eflucimibe), anion exchange resin (e.g., colestyramine), probucol, nicotinic acid drugs (e.g., nicomol, niceritrol), ethyl icosapentate, plant sterols (e.g., soysterol, γ-oryzanol) and the like.

Examples of the antihypertensive agent include angiotensin converting enzyme inhibitors (e.g., captopril, enalapril, delapril), angiotensin II receptor antagonists (e.g., losartan, candesartan cilexetil, eprosartan, valsartan, telmisartan, irbesartan, olmesartan medoxomil, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium channel blockers (e.g., manidipine, nifedipine, amlodipine, efonidipine, nicardipine), Clonidine and the like.

Examples of the antiobestic agent include antiobestic agents acting on the central nervous system (e.g., Dexfenfluramine, fenfluramine, phentermine, Sibutramine, amfepramone, dexamphetamine, Mazindol, phenylpropanolamine, clobenzorex; MCH receptor antagonists (e.g., SB-568849; SNAP-7941; compounds encompassed in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, ATL-962), β3 agonists (e.g., CL-316243, SR-58611-A, UL-TG-307, AJ-9677, AZ40140), peptidic anorexiants (e.g., leptin, CNTF (Ciliary Neurotropic Factor)), cholecystokinin agonists (e.g., lintitript, FPL-15849), feeding deterrent (e.g., P-57) and the like.

Examples of the diuretic include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the chemotherapeutic agent include alkylation agents (e.g., cyclophosphamide, ifosfamide), metabolic antagonists (e.g., methotrexate, 5-fluorouracil or its derivative), anti-cancer antibiotics (e.g., mitomycin, adriamycin), plant-derived anti-cancer agents (e.g., vincristin, vindesine, taxol), cisplatin, carboplatin, etoposide and the like.

Examples of the immunotherapeutic agent include microorganism or bacterial components (e.g., muramyl dipeptide derivatives, picibanil), polysaccharides having immunity potentiating activity (e.g., lentinan, sizofiran, krestin), cytokines obtained by genetic engineering techniques (e.g., interferon, interleukin (IL)), colony stimulating factors (e.g., granulocyte colony stimulating factor, erythropoietin) and the like.

As the antiinflammatory drug, for example, non-steroidal antiinflammatory agents such as aspirin, acetoaminofen, indomethacin and the like can be mentioned.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Examples of the therapeutic agent for osteoporosis include alfacalcidol, calcitriol, elcatonin, calcitonin salmon, estriol, ipriflavone, risedronate disodium, pamidronate disodium, alendronate sodium hydrate, incadronate disodium and the like.

As the vitamin, for example, vitamin B₁, vitamin B₁₂ and the like can be mentioned.

Examples of the antidementia agent include tacrine, donepezil, rivastigmine, galanthamine and the like.

Examples of the therapeutic agent for incontinentia or pollakiuria include flavoxate hydrochloride, oxybutynin hydrochloride, propiverine hydrochloride and the like.

Examples of the therapeutic agent for dysurea include acetylcholine esterase inhibitors (e.g., distigmine) and the like can be mentioned.

Furthermore, drugs having a cachexia-improving action established in animal models and clinical situations, such as cyclooxygenase inhibitors (e.g., Indometacin), Progesterone derivatives (e.g., Megesterol acetate), glucosteroid (e.g., dexamethasone), metoclopramide agents, tetrahydrocannabinol agents, fat metabolism improving agents (e.g., eicosapentaenoic acid), growth hormones, IGF-1, or antibodies to a cachexia-inducing factor such as TNF-α, LIF, IL-6, Oncostatin M and the like, can be used in combination with the compound of the present invention.

Further, glycosylation inhibitors (e.g., ALT-711), nerve regeneration promoting drugs (e.g., Y-128, VX853, prosaptide), antidepressants (e.g., desipramine, amitriptyline, imipramine), anticonvulsants (e.g., lamotrigine, Trileptal, Keppra, Zonegran, Pregabalin, Harkoseride, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), narcotic analgesics (e.g., morphine), GABA receptor agonists (e.g., gabapentin, gabapentin MR agent), α₂ receptor agonists (e.g., clonidine), local analgesics (e.g., capsaicin), antianxiety drugs (e.g., benzothiazepines), phosphodiesterase inhibitors (e.g., sildenafil), dopamine receptor agonists (e.g., apomorphine) and the like can be also used in combination with the compound of the present invention.

Two or more kinds of the above-mentioned drug X may be used in an appropriate combination.

By combining the compound of the present invention and a drug X, a superior effect such as the following can be achieved as compared to single administration of the compound of the present invention or a drug X.
(1) The dose of the compound of the present invention and/or the drug X can be reduced.
(2) The side effect of the compound of the present invention and/or the drug X can be reduced.
(3) The effect (action) of the compound of the present invention and/or the drug X can be enhanced.

When the compound of the present invention and a drug X are used in combination, the administration time of the compound of the present invention and the drug X is not restricted, and the compound of the present invention and the drug X can be administered to an administration subject simultaneously, or may be administered at staggered times. The dosage of the drug X may be determined according to the dose clinically used, and can be appropriately selected depending on an administration subject, administration route, disease, combination and the like.

The administration mode of the compound of the present invention and drug X is not particularly restricted, as long as the compound of the present invention and the drug X are combined in administration. Examples of such administration mode include the following methods: (1) The compound of the present invention and the drug X are simultaneously formulated to give a single preparation which is administered. (2) The compound of the present invention and the drug X are separately formulated to give two kinds of preparations which are administered simultaneously by the same administration route. (3) The compound of the present invention and the drug X are separately formulated to give two kinds of preparations which are administered by the same administration route at staggered times. (4) The compound of the present invention and the drug X are separately formulated to give two kinds of preparations which are administered simultaneously by the different administration routes. (5) The compound of the present invention and the drug X are separately formulated to give two kinds of preparations which are administered by the different administration routes at staggered times (for example, the compound of the present invention and the drug X are administered in this order, or in the reverse order), and the like.

The present invention also relates to "a method of antagonizing a GPR40 receptor, which comprises administering an effective amount of a non-peptidic nitrogen-containing heterocyclic compound to a mammal".

As used herein as the mammal, for example, mouse, rat, hamster, rabbit, cat, dog, bovine, sheep, monkey, human can be mentioned.

As the non-peptidic nitrogen-containing heterocyclic compound, any non-peptidic compound can be used as long as it has a nitrogen-containing heterocycle (preferably nitrogen-containing fused heterocycle) as a partial structure and, as specific examples thereof, the aforementioned compound of the present invention and the like can be mentioned.

The effective amount of the non-peptidic nitrogen-containing heterocyclic compound is, for example, the dose of the aforementioned compound of the present invention.

The present method of antagonizing is useful for the regulation of the physiological function involving a GPR40 receptor or the prophylaxis or treatment of a pathology or disease involving a GPR40 receptor. The present method of antagonizing is particularly used for treating diabetes, diabetic neuropathy, diabetic nephropathy, retinopathy (e.g., diabetic retinopathy), obesity, metabolic syndrome, insulin resistance, impaired glucose tolerance, hyperinsulinemia, hypertension, hyperlipidemia, arteriosclerosis, cardiac failure, cardiac infarction, thrombotic disease (e.g., foot ulcer), deficits in memory and learning, depression and mania, visual disorder, appestat disorder, lipotoxicity, pancreatic fatigue, immune disease (e.g., immunodeficiency), inflammatory disease (e.g., enteritis, arthritis, allergy) or cancer (e.g., breast cancer) in a mammal.

### Examples

The present invention is explained in detail in the following by referring to Reference Examples, Examples, Formulation Examples and Experimental Examples. However, the examples are mere exemplifications and do not limit the present invention in any way. The present invention may be modified without departing from the scope of the invention.

In the following Reference Examples and Examples, the "room temperature" means generally about 10°C to about 35°C, % means mol/mol% for the yield, % by volume for the solvent used for chromatography, and wt% for others.

Other abbreviations used in the text mean the following.
s: singlet
d: doublet
t: triplet
q: quartet
m: multiplet
br: broad
J: coupling constant
Hz: Hertz
CDCl₃: chloroform-d
¹H-NMR: protone nuclear magnetic resonance

In the following Reference Examples and Examples, mass spectrum (MS) and nuclear magnetic resonance spectrum (NMR) were measured under the following conditions.
MS measurement device: Waters Corporation ZMD, Waters Corporation ZQ2000 or Micromass Ltd., platform II ionization: Electron Spray Ionization (ESI), or Atmospheric Pressure Chemical Ionization (APCI) Unless otherwise specified, ESI was used.
NMR measurement device: AVANCE DPX300 BRUKER·BIOSPIN.

In addition, purification by preparative HPLC in Reference Examples and Examples were performed under the following conditions.
Preparative HPLC equipment: high-throughput purified system, GILSON Inc.
column: YMC Combiprep ODS-A S-5 µm, 20 X 50 mm solvent: SOLUTION A; 0.1% trifluoroacetic acid-containing water,

SOLUTION B; 0.1% trifluoroacetic acid-containing acetonitrile
gradient cycle A: 0.00 min (SOLUTION A/SOLUTION B = 90/10), 1.20 min (SOLUTION A/SOLUTION B = 90/10), 4.75 min (SOLUTION A/SOLUTION B = 0/100), 7.30 min (SOLUTION A/SOLUTION B = 0/100), 7.40 min (SOLUTION A/SOLUTION B = 90/10), 7.50 min (SOLUTION A/SOLUTION B = 90/10).
gradient cycle B: 0.00 min (SOLUTION A/SOLUTION B = 95/5), 1.00 min (SOLUTION A/SOLUTION B = 95/5), 5.20 min (SOLUTION A/SOLUTION B = 5/95), 6.40 min (SOLUTION A/SOLUTION B = 5/95), 6.50 min (SOLUTION A/SOLUTION B = 95/5), 6.60 min (SOLUTION A/SOLUTION B = 95/5).
flow rate: 25 ml/min, detection: UV 220 nm

### Reference Example 1

Production of 5-chloro-N-(4-methoxyphenyl)-2-nitroaniline

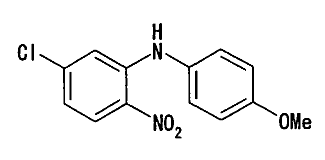

A mixture of 2,4-dichloronitrobenzene (6.2 g, 32.29 mmol), p-anisidine (3.98 g, 32.29 mmol) and potassium fluoride (1.88 g, 32.29 mmol) was stirred for 20 hr at 160°C. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/10) and recrystallized from diethyl ether to give the title compound (5.5 g, yield 61%) as orange needle crystals.
¹H-NMR (300MHz, CDCl₃) δ:3.86 (3H, s), 6.66 (1H, dd, J = 2.2, 9.2 Hz), 6.93-7.01 (3H, m), 7.18 (2H, d, J = 8.8 Hz), 8.14 (1H, d, J = 8.8 Hz), 9.44 (1H, brs).
melting point: 108-109°C

### Reference Example 2

### 4-Chloro-N²-(4-methoxyphenyl)benzene-1,2-diamine

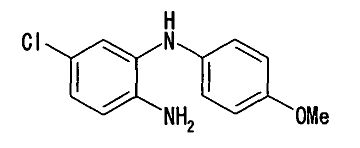

A solution of the compound (2.0 g, 7.18 mmol) of Reference Example 1 in THF (80 mL)-EtOH (40 mL) was added to an aqueous solution (100 mL) of sodium hydrosulfite (20.0 g, 114.88 mmol) under ice-cooling, and the mixture was stirred at room temperature for 1 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure to give the title compound (1.78 g, yield 100%) as a red oil.
¹H-NMR (300MHz, CDCl₃) δ:3.20-3.70 (3H, br), 3.76 (3H, s), 6.65 (1H, d, J = 8.3 Hz), 6.78-6.84 (5H, m), 6.94 (1H, d, J = 2.3 Hz).

### Reference Example 3

### 6-Chloro-1-(4-methoxyphenyl)-1,3-dihydro-2H-benzimidazole-2-thione

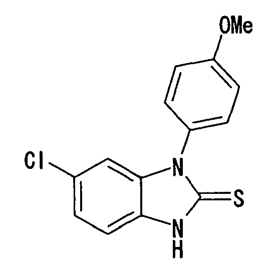

To a solution of the compound (1.78 g, 7.16 mmol) of Reference Example 2 in THF (100 mL) was added 1,1'-thiocarbonyldiimidazole (1.53 g, 8.58 mmol) under ice-cooling, and the mixture was stirred at room temperature for 18 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure to give a pale-brown solid. This solid was washed with a mixed solution of diethyl ether and hexane, and dried under reduced pressure to give the title compound (1.58 g, yield 77%) as a white powder.
¹H-NMR (300MHz, CDCl₃) δ:3.90 (3H, s), 6.93 (1H, d, J = 2.4 Hz), 7.09-7.22 (4H, m), 7.40 (2H, d, J = 8.9 Hz), 10.45-10.55 (1H, br).

### Reference Example 4

### 5-Chloro-N-(4-ethoxyphenyl)-2-nitroaniline

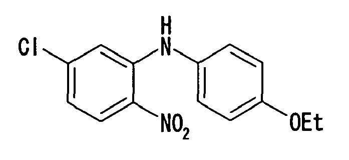

By the reaction in the same manner as in Reference Example 1, the title compound (13.91 g, yield 48%) was obtained as orange needle crystals from 2,4-dichloronitrobenzene (19.2 g, 100 mmol), p-phenetidine (13.7 g, 100 mmol) and potassium fluoride (5.81 g, 100 mmol).
¹H-NMR (300MHz, CDCl₃) δ:1.45 (3H, t, J = 7.1 Hz), 4.07 (2H, q, J = 7.1 Hz), 6.66 (1H, dd, J = 2.1, 9.0 Hz), 6.93-6.99 (3H, m), 7.16 (2H, d, J = 8.7 Hz), 8.14 (1H, d, J = 9.3 Hz), 9.43 (1H, brs).
melting point: 124-125°C

### Reference Example 5

### 4-Chloro-N²-(4-ethoxyphenyl)benzene-1,2-diamine

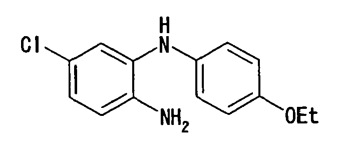

By the reaction in the same manner as in Reference Example 2, the title compound (4.15 g, yield 93%) was obtained as a red oil from the compound (5.0 g, 17.08 mmol) of Reference Example 4.
¹H-NMR (300MHz, CDCl₃) δ:1.26 (3H, t, J = 7.1 Hz), 3.70-3.90 (2H, br), 4.39 (2H, q, J = 7.1 Hz), 5.06 (1H, brs), 6.68 (1H, d, J = 8.4 Hz), 6.80-6.85 (5H, m), 6.95 (1H, d, J = 2.4 Hz).

### Reference Example 6

### 6-Chloro-1-(4-ethoxyphenyl)-1,3-dihydro-2H-benzimidazole-2-thione

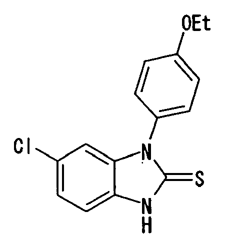

By the reaction in the same manner as in Reference Example 3, the title compound (3.51g, yield 96%) was obtained as a white powder from the compound (3.15 g, 11.99 mmol) of Reference Example 5.
¹H-NMR (300MHz, CDCl₃) δ:1.47 (3H, t, J = 7.0 Hz), 4.11 (2H, q, J = 7.0 Hz), 6.92 (1H, d, J = 1.5 Hz), 7.07 (2H, d, J = 9.0 Hz), 7.14-7.21 (2H, m), 7.38 (2H, d, J = 9.0 Hz), 10.40-10.50 (1H, br).

### Reference Example 7

### 4-[(5-Chloro-2-nitrophenyl)amino]phenol

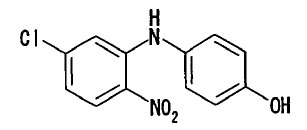

2,4-Dichloronitrobenzene (6.2 g, 32.29 mmol), 4-aminophenol (3.52 g, 32.29 mmol) and potassium fluoride (1.88 g, 32.29 mmol) were subjected to the reaction in the same manner as in Reference Example 1 and purified by silica gel chromatography (eluent; ethyl acetate/hexane=1/2), and the obtained solid was washed with a mixed solvent of diethyl ether and hexane to give the title compound (2.62 g, yield 31%) as a yellow powder.
¹H-NMR (300MHz, CDCl₃) δ:4.89 (1H, s), 6.67 (1H, dd, J = 2.2, 9.1 Hz), 6.91-6.95 (3H, m), 7.12-7.17 (2H, m), 8.15 (1H, d, J = 9.2 Hz), 9.41 (1H, brs).

### Reference Example 8

### 5-Chloro-N-[4-(methoxymethoxy)phenyl]-2-nitroaniline

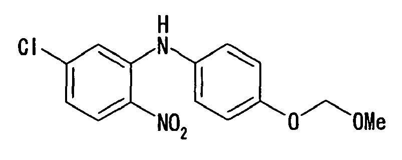

To a solution of the compound (1.0 g, 3.77 mmol) of Reference Example 7 in THF (15 mL) was added sodium hydride (in oil: 60 - 72%) (166 mg) under ice-cooling, and the mixture was stirred for 1 hr. To the reaction mixture was added chloromethyl methyl ether (303 µL, 3.97 mmol) and the mixture was stirred for 16 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/4) to give the title compound (1.01 g, yield 87%) as a red oil.
¹H-NMR (300MHz, CDCI₃) δ:3.52 (3H, s), 5.21 (2H, s), 6.67 (1H, dd, J = 2.1, 9.1 Hz), 6.98 (1H, d, J = 2.1 Hz), 7.10-7.21 (4H, m), 8.15 (1H, d, J = 9.1 Hz), 9.44 (1H, brs).

### Reference Example 9

### 4-Chloro-N²-[4-(methoxymethoxy)phenyl]benzene-1,2-diamine

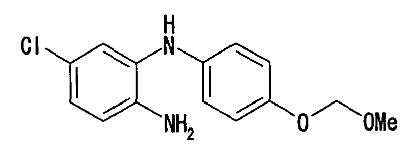

By the reaction in the same manner as in Reference Example 2, the title compound (0.44 g, yield 49%) was obtained as a red oil from the compound (1.0 g, 3.23 mmol) of Reference Example 8.
¹H-NMR (300MHz, CDCl₃) δ:3.40 (3H, s), 3.45-3.65 (3H, br), 5.03 (2H, s), 6.58 (1H, d, J = 8.4 Hz), 6.67-6.71 (2H, m), 6.76 (1H, dd, J = 2.3, 8.4 Hz), 6.86-6.90 (3H, m).

### Reference Example 10

### 6-Chloro-1-[4-(methoxymethoxy)phenyl]-1,3-dihydro-2H-benzimidazole-2-thione

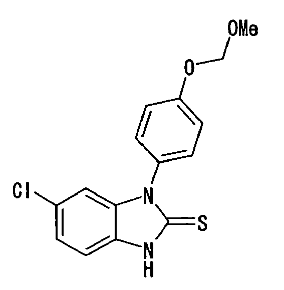

By the reaction in the same manner as in Reference Example 3, the title compound (485 mg, yield 96%) was obtained as a white powder from the compound (440 mg, 1.57 mmol) of Reference Example 9.
¹H-NMR (300MHz, CDCI₃) δ:3.54 (3H, s), 5.26 (2H, s), 6.94 (1H, d, J = 1.6 Hz), 7.14-7.28 (4H, m), 7.41 (2H, d, J = 8.9 Hz), 10.53 (1H, brs).

### Reference Example 11

### 5-Chloro-N-[4-(ethoxymethoxy)phenyl]-2-nitroaniline

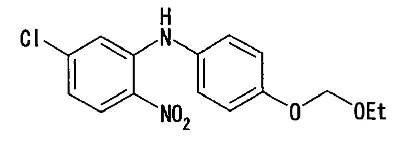

By the reaction in the same manner as in Reference Example 8, the title compound (500 mg, yield 100%) was obtained as a red oil from the compound (400 mg, 1.51 mmol) of Reference Example 7 and chloromethyl ethyl ether (196 µL, 2.27 mmol).
¹H-NMR (300MHz, CDCl₃) δ:1.26 (3H, t, J = 7.1 Hz), 3.77 (2H, q, J = 7.1 Hz), 5.26 (2H, s), 6.67 (1H, dd, J = 2.1, 9.3 Hz), 6.98 (1H, d, J = 2.1 Hz), 7.11-7.20 (4H, m), 8.15 (1H, d, J = 9.3 Hz), 9.44 (1H, brs).

### Reference Example 12

### 4-Chloro-N²-[4-(ethoxymethoxy)phenyl]benzene-1,2-diamine

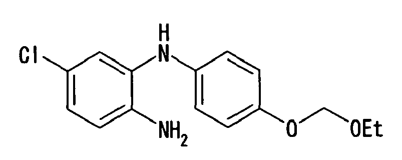

By the reaction in the same manner as in Reference Example 2, the title compound (450 mg, yield 100%) was obtained as a red oil from the compound (500 mg, 1.55 mmol) of Reference Example 11.
¹H-NMR (300MHz, CDCI₃) δ:1.24 (3H, t, J = 6.9 Hz), 2.70-3.70 (3H, br), 3.74 (2H, q, J = 7.1 Hz), 5.17 (2H, s), 6.73-6.83 (4H, m), 6.93-7.00 (3H, m).

### Reference Example 13

### 6-Chloro-1-[4-(ethoxymethoxy)phenyl]-1,3-dihydro-2H-benzimidazole-2-thione

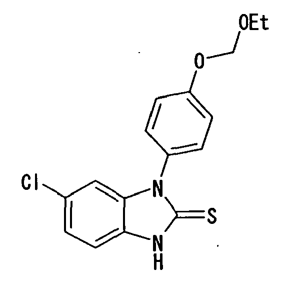

By the reaction in the same manner as in Reference Example 3, the title compound (310 mg, yield 60%) was obtained as a pale-brown powder from the compound (450 mg, 1.54 mmol) of Reference Example 12.
¹H-NMR (300MHz, CDCl₃) δ:1.28 (3H, t, J = 7.1 Hz), 4.07 (2H, q, J = 7.1 Hz), 5.31 (2H, s), 6.94 (1H, s), 7.03-7.27 (4H, m), 7.38-7.42 (2H, m), 10.93 (1H, brs).

### Reference Example 14

### 6-Chloro-1-(3-methoxyphenyl)-1,3-dihydro-2H-benzimidazole-2-thione

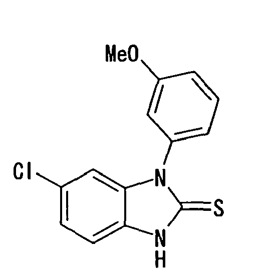

A mixture of 2,4-dichloronitrobenzene (1.92 g, 1.0 mmol), m-anisidine (1.85 g, 1.5 mmol) and potassium fluoride (0.58 g, 1.0 mmol) was stirred for 20 hr at 160°C. After cooling, the reaction mixture was subjected to silica gel chromatography (eluent; chloroform/diethyl ether=9/1) to give a mixture (1.01 g) of 2,4-dichloronitrobenzene and 5-chloro-N-(3-methoxyphenyl)-2-nitroaniline as a red liquid.

A solution of this mixture (1.01 g) in THF (80 mL) and ethanol (40 mL) were added to an aqueous solution (80 mL) of sodium hydrosulfite (8.44 g, 48.48 mmol) under ice-cooling, and the mixture was stirred at room temperature for 30 min. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/3) to give crude 4-chloro-N²-(3-methoxyphenyl)benzene-1,2-diamine (0.58 g) as a pale-brown oil.

By the reaction in the same manner as in Reference Example 3, the title compound (615 mg, yield 21% from 2,4-dichloronitrobenzene) was obtained as a white powder from this oil (0.58 g).
¹H-NMR (300MHz, CDCl₃) δ:3.88 (3H, s), 6.98 (1H, s), 7.04-7.26 (5H, m), 7.52 (1H, t, J = 8.1 Hz), 11.34 (1H, brs).

### Reference Example 15

### 6-Chloro-1-(2-methoxyphenyl)-1,3-dihydro-2H-benzimidazole-2-thione

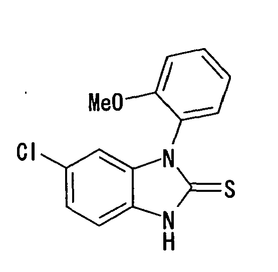

By the reaction in the same manner as in Reference Example 14, the title compound (683 mg, yield 23% from 2,4-dichloronitrobenzene) was obtained as a white powder from 2,4-dichloronitrobenzene (1.92 g, 1.0 mmol) and o-anisidine (1.85 g, 1.5 mmol).
¹H-NMR (300MHz, CDCl₃) δ:3.80 (3H, s), 6.75 (1H, s), 7.14-7.19 (4H, m), 7.40 (1H, dd, J = 1.6, 8.2 Hz), 7.55 (1H, dt, J = 1.7, 7.9 Hz), 10.66 (1H, brs).

### Reference Example 16

### Ethyl 4-[(5-chloro-2-nitrophenyl)amino]benzoate

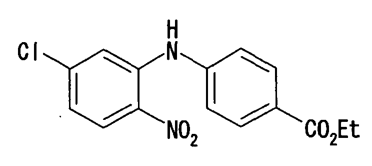

A mixture of 4-chloro-2-fluoronitrobenzene (1.0 g, 5.70 mmol) and ethyl p-aminobenzoate (941 mg, 5.70 mmol) was stirred for 18 hr at 150°C. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) to give the title compound (0.37 g, yield 20%) as an orange solid.
¹H-NMR (300MHz, CDCl₃) δ:1.41 (3H, t, J = 7.1 Hz), 4.40 (2H, q, J = 7.1 Hz), 6.83 (1H, dd, J = 2.1, 9.1 Hz), 7.31-7.35 (3H, m), 8.11 (2H, d, J = 8.6 Hz), 8.18 (1H, d, J = 9.1 Hz), 9.58 (1H, brs).

### Reference Example 17

### Ethyl 4-[(2-amino-5-chlorophenyl)amino]benzoate

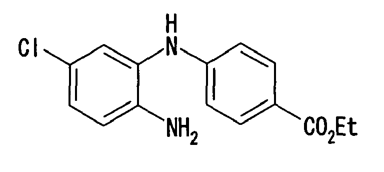

By the reaction in the same manner as in Reference Example 2, the title compound (0.28 g, yield 89%) was obtained as a colorless amorphous substance from the compound (350 mg, 1.09 mmol) of Reference Example 16.
¹H-NMR (300MHz, CDCl₃) δ:1.38 (3H, t, J = 7.1 Hz), 3.20-3.60 (2H, br), 4.34 (2H, q, J = 7.1 Hz), 5.59 (1H, s), 6.68-6.78 (3H, m), 7.04 (1H, dd, J = 2.3, 8.5 Hz), 7.15 (1H, d, J = 2.3 Hz), 7.92 (2H, d, J = 8.7 Hz).

### Reference Example 18

### Ethyl 4-(6-chloro-2-thioxo-2,3-dihydro-1H-benzimidazol-1-yl)benzoate

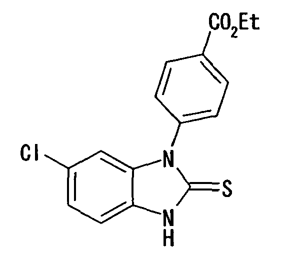

By the reaction in the same manner as in Reference Example 3, the title compound (234 mg, yield 73%) was obtained as a white powder from the compound (280 mg, 0.96 mmol) of Reference Example 17.
¹H-NMR (300MHz, CDCl₃) δ:1.43 (3H, t, J = 7.1 Hz), 4.44 (2H, q, J = 7.1 Hz), 6.95 (1H, d, J = 1.8 Hz), 7.15-7.25 (2H, m), 7.63 (2H, d, J = 8.6 Hz), 8.29 (2H, d, J = 8.7 Hz), 10.05 (1H, brs).

### Reference Example 19

### 5-Methoxy-N-(4-methoxyphenyl)-2-nitroaniline

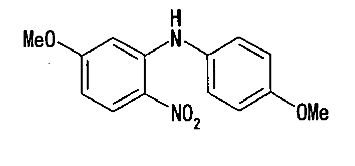

To a solution of the compound (500 mg, 1.79 mmol) of Reference Example 1 in methanol (10 mL) was added a 28% methanol solution (10 mL) of sodium methoxide, and the mixture was heated under reflux for 16 hr. After cooling, to the reaction mixture was added saturated brine, and the mixture was extracted twice with ethyl acetate. The organic layers were combined, washed with saturated brine, dried over magnesium sulfate and concentrated under reduced pressure.

The residue was recrystallized from diethyl ether and hexane to give the title compound (441 mg, yield 90%) as red crystals.
¹H-NMR (300MHz, CDCl₃) δ:3.70 (3H, s), 3.84 (3H, s), 6.26-6.33 (2H, m), 6.95 (2H, d, J = 8.9 Hz), 7.20 (2H, d, J = 8.9 Hz), 8.15 (1H, d, J = 9.4 Hz), 9.65 (1H, brs).
melting point: 99-100°C

### Reference Example 20

### 4-Methoxy-N²-(4-methoxyphenyl)benzene-1,2-diamine

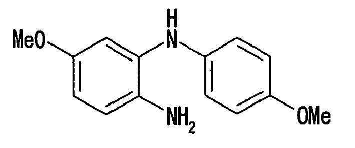

A suspension of the compound (400 mg, 1.46 mmol) of Reference Example 19 and 10% palladium carbon (50% containing water) (100 mg) in ethanol (50 mL) was stirred for 3 hr under hydrogen atmosphere. The catalyst was filtered off, and the filtrate was concentrated to give the title compound (380 mg, yield 100%) as a pale-brown oil.
¹H-NMR (300MHz, CDCl₃) δ:3.66 (3H, s), 3.75 (3H, s), 4.00-4.70 (3H, br), 6.40 (1H, dd, J = 2.7, 8.4 Hz), 6.61 (1H, d, J = 2.7 Hz), 6.72 (1H, d, J = 8.4 Hz), 6.78-6.87 (4H, m).

### Reference Example 21

### 6-Methoxy-1-(4-methoxyphenyl)-1,3-dihydro-2H-benzimidazole-2-thione

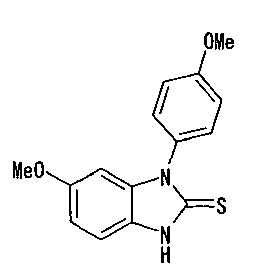

By the reaction in the same manner as in Reference Example 3, the title compound (340 mg, yield 74%) was obtained as a white powder from the compound (380 mg, 0.16 mmol) of Reference Example 20.
¹H-NMR (300MHz, CDCl₃) δ:3.74 (3H, s), 3.90 (3H, s), 6.45 (1H, d, J = 2.3 Hz), 6.80 (1H, dd, J = 2.3, 8.7 Hz), 7.09-7.16 (3H, m),7.40-7.45 (2H, m), 10.86 (1H, brs).

### Reference Example 22

### 4-Chloro-N²-(4-chlorophenyl)benzene-1,2-diamine

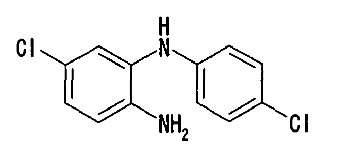

A mixture of 2,4-dichloronitrobenzene (5.0 g, 26.04 mmol), p-chloroaniline (3.32 g, 26.04 mmol) and potassium fluoride (1.51 g, 26.04 mmol) was stirred for 16 hr at 150°C. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/4) to give the mixture (5.02 g) of 5-chloro-N-(4-chlorophenyl)-2-nitroaniline and 2,4-dichloronitrobenzene as an orange solid.

A solution of this mixture (1.0 g) in THF (80 mL)-EtOH (40 mL) was added to an aqueous solution (80 mL) of sodium hydrosulfite (8.0 g, 45.95 mmol) under ice-cooling and the mixture was stirred for 1 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure to give the title compound (0.10 g) as a colorless amorphous substance.
¹H-NMR (300MHz, CDCl₃) δ:3.69 (2H, br), 5.17 (1H, brs), 6.66-6.72 (3H, m), 6.95 (1H, dd, J = 2.3, 8.5 Hz), 7.06 (1H, d, J = 2.3 Hz), 7.15-7.19 (2H, m).

### Reference Example 23

### 6-Chloro-1-(4-chlorophenyl)-1,3-dihydro-2H-benzimidazole-2-thione

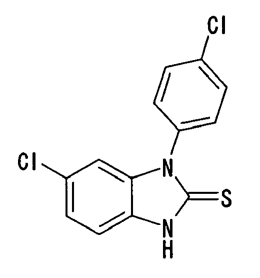

By the reaction in the same manner as in Reference Example 3, the title compound (83 mg, yield 70%) was obtained as a white powder from the compound (100 mg, 0.40 mmol) of Reference Example 22.
¹H-NMR (300MHz, CDCI₃) δ:6.94 (1H, d, J = 1.5 Hz), 7.16-7.26 (2H, m), 7.47 (2H, d, J = 8.7 Hz), 7.58 (2H, d, J = 8.7 Hz), 10.76 (1H, brs).

### Reference Example 24

### 4-Chloro-N¹-(4-methoxyphenyl)benzene-1,2-diamine

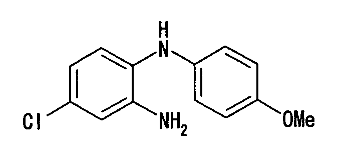

A mixture of 2,5-dichloronitrobenzene (5.0 g, 26.04 mmol), p-anisidine (3.21 g, 26.04 mmol) and potassium fluoride (1.51 g, 26.04 mmol) was stirred for 18 hr at 160°C. After cooling, to the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/4) to give the mixture (5.90 g) of 4-chloro-N-(4-methoxyphenyl)-2-nitroaniline and 2,5-dichloronitrobenzene as a red solid.

A solution of this mixture (1.0 g) in THF (80 mL)-EtOH (40 mL) was added to an aqueous solution (80 mL) of sodium hydrosulfite (8.0 g, 45.95 mmol) under ice-cooling, and the mixture was stirred for 2 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/2) to give the title compound (0.45 g) as a white solid.
¹H-NMR (300MHz, CDCI₃) δ:3.77 (3H, s), 6.70-6.91 (7H, m).

### Reference Example 25

### 5-Chloro-1-(4-methoxyphenyl)-1,3-dihydro-2H-benzimidazole-2-thione

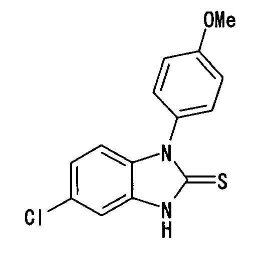

By the reaction in the same manner as in Reference Example 3, the title compound (401 mg, yield 78%) was obtained as a white powder from the compound (440 mg, 1.77 mmol) of Reference Example 24.
¹H-NMR (300MHz, CDCl₃) δ:3.89 (3H, s), 6.83 (1H, d, J = 8.7 Hz), 7.08-7.15 (4H, m), 7.40 (2H, d, J = 9.0 Hz), 10.27 (1H, brs).

### Reference Example 26

### 6-Chloro-1-(4-ethoxyphenyl)-1,3-dihydro-2H-benzimidazol-2-one

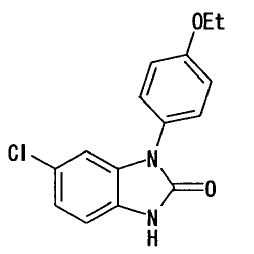

1,1'-Carbonyldiimidazole (1.63 g, 10.05 mmol) was added to a solution of the compound (2.20 g, 8.37 mmol) of Reference Example 5 in THF (150 mL) under ice-cooling, and the mixture was stirred for 16 hr at room temperature. To the reaction mixture was added aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/1 to 2/1), and the obtained solid was washed with a mixed solution of diethyl ether and hexane, and dried under reduced pressure to give the title compound (1.35 g, yield 56%) as a white powder.
¹H-NMR (300MHz, CDCl₃) δ:1.47 (3H, t, J = 7.1 Hz), 4.10 (2H, q, J = 7.1 Hz), 6.95 (1H, s), 7.01-7.07 (4H, m), 7.40 (2H, d, J = 8.7 Hz), 10.09 (1H, s).

### Reference Example 27

### 2,6-Dichloro-1-(4-ethoxyphenyl)-1H-benzimidazole

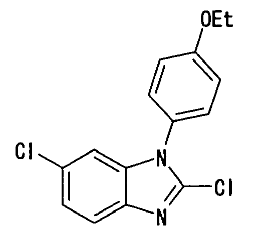

A mixture of the compound (300 mg, 1.04 mmol) of Reference Example 26 and phosphorus oxychloride (3 mL) was heated under reflux for 14 hr. After cooling, the residue was concentrated under reduced pressure. Saturated aqueous sodium hydrogencarbonate was added to the residue, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/4) to give the title compound (220 mg, yield 69%) as a colorless oil.
¹H-NMR (300MHz, CDCI₃) δ:1.49 (3H, t, J = 7.0 Hz), 4.13 (2H, q, J = 7.0 Hz), 7.01-7.12 (3H, m), 7.25-7.31 (3H, m), 7.65 (1H, d, J = 8.7 Hz).

### Reference Example 28

### 1-Bromo-4-(1-bromoethyl)benzene

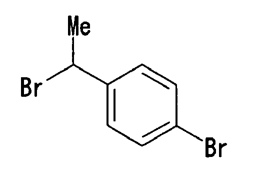

To a suspension of 4-bromoethylbenzene (10.0 g, 54.04 mmol) and N-bromosuccinimide (10.1 g, 56.74 mmol) in carbon tetrachloride (300 mL) was added azobisisobutyronitrile (887 mg, 5.40 mmol) and the mixture was stirred for 3 hr at 90°C. After cooling, the insoluble material was filtered off, and the filtrate was washed successively with water and saturated brine, dried over magnesium sulfate, and concentrated under reduced pressure to give the title compound (14.25 g, yield 100%) as a pale-yellow liquid.
¹H-NMR (300MHz, CDCl₃) δ:2.02 (3H, d, J = 7.2 Hz), 5.15 (1H, q, J = 6.9 Hz), 7.30 (2H, d, J = 9.0 Hz), 7.46 (2H, d, J = 9.0 Hz).

### Reference Example 29

### 6-Chloro-1-phenyl-1,3-dihydro-2H-benzimidazole-2-thione

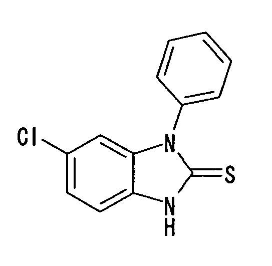

By the reaction in the same manner as in Reference Example 1 and Reference Example 2, 4-chloro-N²-phenylbenzene-1,2-diamine was obtained from 2,4-dichloronitrobenzene (1.92 g, 10.00 mmol) and aniline (931 mg, 10.00 mmol), and further, by the reaction in the same manner as in Reference Example 3, the title compound (551 mg, yield 34%) was obtained as a white powder.
¹H-NMR (300MHz, CDCl₃) δ:6.85-6.91 (1H, m), 7.10-7.29 (3H, m), 7.51-7.67 (4H, m), 10.40 (1H, br).

### Reference Example 30

### 1-(4-Methoxyphenyl)-1,3-dihydro-2H-imidazo[4,5-b]pyridine-2-thione

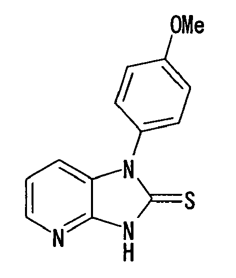

By the reaction in the same manner as in Reference Example 14, the title compound (142 mg, yield 9%) was obtained as a white powder from 3-chloro-2-nitropyridine (1.00 g, 6.31 mmol) and p-anisidine (777 mg, 6.31 mmol).
¹H-NMR (300MHz, CDCl₃) δ:3.90 (3H, s), 7.08-7.13 (3H, m), 7.20 (1H, d, J = 7.8 Hz), 7.41 (2H, d, J = 7.5 Hz), 8.22 (1H, s).

### Reference Example 31

### 6-Chloro-1-(4-methoxyphenyl)-2-(2-phenylethyl)-1H-benzimidazole

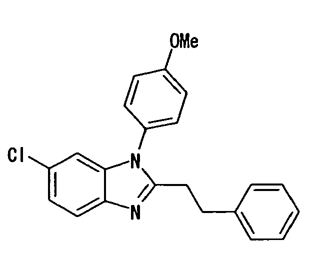

To a solution of the compound (100 mg, 0.40 mmol) of Reference Example 2 in dichloromethane (3 mL) was added 3-phenylpropionyl chloride (81 mg, 0.48 mmol) under ice-cooling, and the mixture was stirred for 16 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/4) to give the title compound (60 mg, yield 41%) as a pale-blue oil.
¹H-NMR (300MHz, CDCl₃) δ:2.98-3.04 (2H, m), 3.10-3.16 (2H, m), 3.88 (3H, s), 6.98-7.08 (7H, m), 7.17-7.26 (4H, m), 7.69 (1H, d, J = 8.6 Hz).

### Reference Example 32

### N-(4-Chlorobenzyl)-N'-{4-chloro-2-[(4-ethoxyphenyl)amino]phenyl}thiourea

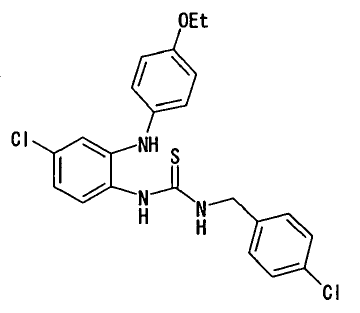

To a solution of the compound (1.0 g, 3.81 mmol) of Reference Example 5 in THF (70 mL) was added 4-chlorobenzyl thiocyanate (839 mg, 4.57 mmol) and the mixture was stirred at room temperature for 16 hr and at 70°C for 4 hr. After cooling, to the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/2) to give the title compound (1.70 g, yield 100%) as a yellow oil.
¹H-NMR (300MHz, CDCl₃) δ:1.44 (3H, t, J = 7.1 Hz), 4.04 (2H, q, J = 7.1 Hz), 4.83 (2H, d, J = 5.7 Hz), 5.93 (1H, s), 6.71 (1H, brs), 6.72 (1H, dd, J = 1.8, 8.1 Hz), 6.86-6.95 (5H, m), 7.05 (1H, d, J = 8.1 Hz), 7.27 (4H, s), 7.55 (1H,s).

### Reference Example 33

### Ethyl 4-(6-chloro-2-thioxo-2,3-dihydro-1H-benzimidazol-1-yl)piperidine-1-carboxylate

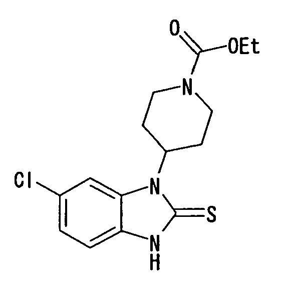

By the reaction in the same manner as in Reference Example 14, the title compound (3.3 g, yield 61% from 2,4-dichloronitrobenzene) was obtained as a white powder from 2,4-dichloronitrobenzene (3.84 g, 20 mmol) and ethyl 4-aminopiperidine-1-carboxylate (3.45 g, 20 mmol).
¹H-NMR (300MHz, CDCl₃) δ:1.33 (3H, t, J = 7.2 Hz), 1.90-2.00 (2H, m), 2.20-2.40 (2H, m), 2.90-3.10 (2H, m), 4.22 (2H, q, J = 7.2 Hz), 4.30-4.55 (2H, m), 5.38 (1H, m), 7.10-7.24 (2H, m), 7.36 (1H, s), 10.75 (1H, brs).

### Reference Example 34

### 1-(1-Benzylpiperidin-4-yl)-6-chloro-1,3-dihydro-2H-benzimidazole-2-thione

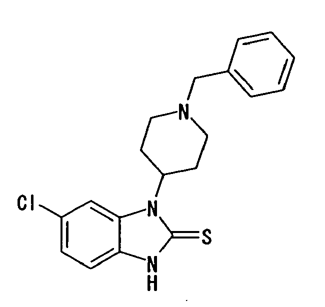

By the reaction in the same manner as in Reference Example 14, the title compound (4.89 g, yield 46% from 2,4-dichloronitrobenzene) was obtained as a white powder from ethyl 2,4-dichloronitrobenzene (5.76 g, 30 mmol) and 4-amino-1-benzylpiperidine (5.80 g, 30 mmol).
¹H-NMR (300MHz, CDCl₃) δ:1.80-1.90 (2H, m), 2.20-2.50 (4H, m), 3.05-3.13 (2H, m), 3.62 (2H, s), 5.21 (1H, m), 7.10-7.20 (2H, m), 7.25-7.40 (5H, m), 7.56 (1H, s), 10.77 (1H, brs).

### Reference Example 35

### 5-Bromo-1-(4-methoxyphenyl)-1,3-dihydro-2H-benzimidazole-2-thione

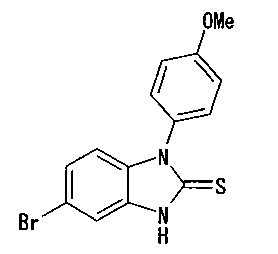

By the reaction in the same manner as in Reference Example 14, the title compound (1.68 g, yield 29%) was obtained as a white powder from 5-bromo-2-fluoronitrobenzene (3.83 g, 17.4 mmol) and p-anisidine (2.18 g, 17.4 mmol).
¹H-NMR (300MHz, CDCl₃) δ:3.90 (3H, s), 6.80 (1H, d, J = 8.4 Hz), 7.20 (2H, d, J = 9.0 Hz), 7.26 (1H, dd, J = 1.8, 8.4 Hz), 7.37-7.45 (3H, m), 11.11 (1H, brs).

### Reference Example 36

### 2-(Benzylthio)-1-phenyl-1H-benzimidazole

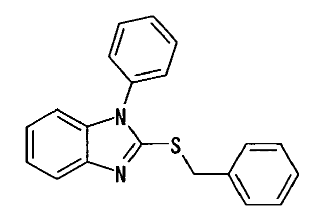

The title compound was synthesized by the method described in Yakugaku Zasshi (Journal of the Pharmaceutical Society of Japan), vol. 78, page 1378 (1958).

### Reference Example 37

### 2-(Benzylthio)-N,N-diethyl-1-(2-methoxyphenyl)-1H-benzimidazole-5-sulfonamide

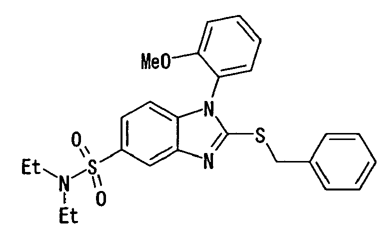

The title compound was purchased from Enamine.

### Reference Example 38

### 1-(2-Methoxyphenyl)-5-nitro-2-[(3-phenoxybenzyl)thio]-1H-benzimidazole

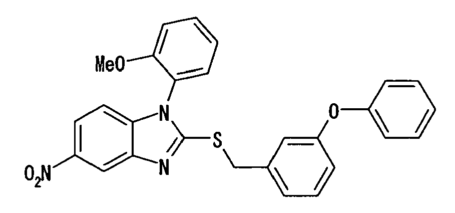

The title compound was purchased from Enamine.

### Reference Example 39

### 1-[4-Methoxy-3-({[1-(4-methoxyphenyl)-1H-benzimidazol-2-yl]thio}methyl)phenyl]ethanone

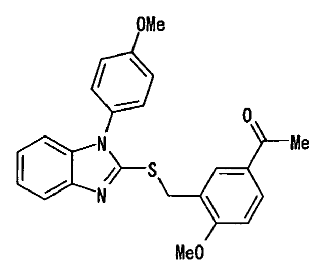

The title compound was purchased from Ivonin.

### Reference Example 40

### 2-[(4-Fluorobenzyl)thio]-1-(4-methoxyphenyl)-1H-benzimidazole

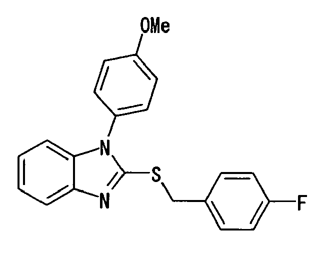

The title compound was purchased from Ivonin.

### Reference Example 41

### 2-[(4-Chlorobenzyl)thio]-1-(4-methoxyphenyl)-1H-benzimidazole

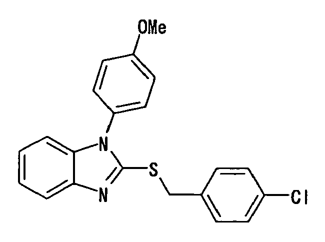

The title compound was purchased Enamine.

### Reference Example 42

### 2-{[5-Chloro-2-(difluoromethoxy)benzyl]thio}-N,N-diethyl-l-(2-methoxyphenyl)-1H-benzimidazole-5-sulfonamide

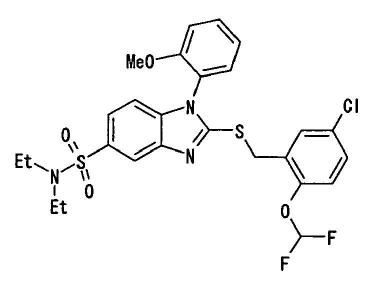

The title compound was purchased from Ivonin.

### Reference Example 43

### N-(2,5-Dimethylphenyl)-2-{[1-(4-methoxyphenyl)-1H-benzimidazol-2-yl]thio}-2-phenylacetamide

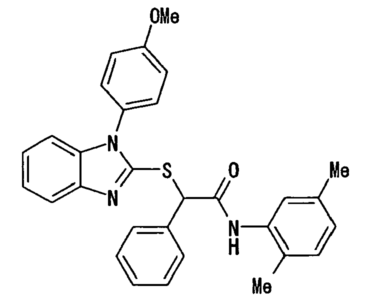

The title compound was purchased Enamine.

### Reference Example 44

### 1-(4-Ethoxyphenyl)-2-[(4-methylbenzyl)thio]-1H-benzimidazole

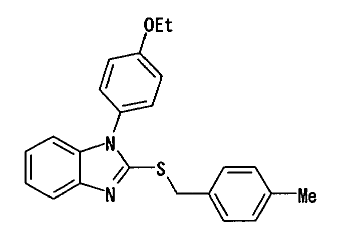

The title compound was purchased from Ivonin.

### Reference Example 45

### 2-[(4-tert-Butylbenzyl)thio]-1-(3-methylphenyl)-1H-benzimidazole

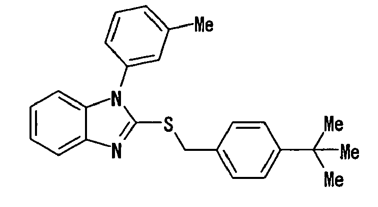

The title compound was purchased from Enamine.

### Reference Example 46

### 2-[(4-Methylbenzyl)thio]-1-(3-methylphenyl)-1H-benzimidazole

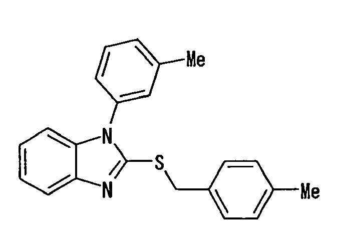

The title compound was purchased from Ivonin.

### Reference Example 47

### 1-(1H-Indol-3-yl)-2-{[1-(4-methylphenyl)-1H-benzimidazol-2-yl]thio}-2-phenylethanone

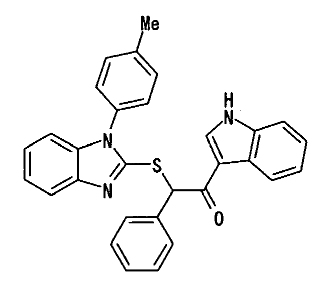

The title compound was purchased from Ivonin.

### Reference Example 48

### 2-(Benzylthio)-1-cyclohexyl-1H-benzimidazole

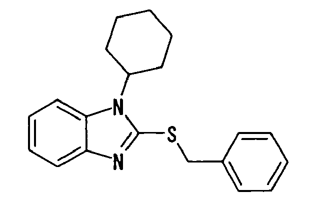

The title compound was purchased from SPECS&BIOSPECS.

### Reference Example 49

### 1,1-Diphenyl-2-(1-phenyl-1H-benzimidazol-2-yl)ethanol

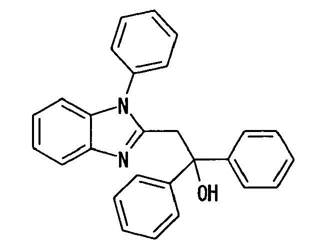

The title compound was purchased from IF LTD.

### Reference Example 50

### N,N-Diethyl-1-(2-methoxyphenyl)-2-(phenoxymethyl)-1H-benzimidazole-5-sulfonamide

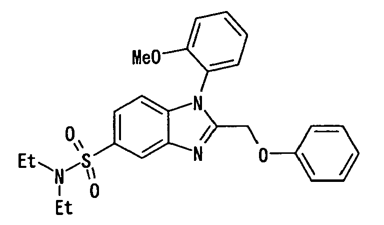

The title compound was purchased from Enamine.

### Reference Example 51

### 2-[(4-Chlorophenoxy)methyl]-N,N-diethyl-1-(2-methoxyphenyl)-1H-benzimidazole-5-sulfonamide

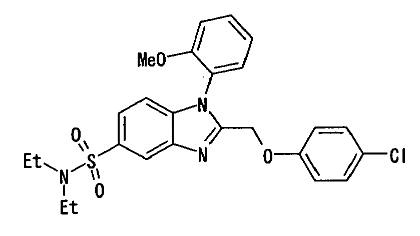

The title compound was purchased from Enamine.

### Reference Example 52

### 2-[(2-Fluorophenoxy)methyl]-1-(4-methoxyphenyl)-1H-benzimidazole

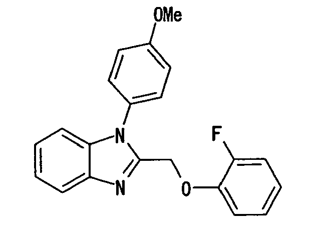

The title compound was purchased from Enamine.

### Reference Example 53

### 2-[(Biphenyl-2-yloxy)methyl]-1-(4-methylphenyl)-1H-benzimidazole

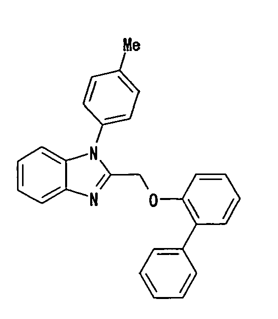

The title compound was purchased from Ivonin.

### Reference Example 54

### 2-[(Biphenyl-2-yloxy)methyl]-1-(4-methoxyphenyl)-1H-benzimidazole

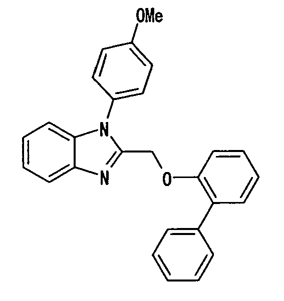

The title compound was purchased from Ivonin.

### Reference Example 55

### 2-{[(2,2-Dimethyl-2,3-dihydro-1-benzofuran-7-yl)oxy]methyl}-1-(4-methoxyphenyl)-1H-benzimidazole

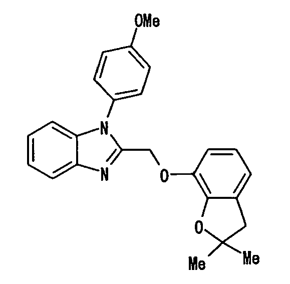

The title compound was purchased from Volovenko.

### Reference Example 56

### 2-[(3-Bromophenoxy)methyl]-1-(4-methoxyphenyl)-1H-benzimidazole

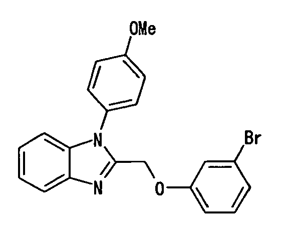

The title compound was purchased from Volovenko.

### Reference Example 57

### 1-(2,4-Difluorophenyl)-2-[(1-phenyl-1H-benzimidazol-2-yl)thio]ethanone

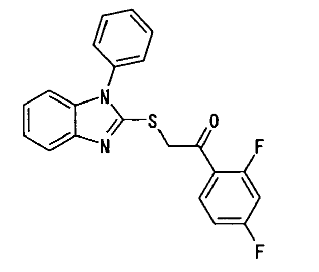

The title compound was purchased from INTELBIOSCAN.

### Reference Example 58

### 6-Chloro-1-(4-methoxyphenyl)-1,3-dihydro-2H-benzimidazol-2-one

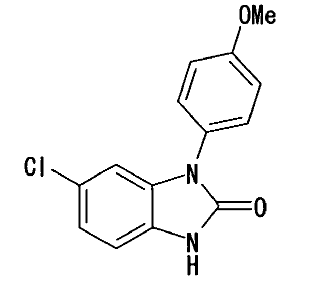

By the reaction in the same manner as in Reference Example 26, the title compound (1.07 g, yield 88%) was obtained as a pale-pink powder from the compound (1.1 g, 4.42 mmol) of Reference Example 2.
¹H-NMR (300MHz, CDCl₃) δ:3.88 (3H, s), 6.95 (1H, d, J = 1.5 Hz), 7.00-7.12 (4H, m), 7.40 (2H, d, J = 8.7 Hz), 9.21 (1H, brs).

### Reference Example 59

### 2,6-Dichloro-1-(4-methoxyphenyl)-1H-benzimidazole

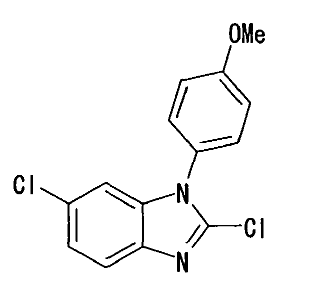

By the reaction in the same manner as in Reference Example 27, the title compound (0.85 g, yield 74%) was obtained as a pale-yellow oil from the compound (1.07 g, 3.90 mmol) of Reference Example 58.
¹H-NMR (300MHz, CDCl₃) δ:3.91 (3H, s), 7.07-7.12 (3H, m), 7.25-7.34 (3H, m), 7.65 (1H, d, J = 8.4 Hz).

### Reference Example 60

### 5-Chloro-2-nitro-N-(4-propoxyphenyl)aniline

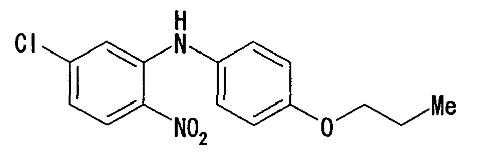

By the reaction in the same manner as in Reference Example 1, the title compound (1.39 g, yield 45%) was obtained as orange needle crystals from 2,4-dichloronitrobenzene (1.92 g, 10.0 mmol), 4-propoxyaniline (1.51 g, 10.0 mmol) and potassium fluoride (581 mg, 10.0 mmol).
¹H-NMR (300MHz, CDCl₃) δ:1.07 (3H, t, J = 7.5 Hz), 1.78-1.90 (2H, m), 3.96 (2H, t, J = 6.6 Hz), 6.66 (1H, dd, J = 2.1, 9.0 Hz), 6.93-6.99 (3H, m), 7.17 (2H, d, J = 9.0 Hz), 7.88 (1H, d, J = 8.7 Hz), 9.43 (1H, s).
melting point: 94-95°C

### Reference Example 61

### 4-Chloro-N²-(4-propoxyphenyl)benzene-1,2-diamine

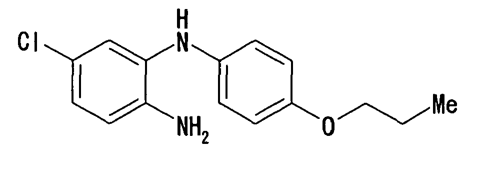

By the reaction in the same manner as in Reference Example 2, the title compound (1.15 g, yield 98%) was obtained as a red oil from the compound (1.30 g, 4.24 mmol) of Reference Example 60.
¹H-NMR (300MHz, CDCI₃) δ:1.03 (3H, t, J = 7.4 Hz), 1.73-1.85 (2H, m), 3.50-3.70 (2H, br), 3.88 (2H, t, J = 6.6 Hz), 5.08 (1H, brs), 6.70 (1H, d, J = 8.4 Hz), 6.78-6.87 (3H, m), 7.03 (1H, s), 7.16-7.28 (2H, m).

### Reference Example 62

### 6-Chloro-1-(4-propoxyphenyl)-1,3-dihydro-2H-benzimidazol-2-one

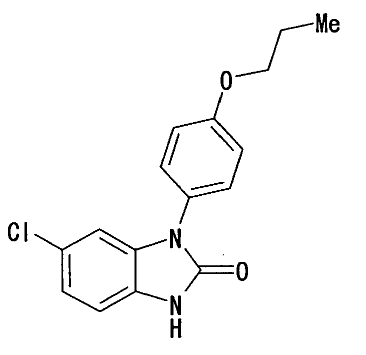

By the reaction in the same manner as in Reference Example 26, the title compound (0.88 g, yield 70%) was obtained as a pale-pink powder from the compound (1.30 g, 4.24 mmol) of Reference Example 61.
¹H-NMR (300MHz, CDCl₃) δ:1.07 (3H, t, J = 7.5 Hz), 1.78-1.88 (2H, m), 3.97 (2H, t, J = 6.6 Hz), 6.91 (1H, d, J = 1.5 Hz), 6.95-7.08 (4H, m), 7.32-7.40 (2H, m), 10.36 (1H, brs).

### Reference Example 63

### 2,6-Dichloro-1-(4-propoxyphenyl)-1H-benzimidazole

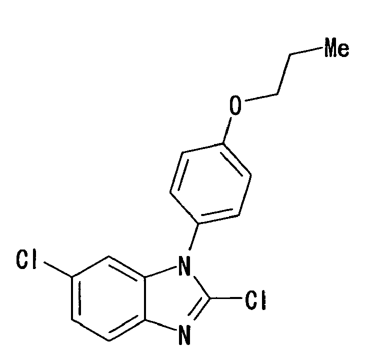

By the reaction in the same manner as in Reference Example 27, the title compound (0.30 g, yield 35%) was obtained as a pale-green solid from the compound (800 mg, 2.64 mmol) of Reference Example 62.
¹H-NMR (300MHz, CDCl₃) δ:1.09 (3H, t, J = 7.5 Hz), 1.82-1.94 (2H, m), 4.02 (2H, t, J = 6.5 Hz), 7.05-7.12 (3H, m), 7.25-7.32 (3H, m), 7.64 (1H, d, J = 8.7 Hz).

### Reference Example 64

### N-(4-Butoxyphenyl)-5-chloro-2-nitroaniline

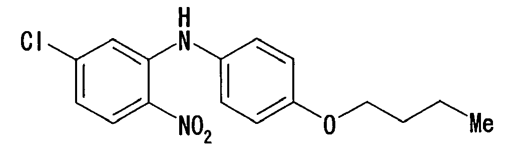

By the reaction in the same manner as in Reference Example 1, the title compound (3.51 g, yield 42%) was obtained as orange needle crystals from 2,4-dichloronitrobenzene (5.0 g, 26.04 mmol), 4-butoxyaniline (4.30 g, 26.04 mmol) and potassium fluoride (1.51 g, 26.04 mmol).
¹H-NMR (300MHz, CDCI₃) δ:1.00 (3H, t, J = 7.4 Hz), 1.45-1.59 (2H, m), 1.76-1.85 (2H, m), 4.00 (2H, t, J = 6.5 Hz), 6.66 (1H, dd, J = 2.1, 9.0 Hz), 6.91-6.99 (3H, m), 7.15-7.20 (2H, m), 8.14 (1H, d, J = 9.0 Hz), 9.43 (1H, s).
melting point: 83-84°C

### Reference Example 65

### N²-(4-Butoxyphenyl)-4-chlorobenzene-1,2-diamine

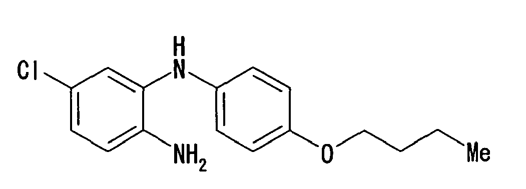

By the reaction in the same manner as in Reference Example 2, the title compound (3.01 g, yield 100%) was obtained as a red oil from the compound (3.0 g, 9.35 mmol) of Reference Example 64.
¹H-NMR (300MHz, CDCl₃) δ:0.98 (3H, t, J = 7.4 Hz), 1.48-1.55 (2H, m), 1.67-1.80 (2H, m), 3.40-3.80 (2H, br), 3.93 (2H, t, J = 6.5 Hz), 5.04 (1H, br s), 6.68 (1H, d, J = 8.1 Hz), 6.81-6.91 (3H, m), 6.95 (1H, d, J = 1.8 Hz), 7.13-7.19 (1H, m), 7.23-7.28 (1H, m).

### Reference Example 66

### 1-(4-Butoxyphenyl)-6-chloro-1,3-dihydro-2H-benzimidazol-2-one

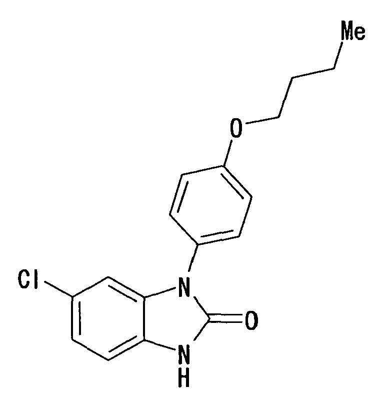

By the reaction in the same manner as in Reference Example 26, the title compound (1.25 g, yield 38%) was obtained as a pale-brown powder from the compound (3.01 g, 10.35 mmol) of Reference Example 65.
¹H-NMR (300MHz, CDCl₃) δ:1.00 (3H, t, J = 7.4 Hz), 1.46-1.59 (2H, m), 1.77-1.86 (2H, m), 4.03 (2H, t, J = 6.5 Hz), 6.94 (1H, d, J = 1.2 Hz), 7.00-7.08 (4H, m), 7.36-7.41 (2H, m), 9.41 (1H, brs).

### Reference Example 67

### 1-(4-Butoxyphenyl)-2,6-dichloro-1H-benzimidazole

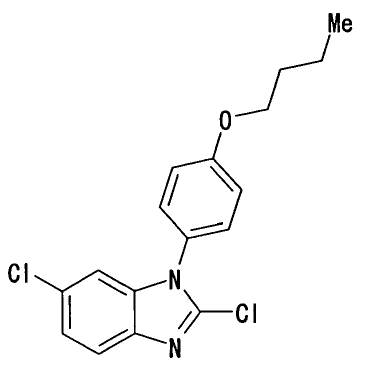

By the reaction in the same manner as in Reference Example 27, the title compound (0.93 g, yield 73%) was obtained as a pale-brown solid from the compound (1.20 g, 3.79 mmol) of Reference Example 66.
¹H-NMR (300MHz, CDCl₃) δ:1.02 (3H, t, J = 7.4 Hz), 1.46-1.61 (2H, m), 1.79-1.88 (2H, m), 4.06 (2H, t, J = 6.5 Hz), 7.05-7.12 (3H, m), 7.25-7.31 (3H, m), 7.64 (1H, d, J = 8.4 Hz).

### Reference Example 68

### 4-(2,2,2-Trifluoroethoxy)nitrobenzene

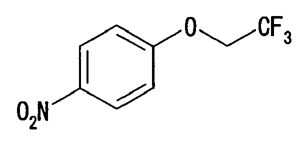

To a solution of 2,2,2-trifluoroethanol (26.49 g, 0.26 mol) in THF (300 mL) was added sodium hydride (in oil, 65%, 10.27 g, 0.28 mol) under ice-cooling, and the mixture was stirred for 1 hr at room temperature. To this mixture was added p-fluoronitrobenzene (37.36 g, 0.26 mol), and the mixture was stirred for 3 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure to give the title compound (56.13 g, yield 98%) as a yellow amorphous solid.
¹H-NMR (300MHz, CDCl₃) δ:4.46 (2H, q, J = 7.8 Hz), 7.04 (2H, d, J = 9.2 Hz), 8.25(2H, d, J = 9.2 Hz).

### Reference Example 69

### 4-(2,2,2-Trifluoroethoxy)aniline

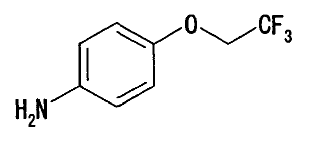

A mixture of the compound (56.13 g, 0.25 mol) of Reference Example 68 and palladium carbon (6.0 g) in ethanol (200 mL)-ethyl acetate (100 mL) was stirred for 24 hr at room temperature under hydrogen atmosphere under ice-cooling. The catalyst was filtered off, and the filtrate was concentrated under reduced pressure to give the title compound (47.32 g, yield 98%) as a red liquid.
¹H-NMR (300MHz, CDCl₃) δ:3.20-3.60 (2H, br), 4.25 (2H, q, J = 8.3 Hz), 6.58-6.66 (2H, m), 6.70-6.80(2H, m).

### Reference Example 70

### 5-Chloro-2-nitro-N-[4-(2,2,2-trifluoroethoxy)phenyl]aniline

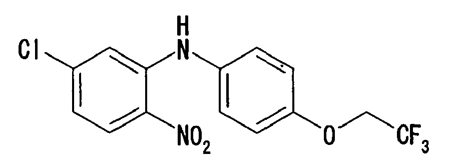

By the reaction in the same manner as in Reference Example 1, the title compound (8.38 g, yield 46%) was obtained as orange needle crystals from the compound (10.0 g, 52.31 mmol) of Reference Example 69, 2,4-dichloronitrobenzene (10.04 g, 52.31 mmol) and potassium fluoride (3.03 g, 52.31 mmol).
¹H-NMR (300MHz, CDCl₃) δ:4.40(2H, q, J = 8.0 Hz), 6.70 (1H, dd, J = 2.1, 9.3 Hz), 6.91 (1H, s), 7.02-7.06 (2H, m), 7.24 (2H, d, J = 9.3 Hz), 8.15 (1H, d, J = 9.0 Hz), 9.43 (1H, s).
melting point: 106-107°C

### Reference Example 71

### 4-Chloro-N²-[4-(2,2,2-trifluoroethoxy)phenyl]benzene-1,2-diamine

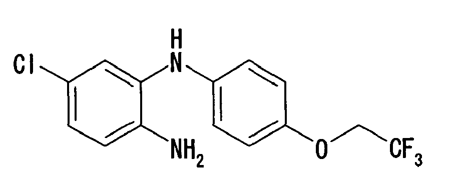

By the reaction in the same manner as in Reference Example 2, the title compound (4.80 g, yield 100%) was obtained as a red oil from the compound (5.0 g, 14.42 mmol) of Reference Example 70.
¹H-NMR (300MHz, CDCl₃) δ:4.29 (2H, q, J = 8.0 Hz), 4.60-.10 (2H, br), 5.30-5.70 (1H, br), 6.72-6.86 (5H, m), 6.99 (1H, s), 7.18-7.24 (1H, m).

### Reference Example 72

### 6-Chloro-1-[4-(2,2,2-trifluoroethoxy)phenyl]-1,3-dihydro-2H-benzimidazol-2-one

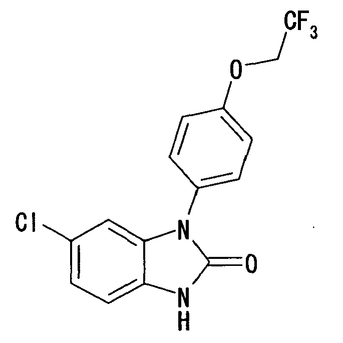

By the reaction in the same manner as in Reference Example 26, the title compound (3.43 g, yield 64%) was obtained as a pale-brown powder from the compound (4.80 g, 15.52 mmol) of Reference Example 71.
¹H-NMR (300MHz, CDCl₃) δ:4.42 (2H, q, J = 8.0 Hz), 6.96-7.15 (5H, m), 7.47 (2H, d, J = 9.0 Hz), 8.85 (1H, s).

### Reference Example 73

### 2,6-Dichloro-1-[4-(2,2,2-trifluoroethoxy)phenyl]-1H-benzimidazole

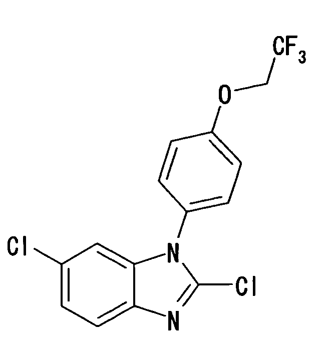

By the reaction in the same manner as in Reference Example 27, the title compound (1.95 g, yield 62%) was obtained as a white amorphous solid from the compound (3.0 g, 8.75 mmol) of Reference Example 72.
¹H-NMR (300MHz, CDCl₃) δ:4.45 (2H, q, J = 8.0 Hz), 7.03 (1H, d, J = 8.7 Hz), 7.15 (2H, d, J = 8.7 Hz), 7.22 (1H, dd, J = 2.1, 9.0 Hz), 7.36-7.41 (2H, m), 7.73 (1H, d, J = 2.1 Hz).

### Reference Example 74

### 6-Chloro-1-[4-(trifluoromethoxy)phenyl]-1,3-dihydro-2H-benzimidazole-2-thione

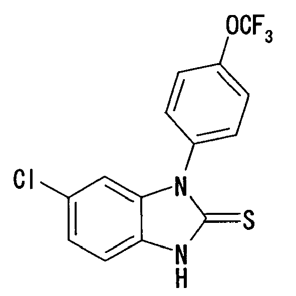

By the reaction in the same manner as in Reference Example 1, crude 5-chloro-2-nitro-N-[4-(trifluoromethoxy)phenyl]aniline (3.20 g) was obtained as a red oil from 2,4-dichloronitrobenzene (5.42 g, 28.23 mmol), 4-(trifluoromethyl)aniline (5.0 g, 28.23 mmol) and potassium fluoride (1.64 g, 28.23 mmol).

By the reaction in the same manner as in Reference Example 2, crude 4-chloro-N²-[4-(trifluoromethoxy)phenyl]benzene-1,2-diamine mixture (2.28 g) was obtained as a brown oil from 5-chloro-2-nitro-N-[4-(trifluoromethoxy)phenyl]aniline (3.20 g).

By the reaction in the same manner as in Reference Example 3, the title compound (460 mg) was obtained as a white amorphous solid from 4-chloro-N²-[4-(trifluoromethoxy)phenyl]benzene-1,2-diamine (500 mg).
¹H-NMR (300MHz, CDCl₃) δ:7.18 (3H, s), 7.33 (1H, d, J = 8.4 Hz), 7.38 (1H, s), 7.77 (2H, s), 7.95 (1H, d, J = 8.4 Hz).

### Reference Example 75

### 6-Chloro-1-[4-(trifluoromethoxy)phenyl]-1,3-dihydro-2H-benzimidazol-2-one

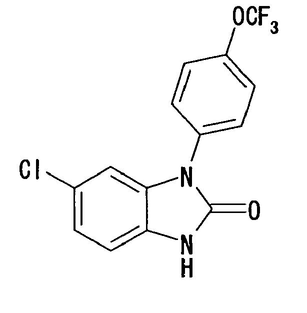

By the reaction in the same manner as in Reference Example 26, the title compound (70 mg) was obtained as a white amorphous solid from 4-chloro-N²-[4-(trifluoromethoxy)phenyl]benzene-1,2-diamine (1.78 g) obtained in the production process of Reference Example 74.
¹H-NMR (300MHz, CDCl₃) δ:7.00-7.16 (3H, m), 7.46-7.49 (2H, m), 7.51-7.64 (2H, m), 9.30-9.40 (1H, br).

### Reference Example 76

### 2,6-Dichloro-1-[4-(trifluoromethoxy)phenyl]-1H-benzimidazole

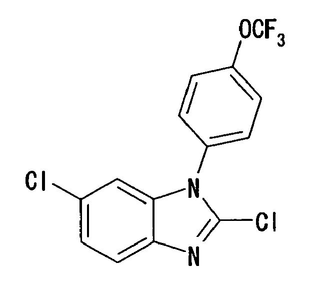

By the reaction in the same manner as in Reference Example 27, the title compound (60 mg, yield 82%) was obtained as a colorless oil from the compound (70 mg, 0.21 mmol) of Reference Example 75.
¹H-NMR (300MHz, CDCl₃) δ:7.14 (1H, d, J = 1.8 Hz), 7.27-7.32 (1H, m), 7.44-7.51 (4H, m), 7.66 (1H, d, J = 8.7 Hz).

### Reference Example 77

### 4-Chloro-N¹-(4-ethoxyphenyl)benzene-1,2-diamine

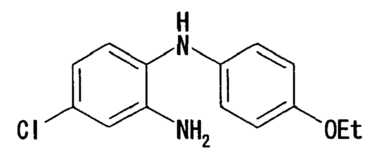

By the reaction in the same manner as in Reference Example 24, the title compound (2.32 g, yield 34%) was obtained as a red oil from 2,5-dichloronitrobenzene (5.0 g, 26.04 mmol) and p-phenetidine (3.57 g, 26.04 mmol).
¹H-NMR (300MHz, CDCl₃) δ:1.39 (3H, t, J = 6.9 Hz), 3.78 (2H, br s), 3.98 (2H, q, J = 6.9 Hz), 4.89 (1H, br s), 6.63-6.82 (6H, m), 6.91 (1H, d, J = 8.4 Hz).

### Reference Example 78

### 5-Chloro-1-(4-ethoxyphenyl)-1,3-dihydro-2H-benzimidazol-2-one

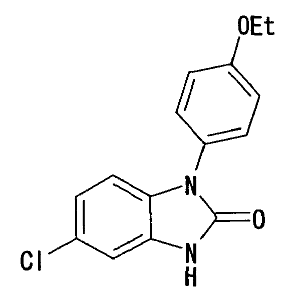

By the reaction in the same manner as in Reference Example 26, the title compound (1.28 g, yield 50%) was obtained as a white solid from the compound (2.32 g, 8.83 mmol) of Reference Example 77.
¹H-NMR (300MHz, CDCl₃) δ:1.46 (3H, t, J = 7.0 Hz), 4.00 (2H, q, J = 7.0 Hz), 6.87 (1H, d, J = 8.4 Hz), 7.01-7.06 (3H, m), 7.12 (1H, d, J = 1.5 Hz), 7.39 (2H, d, J = 8.4 Hz), 9.21 (1H, brs).

### Reference Example 79

### 2,5-Dichloro-1-(4-ethoxyphenyl)-1H-benzimidazole

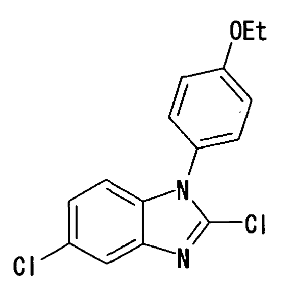

By the reaction in the same manner as in Reference Example 27, the title compound (0.82 g, yield 70%) was obtained as a white amorphous solid from the compound (1.10 g, 3.81 mmol) of Reference Example 78.
¹H-NMR (300MHz, CDCl₃) δ:1.48 (3H, t, J = 7.0 Hz), 4.12 (2H, q, J = 7.0 Hz), 7.02-7.09 (3H, m), 7.21-7.37 (3H, m), 7.72 (1H, d, J = 1.8 Hz).

### Reference Example 80

### 4-Chloro-N¹-(4-propoxyphenyl)benzene-1,2-diamine

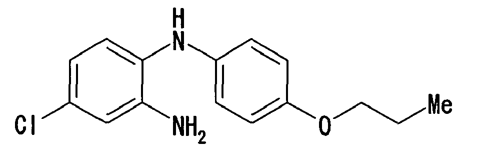

By the reaction in the same manner as in Reference Example 24, the title compound (1.92 g, yield 52%) was obtained as a pale-brown amorphous solid from 2,5-dichloronitrobenzene (2.54 g, 13.23 mmol) and 4-propoxyaniline (2.0 g, 13.23 mmol).
¹H-NMR (300MHz, CDCl₃) δ:1.02 (3H, t, J = 7.5 Hz), 1.74-1.81 (2H, m), 3.78 (2H, brs), 3.87 (2H, t, J = 6.6 Hz), 4.89 (1H, br s), 6.63-6.82 (6H, m), 6.92 (1H, d, J = 8.4 Hz).

### Reference Example 81

### 5-Chloro-1-(4-propoxyphenyl)-1,3-dihydro-2H-benzimidazol-2-one

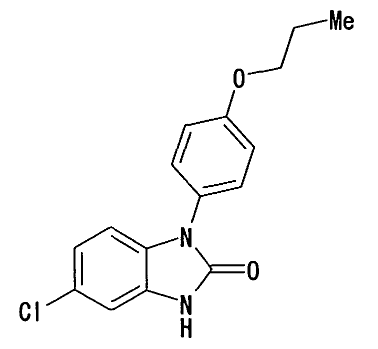

By the reaction in the same manner as in Reference Example 26, the title compound (1.30 g, yield 62%) was obtained as a pink powder from the compound (1.92 g, 6.94 mmol) of Reference Example 80.
¹H-NMR (300MHz, CDCI₃) δ:1.07 (3H, t, J = 7.4 Hz), 1.80-1.91 (2H, m), 3.98 (2H, t, J = 6.6 Hz), 6.87 (1H, d, J = 8.4 Hz), 7.00-7.1 3 (4H, m), 7.36 (2H, d, J = 8.7 Hz), 9.49 (1H, s).

### Reference Example 82

### 2,5-Dichloro-1-(4-propoxyphenyl)-1H-benzimidazole

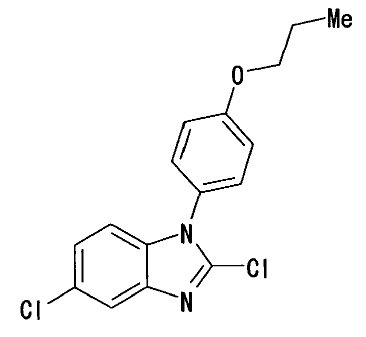

By the reaction in the same manner as in Reference Example 27, the title compound (0.89 g, yield 70%) was obtained as a pale-yellow oil from the compound (1.20 g, 3.96 mmol) of Reference Example 81.
¹H-NMR (300MHz, CDCI₃) δ:1.09 (3H, t, J = 7.4 Hz), 1.82-1.93 (2H, m), 4.01 (2H, t, J = 6.6 Hz), 7.02-7.09 (3H, m), 7.21-7.32 (3H, m), 7.71 (1H, d, J = 1.8 Hz).

### Reference Example 83

### 1-(4-Butoxyphenyl)-5-chloro-1,3-dihydro-2H-benzimidazol-2-one

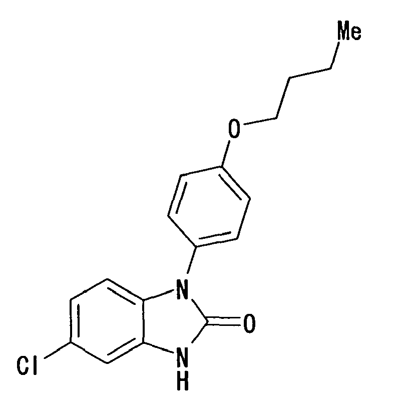

By the reaction in the same manner as in Reference Example 24, crude 4-chloro-N¹-(4-propoxyphenyl)benzene-1,2-diamine (2.86 g) was obtained as a red amorphous solid from 2,5-dichloronitrobenzene (5.0 g, 26.04 mmol) and 4-butoxy aniline (4.30 g, 26.04 mmol).

By the reaction in the same manner as in Reference Example 26, the title compound (1.65 g, yield 20%) was obtained as a white amorphous solid from 4-chloro-N¹-(4-propoxyphenyl)benzene-1,2-diamine (2.86 g).
¹H-NMR (300MHz, CDCl₃) δ:1.00 (3H, t, J = 7.4 Hz), 1.46-1.59 (2H, m), 1.76-1.86 (2H, m), 4.02 (2H, t, J = 6.6 Hz), 6.87 (1H, d, J = 8.4 Hz), 7.00-7.07 (3H, m), 7.12 (1H, d, J = 2.1 Hz), 7.36-7.41 (2H, m), 9.41 (1H, brs).

### Reference Example 84

### 1-(4-Butoxyphenyl)-2,5-dichloro-1H-benzimidazole

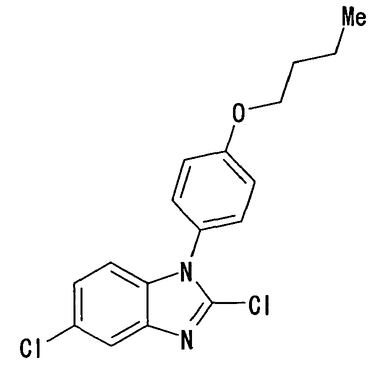

By the reaction in the same manner as in Reference Example 27, the title compound (0.82 g, yield 77%) was obtained as a white amorphous solid from the compound (1.0 g, 3.16 mmol) of Reference Example 83.
¹H-NMR (300MHz, CDCl₃) δ:1.02 (3H, t, J = 7.5 Hz), 1.48-1.60 (2H, m), 1.79-1.88 (2H, m), 4.05 (2H, t, J = 6. 5 Hz), 7.03-7.08 (3H, m), 7.21-7.31 (3H, m), 7.72 (1H, d, J = 1.8 Hz).

### Reference Example 85

### 5-Chloro-1-[4-(2,2,2-trifluoroethoxy)phenyl]-1,3-dihydro-2H-benzimidazole-2-thione

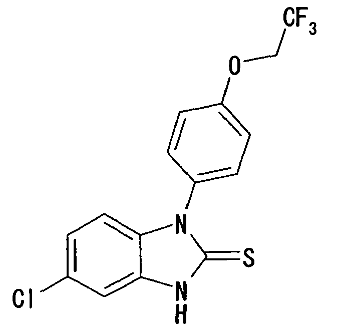

By the reaction in the same manner as in Reference Example 24, crude 4-chloro-N¹-[4-(2,2,2-trifluoroethoxy)phenyl]benzene-1,2-diamine (6.48 g) was obtained as a red liquid from 2,5-dichloronitrobenzene (14.9 g, 77.6 mmol) and compound (14.6 g, 77.6 mmol) of Reference Example 69.

By the reaction in the same manner as in Reference Example 3, the title compound (1.21 g, yield 4%) was obtained as a white amorphous solid from 4-chloro-N¹-[4-(2,2,2-trifluoroethoxy)phenyl]benzene-1,2-diamine (6.48 g).
¹H-NMR (300MHz, CDCl₃) δ:4.44 (2H, q, J = 8.0 Hz), 6.84 (1H, d, J = 8.4 Hz), 7.12-7.20 (3H, m), 7.26-7.27 (1H, m), 7.49 (2H, d, J = 8.4 Hz), 10.74 (1H, brs).

### Reference Example 86

### 5-Chloro-1-[4-(2,2,2-trifluoroethoxy)phenyl]-1,3-dihydro-2H-benzimidazol-2-one

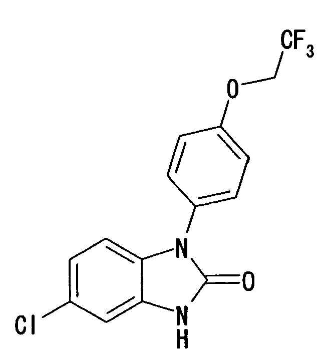

By the reaction in the same manner as in Reference Example 26, the title compound (3.58 g, yield 67%) was obtained as a white amorphous solid from 4-chloro-N¹-[4-(2,2,2-trifluoroethoxy)phenyl]benzene-1,2-diamine (4.95 g, 15.63 mmol) obtained in the production process of Reference Example 85.
¹H-NMR (300MHz, CDCl₃) δ:4.42 (2H, q, J = 8.0 Hz), 6.83-6.90 (1H, m), 7.02-7.14 (4H, m), 7.35-7.48 (2H, m), 9.57 (1H, s).

### Reference Example 87

### 2,5-Dichloro-1-[4-(2,2,2-trifluoroethoxy)phenyl]-1H-benzimidazole

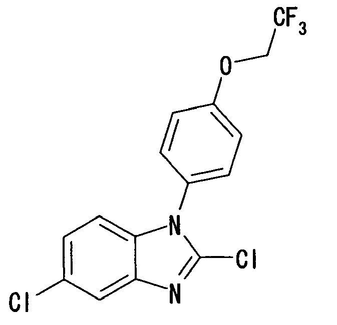

By the reaction in the same manner as in Reference Example 27, the title compound (1.95 g, yield 62%) was obtained as a white amorphous solid from the compound (3.0 g, 8.75 mmol) of Reference Example 86.
¹H-NMR (300MHz, CDCI₃) δ:4.45 (2H, q, J = 8.0 Hz), 7.03 (1H, d, J = 8.7 Hz), 7.15 (2H, d, J = 8.7 Hz), 7.22 (1H, dd, J = 2.1, 9.0 Hz), 7.36-7.41 (2H, m), 7.73 (1H, d, J = 2.1 Hz).

### Reference Example 88

### 4,5-Dichloro-N-(4-methoxyphenyl)-2-nitroaniline

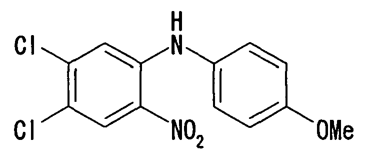

By the reaction in the same manner as in Reference Example 1, the title compound (5.22 g, yield 38%) was obtained as an orange solid from 1,2,4-trichloro-5-nitrobenzene (10.0 g, 44.16 mmol), p-anisidine (5.43 g, 44.16 mmol) and potassium fluoride (2.56 g, 44.16 mmol).
¹H-NMR (300MHz, CDCl₃) δ:3.86 (3H, s), 6.97 (2H, d, J = 9.0 Hz), 7.06 (1H, s), 7.18 (2H, d, J = 8.7 Hz), 8.31 (1H, s), 9.32 (1H, brs).

### Reference Example 89

### 4,5-Dichloro-N-(4-methoxyphenyl)benzene-1,2-diamine

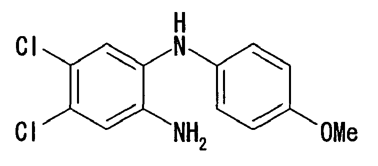

By the reaction in the same manner as in Reference Example 2, the title compound (2.72 g, yield 100%) was obtained as a black amorphous solid from the compound (3.0 g, 9.58 mmol) of Reference Example 88.
¹H-NMR (300MHz, CDCl₃) δ:3.55 (2H, brs), 3.75 (3H, s), 5.04 (1H, brs), 6.84 (4H, s), 6.94 (1H, s), 7.04 (1H, s).

### Reference Example 90

### 5,6-Dichloro-1-(4-methoxyphenyl)-1,3-dihydro-2H-benzimidazol-2-one

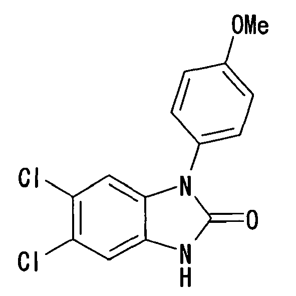

By the reaction in the same manner as in Reference Example 26, the title compound (1.64 g, yield 85%) was obtained as a pale-pink powder from the compound (1.76 g, 6.22 mmol) of Reference Example 89.
¹H-NMR (300MHz, CDCl₃) δ:3.88 (3H, s), 7.03 (1H, s), 7.07 (2H, d, J = 9.0 Hz), 7.20 (1H, s), 7.38 (2H, d, J = 8.7 Hz), 8.74 (1H, brs).

### Reference Example 91

### 2,5,6-Trichloro-1-(4-methoxyphenyl)-1H-benzimidazole

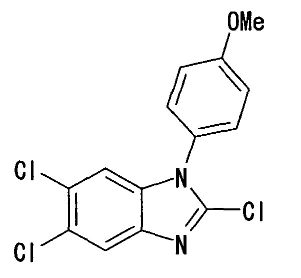

By the reaction in the same manner as in Reference Example 27, the title compound (1.55 g, yield 68%) was obtained as a white solid from the compound (2.15 g, 6.95 mmol) of Reference Example 90.
¹H-NMR (300MHz, CDCl₃) δ:3.91 (3H, s), 7.09 (2H, d, J = 11.4 Hz), 7.22-7.35 (3H, m), 7.83 (1H, s).

### Reference Example 92

### 5,6-Dichloro-1-(4-methoxyphenyl)-1,3-dihydro-2H-benzimidazole-2-thione

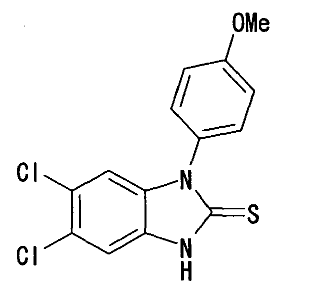

By the reaction in the same manner as in Reference Example 3, the title compound (1.95 g, yield 85%) was obtained as a pale-blue amorphous solid from the compound (2.0 g, 7.06 mmol) of Reference Example 89.
¹H-NMR (300MHz, CDCl₃) δ:3.90 (3H, s), 7.00 (1H, s), 7.08-7.13 (3H, m), 7.33-7.41 (3H, m).

### Reference Example 93

### 4,5-Dichloro-N-(4-ethoxyphenyl)-2-nitroaniline

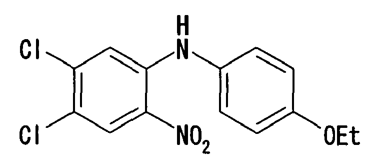

By the reaction in the same manner as in Reference Example 1, the title compound (3.47 g, yield 41%) was obtained as red crystals from 1,2,4-trichloro-5-nitrobenzene (5.89 g, 26.04 mmol), p-phenetidine (3.57 g, 26.04 mmol) and potassium fluoride (1.51 g, 26.04 mmol).
¹H-NMR (300MHz, CDCl₃) δ:1.45 (3H, t, J = 7.0 Hz), 4.07 (2H, q, J = 7.0 Hz), 6.95 (2H, d, J = 8.4 Hz), 7.06 (1H, s), 7.14-7.19 (2H, m), 8.31 (1H, s), 9.32 (1H, brs).
melting point: 148-149°C

### Reference Example 94

### 4,5-Dichloro-N-(4-ethoxyphenyl)benzene-1,2-diamine

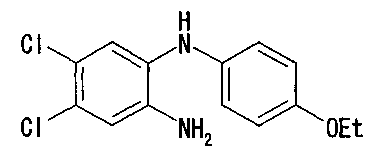

By the reaction in the same manner as in Reference Example 2, the title compound (2.70 g, yield 100%) was obtained as a pale-brown amorphous solid from the compound (3.0 g, 9.17 mmol) of Reference Example 93.
¹H-NMR (300MHz, CDCl₃) δ:1.40 (3H, t, J = 7.0 Hz), 3.60-3.80 (2H, br), 4.00 (2H, q, J = 7.0 Hz), 4.90-5.10 (1H, br), 6.78-6.86 (5H, m), 7.03 (1H, s).

### Reference Example 95

### 5,6-Dichloro-1-(4-ethoxyphenyl)-1,3-dihydro-2H-benzimidazol-2-one

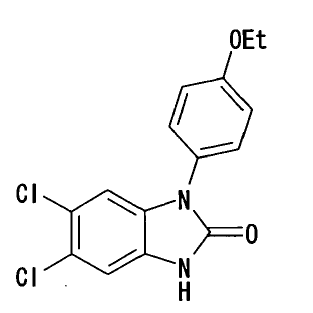

By the reaction in the same manner as in Reference Example 26, the title compound (1.22 g, yield 42%) was obtained as a white powder from the compound (2.70 g, 9.09 mmol) of Reference Example 94.
¹H-NMR (300MHz, DMSO-d₆) δ:1.37 (3H, t, J = 6.5 Hz), 4.07-4.12 (2H, m), 7.00 (1H, s), 7.08-7.10 (2H, m), 7.25 (1H, s), 7.40-7.43 (2H, m), 11.41 (1H, brs).

### Reference Example 96

### 2,5,6-Trichloro-1-(4-ethoxyphenyl)-1H-benzimidazole

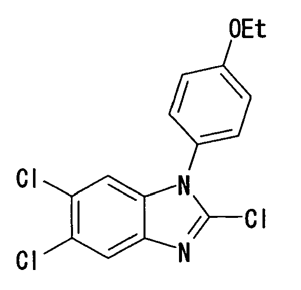

By the reaction in the same manner as in Reference Example 27, the title compound (0.80 g, yield 76%) was obtained as a pale-brown solid from the compound (1.0 g, 3.09 mmol) Reference Example 95.
¹H-NMR (300MHz, CDCl₃) δ:1.49 (3H, t, J = 7.1 Hz), 4.13 (2H, q, J = 7.1 Hz), 7.07 (2H, d, J = 8.7 Hz), 7.22-7.32 (3H, m), 7.83 (1H, s).

### Reference Example 97

### 4,5-Dichloro-2-nitro-N-(4-propoxyphenyl)aniline

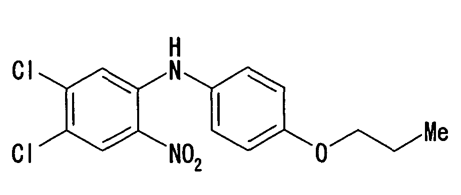

By the reaction in the same manner as in Reference Example 1, the title compound (1.38 g, yield 40%) was obtained as a red amorphous solid from 1,2,4-trichloro-5-nitrobenzene (2.26 g, 10.0 mmol), 4-propoxyaniline (1.51 g, 10.0 mmol) and potassium fluoride (581 mg, 10.0 mmol).
¹H-NMR (300MHz, CDCl₃) δ:1.07 (3H, t, J = 7.4 Hz), 1.79-1.90 (2H, m), 3.96 (2H, t, J = 6.5 Hz), 6.97 (2H, d, J = 9.0 Hz), 7.06 (1H, s), 7.16 (2H, d, J = 9.0 Hz), 8.31 (1H, s), 9.32 (1H, brs).

### Reference Example 98

### 4,5-Dichloro-N-(4-propoxyphenyl)benzene-1,2-diamine

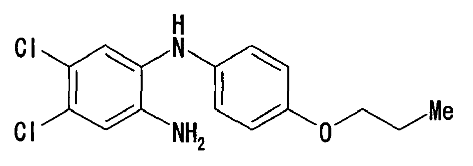

By the reaction in the same manner as in Reference Example 2, the title compound (1.16 g, yield 98%) was obtained as a brown amorphous solid from the compound (1.30 g, 3.81 mmol) of Reference Example 97.
¹H-NMR (300MHz, CDCl₃) δ:1.03 (3H, t, J = 7.4 Hz), 1.74-1.85 (2H, m), 3.88 (2H, t, J = 6.6 Hz), 4.40-4.70 (3H, br), 7.03 (1H, s), 7.13 (1H, s), 7.23-7.32 (4H, m).

### Reference Example 99

### 5,6-Dichloro-1-(4-propoxyphenyl)-1,3-dihydro-2H-benzimidazol-2-one

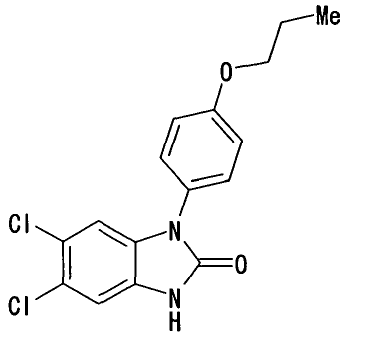

By the reaction in the same manner as in Reference Example 26, the title compound (0.65 g, yield 52%) was obtained as a brown powder from the compound (1.16 g, 3.73 mmol) of Reference Example 98.
¹H-NMR (300MHz, DMSO-d₆) δ:1.06 (3H, t, J = 7.5 Hz), 1.75-1.90 (2H, m), 3.98 (2H, t, J = 6.6 Hz), 6.91-7.05 (3H, m), 7.17 (1H, s), 7.32-7.38 (2H, m), 10.90 (1H, s).

### Reference Example 100

### 2,5,6-Trichloro-1-(4-propoxyphenyl)-1H-benzimidazole

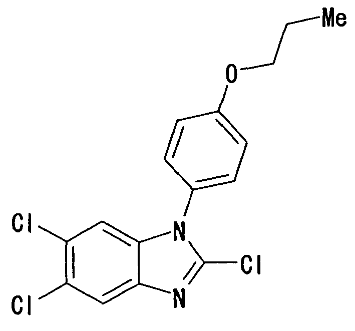

By the reaction in the same manner as in Reference Example 27, the title compound (390 mg, yield 62%) was obtained as a pale-green solid from the compound (600 mg, 1.78 mmol) of Reference Example 99.
¹H-NMR (300MHz, CDCl₃) δ:1.09 (3H, t, J = 7.4 Hz), 1.82-1.94 (2H, m), 4.01 (2H, t, J = 6.5 Hz), 7.05-7.11 (2H, m), 7.22-7.34 (3H, m), 7.82 (1H, s).

### Reference Example 101

### N-(4-Butoxyphenyl)-4,5-dichlorobenzene-1,2-diamine

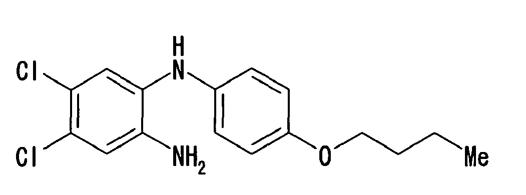

By the reaction in the same manner as in Reference Example 1, crude N-(4-butoxyphenyl)-4,5-dichloro-2-nitroaniline (4.77 g) was obtained as a red amorphous solid from 1,2,4-trichloro-5-nitrobenzene (5.90 g, 26.04 mmol), 4-butoxy aniline (4.30 g, 26.04 mmol) and potassium fluoride (1.51 g, 26.04 mmol).

By the reaction in the same manner as in Reference Example 2, the title compound (4.28 g, yield 51%) was obtained as a red oil from N-(4-butoxyphenyl)-4,5-dichloro-2-nitroaniline (4.77 g).
¹H-NMR (300MHz, CDCl₃) δ:0.97 (3H, t, J = 7.4 Hz), 1.43-1.55 (2H, m), 1.71-1.83 (2H, m), 3.69 (2H, br s), 3.93 (2H, t, J = 6.5 Hz), 4.96 (1H, br s), 6.77-6.87 (5H, m), 7.02 (1H, s).

### Reference Example 102

### 1-(4-Butoxyphenyl)-5,6-dichloro-1,3-dihydro-2H-benzimidazol-2-one

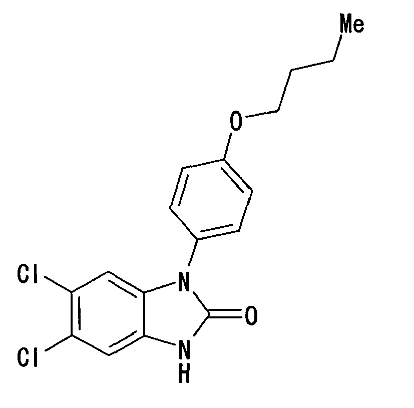

By the reaction in the same manner as in Reference Example 26, the title compound (1.50 g, yield 32%) was obtained as a pale-brown powder from the compound (4.28 g, 13.16 mmol) of Reference Example 101.
¹H-NMR (300MHz, DMSO-d₆) δ:1.01 (3H, t, J = 7.4 Hz), 1.47-1.59 (2H, m), 1.77-1.86 (2H, m), 4.03 (2H, t, J = 6.5 Hz), 7.03-7.08 (3H, m), 7.21 (1H, s), 7.36 (2H, d, J = 9.0 Hz), 9.76 (1H, brs).

### Reference Example 103

### 1-(4-Butoxyphenyl)-2,5,6-trichloro-1H-benzimidazole

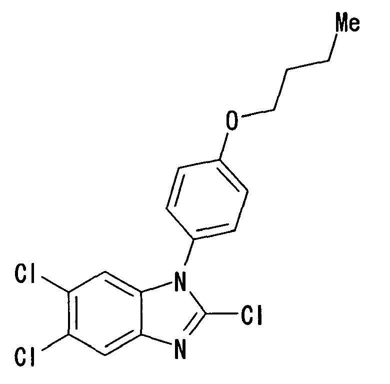

By the reaction in the same manner as in Reference Example 27, the title compound (1.03 g, yield 70%) was obtained as a pale-pink solid from the compound (1.40 g, 3.99 mmol) of Reference Example 102.
¹H-NMR (300MHz, CDCl₃) δ:1.02 (3H, t, J = 7.5 Hz), 1.48-1.58 (2H, m), 1.79-1.88 (2H, m), 4.05 (2H, t, J = 6.5 Hz), 7.05-7.10 (2H, m), 7.22-7.31 (3H, m), 7.83 (1H, s).

### Reference Example 104

### 4,5-Dichloro-2-nitro-N-[4-(2,2,2-trifluoroethoxy)phenyl]aniline

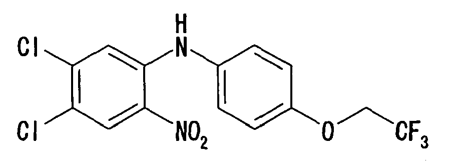

By the reaction in the same manner as in Reference Example 1, the title compound (8.46 g, yield 42%) was obtained as orange crystals from the compound (10.0 g, 52.31 mmol) of Reference Example 69, 1,2,4-trichloro-5-nitrobenzene (11.85 g, 52.31 mmol) and potassium fluoride (3.03 g, 52.31 mmol).
¹H-NMR (300MHz, CDCl₃) δ:4.40 (2H, q, J = 8.1 Hz), 7.03-7.09 (3H, m), 7.21-7.26 (2H, m), 8.32 (1H, s), 9.32 (1H, s).
melting point: 144-145°C

### Reference Example 105

### 4,5-Dichloro-N-[4-(2,2,2-trifluoroethoxy)phenyl]benzene-1,2-diamine

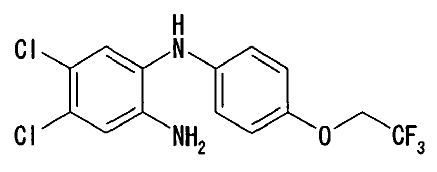

By the reaction in the same manner as in Reference Example 2, the title compound (4.60 g, yield 100%) was obtained as a blue amorphous solid from the compound (5.0 g, 13.11 mmol) of Reference Example 104.
¹H-NMR (300MHz, CDCl₃) δ: 3.40-3.90 (2H, br), 4.30 (2H, q, J = 8.0 Hz), 5.01 (1H, brs), 6.76-6.80 (2H, m), 6.86-6.91 (2H, m), 7.18-7.28 (2H, m).

### Reference Example 106

### 5,6-Dichloro-1-[4-(2,2,2-trifluoroethoxy)phenyl]-1,3-dihydro-2H-benzimidazol-2-one

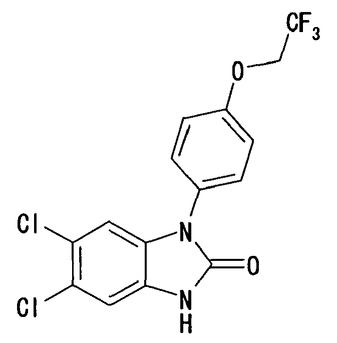

By the reaction in the same manner as in Reference Example 26, the title compound (3.57 g, yield 72%) was obtained as a pale-purple powder from the compound (4.60 g, 13.10 mmol) of Reference Example 105.
¹H-NMR (300MHz, DMSO-d₆) δ:4.40 (2H, q, J = 8.0 Hz), 7.02-7.15 (3H, m), 7.41 (2H, d, J = 8.7 Hz), 7.61 (1H, s), 8.60-8.80 (1H, br).

### Reference Example 107

### 2,5,6-Trichloro-1-[4-(2,2,2-trifluoroethoxy)phenyl]-1H-benzimidazole

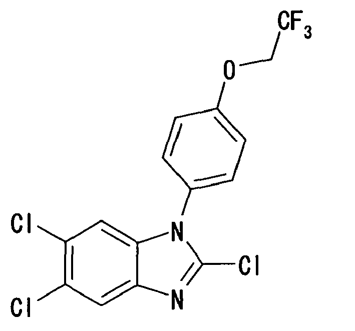

By the reaction in the same manner as in Reference Example 27, the title compound (1.60 g, yield 51%) was obtained as a pale-pink solid from the compound (3.0 g, 7.95 mmol) of Reference Example 106.
¹H-NMR (300MHz, CDCl₃) δ:4.46 (2H, q, J = 8.0 Hz), 7.13-7.22 (3H, m), 7.35 (2H, d, J = 8.7 Hz), 7.83 (1H, s).

### Reference Example 108

### 5,6-Dichloro-1-[4-(trifluoromethoxy)phenyl]-1,3-dihydro-2H-benzimidazol-2-one

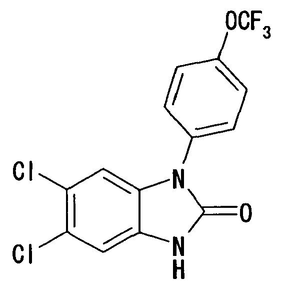

By the reaction in the same manner as in Reference Example 1, crude 4,5-dichloro-2-nitro-N-[4-(trifluoromethoxy)phenyl]aniline (4.83 g) was obtained as an orange amorphous solid from 1,2,4-trichloro-5-nitrobenzene (5.11 g, 22.58 mmol), p-trifluoromethoxyaniline (4.0 g, 22.58 mmol) and potassium fluoride (1.31 g, 22.58 mmol).

By the reaction in the same manner as in Reference Example 2, crude 4,5-dichloro-N-[4-(trifluoromethoxy)phenyl]benzene-1,2-diamine (2.27 g) was obtained as a brown amorphous solid from 4,5-dichloro-2-nitro-N-[4-(trifluoromethoxy)phenyl]aniline (4.83 g).

By the reaction in the same manner as in Reference Example 26, the title compound (162 mg, yield 4%) was obtained as a white powder from 4,5-dichloro-N-[4-(trifluoromethoxy)phenyl]benzene-1,2-diamine (1.13 g).
¹H-NMR (300MHz, DMSO-d₆) δ:7.08 (1H, s), 7.20 (1H, s), 7.39 (2H, d, J = 8.7 Hz), 7.56 (2H, d, J = 8.7 Hz), 11.01 (1H, brs).

### Reference Example 109

### 2,5,6-Trichloro-1-[4-(trifluoromethoxy)phenyl]-1H-benzimidazole

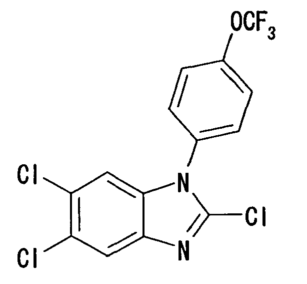

By the reaction in the same manner as in Reference Example 27, the title compound (80 mg, yield 51%) was obtained as a white solid from the compound (150 mg, 0.41 mmol) of Reference Example 108.
¹H-NMR (300MHz, CDCl₃) δ:7.26(1H, s), 7.47 (4H, s), 7.86 (1H, s).

### Reference Example 110

### 5,6-Dichloro-1-[4-(trifluoromethoxy)phenyl]-1,3-dihydro-2H-benzimidazole-2-thione

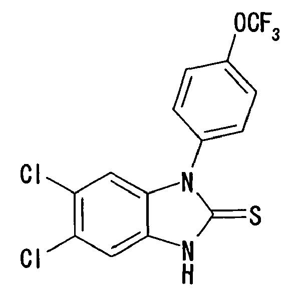

By the reaction in the same manner as in Reference Example 3, the title compound (202 mg, yield 16%) was obtained as a white solid from 4,5-dichloro-N-[4-(trifluoromethoxy)phenyl]benzene-1,2-diamine (1.13 g) obtained in the production process of Reference Example 108.
¹H-NMR (300MHz, DMSO-d₆) δ:6.99 (1H, s), 7.36 (1H, s), 7.44 (2H, d, J = 8.4 Hz), 7.56 (2H, d, J = 8.4 Hz), 12.96 (1H, br s).

### Reference Example 111

### 6-Chloro-1-[4-(1,1,2,2-tetrafluoroethoxy)phenyl]-1,3-dihydro-2H-benzimidazole-2-thione

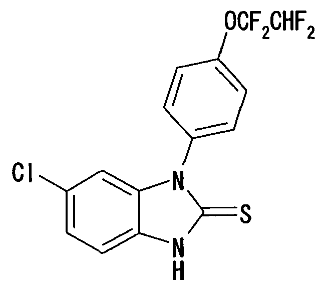

By the reaction in the same manner as in Reference Example 1, crude 5-chloro-2-nitro-N-[4-(1,1,2,2-tetrafluoroethoxy)phenyl]aniline (510 mg) was obtained as a red oil from 2,4-dichloronitrobenzene (918 mg, 4.78 mmol), 4-(1,1,2,2-tetrafluoroethoxy)aniline (1.0 g, 4.78 mmol) and potassium fluoride (278 mg, 4.78 mmol).

By the reaction in the same manner as in Reference Example 2, crude 4-chloro-N²-[4-(1,1,2,2-tetrafluoroethoxy)phenyl]benzene-1,2-diamine (390 mg) was obtained as a red oil from 5-chloro-2-nitro-N-[4-(1,1,2,2-tetrafluoroethoxy)phenyl]aniline (510 mg).

By the reaction in the same manner as in Reference Example 3, the title compound (30 mg, yield 2%) was obtained as a pale-brown powder from 4-chloro-N²-[4-(1,1,2,2-tetrafluoroethoxy)phenyl]benzene-1,2-diamine (160 mg).
¹H-NMR (300MHz, CDCl₃) δ:5.98 (1H, tt, J = 53.1, 2.1 Hz), 6.96 (1H, s), 7.05-7.26 (2H, m), 7.32-7.35 (2H, m), 7.40-7.47 (2H, m), 10.30-10.70 (1H, br).

### Reference Example 112

### Ethyl 4-[(4-ethoxyphenyl)amino]-3-nitrobenzoate

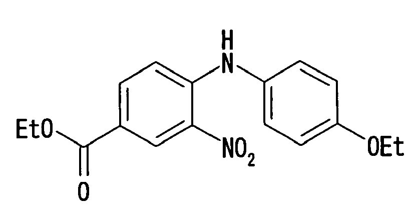

By the reaction in the same manner as in Reference Example 1, the title compound (4.36 g, yield 61%) was obtained as red crystals from ethyl 4-chloro-3-nitrobenzoate (5.0 g, 21.78 mmol), p-phenetidine (2.99 g, 21.78 mmol) and potassium fluoride (1.27 g, 21.78 mmol).
¹H-NMR (300MHz, CDCI₃) δ:1.39 (3H, t, J = 7.1 Hz), 1.45 (3H, t, J = 6.9 Hz), 4.07 (2H, q, J = 6.9 Hz), 4.36 (2H, q, J = 7.1 Hz), 6.94-7.00 (3H, m), 7.19 (2H, d, J = 8.7 Hz), 7.93 (1H, dd, J = 1.8, 9.0 Hz), 8.90 (1H, d, J = 1.8 Hz), 9.68 (1H, s).
melting point: 109-110°C

### Reference Example 113

### Ethyl 3-amino-4-[(4-ethoxyphenyl)amino]benzoate

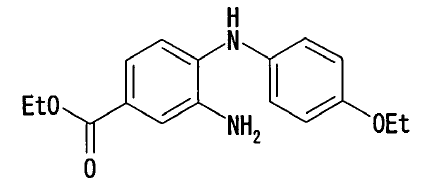

A mixture of the compound (2.50 g, 7.57 mmol) of Reference Example 112 and palladium carbon (500 mg) in THF (50 mL)-EtOH (100 mL) was stirred for 3 hr under hydrogen atmosphere at room temperature. The catalyst was filtered off, and the filtrate was concentrated under reduced pressure to give the title compound (1.78 g, yield 100%) as a white amorphous solid.
¹H-NMR (300MHz, CDCl₃) δ:1.24 (3H, t, J = 7.1 Hz), 1.42 (3H, t, J = 7.1 Hz), 3.53 (2H, br s), 4.02 (2H, q, J = 7.1 Hz), 4.32 (2H, q, J = 7.1 Hz), 5.47 (1H, s), 6.84-7.00 (2H, m), 6.95-7.00 (3H, m), 7.45-7.49 (2H, m).

### Reference Example 114

### Ethyl 1-(4-ethoxyphenyl)-2-thioxo-2,3-dihydro-1H-benzimidazole-5-carboxylate

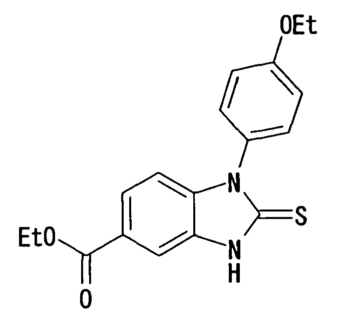

By the reaction in the same manner as in Reference Example 3, the title compound (1.51 g, yield 88%) was obtained as a white powder from the compound (1.50 g, 4.99 mmol) of Reference Example 113.
¹H-NMR (300MHz, CDCl₃) δ:1.39 (3H, t, J = 7.2 Hz), 1.45 (3H, t, J = 6.9 Hz), 4.12 (2H, q, J = 6.9 Hz), 4.40 (2H, q, J = 7.2 Hz), 6.95 (1H, d, J = 8.4 Hz), 7.07-7.13 (2H, m), 7.38-7.42 (2H, m), 7.89 (1H, dd, J = 1.5, 8.4 Hz), 7.93(1H, s), 10.34 (1H, brs).

### Reference Example 115

### N-(5-Chloro-2-nitrophenyl)-6-methoxypyridin-3-amine

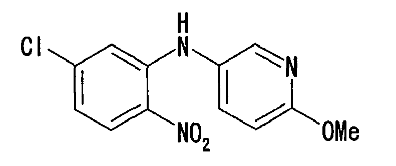

By the reaction in the same manner as in Reference Example 1, the title compound (3.38 g, yield 31%) was obtained as red crystals from 2,4-dichloronitrobenzene (7.44 g, 38.75 mmol), 5-amino-2-methoxypyridine (4.81 g, 38.75 mmol) and potassium fluoride (2.25 g, 38.75 mmol).
¹H-NMR (300MHz, CDCl₃) δ:3.99 (3H, s), 6.73 (1H, dd, J = 1.5, 9.0 Hz), 6.84-6.88 (2H, m), 7.50 (1H, dd, J = 2.7, 8.7 Hz), 8.12-8.18 (2H, m), 9.32 (1H, s).
melting point: 111-112°C

### Reference Example 116

### 4-Chloro-N²-(6-methoxypyridin-3-yl)benzene-1,2-diamine

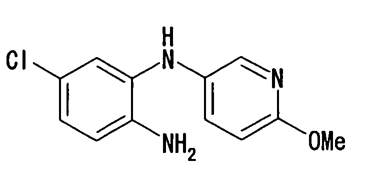

By the reaction in the same manner as in Reference Example 2, the title compound (0.68 g, yield 76%) was obtained as a pink amorphous solid from the compound (1.0 g, 3.58 mmol) of Reference Example 115.
¹H-NMR (300MHz, CDCl₃) δ:3.92 (3H, s), 4.20-4.60 (3H, br), 6.66 (1H, d, J = 8.7 Hz), 6.69 (1H, d, J = 8.1 Hz), 6.79-6.86 (2H, m), 7.18 (1H, dd, J = 2.7, 9.0 Hz), 7.79 (1H, d, J = 2.7 Hz).

### Reference Example 117

### 6-Chloro-1-(6-methoxypyridin-3-yl)-1,3-dihydro-2H-benzimidazole-2-thione

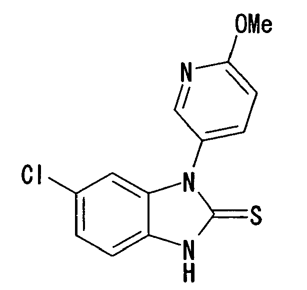

By the reaction in the same manner as in Reference Example 3, the title compound (580 mg, yield 73%) was obtained as a pale-brown powder from the compound (680 mg, 2.72 mmol) of Reference Example 116.
¹H-NMR (300MHz, CDCl₃) δ:4.04 (3H, s), 6.97 (2H, d, J = 8.7 Hz), 7.13-7.26 (2H, m), 7.74 (1H, dd, J = 2.4, 8.4 Hz), 8.30 (1H, d, J = 2.7 Hz), 10.30-10.80 (1H, br).

### Reference Example 118

### 6-Chloro-1-(pyridin-2-yl)-1,3-dihydro-2H-benzimidazole-2-thione

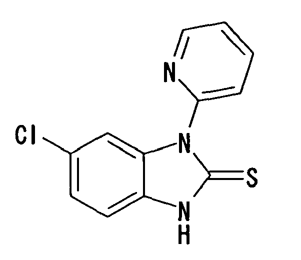

By the reaction in the same manner as in Reference Example 1, N-(5-chloro-2-nitrophenyl)pyridin-2-amine (780 mg) was obtained as an orange solid from 2,4-dichloronitrobenzene (3.84 g, 20.0 mmol), 2-aminopyridine (1.90 g, 20.0 mmol) and potassium fluoride (1.16 g, 20.0 mmol).

By the reaction in the same manner as in Reference Example 2, 4-chloro-N²-(pyridin-2-yl)benzene-1,2-diamine was obtained from N-(5-chloro-2-nitrophenyl)pyridin-2-amine (780 mg). Then, by the reaction in the same manner as in Reference Example 3, the title compound (280 mg, yield 5%) was obtained as a pale-brown powder from 4-chloro-N²-(pyridin-2-yl)benzene-1,2-diamine.
¹H-NMR (300MHz, CDCl₃) δ:7.15 (1H, d, J = 8.4 Hz), 7.23 (1H, dd, J = 1.8, 8.4 Hz), 7.41-7.48 (2H, m), 7.98 (1H, m), 8.12 (1H, m), 8.70 (1H, m), 10.10 (1H, brs).

### Reference Example 119

### 2-Chloro-N-(4-ethoxyphenyl)-6-nitroaniline

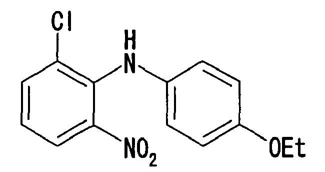

By the reaction in the same manner as in Reference Example 1, the title compound (1.75 g, yield 23%) was obtained as a pale-brown solid from 2,3-dichloronitrobenzene (5.0 g, 26.04 mmol), p-phenetidine (3.57 g, 26.04 mmol) and potassium fluoride (1.51 g, 26.04 mmol).
¹H-NMR (300MHz, CDCl₃) δ:1.40 (3H, t, J = 6.9 Hz), 4.01 (2H, q, J = 6.9 Hz), 6.79-6.87 (4H, m), 6.95 (1H, t, J = 8.1 Hz), 7.57 (1H, dd, J = 1.5, 8.4 Hz), 7.68-7.72 (1H, m), 8.26 (1H, brs).

### Reference Example 120

### 3-Chloro-N²-(4-ethoxyphenyl)benzene-1,2-diamine

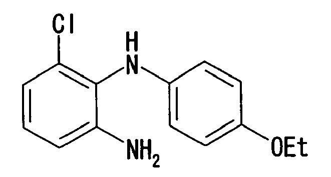

By the reaction in the same manner as in Reference Example 2, the title compound (1.02 g, yield 65%) was obtained as a red oil from the compound (1.75 g, 5.98 mmol) of Reference Example 119.
¹H-NMR (300MHz, CDCl₃) δ:1.37 (3H, t, J = 7.1 Hz), 3.88-3.99 (4H, m), 5.28 (1H, brs), 6.59-6.68 (3H, m), 6.75-6.85 (3H, m), 6.91-6.98 (1H, m).

### Reference Example 121

### 7-Chloro-1-(4-ethoxyphenyl)-1,3-dihydro-2H-benzimidazole-2-thione

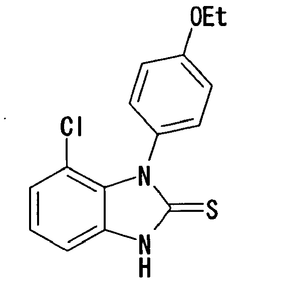

By the reaction in the same manner as in Reference Example 3, the title compound (611 mg, yield 52%) was obtained as a pale-brown powder from the compound (1.02 g, 3.88 mmol) of Reference Example 120.
¹H-NMR (300MHz, CDCl₃) δ:1.47 (3H, t, J = 7.0 Hz), 4.11 (2H, q, J = 7.0 Hz), 7.00-7.06 (2H, m), 7.10-7.19 (3H, m), 7.26-7.35 (2H, m), 10.90 (1H, brs).

### Reference Example 122

### 5-Chloro-1-[4-(trifluoromethoxy)phenyl]-1,3-dihydro-2H-benzimidazole-2-thione

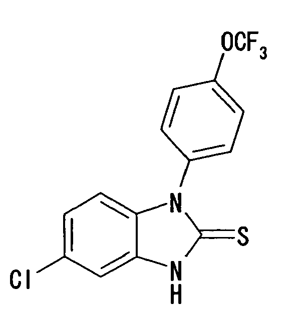

By the reaction in the same manner as in Reference Example 1, crude 4-chloro-2-nitro-N-[4-(trifluoromethoxy)phenyl]aniline (0.58 g) was obtained as a red solid from 2,5-dichloronitrobenzene (4.34 g, 22.58 mmol), 4-trifluoromethoxyaniline (4.0 g, 22.58 mmol) and potassium fluoride (1.31 g, 22.58 mmol).

By the reaction in the same manner as in Reference Example 2, crude 4-chloro-N¹-[4-(trifluoromethoxy)phenyl]benzene-1,2-diamine (0.27 g) was obtained as a red oil from 4-chloro-2-nitro-N-[4-(trifluoromethoxy)phenyl]aniline (0.58 g).

By the reaction in the same manner as in Reference Example 3, the title compound (36 mg, yield 1%) was obtained as a white powder from 4-chloro-N¹-[4-(trifluoromethoxy)phenyl]benzene-1,2-diamine (0.27g).
¹H-NMR (300MHz, CDCl₃) δ:6.88 (1H, d, J = 8.4 Hz), 7.16 (1H, dd, J = 1.8, 8.7 Hz), 7.26-7.28 (1H, m), 7.45 (2H, d, J = 8.7 Hz), 7.59 (2H, d, J = 9.0 Hz), 10.21 (1H, brs).

### Reference Example 123

### N-(4-Methoxyphenyl)-3-nitropyridin-4-amine

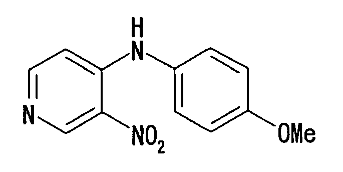

A mixture of 4-chloro-3-nitropyridine (1.0 g, 6.31 mmol) and p-anisidine (777 mg, 6.31 mmol) was stirred for 16 hr at room temperature. To the reaction mixture was added saturated aqueous sodium hydrogencarbonate, and the mixture was extracted with ethyl acetate. The extract was washed with saturated brine, dried over magnesium sulfate and concentrated under reduced pressure.

The residue was purified by silica gel chromatography (eluent; ethyl acetate/hexane=2/3) to give the title compound (1.25 g, yield 81%) as a pale-brown solid.
¹H-NMR (300MHz, CDCl₃) δ:3.86 (3H, s), 6.78 (1H, d, J = 6.2 Hz), 7.00 (2H, d, J = 8.9 Hz), 7.20 (2H, d, J = 8.8 Hz), 8.20 (1H, d, J = 6.0 Hz), 9.27 (1H, s), 9.53 (1H, brs).

### Reference Example 124

### N⁴-(4-Methoxyphenyl)pyridine-3,4-diamine

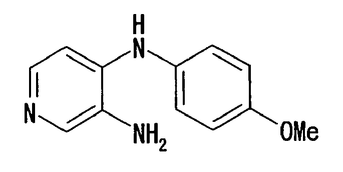

A mixture of the compound (500 mg, 2.04 mmol) of Reference Example 123 and palladium carbon (100 mg) in ethanol (50 mL) was stirred for 18 hr at room temperature under hydrogen atmosphere. The catalyst was filtered off, and the filtrate was concentrated under reduced pressure to give the title compound (440 mg, yield 100%) as a pale-purple solid.
¹H-NMR (300MHz, CDCI₃) δ:3.27 (2H, br s), 3.83 (3H, s), 5.77 (1H, s), 6.76 (1H, d, J = 5.4 Hz), 6.91 (2H, d, J = 8.9 Hz), 7.07 (2H, d, J = 8.9 Hz), 7.92 (1H, d, J = 5.4 Hz), 8.02 (1H, s).

### Reference Example 125

### 2-[(4-Chlorobenzyl)thio]-1-(4-methoxyphenyl)-1H-imidazo[4,5-c]pyridine

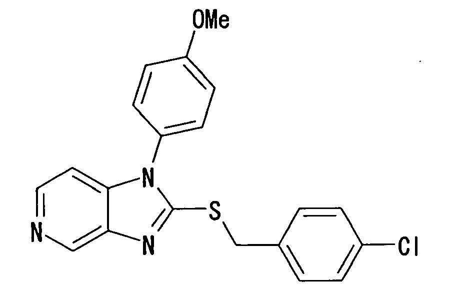

By the reaction in the same manner as in Reference Example 3, 1-(4-methoxyphenyl)-1,3-dihydro-2H-imidazo[4,5-c]pyridine-2-thione (142 mg, yield 27%) was obtained as a white powder from the compound (440 mg, 2.04 mmol) of Reference Example 124.

By the reaction in the same manner as in Example 8, the title compound (87 mg, yield 58%) was obtained as white crystals from 1-(4-methoxyphenyl)-1,3-dihydro-2H-imidazo[4,5-c]pyridine-2-thione (100 mg, 0.39 mmol) and p-chlorobenzyl chloride (69 mg, 0.43 mmol).
¹H-NMR (300MHz, CDCl₃) δ: 3.88 (3H, s), 4.58 (2H, s), 7.03-7.06 (3H, m), 7.25-7.30 (4H, m), 7.36-7.39 (2H, m), 8.34 (1H, d, J = 5.7 Hz), 9.02 (1H, s).
melting point: 141-143°C

### Reference Example 126

### N-(4-Methoxyphenyl)-3-nitropyridin-2-amine

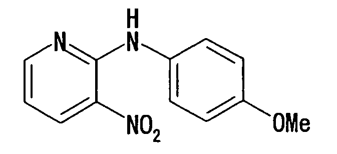

By the reaction in the same manner as in Reference Example 123, the title compound (6.51 g, yield 84%) was obtained as a black solid from 2-chloro-3-nitropyridine (5.0 g, 31.54 mmol) and p-anisidine (777 mg, 6.31 mmol).
¹H-NMR (300MHz, CDCI₃) δ:3.83 (3H, s), 6.75-6.80 (1H, m), 6.93 (2H, d, J = 9.0 Hz), 7.49 (2H, d, J = 9.0 Hz), 8.30-8.45 (1H, m), 8.50 (1H, dd, J = 1.8, 8.3 Hz), 9.96 (1H, brs).

### Reference Example 127

### N²-(4-Methoxyphenyl)pyridine-2,3-diamine

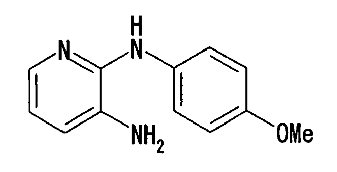

By the reaction in the same manner as in Reference Example 124, the title compound (750 mg, yield 85%) was obtained as a white solid from the compound (1.0 g, 4.08 mmol) of Reference Example 126.
¹H-NMR (300MHz, CDCl₃) δ:3.35 (2H, brs), 3.79 (3H, s), 6.06 (1H, br s), 6.69-6.73 (1H, m), 6.88(2H, d, J = 8.9 Hz), 6.97 (1H, dd, J = 1.5, 7.5 Hz), 7.20-7.26 (2H, m), 7.79 (1H, dd, J = 1.5, 5.0 Hz).

### Reference Example 128

### 3-(4-Methoxyphenyl)-1,3-dihydro-2H-imidazo[4,5-b]pyridine-2-thione

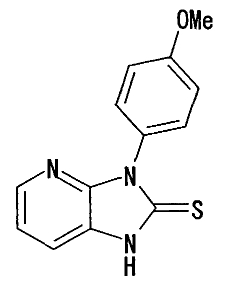

By the reaction in the same manner as in Reference Example 3, the title compound (538 mg, yield 60%) was obtained as a white powder from the compound (750 mg, 3.48 mmol) of Reference Example 127.
¹H-NMR (300MHz, CDCI₃) δ:3.88 (3H, s), 7.06-7.16 (3H, m), 7.44-7.54 (3H, m), 8.13 (1H, d, J = 5.0 Hz), 9.20-9.30 (1H, br).

### Reference Example 129

### 2-[(4-Chlorobenzyl)thio]-3-(4-methoxyphenyl)-3H-imidazo[4,5-b]pyridine

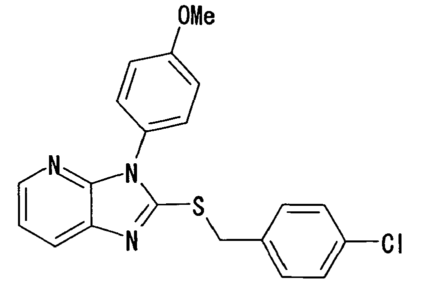

By the reaction in the same manner as in Example 8, the title compound (92 mg, yield 62%) was obtained as white crystals from the compound (100 mg, 0.39 mmol) of Reference Example 128 and p-chlorobenzyl chloride (69 mg, 0.43 mmol). ¹H-NMR (300MHz, CDCI₃) δ: 3.86 (3H, s), 4.58 (2H, s), 7.05 (2H, d, J = 8.9 Hz), 7.19-7.28 (3H, m), 7.34-7.39 (4H, m), 7.96 (1H, dd, J = 1.4, 8.0 Hz), 8.24 (1H, dd, J = 1.4, 4.9 Hz).
melting point: 134-135°C
Elemental analysis:
Calculated (%) for C₂₀H₁₆N₃OSCl:C, 62.90; H, 4.22; N, 11.00. Found (%) :C, 62.82; H, 4.23; N, 10.98.

### Example 1

### 6-Chloro-2-[(4-chlorobenzyl)thio]-1-(4-methoxyphenyl)-1H-benzimidazole

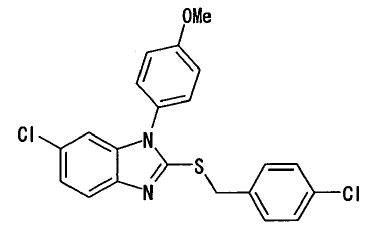

To a suspension of the compound (600 mg, 2.06 mmol) of Reference Example 3 in methanol (30 mL) was added 1N aqueous sodium hydroxide solution (4 mL), a solution of p-chlorobenzyl chloride (399 mg, 2.48 mmol) in diethyl ether (8 mL) and sodium bromide (10 mg) were added and the mixture was stirred at room temperature for 2 days. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) and recrystallized from a mixed solvent of diethyl ether and hexane to give the title compound (541 mg, yield 63%) as white crystals.
¹H-NMR (300MHz, CDCl₃) δ:3.88 (3H, s), 4.54 (2H, s), 7.02-7.07 (3H, m), 7.19-7.27 (5H, m), 7.34(2H, d, J = 8.5 Hz), 7.61 (1H, d, J = 8.5 Hz).
melting point: 116-117°C
Elemental analysis:
Calculated (%) for C₂₁H₁₆N₂OSCl₂:C, 60.73; H, 3.88; N, 6.74. Found (%):C, 60.98; H, 3.88; N, 6.64.

### Example 2

### 6-Chloro-2-[(3-chlorobenzyl)thio]-1-(4-methoxyphenyl)-1H-benzimidazole

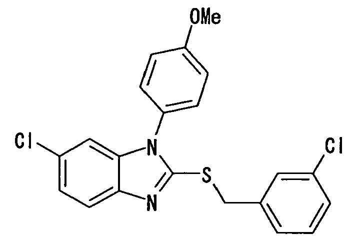

By the reaction in the same manner as in Example 1, the title compound (58 mg, yield 82%) was obtained as a colorless oil from the compound (50 mg, 0.17 mmol) of Reference Example 3 and m-chlorobenzyl chloride (30 mg, 0.19 mmol).
¹H-NMR (300MHz, CDCl₃) δ:3.88 (3H, s), 4.54 (2H, s), 7.02-7.07 (3H, m), 7.20-7.29 (6H, m), 7.40 (1H, s), 7.62 (1H, d, J = 8.5 Hz).

### Example 3

### 6-Chloro-2-[(2-chlorobenzyl)thio]-1-(4-methoxyphenyl)-1H-benzimidazole

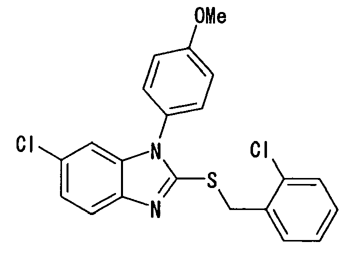

By the reaction in the same manner as in Example 1, the title compound (45 mg, yield 64%) was obtained as white needle crystals from the compound (50 mg, 0.17 mmol) of Reference Example 3 and o-chlorobenzyl chloride (30 mg, 0.19 mmol).
¹H-NMR (300MHz, CDCl₃) δ:3.87 (3H, s), 4.71 (2H, s), 7.01-7.06 (3H, m), 7.18-7.27 (5H, m), 7.34-7.40 (1H, m), 7.55-7.58 (1H, m), 7.63 (1H, d, J = 8.5 Hz).
melting point: 94-95°C
Elemental analysis:
Calculated (%) for C₂₁H₁₆N₂OSCl₂:C, 60.73; H, 3.88; N, 6.74. Found (%):C, 60.74; H, 3.76; N, 6.70.

### Example 4

### 6-Chloro-2-[(2-methoxybenzyl)thio]-1-(4-methoxyphenyl)-1H-benzimidazole

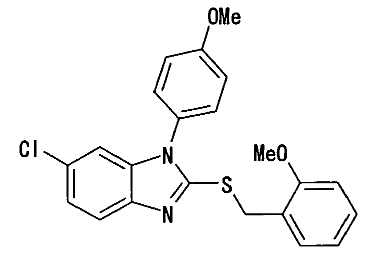

By the reaction in the same manner as in Example 1, the title compound (70 mg, 50%) was obtained as a colorless oil from the compound (100 mg, 0.34 mmol) of Reference Example 3 and o-methoxybenzyl chloride (65 mg, 0.41 mmol).
¹H-NMR (300MHz, CDCl₃) δ:3.78 (3H, s), 3.83 (3H, s), 4.63 (2H, s), 6.81-6.88 (2H, m), 6.98-7.04 (3H, m), 7.18-7.25 (4H, m), 7.41 (1H, d, J = 7.0 Hz), 7.63 (1H, d, J = 8.4Hz).

### Example 5

### 6-Chloro-2-[(3-methoxybenzyl)thio]-1-(4-methoxyphenyl)-1H-benzimidazole

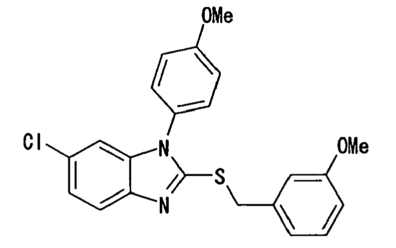

By the reaction in the same manner as in Example 1, the title compound (91 mg, yield 65%) was obtained as white crystals from the compound (100 mg, 0.34 mmol) of Reference Example 3 and m-methoxybenzyl chloride (65 mg, 0.41 mmol).
¹H-NMR (300MHz, CDCl₃) δ:3.76 (3H, s), 3.87 (3H, s), 4.55 (2H, s), 6.75-6.82 (1H, s), 6.95-7.07 (5H, m), 7.19-7.28 (4H, m), 7.63 (1H, d, J = 8. 6 Hz).
melting point: 93-94°C
Elemental analysis:
Calculated (%) for C₂₂H₁₉N₂O₂SCl:C, 64.30; H, 4.66; N, 6.82. Found (%):C, 64.26; H, 4.62; N, 6.86.

### Example 6

### 6-Chloro-2-[(4-methoxybenzyl)thio]-1-(4-methoxyphenyl)-1H-benzimidazole

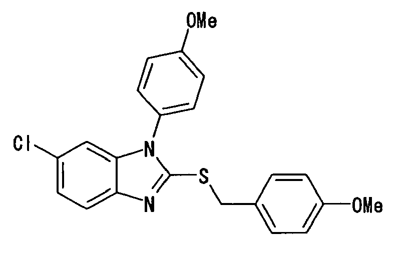

By the reaction in the same manner as in Example 1, the title compound (84 mg, yield 60%) was obtained as white crystals from the compound (100 mg, 0.34 mmol) of Reference Example 3 and p-methoxybenzyl chloride (65 mg, 0.41 mmol).
¹H-NMR (300MHz, CDCl₃) δ:3.77 (3H, s), 3.87 (3H, s), 4.54 (2H, s), 6.81 (2H, d, J = 8.6 Hz), 7.01-7.06 (3H, m), 7.19-7.33 (5H, m), 7.63 (1H, d, J = 8.5 Hz).
melting point: 119-120°C
Elemental analysis:
Calculated (%) for C₂₂H₁₉N₂O₂SCl:C, 64.30; H, 4.66; N, 6.82. Found (%):C, 64.36; H, 4.78; N, 6.70.

### Example 7

### 6-Chloro-2-[(4-chlorobenzyl)thio]-1-[4-(methoxymethoxy)phenyl]-1H-benzimidazole

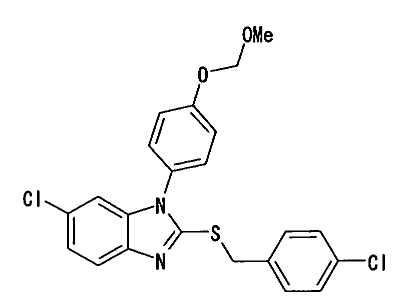

By the reaction in the same manner as in Example 1, the title compound (533 mg, yield 85%) was obtained as white crystals from the compound (450 mg, 1.40 mmol) of Reference Example 10 and p-chlorobenzyl chloride (248 mg, 1.54 mmol).
¹H-NMR (300MHz, CDCl₃) δ:3.52 (3H, s), 4.54 (2H, s), 5.24 (2H, s), 7.08 (1H, d, J = 1.9 Hz), 7.16-7.28 (7H, m), 7.34 (2H, d, J = 8.5 Hz), 7.61 (1H, d, J = 8.6 Hz).
melting point: 109-110°C
Elemental analysis:
Calculated (%) for C₂₂H₁₈N₂O₂SCl₂:C, 59.33; H, 4.07; N, 6.29. Found (%) :C, 59.48; H, 4.01; N, 6.17.

### Example 8

### 2-[(4-Bromobenzyl)thio]-6-chloro-1-[4-(methoxymethoxy)phenyl]-1H-benzimidazole

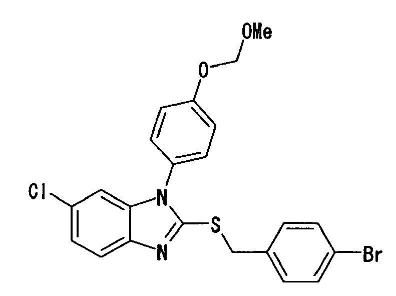

To a suspension of the compound (80 mg, 0.25 mmol) of Reference Example 10 in methanol (3 mL) was added 1N aqueous sodium hydroxide solution (0.45 mL), a solution of p-bromobenzyl bromide (68 mg, 0.27 mmol) in diethyl ether (0.8 mL) was added, and the mixture was stirred for 16 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/4) and recrystallized from a mixed solvent of diethyl ether and hexane to give the title compound (103 mg, yield 84%) as white crystals.
¹H-NMR (300MHz, CDCl₃) δ:3.52 (3H, s), 4.52 (2H, s), 5.24 (2H, s), 7.08 (1H, d, J = 1.9 Hz), 7.16-7.29 (7H, m), 7.40 (2H, d, J = 8.3 Hz), 7.61 (1H, d, J = 8.4 Hz).
melting point: 123-124°C
Elemental analysis:
Calculated (%) for C₂₂H₁₈N₂O₂SBrCl:C, 53.95; H, 3.70; N, 5.72. Found (%):C, 53.88; H, 3.53; N, 5.61.

### Example 9

### 4-{6-Chloro-2-[(4-chlorobenzyl)thio]-1H-benzimidazol-1-yl}phenol

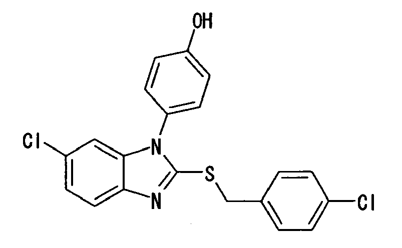

To a solution of the compound (100 mg, 0.22 mmol) of Example 7 in THF (5 mL) was added concentrated hydrochloric acid (0.5 mL) under ice-cooling, and the mixture was stirred for 3 hr at room temperature. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was recrystallized from chloroform and diethyl ether to give the title compound (47 mg, yield 53%) as white crystals.
¹H-NMR (300MHz, CDCl₃) δ:4.53 (2H, s), 5.44 (1H, s), 6.98 (2H, d, J = 8.7 Hz), 7.07 (1H, d, J = 2.0 Hz), 7.20-7.26 (5H, m), 7.33 (2H, d, J = 8.5 Hz), 7.62 (1H, d, J = 8.5 Hz).
melting point: 210-212°C
Elemental analysis:
Calculated (%) for C₂₀H₁₄N₂OSCl₂·0.3H₂O: C, 59.06; H, 3.62; N, 6.89.
Found (%):C, 58.82; H, 3.39; N, 6.82.

### Example 10

### 6-Chloro-2-[(4-chlorobenzyl)thio]-1-(4-ethoxyphenyl)-1H-benzimidazole

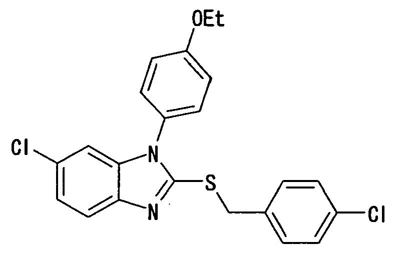

A mixture of the compound (40 mg, 0.10 mmol) of Example 9, ethyl iodide (78 mg, 0.50 mmol) and potassium carbonate (28 mg, 0.20 mmol) in DMF (2 mL) was stirred for 6 hr at 50°C. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) and recrystallized from diethyl ether and hexane to give the title compound (26 mg, yield 61%) as white needle crystals.
¹H-NMR (300MHz, CDCl₃) δ:1.46 (3H, t, J = 7.0 Hz), 4.09 (2H, q, J = 7.0 Hz), 4.53 (2H, s), 7.00 (2H, d, J = 6.9 Hz), 7.07 (1H, s), 7.19-7.26 (5H, m), 7.34 (2H, d, J = 8.4 Hz), 7.61 (1H, d, J = 8.6 Hz).
melting point: 129-130°C
Elemental analysis:
Calculated (%) for C₂₂H₁₈N₂OSCl₂:C, 61.54; H, 4.23; N, 6.52. Found (%):C, 61.38; H, 4.15; N, 6.55.

### Example 11

### 6-Chloro-2-[(4-chlorobenzyl)thio]-1-[4-(cyclopropylmethoxy)phenyl]-1H-benzimidazole

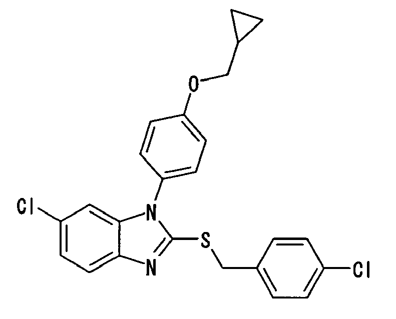

A mixture of the compound (40 mg, 0.10 mmol) of Example 9, cyclopropylmethyl bromide (67 mg, 0.50 mmol), potassium carbonate (28 mg, 0.20 mmol) and potassium iodide (8 mg, 0.05 mmol) in DMF (2 mL) was stirred for 6 hr at 50°C. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) and recrystallized from diethyl ether and hexane to give the title compound (31 mg, yield 68%) as white needle crystals.
¹H-NMR (300MHz, CDCl₃) δ:0.36-0.41 (2H, m), 0.66-0.72 (2H, m), 1.26-1.33 (1H, m), 3.86 (2H, d, J = 6.9 Hz), 4.53 (2H, s), 7.01-7.06 (3H, m), 7.20-7.26 (5H, m), 7.33 (2H, d, J = 8.3 Hz), 7. 61 (1H, d, J = 8.5 Hz).
melting point: 137-138°C
Elemental analysis:
Calculated (%) for C₂₄H₂₀N₂OSCl₂:C, 63.30; H, 4.43; N, 6.15. Found (%):C, 63.18; H, 4.44; N, 6.01.

### Example 12

### 6-Chloro-2-[(4-chlorobenzyl)thio]-1-(4-propoxyphenyl)-1H-benzimidazole

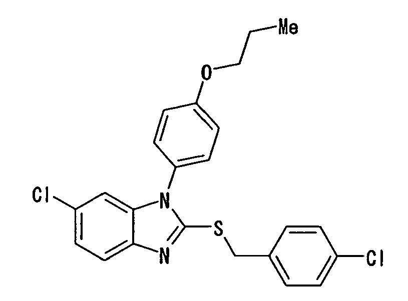

By the reaction in the same manner as in Example 10, the title compound (26 mg, yield 59%) was obtained as white needle crystals from the compound (41 mg, 0.10 mmol) of Example 9 and n-propyl iodide (85 mg, 0.50 mmol).
¹H-NMR (300MHz, CDCI₃) δ:1.07 (3H, t, J = 7.4 Hz), 1.81-1.89 (2H, m), 3.98 (2H, t, J = 6.5 Hz), 4.53 (2H, s), 7.00-7.07 (3H, m), 7.19-7.26 (5H, m), 7.34 (2H, d, J = 8.4 Hz), 7.61 (1H, d, J = 8.5 Hz).
melting point: 102-103°C
Elemental analysis:
Calculated (%) for C₂₃H₂₀N₂OSCl₂:C, 62.30; H, 4.55; N, 6.32. Found (%):C, 62.26; H, 4.50; N, 6.18.

### Example 13

### Ethyl (4-{6-chloro-2-[(4-chlorobenzyl)thio]-1H-benzimidazol-1-yl}phenoxy)acetate

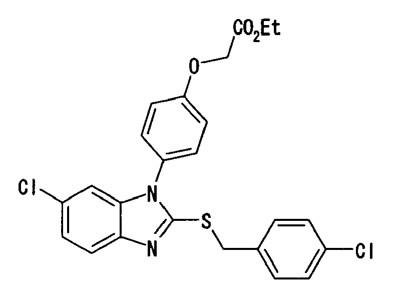

By the reaction in the same manner as in Example 10, the title compound (70 mg, yield 96%) was obtained as a colorless amorphous substance from the compound (60 mg, 0.15 mmol) of Example 9 and ethyl bromoacetate (125 mg, 0.75 mmol).
¹H-NMR (300MHz, CDCl₃) δ:1.32 (3H, t, J = 7.2 Hz), 4.30 (2H, q, J = 7.2 Hz), 4.52 (2H, s), 4.68 (2H, s), 7.02-7.06 (3H, m), 7.19-7.34 (7H, m), 7.61 (1H, d, J = 8.6 Hz).

### Example 14

### (4-{6-Chloro-2-[(4-chlorobenzyl)thio]-1H-benzimidazol-1-yl}phenoxy)acetic acid

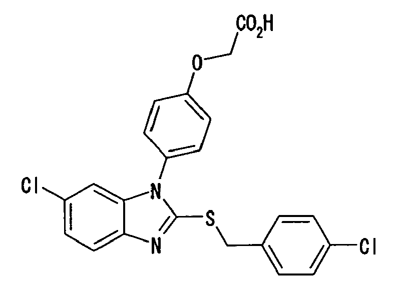

To a solution of the compound (870 mg, 0.14 mmol) of Example 13 in THF (4 mL) was added a 4N aqueous sodium hydroxide solution (2 mL) and the mixture was stirred at room temperature for 1 hr. To the reaction mixture was added saturated aqueous ammonium chloride, and the mixture was extracted twice with ethyl acetate. The organic layers were combined, washed with saturated brine, dried over magnesium sulfate and concentrated under reduced pressure.

The residue was washed with a mixed solvent of dichloromethane and diethyl ether to give the title compound (31 mg, yield 48%) as a white powder.
¹H-NMR (300MHz, CDCl₃) δ:4.46 (4H, s), 6.90-7.30 (9H, m), 7.48 (1H, s), 7.56 (1H, d, J = 8.3 Hz).
melting point: 262-265°C

### Example 15

### 2-(4-{6-Chloro-2-[(4-chlorobenzyl)thio]-1H-benzimidazol-1-yl}phenoxy)ethyl acetate

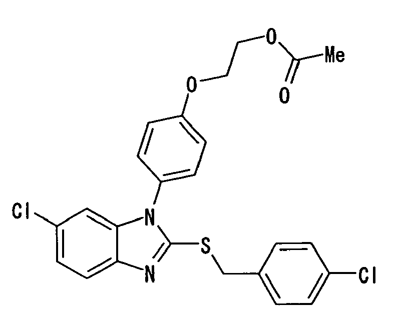

By the reaction in the same manner as in Example 11, the title compound (50 mg, yield 68%) was obtained as white crystals from the compound (60 mg, 0.15 mmol) of Example 9 and 2-bromoethyl acetate (125 mg, 0.75 mmol).
¹H-NMR (300MHz, CDCl₃) δ:2.12 (3H, s), 4.23 (2H, t, J = 4.7 Hz), 4.46 (2H, t, J = 4.7 Hz), 4.54 (2H, s), 7.03-7.07 (3H, m), 7.20-7.28 (6H, m), 7.33 (1H, d, J = 8.5 Hz), 7.62 (1H, d, J = 8.5 Hz).
melting point: 126-127°C
Elemental analysis:
Calculated (%) for C₂₄H₂₀N₂O₃SCl₂·0.6H₂O:C, 57.86; H, 4.28; N, 5.62.
Found (%) :C, 57.71; H, 4.04; N, 5.55.

### Example 16

### 2-(4-{6-Chloro-2-[(4-chlorobenzyl)thio]-1H-benzimidazol-1-yl}phenoxy)ethanol

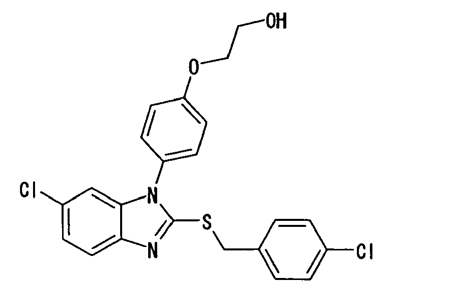

To a mixed solution of the compound (40 mg, 0.082 mmol) of Example 15 in THF (4 mL)-EtOH (1 mL) was added a 4N aqueous sodium hydroxide solution (2 mL), and the mixture was stirred at room temperature for 1 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was recrystallized from a mixed solvent of diethyl ether and hexane to give the title compound (15 mg, yield 41%) as white crystals.
¹H-NMR (300MHz, CDCl₃) δ:2.00 (1H, t, J = 6.2 Hz), 3.99-4.04 (2H, m), 4.16 (2H, t, J = 4.8 Hz), 4.54 (2H, s), 7.04-7.07 (3H, m), 7.20-7.35 (7H, m), 7.62 (1H, d, J = 8.6 Hz).
melting point: 113-114°C
Elemental analysis:
Calculated (%) for C₂₂H₁₈N₂O₂SCl₂:C, 59.33; H, 4.07; N, 6.29. Found (%):C, 59.35; H, 4.06; N, 6.14.

### Example 17

### 6-Chloro-2-[(4-chlorobenzyl)thio]-1-(3-methoxyphenyl)-1H-benzimidazole

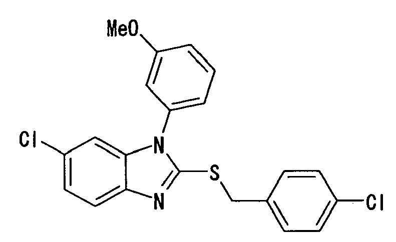

By the reaction in the same manner as in Example 1, the title compound (75 mg, yield 53%) was obtained as white crystals from the compound (100 mg, 0.34 mmol) of Reference Example 14 and p-chlorobenzyl chloride (61 mg, 0.38 mmol).
¹H-NMR (300MHz, CDCl₃) δ:3.84 (3H, s), 4.55 (2H, s), 6.87 (1H, d, J = 2.1 Hz), 6.94 (1H, d, J = 8.8 Hz), 7.05 (1H, d, J = 8.8 Hz), 7.14 (1H, d, J = 1.8 Hz), 7.21-7.26 (3H, m), 7.34 (2H, d, J = 8.4 Hz), 7.44 (1H, d, J = 8.1 Hz), 7.62 (1H, d, J = 8.6 Hz).
melting point: 100-101°C
Elemental analysis:
Calculated (%) for C₂₁H₁₆N₂OSCl₂:C, 60.73; H, 3.88; N, 6.74. Found (%):C, 60.74; H, 3.79; N, 6.70.

### Example 18

### 6-Chloro-2-[(4-chlorobenzyl)thio]-1-(2-methoxyphenyl)-1H-benzimidazole

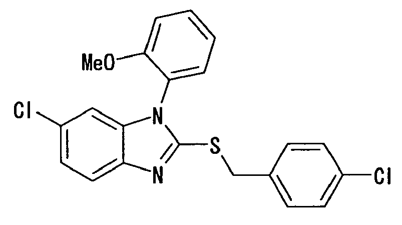

By the reaction in the same manner as in Example 1, the title compound (96 mg, yield 68%) was obtained as white crystals from the compound (100 mg, 0.34 mmol) of Reference Example 15 and p-chlorobenzyl chloride (61 mg, 0.38 mmol).
¹H-NMR (300MHz, CDCl₃) δ:3.72 (3H, s), 4.52 (2H, s), 6.93 (1H, d, J = 1.5 Hz), 7.07 (2H, t, J = 7.2 Hz), 7.19-7.32 (6H, m), 7.49 (1H, d, J = 8.1 Hz), 7.62 (1H, d, J = 8.6 Hz).
melting point: 124-125°C
Elemental analysis:
Calculated (%) for C₂₁H₁₆N₂OSCl₂:C, 60.73; H, 3.88; N, 6.74. Found (%):C, 60.78; H, 3.85; N, 6.72.

### Example 19

### Ethyl 4-{6-chloro-2-[(4-chlorobenzyl)thio]-1H-benzimidazol-1-yl}benzoate

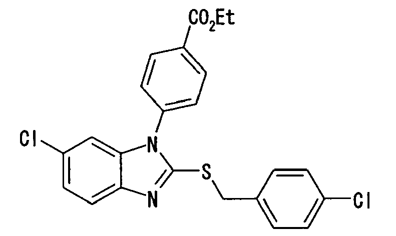

A mixed solution of the compound (50 mg, 0.15 mmol) of Reference Example 18, p-chlorobenzyl chloride (27 mg, 0.17 mmol) and potassium carbonate (25 mg, 0.18 mmol) in DMF (3 mL) was stirred at room temperature for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/4) and recrystallized from a mixed solvent of diethyl ether and hexane to give the title compound (56 mg, yield 82%) as white crystals. ¹H-NMR (300MHz, CDCl₃) δ:1.43 (3H, t, J = 7.1 Hz), 4.43 (2H, q, J = 7.1 Hz), 4.55 (2H, s), 7.13 (1H, d, J = 1.9 Hz), 7.23-7.26 (3H, m), 7.33 (2H, d, J = 8.5 Hz), 7.45 (2H, d, J = 8.5 Hz), 7.64 (1H, d, J = 8.6 Hz), 8.22 (2H, d, J = 8.6 Hz).
melting point: 100-101°C
Elemental analysis:
Calculated (%) for C₂₃H₁₈N₂O₂SCl₂:C, 60.40; H, 3.97; N, 6.12. Found (%):C, 60.32; H, 3.77; N, 6.05.

### Example 20

### 2-[(4-Chlorobenzyl)thio]-6-methoxy-1-(4-methoxyphenyl)-1H-benzimidazole

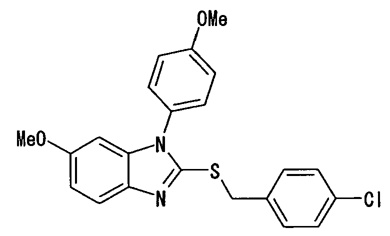

By the reaction in the same manner as in Example 1, the title compound (115 mg, yield 80%) was obtained as white crystals from the compound (100 mg, 0.35 mmol) of Reference Example 21 and p-chlorobenzyl chloride (67 mg, 0.42 mmol).
¹H-NMR (300MHz, CDCl₃) δ:3.77 (3H, s), 3.88 (3H, s), 4.50 (2H, s), 6.55 (1H, d, J = 2.4 Hz), 6.88 (1H, dd, J = 2.4, 8.8 Hz), 7.02 (2H, d, J = 8.9 Hz), 7.22-7.33 (6H, m), 7.61 (1H, d, J = 8.7 Hz).
melting point: 80-81°C
Elemental analysis:
Calculated (%) for C₂₂H₁₉N₂O₂SCl:C, 64.30; H, 4.66; N, 6.82. Found (%):C, 64.14; H, 4.55; N, 6.78.

### Example 21

### 2-(Benzylthio)-6-chloro-1-(4-methoxyphenyl)-1H-benzimidazole

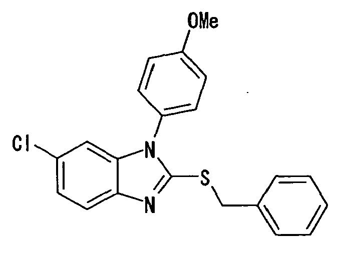

By the reaction in the same manner as in Example 8, the title compound (93 mg, yield 72%) was obtained as white crystals from the compound (100 mg, 0.34 mmol) of Reference Example 3 and benzyl bromide (71 mg, 0.41 mmol). ¹H-NMR (300MHz, CDCl₃) δ:3.87 (3H, s), 4.58 (2H, s), 7.01-7.06 (3H, m), 7.20-7.29 (6H, m), 7.40-7.41 (2H, m), 7.63 (1H, d, J = 8.5 Hz).
melting point: 89-90°C
Elemental analysis:
Calculated (%) for C₂₁H₁₇N₂OSCl:C, 66.22; H, 4.50; N, 7.35. Found value (%):C, 66.15; H, 4.41; N, 7.36.

### Example 22

### 1-(4-Butoxyphenyl)-6-chloro-2-[(4-chlorobenzyl)thio]-1H-benzimidazole

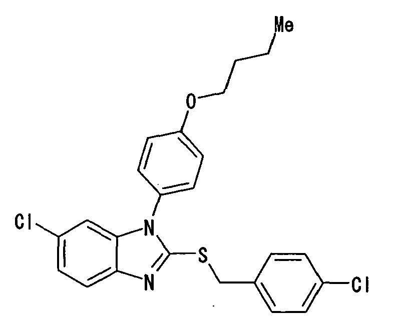

By the reaction in the same manner as in Example 10, the title compound (46 mg, yield 50%) was obtained as white needle crystals from the compound (80 mg, 0.20 mmol) of Example 9 and n-butyl bromide (136 mg, 1.0 mmol).
¹H-NMR (300MHz, CDCl₃) δ:1.02 (3H, t, J = 7.4 Hz), 1.48-1.61 (2H, m), 1.78-1.88 (2H, m), 4.04 (2H, t, J = 6.4 Hz), 4.56 (2H, s), 7.02-7.09 (3H, m), 7.21-7.28 (5H, m), 7.35 (2H, d, J = 8.4 Hz), 7.63 (1H, d, J = 8.6 Hz).
melting point: 96-97°C
Elemental analysis:
Calculated (%) for C₂₄H₂₂N₂OSCl₂:C, 63.02; H, 4.85; N, 6.12. Found (%):C, 62.98; H, 4.82; N, 5.97.

### Example 23

### 6-Chloro-2-[(4-chlorobenzyl)thio]-1-(4-isopropoxyphenyl)-1H-benzimidazole

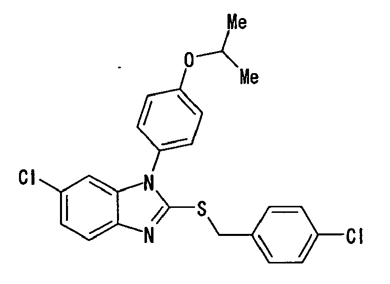

By the reaction in the same manner as in Example 10, the title compound (14 mg, yield 16%) was obtained as white needle crystals from the compound (80 mg, 0.20 mmol) of Example 9 and isopropyl bromide (123 mg, 1.0 mmol). ¹H-NMR (300MHz, CDCl₃) δ:1.39 (6H, d, J = 6.0 Hz), 4.54 (2H, s), 4.56-4.64 (1H, m), 6.99 (2H, d, J = 8.8 Hz), 7.07 (1H, d, J = 1.9 Hz), 7.19-7.26 (5H, m), 7.34 (2H, d, J = 8.4 Hz), 7.61 (1H, d, J = 8.5 Hz).
melting point: 102-103°C
Elemental analysis:
Calculated (%) for C₂₃H₂₀N₂OSCl₂:C, 62.30; H, 4.55; N, 6.32. Found (%):C, 62.29; H, 4.56; N, 6.23.

### Example 24

### 6-Chloro-2-[(4-chlorobenzyl)thio]-1-[4-(2-methoxyethoxy)phenyl]-1H-benzimidazole

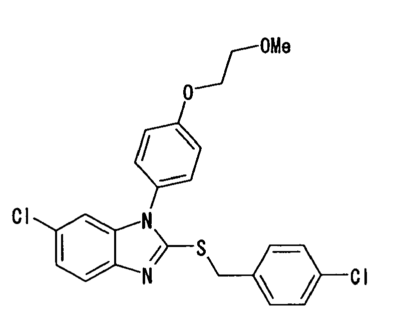

By the reaction in the same manner as in Example 10, the title compound (34 mg, yield 37%) was obtained as white needle crystals from the compound (80 mg, 0.20 mmol) of Example 9 and 2-methoxyethyl bromide (95 mg, 1.0 mmol).
¹H-NMR (300MHz, CDCI₃) δ:3.49 (3H, s), 3.79 (2H, t, J = 4.7 Hz), 4.18 (2H, t, J = 4.7 Hz), 4.53 (2H, s), 7.03-7.09 (3H, m), 7.19-7.26 (5H, m), 7.33 (2H, d, J = 8.4 Hz), 7.62 (1H, d, J = 8.6 Hz).
melting point: 88-90°C
Elemental analysis:
Calculated (%) for C₂₃H₂₀N₂O₂SCl₂·0.7Et₂O:C, 60.61; H, 5.32; N, 5.48.
Found (%):C, 60.91; H, 5.21; N, 5.60.

### Example 25

### 4-{6-Chloro-2-[(4-chlorobenzyl)thio]-1H-benzimidazol-1-yl}phenyl methanesulfonate

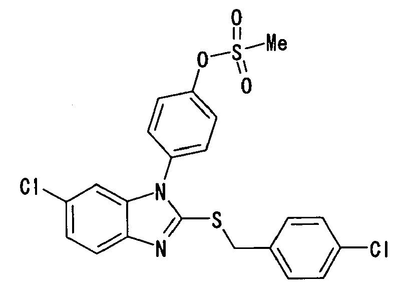

To a solution of the compound (80 mg, 0.20 mmol) of Example 9 and triethylamine (61 mg, 0,60 mmol) in dichloromethane (10 mL) was added methanesulfonyl chloride (46 µL, 0.60 mmol) under ice-cooling, and the mixture was stirred for 1 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/2) and recrystallized from diethyl ether to give the title compound (72 mg, yield 75%) as white crystals.
¹H-NMR (300MHz, CDCl₃) δ:3.27 (3H, s), 4.57 (2H, s), 7.12 (1H, d, J = 2.0 Hz), 7.25-7.29 (3H, m), 7.35 (2H, d, J = 8.4 Hz), 7.43-7.51 (4H, m), 7.65 (1H, d, J = 8.6 Hz).
melting point: 128-129°C
Elemental analysis:
Calculated (%) for C₂₁H₁₆N₂O₃S₂Cl₂:C, 52.61; H, 3.36; N, 5.84. Found (%) :C, 52.63; H, 3.40; N, 5.70.

### Example 26

### 6-Chloro-2-[(4-chlorobenzyl)thio]-1-(4-chlorophenyl)-1H-benzimidazole

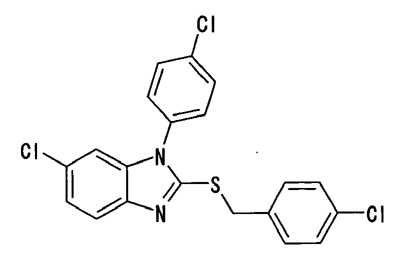

By the reaction in the same manner as in Example 8, the title compound (47 mg, yield 56%) was obtained as white crystals from the compound (60 mg, 0.20 mmol)of Reference Example 23 and p-chlorobenzyl chloride (36 mg, 0.22 mmol).
¹H-NMR (300MHz, CDCI₃) δ:4.54 (2H, s), 7.08 (1H, d, J = 1.8 Hz), 7.22-7.35 (7H, m), 7.52 (2H, d, J = 8.6 Hz), 7.62 (1H, d, J = 8.4 Hz).
melting point: 156-157°C
Elemental analysis:
Calculated (%) for C₂₀H₁₃N₂SCl₃:C, 57.23; H, 3.12; N, 6.67. Found value (%):C, 57.26; H, 3.22; N, 6.57.

### Example 27

### 4-{6-Chloro-2-[(4-chlorobenzyl)thio]-1H-benzimidazol-1-yl}-N,N-dimethylbenzamide

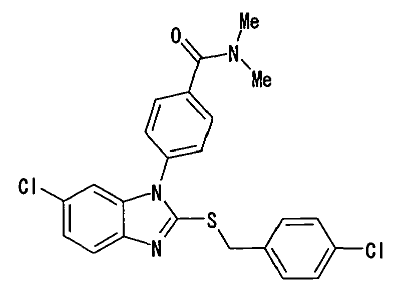

To a solution of dimethylamine hydrochloride (80 mg, 1.0 mmol) and triethylamine (100 mg, 1.0 mmol) in dichloromethane (5 mL) was added a toluene solution (about 15%, 0.33 mL, 1.0 mmol) of trimethylaluminum under ice-cooling, and the mixture was stirred at room temperature for 1 hr. To the reaction mixture was added a solution of the compound (90 mg, 0.20 mmol) of Example 19 in dichloromethane (2 mL), and the mixture was stirred at room temperature for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/1 to 2/1) and recrystallized from diethyl ether to give the title compound (82 mg, yield 90%) as white crystals.
¹H-NMR (300MHz, CDCl₃) δ:3.06 (3H, s), 3.15 (3H, s), 4.55 (2H, s), 7.12 (1H, d, J = 1.9 Hz), 7.22-7.27 (3H, m), 7.34 (2H, d, J = 8.5 Hz), 7.41 (2H, d, J = 8.4 Hz), 7.59-7.65 (3H, m).
melting point: 139-140°C
Elemental analysis:
Calculated (%) for C₂₃H₁₉N₃OSCl₂:C, 60.53; H, 4.20; N, 9.21. Found (%):C, 60.54; H, 4.19; N, 9.16.

### Example 28

### 4-{6-Chloro-2-[(4-chlorobenzyl)thio]-1H-benzimidazol-1-yl}-N-methylbenzamide

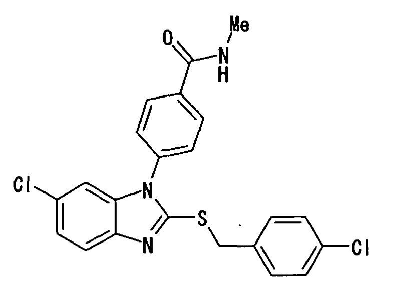

By the reaction in the same manner as in Example 27, the title compound (64 mg, yield 72%) was obtained as white crystals from the compound (90 mg, 0.20 mmol) of Example 19 and methylamine hydrochloride (66 mg, 1.0 mmol).
¹H-NMR (300MHz, CDCl₃) δ:3.05 (3H, d, J = 4.9 Hz), 4.53 (2H, s), 6.37 (1H, brs), 7.09 (1H, d, J = 1.9 Hz), 7.22-7.33 (5H, m), 7.42 (2H, d, J = 8.5 Hz), 7.63 (1H, d, J = 8.6 Hz), 7.93 (2H, d, J = 8.5 Hz).
melting point: 187-188°C

### Example 29

### 5-Chloro-2-[(4-chlorobenzyl)thio]-1-(4-methoxyphenyl)-1H-benzimidazole

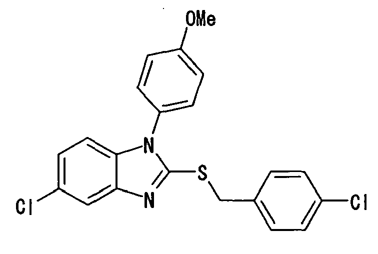

By the reaction in the same manner as in Example 8, the title compound (200 mg, yield 93%) was obtained as a colorless oil from the compound (150 mg, 0.52 mmol) of Reference Example 25 and p-chlorobenzyl chloride (100 mg, 0.62 mmol).
¹H-NMR (300MHz, CDCl₃) δ:3.85 (3H, s), 4.52 (2H, s), 6.95-7.10 (3H, m), 7.10 (1H, dd, J = 2.1, 8.7 Hz), 7.20-7.34 (6H, m), 7.69 (1H, d, J = 2.1 Hz).

### Example 30

### 2-[(4-Bromobenzyl)thio]-6-chloro-1-(4-ethoxyphenyl)-1H-benzimidazole

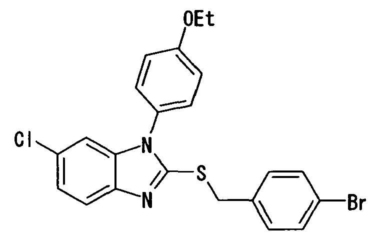

By the reaction in the same manner as in Example 8, the title compound (162 mg, yield 70%) was obtained as white crystals from the compound (150 mg, 0.49 mmol) of Reference Example 6 and p-bromobenzyl bromide (135 mg, 0.54 mmol).
¹H-NMR (300MHz, CDCl₃) δ:1.21 (3H, t, J = 7.0 Hz), 4.09 (2H, q, J = 7.0 Hz), 4.52 (2H, s), 7.03 (3H, t, J = 9.3 Hz), 7.19-7.29 (5H, m), 7.40 (2H, d, J = 8.4 Hz), 7.62 (1H, d, J = 8.7 Hz).
melting point: 136-137°C
Elemental analysis:
Calculated (%) for C₂₂H₁₉N₂OSBrCl:C, 55.77; H, 3.83; N, 5.91. Found (%):C, 55.90; H, 3.82; N, 5.82.

### Example 31

### 6-Chloro-2-[(4-chlorobenzyl)thio]-1-[4-(ethoxymethoxy)phenyl]-1H-benzimidazole

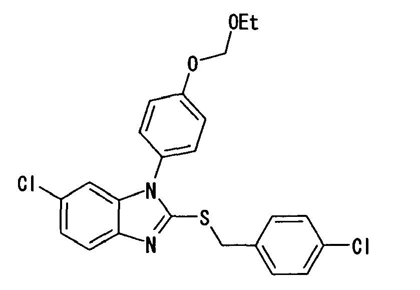

By the reaction in the same manner as in Example 8, the title compound (144 mg, yield 68%) was obtained as white crystals from the compound (155 mg, 0.46 mmol) of Reference Example 13 and p-chlorobenzyl chloride (89 mg, 0.56 mmol).
¹H-NMR (300MHz, CDCl₃) δ:1.26 (3H, t, J = 7.1 Hz), 3.77 (2H, q, J = 7.1 Hz), 4.54 (2H, s), 5.28 (2H, s), 7.07 (1H, q, J = 2.1 Hz), 7.16-7.27 (7H, m), 7.33 (2H, d, J = 8.7 Hz), 7.62 (1H, d, J = 8.4 Hz).
melting point: 130-131°C
Elemental analysis:
Calculated (%) for C₂₃H₂₀N₂O₂SCl₂:C, 60.13; H, 4.39; N, 6.10. Found (%):C, 59.99; H, 4.45; N, 6.01.

### Example 32

### 2-[(4-Bromobenzyl)thio]-6-chloro-1-[4-(ethoxymethoxy)phenyl]-1H-benzimidazole

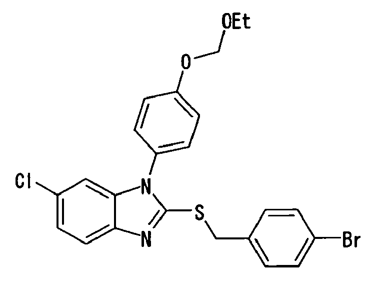

By the reaction in the same manner as in Example 8, the title compound (158 mg, yield 68%) was obtained as white crystals from the compound (155 mg, 0.46 mmol) of Reference Example 13 and p-bromobenzyl bromide (138 mg, 0.56 mmol).
¹H-NMR (300MHz, CDCl₃) δ:1.25 (3H, t, J = 7.1 Hz), 3.76 (2H, q, J = 7.1 Hz), 4.51 (2H, s), 5.27 (2H, s), 7.07 (1H, q, J = 1.8 Hz), 7.15-7.28 (7H, m), 7.39 (2H, d, J = 9.0 Hz), 7.60 (1H, d, J = 8.4 Hz).
melting point: 145-146°C
Elemental analysis:
Calculated (%) for C₂₃H₂₀N₂O₂SBrCl:C, 54.83; H, 4.00; N, 5.56. Found (%):C, 54.82; H, 3.90; N, 5.36.

### Example 33

### 6-Chloro-1-(4-ethoxyphenyl)-2-{[4-(trifluoromethyl)benzyl]thio}-1H-benzimidazole

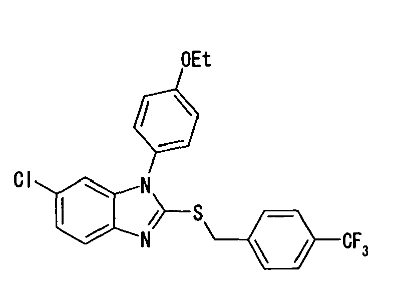

By the reaction in the same manner as in Example 8, the title compound (104 mg, yield 46%) was obtained as white needle crystals from the compound (150 mg, 0.49 mmol) of Reference Example 6 and 4-(trifluoromethyl)benzyl bromide (141 mg, 0.59 mmol).
¹H-NMR (300MHz, CDCl₃) δ:1.46 (3H, t, J = 7.1 Hz), 4.09 (2H, q, J = 7.1 Hz), 4.60 (2H, s), 7.00-7.07 (3H, m), 7.20-7.26 (4H, m), 7.54 (3H, s), 7.61 (1H, d, J = 8.4 Hz).
melting point: 103-104°C
Elemental analysis:
Calculated (%) for C₂₃H₁₈N₂OSClF₃:C, 59.68; H, 3.92; N, 6.05. Found (%):C, 59.66; H, 3.93; N, 5.99.

### Example 34

### 2-[(Biphenyl-4-ylmethyl)thio]-6-chloro-1-(4-ethoxyphenyl)-1H-benzimidazole

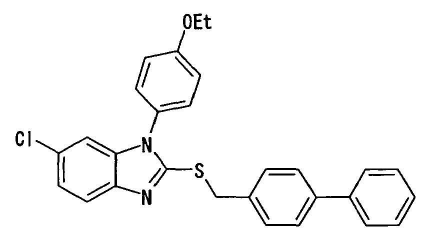

By the reaction in the same manner as in Example 8, the title compound (156 mg, yield 68%) was obtained as white needle crystals from the compound (150 mg, 0.49 mmol) of Reference Example 6 and 4-(chloromethyl)biphenyl (120 mg, 0.59 mmol).
¹H-NMR (300MHz, CDCl₃) δ:1.46 (3H, t, J = 6.9 Hz), 4.08 (2H, q, J = 6.9 Hz), 4.63 (2H, s), 7.00 (2H, d, J = 9.0 Hz), 7.07 (1H, d, J = 2.1 Hz), 7.20-7.56 (12H, m), 7.64 (1H, d, J = 8.47 Hz). melting point: 116-117°C
Elemental analysis:
Calculated (%) for C₂₈H₂₃N₂OSCl:C, 71.40; H, 4.92; N, 5.95. Found (%):C, 71.32; H, 4.97; N, 5.88.

### Example 35

### 4-({[6-Chloro-1-(4-ethoxyphenyl)-1H-benzimidazol-2-yl]thio}methyl)benzonitrile

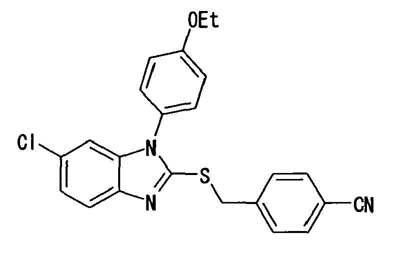

By the reaction in the same manner as in Example 8, the title compound (108 mg, yield 52%) was obtained as white needle crystals from the compound (150 mg, 0.49 mmol) of Reference Example 6 and 4-cyanobenzyl bromide (116 mg, 0.59 mmol).
¹H-NMR (300MHz, CDCl₃) δ:1.47 (3H, t, J = 6.9 Hz), 4.10 (2H, q, J = 6.9 Hz), 4.59 (2H, s), 7.00-7.07 (3H, m), 7.20-7.26 (3H, m), 7.52-7.62 (5H, m).
melting point: 154-155°C
Elemental analysis:
Calculated (%) for C₂₃H₁₈N₃OSCl:C, 65.78; H, 4.32; N, 10.01. Found (%):C, 65.69; H, 4.33; N, 10.02.

### Example 36

### 6-Chloro-1-(4-ethoxyphenyl)-2-({[6-(trifluoromethyl)pyridin-3-yl]methyl}thio)-1H-benzimidazole

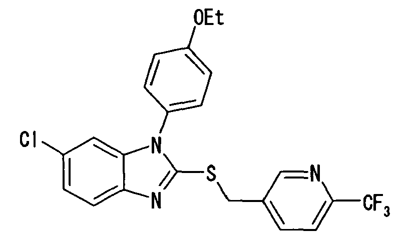

By the reaction in the same manner as in Example 8, the title compound (129 mg, yield 57%) was obtained as white crystals from the compound (150 mg, 0.49 mmol) of Reference Example 6 and 5-(chloromethyl)-2-(trifluoromethyl)pyridine (115 mg, 0.59 mmol).
¹H-NMR (300MHz, CDCl₃) δ:1.47 (3H, t, J = 6.9 Hz), 4.10 (2H, q, J = 6.9 Hz), 4.60 (2H, s), 7.00-7.07 (3H, m), 7.20-7.26 (3H, m), 7.60 (2H, d, J = 8.4 Hz), 7.99 (1H, dd, J = 1.8, 8.1 Hz), 8.81 (1H, d, J = 1.5 Hz).
melting point: 104-105°C
Elemental analysis:
Calculated (%) for C₂₂H₁₇N₃OSClF₃:C, 56.96; H, 3.69; N, 9.06. Found (%):C, 56.96; H, 3.71; N, 9.09.

### Example 37

### Methyl 4-({[6-chloro-1-(4-ethoxyphenyl)-1H-benzimidazol-2-yl]thio}methyl)benzoate

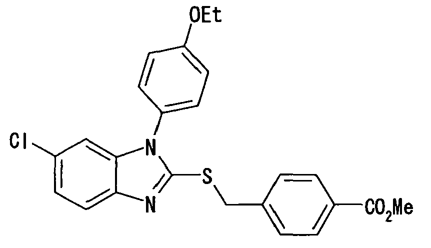

To a suspension of the compound (600 mg, 1.97 mmol) of Reference Example 6 in methanol (30 mL) was added a 1N aqueous sodium hydroxide solution, a solution of methyl 4-(bromomethyl)benzoate (541 mg, 2.36 mmol) in diethyl ether (8 mL) was added, and the mixture was stirred at room temperature for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/4) and recrystallized from a mixed solvent of diethyl ether and hexane to give the title compound (541 mg, yield 63%) as white crystals.
¹H-NMR (300MHz, CDCl₃) δ:1.46 (3H, t, J = 7.1 Hz), 3.89 (3H, s), 4.09 (2H, q, J=7.0Hz), 4.61 (2H, s), 6.99-7.06 (3H, m), 7.19-7.26 (3H, m), 7.47 (2H, d, J = 8.1 Hz), 7.62 (1H, d, J= 8.7 Hz), 7.95 (2H, dd, J = 1.5, 6.6 Hz).
melting point: 107-109°C
Elemental analysis:
Calculated (%) for C₂₄H₂₁N₂O₃SCl:C, 63.64; H, 4.67; N, 6.18. Found (%):C, 63.54; H, 4.70; N, 6.15.

### Example 38

### 2-[(4-tert-Butylbenzyl)thio]-6-chloro-1-(4-ethoxyphenyl)-1H-benzimidazole

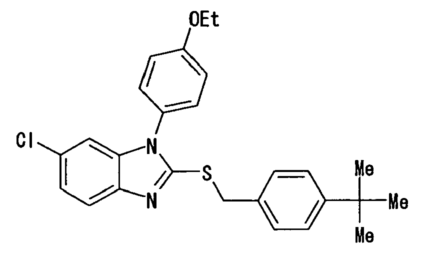

By the reaction in the same manner as in Example 8, the title compound (113 mg, yield 51%) was obtained as white needle crystals from the compound (150 mg, 0.49 mmol) of Reference Example 6 and 4-tert-butylbenzyl chloride (108 mg, 0.59 mmol).
¹H-NMR (300MHz, CDCl₃) δ:1.30 (9H, s), 1.46 (3H, t, J = 7.1 Hz), 4.09 (2H, q, J = 7.0 Hz), 4.57 (2H, s), 7.00 (2H, d, J = 9.0 Hz), 7.07 (1H, d, J = 1.8 Hz), 7.20-7.28 (3H, m), 7.32 (4H, m), 7.64 (1H, d, J = 8.4 Hz).
melting point: 142-143°C
Elemental analysis:
Calculated (%) for C₂₆H₂₇N₂OSCl:C, 69.24; H, 6.03; N, 6.21.
Found value (%):C, 69.48; H, 6.16; N, 6.03.

### Example 39

### 4-{6-Chloro-2-[(4-chlorobenzyl)thio]-1H-benzimidazol-1-yl}-N-methoxy-N-methylbenzamide

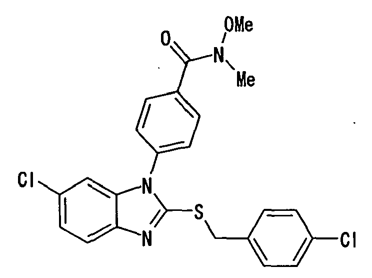

By the reaction in the same manner as in Example 27, the title compound (272 mg, yield 53%) was obtained as white needle crystals from the compound (500 mg, 1.09 mmol) of Example 19 and N,O-dimethylhydroxylamine hydrochloride (320 mg, 3.28 mmol).
¹H-NMR (300MHz, CDCl₃) δ:3.42 (3H, s), 3.62 (3H, s), 4.56 (2H, s), 7.14 (1H, d, J = 2.1 Hz), 7.23-7.27 (3H, m), 7.34 (2H, d, J = 8.4 Hz), 7.42 (2H, d, J = 8.4 Hz), 7.63 (1H, d, J = 8.7 Hz), 7.88 (2H, d, J = 8.4 Hz).
melting point: 125-126°C
Elemental analysis:
Calculated (%) for C₂₃H₁₉N₃O₂SCl₂:C, 58.48; H, 4.05; N, 8.90. Found (%):C, 58.52; H, 4.07; N, 8.97.

### Example 40

### 1-(4-{6-Chloro-2-[(4-chlorobenzyl)thio]-1H-benzimidazol-1-yl}phenyl)propan-1-one

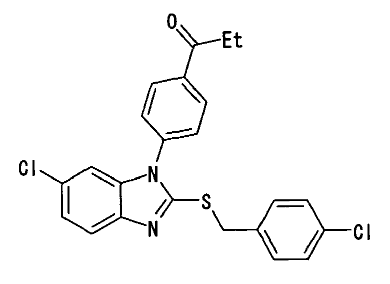

To a solution of the compound (100 mg, 0.37 mmol) of Example 39 in THF (5 mL) was added a THF solution (1.0 M, 880 µL, 0.88 mmol) of ethylmagnesium bromide under ice-cooling, and the mixture was stirred for 3 hr. To the reaction mixture was added 1N hydrochloric acid, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/4) to give the title compound (80 mg, yield 49%) as a white solid.
¹H-NMR (300MHz, CDCl₃) δ:1.29 (3H, t, J = 7.2 Hz), 3.07 (2H, q, J = 7:2 Hz), 4.57 (2H, s), 7.15 (1H, d, J = 1.8 Hz), 7.25-7.28 (3H, m), 7.35 (2H, d, J = 8.4 Hz), 7.50 (2H, d, J = 8.4 Hz), 7.65 (1H, d, J = 8.4 Hz), 8.15 (2H, d, J = 8.4 Hz). melting point: 176-177°C
Elemental analysis:
Calculated (%) for C₂₃H₁₈N₂OSCl₂:C, 62.59; H, 4.11; N, 6.35. Found (%):C, 62.96; H, 4.22; N, 6.35.

### Example 41

### 1-(4-{6-Chloro-2-[(4-chlorobenzyl)thio]-1H-benzimidazol-1-yl}phenyl)-2-methylpropan-1-one

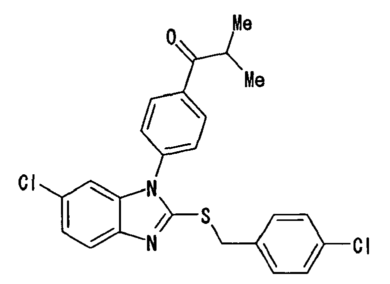

By the reaction in the same manner as in Example 40, the title compound (20 mg, yield 12%) was obtained as white crystals from the compound (100 mg, 0.37 mmol) of Example 39 and a THF solution (2.0 M, 265 µL, 0.53 mmol) of isopropylmagnesium chloride.
¹H-NMR (300MHz, CDCl₃) δ:1.27 (6H, d, J = 6.9 Hz), 3.58 (1H, m), 4.56 (2H, s), 7.14 (1H, d, J = 1.8 Hz), 7.23-7.27 (3H, m), 7.33 (2H, d, J = 8.4 Hz), 7.49 (2H, d, J = 8.4 Hz), 7.64 (1H, d, J = 8.7 Hz), 8.13 (2H, d, J = 8.7 Hz).
melting point: 162-163°C
Elemental analysis:
Calculated (%) for C₂₄H₂₀N₂OSCl₂·0.1H₂O:C, 63.05; H, 4.45; N, 6.13.
Found (%):C, 62.83; H, 4.41; N, 6.07.

### Example 42

### 4-{6-Chloro-2-[(4-chlorobenzyl)thio]-1H-benzimidazol-1-yl}benzoic acid

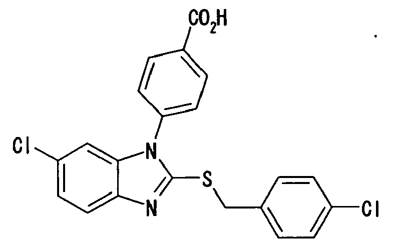

To a solution of the compound (400 mg, 0.87 mmol) of Example 19 in a mixed solvent of THF (5 mL)-EtOH (3 mL) was added 4N sodium hydroxide (2.5 mL), and the mixture was stirred at room temperature for 3 hr. The reaction mixture was partitioned between dilute hydrochloric acid and ethyl acetate. The organic layer was washed with water, dried over magnesium sulfate and concentrated under reduced pressure. The obtained solid was washed with diethyl ether to give the title compound (292 mg, yield 78%) as a white powder.
¹H-NMR (300MHz, CDCl₃) δ:4.55 (2H, s), 7.14 (1H, s), 7.19-7.27 (3H, m), 7.29-7.47 (2H, m), 7.45 (2H, d, J = 9.0 Hz), 7.63 (1H, d, J = 8.4 Hz), 8.23 (2H, dd, J = 1.5, 6.9 Hz).
melting point: 265-266°C
Elemental analysis:
Calculated (%) for C₂₁H₁₄N₂O₂SCl₂:C, 58.75; H, 3.29; N, 6.53. Found (%) :C, 58.51; H, 3.39; N, 6.43.

### Example 43

### 4-{6-Chloro-2-[(4-chlorobenzyl)thio]-1H-benzimidazol-1-yl}-N-(2-hydroxyethyl)benzamide

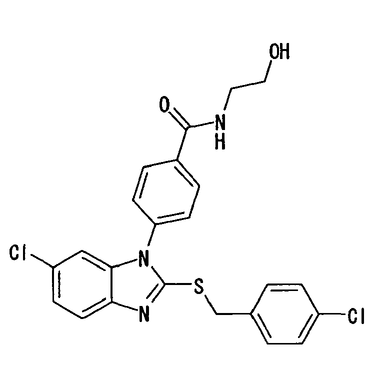

To a solution of the compound (100 mg, 0.23 mmol) of Example 42, 2-aminoethanol (32 mg, 0.52 mmol) and 1-hydroxybenzotriazole (70 mg, 0.52 mmol) in DMF (5 mL) was added 1-ethyl-3-(3-dimethylaminopropyl)carbodiimide hydrochloride (98 mg, 0.52 mmol) under ice-cooling, and the mixture was stirred at room temperature for 4 days. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=2/1) to give the title compound (59 mg, yield 54%) as a white solid.
¹H-NMR (300MHz, CDCl₃) δ:2.16 (1H, t, J = 3.5 Hz), 3.69 (2H, q, J = 5.2 Hz), 3.88 (2H, q, J = 5.0 Hz), 4.55 (2H, s), 6.62 (1H, brs), 7.11 (1H, d, J = 1.8 Hz), 7.23-7.26 (3H, m), 7.33 (2H, d, J = 8.7 Hz), 7.45 (2H, d, J = 8.4 Hz), 7.64 (1H, d, J = 8.7
Hz), 7.96 (2H, d, J = 8.4 Hz).
melting point: 185-186°C
Elemental analysis:
Calculated (%) for C₂₃H₁₉N₃O₂SCl₂:C, 58.48; H, 4.05; N, 8.90. Found (%):C, 58.36; H, 4.09; N, 8.64.

### Example 44

### 4-{5-Chloro-2-[(4-chlorobenzyl)thio]-1H-benzimidazol-1-yl}phenol

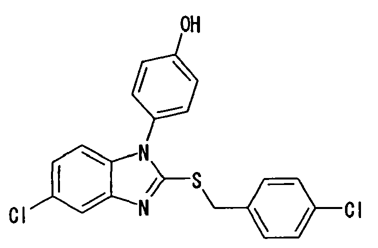

A solution of the compound (1.60 g, 3.85 mmol) of Example 29 in dichloromethane (80 mL) was cooled in a dry ice-acetone bath, and a hexane solution (1.0 M, 11.56 mL, 11.56 mmol) of boron tribromide was added to the solution. The mixture was allowed to gradually warm to room temperature, and the mixture was stirred at room temperature for 3 hr. The reaction mixture was poured into ice water and extracted with ethyl acetate twice. The organic layers were combined, washed with saturated brine, dried over magnesium sulfate and concentrated under reduced pressure.

The obtained solid was washed with a mixed solvent of diethyl ether and hexane to give the title compound (1.32 g, yield 85%) as a white powder.
¹H-NMR (300MHz, CDCl₃) δ:4.53 (2H, s), 6.99-7.07 (3H, m), 7.15-7.40 (7H, m), 7.73 (1H, d, J = 1.5 Hz).
melting point: 212-214°C

### Example 45

### 5-Chloro-2-[(4-chlorobenzyl)thio]-1-(4-ethoxyphenyl)-1H-benzimidazole

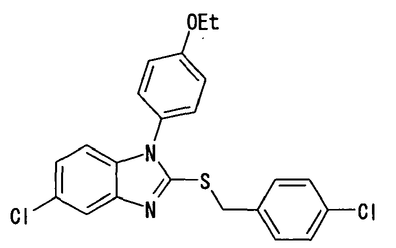

By the reaction in the same manner as in Example 10, the title compound (80 mg, yield 85%) was obtained as a colorless oil from the compound (89 mg, 0.22 mmol) of Example 44 and ethyl iodide (172 mg, 1.11 mmol).
¹H-NMR (300MHz, CDCl₃) δ:1.46 (3H, t, J = 7.1 Hz), 4.09 (2H, q, J = 7.1 Hz), 4.54 (2H, s), 6.98-7.04 (3H, m), 7.13 (1H, dd, J = 1.8, 8.4 Hz), 7.21-7.26 (4H, m), 7.34 (2H, d, J = 8.4 Hz), 7.70 (1H, d, J = 1.8 Hz).

### Example 46

### 5-Chloro-2-[(4-chlorobenzyl)thio]-1-(4-isopropoxyphenyl)-1H-benzimidazole

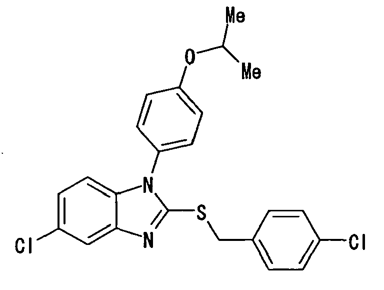

By the reaction in the same manner as in Example 10, the title compound (70 mg, yield 72%) was obtained as a colorless oil from the compound (89 mg, 0.22 mmol) of Example 44 and isopropyl bromide (272 mg, 2.22 mmol).
¹H-NMR (300MHz, CDCl₃) δ:1.38 (6H, d, J = 6.3 Hz), 4.54 (2H, s), 4.56-4.64 (1H, m), 6.98 (3H, d, J = 9.0 Hz), 7.13 (1H, dd, J = 1.8, 8.7 Hz), 7.20-7.26 (4H, m), 7.35 (2H, d, J = 8.4 Hz), 7.70 (1H, d, J = 1.8 Hz).

### Example 47

### 6-Chloro-N-(4-chlorobenzyl)-1-(4-ethoxyphenyl)-1H-benzimidazol-2-amine

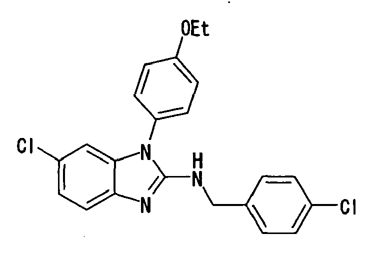

A solution of the compound (1.70 g, 3.81 mmol) of Reference Example 32, methyl iodide (2.16 g, 15.23 mmol) and triethylamine (462 mg, 4.57 mmol) in a mixed solvent of ethanol (20 mL)-THF (10 mL) was stirred at room temperature for 17 hr and at 60°C for 18 hr. The reaction mixture was heated under reflux for 24 hr. After cooling, to the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/3 to 1/1) and the obtained solid was washed with diethyl ether-hexane to give the title compound (843 mg, 54%) as a white solid.
¹H-NMR (300MHz, CDCl₃) δ:1.45 (3H, t, J = 7.1 Hz), 4.08 (2H, q, J = 7.1 Hz), 4.39 (1H, t, J = 6.0 Hz), 4.67 (2H, d, J = 6.0 Hz), 6.86 (1H, d, J = 1.8 Hz), 7.03-7.11 (3H, m), 7.26-7.30 (6H, m), 7.39 (1H, d, J = 8.4 Hz).
melting point: 135-136°C
Elemental analysis:
Calculated (%) for C₂₂H₁₉N₃OCl₂:C, 64.09; H, 4.64; N, 10.19. Found (%):C, 63.94; H, 4.64; N, 10.19.

### Example 48

### 2-{[1-(4-Bromophenyl)ethyl]thio}-6-chloro-1-(4-ethoxyphenyl)-1H-benzimidazole

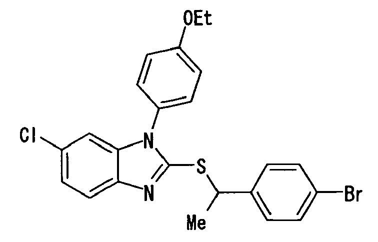

By the reaction in the same manner as in Example 8, the title compound (99 mg, yield 41%) was obtained as white crystals from the compound (150 mg, 0.49 mmol) of Reference Example 6 and the compound (156 mg, 0.59 mmol) of Reference Example 28.
¹H-NMR (300MHz, CDCl₃) δ:1.47 (3H, t, J = 7.0 Hz), 1.79 (3H, d, J = 6.9 Hz), 4.09 (2H, q, J = 7.0Hz), 5.16 (1H, q, J = 6.9 Hz), 6.98-7.04 (3H, m), 7.16-7.28 (5H, m), 7.39 (2H, d, J = 8.4 Hz), 7.61 (1H, d, J = 8.7 Hz).
melting point: 95-98°C
Elemental analysis:
Calculated (%) for C₂₃H₂₀N₂OSBrCl:C, 56.63; H, 4.13; N, 5.74. Found (%):C, 57.09; H, 4.32; N, 5.66.

### Example 49

### 2-(Benzylthio)-6-chloro-1-phenyl-1H-benzimidazole

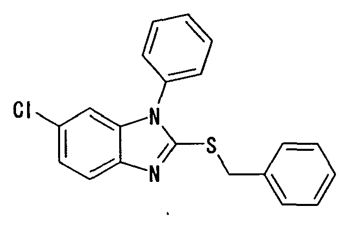

By the reaction in the same manner as in Example 1, the title compound (142 mg, yield 20%) was obtained as a white solid from the compound (515 mg, 0.65 mmol) of Reference Example 29 and benzyl bromide (386 mg, mmol).
¹H-NMR (300MHz, CDCl₃) δ:4.60 (2H, s), 7.10 (1H, d, J = 2.0 Hz), 7.22 (1H, dd, J = 2.0, 8.4 Hz), 7.25-7.41 (7H, m), 7.48-7.61(3H, m), 7. 64 (1H, d, J = 8.4 Hz).
melting point: 82-83°C
Elemental analysis:
Calculated (%) for C₂₀H₁₅N₂SCl:C, 68.46; H, 4.31; N, 7.98
Found (%):C, 68.48; H, 4.24; N, 8.03

### Example 50

### N-(4-Bromobenzyl)-6-chloro-1-(4-ethoxyphenyl)-1H-benzimidazol-2-amine

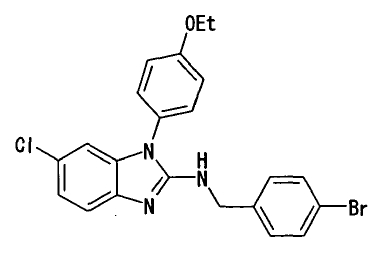

A mixture of the compound (200 mg, 0.65 mmol) of Reference Example 27 and p-bromobenzylamine (1 mL) was stirred at 120°C for 18 hr. After cooling, to the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/2) to give the title compound (101 mg, yield 34%) as a white solid.
¹H-NMR (300MHz, CDCl₃) δ:1.46 (3H, t, J = 7.1 Hz), 4.09 (2H, q, J = 7.1 Hz), 4.41 (1H, t, J = 5.9 Hz), 4.66 (2H, d, J = 6.0 Hz), 6.87 (1H, d, J = 2.1 Hz), 7.02-7.11 (3H, m), 7.21-7.31 (4H, m), 7.39-7.46 (3H, m).
melting point: 140-141°C

### Example 51

### N-(4-tert-Butylbenzyl)-6-chloro-1-(4-ethoxyphenyl)-1H-benzimidazol-2-amine

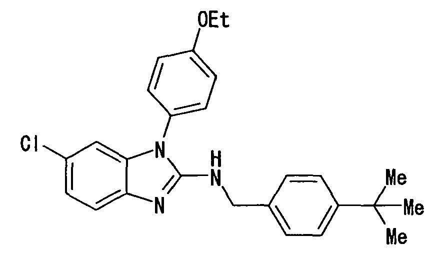

By the reaction in the same manner as in Example 50, the title compound (126 mg, yield 45%) was obtained as a white solid from the compound (200 mg, 0.65 mmol) of Reference Example 27 and 4-tert-butylbenzylamine (1 mL).
¹H-NMR (300MHz, CDCl₃) : δ1.30 (9H, s), 1.46 (3H, t, J = 6.9 Hz), 4.08 (2H, q, J = 7.0 Hz), 4.37 (1H, t, J = 5.4 Hz), 4.67 (2H, d, J = 5.4 Hz), 6.86 (1H, d, J = 2.1 Hz), 7.01-7.11 (3H, m), 7.26-7.43 (7H, m).
melting point: 188-189°C

### Example 52

### N-Benzyl-6-chloro-1-phenyl-1H-benzimidazol-2-amine

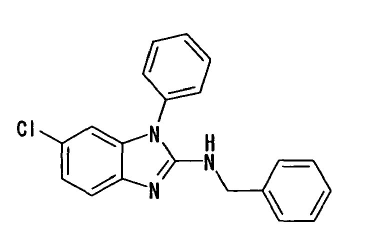

By the reactions in the same manner as in Reference Example 26 and Reference Example 27, 2,6-dichloro-1-phenyl-1H-benzimidazole was obtained from 4-chloro-N²-phenylbenzene-1,2-diamine (500 mg, 2.14 mmol) obtained in the production process of the compound of Reference Example 29. By the reaction in the same manner as in Example 50, the title compound (160 mg, yield 58%) was obtained as a white solid from 2,6-dichloro-1-phenyl-1H-benzimidazole (200 mg, 0.72 mmol) and benzylamine (1 mL).
¹H-NMR (300MHz, CDCl₃) : δ4.47 (1H, t, J = 4.8 Hz), 4.73 (2H, d, J = 5.8 Hz), 6.94 (1H, d, J = 2.2 Hz), 7.12 (1H, dd, J = 2.2, 8.4 Hz), 7.23-7.48 (8H, m), 7.48-7.64(3H, m).
Elemental analysis:
Calculated (%) for C₂₀H₁₆N₂Cl:C, 71.96; H, 4.83; N, 12.59
Found (%):C, 71.78; H, 4.78; N, 12.53

### Example 53

### 6-Chloro-2-[(4-chlorobenzyl)oxy]-1-(4-ethoxyphenyl)-1H-benzimidazole

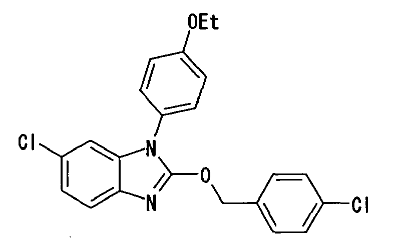

To a solution of p-chlorobenzyl alcohol (186 mg, 1.30 mmol) in DMF (5 mL) was added sodium hydride (in oil: 60 - 72%) (65 mg), and the mixture was stirred at room temperature for 30 min. The compound (200 mg, 0.65 mmol) of Reference Example 27 was added to the mixture, and the mixture was stirred at room temperature for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/5) and recrystallized from diethyl ether and hexane to give the title compound (181 mg, yield 67%) as white crystals.
¹H-NMR (300MHz, CDCl₃) δ:1.46 (3H, t, J = 6.9 Hz), 4.09 (2H, q, J = 6.9 Hz), 5.55 (2H, s), 6.99-7.04 (2H, m), 7.10 (1H, d, J = 1.8Hz), 7.17 (1H, dd, J = 2.1, 8.4 Hz), 7.26-7.36 (6H, m), 7.48 (1H, d, J = 8.4 Hz).
melting point: 134-135°C

### Example 54

### 6-Chloro-1-[4-(methoxymethoxy)phenyl]-2-[(2-phenylethyl)thio]-1H-benzimidazole

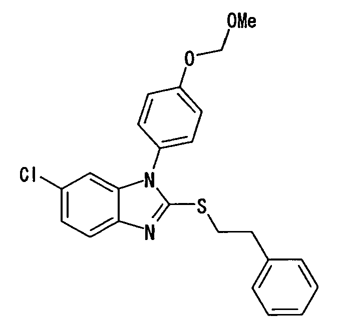

By the reaction in the same manner as in Example 1, the title compound (80 mg, yield 75%) was obtained as a colorless oil from the compound (80 mg, 0.25 mmol) of Reference Example 10 and phenethyl bromide (51 mg, 0.27 mmol).
¹H-NMR (300MHz, CDCl₃) δ:3.09 (2H, t, J = 7.6 Hz), 3.53 (3H, s), 3.58 (2H, t, J = 7.6 Hz), 5.24 (2H, s), 7.07 (1H, d, J = 2.0 Hz), 7.17-7.31 (10H, m), 7.60 (1H, d, J = 8.5 Hz).

### Example 55

### 6-Chloro-1-[4-(methoxymethoxy)phenyl]-2-[(pyridin-2-ylmethyl)thio]-1H-benzimidazole

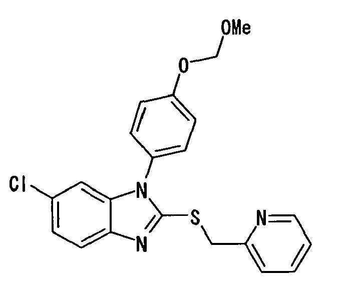

By the reaction in the same manner as in Example 1, the title compound (34 mg, yield 33%) was obtained as white crystals from the compound (80 mg, 0.25 mmol) of Reference Example 10 and 2-(chloromethyl)pyridine hydrochloride (45 mg, 0.27 mmol).
¹H-NMR (300MHz, CDCl₃) δ:3.52 (3H, s), 4.75 (2H, s), 5.23 (2H, s), 7.08 (1H, d, J = 1.7 Hz), 7.14-7.22 (4H, m), 7.29 (2H, d, J = 8.8 Hz), 7.50 (1H, d, J = 7.7 Hz), 7.59-7.63 (2H, m), 8.53 (1H, d, J = 4.8 Hz).
melting point: 95-96°C
Elemental analysis:
Calculated (%) for C₂₁H₁₈N₃O₂SCl:C, 61.23; H, 4.40; N, 10.20. Found (%):C, 61.15; H, 4.22; N, 10.19.

### Example 56

### 6-Chloro-1-[4-(methoxymethoxy)phenyl]-2-[(pyridin-3-ylmethyl)thio]-1H-benzimidazole

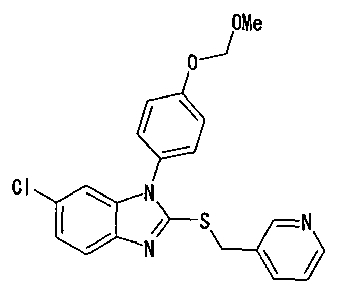

By the reaction in the same manner as in Example 1, the title compound (47 mg, yield 46%) was obtained as white crystals from the compound (80 mg, 0.25 mmol) of Reference Example 10 and 3-(chloromethyl)pyridine hydrochloride (45 mg, 0.27 mmol).
¹H-NMR (300MHz, CDCl₃) δ:3.53 (3H, s), 4.56 (2H, s), 5.24 (2H, s), 7.07 (1H, d, J = 1.9 Hz), 7.17-7.28 (6H, m), 7.62 (1H, d, J = 8.5 Hz), 7.76 (1H, d, J = 7.8 Hz), 8.49 (1H, dd, J = 1.6, 4.8 Hz), 8.66 (1H, d, J = 2.0 Hz).
melting point: 101-102°C
Elemental analysis:
Calculated (%) for C₂₁H₁₈N₃O₂SCl:C, 61.23; H, 4.40; N, 10.20. Found (%):C, 61.09; H, 4.31; N, 10.18.

### Example 57

### 6-Chloro-1-[4-(methoxymethoxy)phenyl]-2-[(pyridin-4-ylmethyl)thio]-1H-benzimidazole

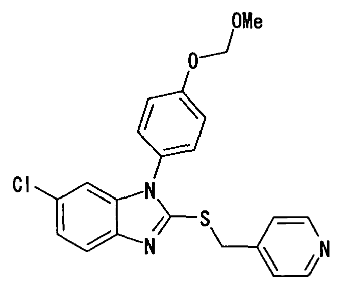

By the reaction in the same manner as in Example 1, the title compound (70 mg, yield 68%) was obtained as a colorless oil from the compound (80 mg, 0.25 mmol) of Reference Example 10 and 4-(chloromethyl)pyridine hydrochloride (45 mg, 0.27 mmol).
¹H-NMR (300MHz, CDCl₃) δ:3.52 (3H, s), 4.53 (2H, s), 5.24 (2H, s), 7.08 (1H, d, J = 1.7 Hz), 7.17-7.28 (5H, m), 7.33 (2H, d, J = 5.7 Hz), 7.60 (1H, d, J = 8.5 Hz), 8.51 (2H, d, J = 5.8 Hz).

### Example 58

### Ethyl 4-{6-chloro-2-[(4-chlorobenzyl)thio]-1H-benzimidazol-1-yl}piperidine-1-carboxylate

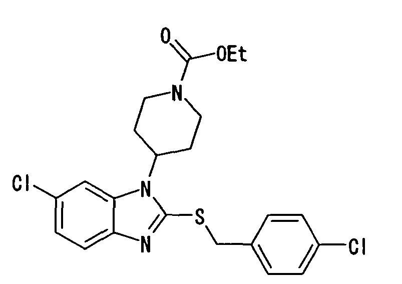

By the reaction in the same manner as in Example 1, the title compound (440 mg, yield 95%) was obtained as colorless crystals from the compound (340 mg, 1 mmol) of Reference Example 33 and p-chlorobenzyl chloride (177 mg, 1.1 mmol).
¹H-NMR (300MHz, CDCl₃) δ:1.31 (3H, t, J = 7.2 Hz), 1.70-1.80 (2H, m), 2.20-2.40 (2H, m), 2.80-2.90 (2H, m), 4.15-4.48 (5H, m), 4.56 (2H, s), 7.19 (1H, dd, J = 2.1, 8.4 Hz), 7.26-7.30 (3H, m), 7.35 (2H, d, J = 8.7 Hz), 7.58 (1H, d, J = 8.4 Hz).
melting point: 118-119°C
Elemental analysis:
Calculated (%) for C₂₂H₂₃N₃O₂SCl₂:C, 56.90; H, 4.99; N, 9.05. Found (%):C, 56.87; H, 4.96; N, 9.11.

### Example 59

### Ethyl 4-{2-[(4-bromobenzyl)thio]-6-chloro-1H-benzimidazol-1-yl}piperidine-1-carboxylate

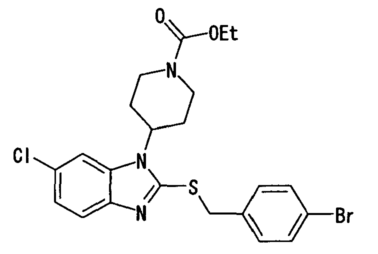

By the reaction in the same manner as in Example 1, the title compound (470 mg, yield 92.5%) was obtained as colorless crystals from the compound (340 mg, 1 mmol) of Reference Example 33 and p-bromobenzyl bromide (275 mg, 1.1 mmol). ¹H-NMR (300MHz, CDCl₃) δ:1.31 (3H, t, J = 7.2 Hz), 1.70-1.80 (2H, m), 2.20-2.40 (2H, m), 2.80-2.90 (2H, m), 4.15-4.45 (5H, m), 4.54 (2H, s), 7.19 (1H, dd, J = 2.1, 8.4 Hz), 7.28 (2H, d, J = 8.4 Hz), 7.36 (1H, d, J = 2.1 Hz), 7.42 (2H, d, J = 8.4 Hz), 7.58 (1H, d, J = 8.4 Hz).
melting point: 123-124°C
Elemental analysis:
Calculated (%) for C₂₂H₂₃N₃O₂SBrCl:C, 51.93; H, 4.56; N, 8.26. Found (%):C, 51.84; H, 4.50; N, 8.29.

### Example 60

### 1-(1-Benzylpiperidin-4-yl)-6-chloro-2-[(4-chlorobenzyl)thio]-1H-benzimidazole

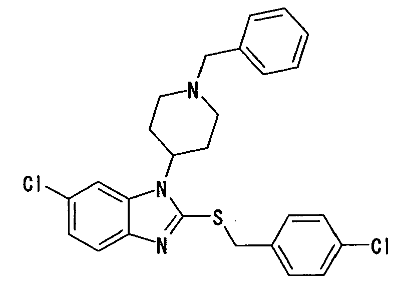

By the reaction in the same manner as in Example 1, the title compound (1.36 g, yield 94%) was obtained as a colorless oil from the compound (1.07 g, 3.0 mmol) of Reference Example 34 and p-chloroberizyl chloride (500 mg, 3.3 mmol).
¹H-NMR (300MHz, CDCl₃) δ:1.70-1.80 (2H, m), 2.05-2.15 (2H, m), 2.35-2.53 (2H, m), 3.00-3.10 (2H, m), 3.38 (2H, s), 4.14 (1H, m), 4.55 (2H, s), 7.17 (1H, dd, J = 1.8, 8.4 Hz), 7.23-7.40 (9H, m), 7.50-7.60 (2H, m).

### Example 61

### 5-Bromo-2-[(4-chlorobenzyl)thio]-1-(4-methoxyphenyl)-1H-benzimidazole hydrochloride

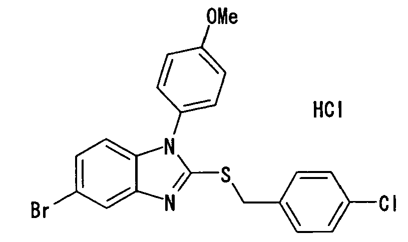

By the reaction in the same manner as in Example 1, 5-bromo-2-[(4-chlorobenzyl)thio]-1-(4-methoxyphenyl)-1H-benzimidazole (1.08 g, yield 98%) was obtained as a colorless oil from the compound (800 mg, 2.38 mmol) of Reference Example 35 and p-chlorobenzyl chloride (385 mg, 2.85 mmol).
¹H-NMR (300MHz, CDCl₃) δ:3.87 (3H, s), 4.54 (2H, s), 6.93 (1H, d, J = 8.4 Hz), 7.02 (2H, d, J = 8.7 Hz), 7.20-7.38 (7H, m), 7.86 (1H, d, J = 1.8 Hz).

The obtained 5-bromo-2-[(4-chlorobenzyl)thio]-1-(4-methoxyphenyl)-1H-benzimidazole (1.08 g) was dissolved in diethyl ether and a 4N hydrogen chloride-ethyl acetate solution was added. The resulting colorless crystals were collected by filtration to give the title compound (980 mg).

### Example 62

### 5-Bromo-2-[(4-bromobenzyl)thio]-1-(4-methoxyphenyl)-1H-benzimidazole hydrochloride

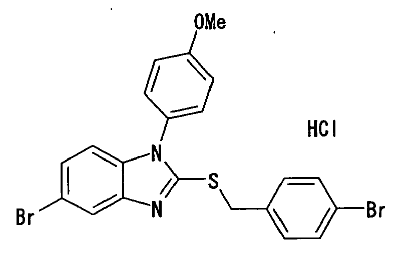

By the reaction in the same manner as in Example 1, 5-bromo-2-[(4-bromobenzyl)thio]-1-(4-methoxyphenyl)-1H-benzimidazole (1.21 g, yield 100%) was obtained as a colorless oil from the compound (800 mg, 2.38 mmol) of Reference Example 35 and p-bromobenzyl bromide (716 mg, 2.85 mmol).
1H-NMR (300MHz, CDCl₃) δ:3.87 (3H, s), 4.54 (2H, s), 6.93 (1H, d, J = 8.7 Hz), 7.02 (2H, d, J = 9.0 Hz), 7.20-7.30 (5H, m), 7.41 (2H, d, J = 8.4 Hz), 7.86 (1H, d, J = 1.5 Hz).

The obtained 5-bromo-2-[(4-bromobenzyl)thio]-1-(4-methoxyphenyl)-1H-benzimidazole (1.21 g) was dissolved in diethyl ether and a 4N hydrogen chloride-ethyl acetate solution was added. The resulting colorless crystals were collected by filtration to give the title compound (1.06 g).

### Example 63

### 2-[(4-Chlorobenzyl)thio]-1-(4-methoxyphenyl)-1H-imidazo[4,5-b]pyridine

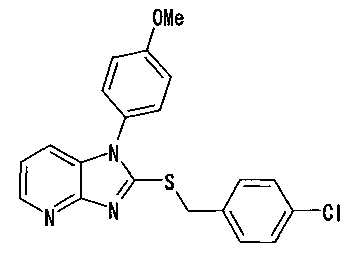

By the reaction in the same manner as in Example 1, the title compound (90 mg, yield 100%) was obtained as a colorless oil from the compound (60 mg, 0.23 mmol) of Reference Example 30 and p-chlorobenzyl chloride (41 mg, 0.26 mmol).
¹H-NMR (300MHz, CDCI₃) δ:3.87 (3H, s), 4.63 (2H, s), 7.00-7.10 (3H, m), 7.20-7.35 (4H, m), 7.36 (1H, dd, J = 1.8, 8.1 Hz), 7.42 (2H, dd, J = 1.5, 6.3 Hz), 8.45 (1H, dd, J = 1.5, 4.8 Hz).

### Example 64

### 5-Chloro-2-[(4-chlorobenzyl)thio]-1-(4-methoxyphenyl)-1H-benzimidazole hydrochloride

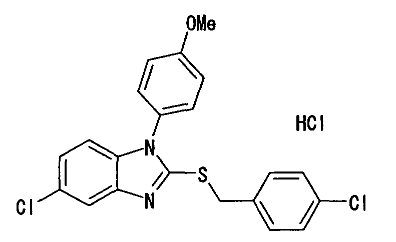

To a solution of the compound (100 mg, 0.24 mmol) of Example 29 in diethyl ether (10 mL)was added a 4N hydrogen chloride-ethyl acetate solution (0.8 mL), and the mixture was concentrated under reduced pressure. To the residue was added diisopropyl ether, and the obtained white solid was collected by filtration to give the title compound (82 mg, yield 76%).
¹H-NMR (300MHz, CDCl₃) δ:3.90 (3H, s), 4.90 (2H, brs), 7.05-7.09 (3H, m), 7.23-7.38 (5H, m), 7.42 (2H, d, J = 8.1 Hz), 8.04 (1H, s).

### Example 65

### 5-Chloro-2-[(4-chlorobenzyl)thio]-1-(4-ethoxyphenyl)-1H-benzimidazole hydrochloride

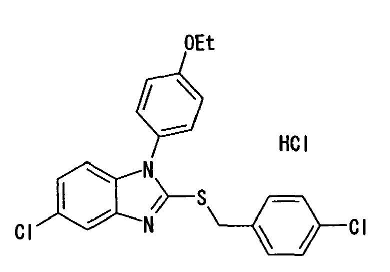

By the method in the same manner as in Example 64, the title compound (37 mg, yield 42%) was obtained as a white powder from the compound (80 mg, 0.19 mmol) of Example 45.
¹H-NMR (300MHz, CDCI₃) δ:1.48 (3H, t, J = 6.9 Hz), 4.12 (2H, q, J = 6.9 Hz), 5.09 (2H, s), 7.05-7.10 (3H, m), 7.20-7.29 (4H, m), 7.37 (1H, dd, J = 1.8, 8.7 Hz), 7.47 (2H, d, J = 8.4 Hz), 8.22 (1H, d, J = 1.5 Hz).

### Example 66

### 5-Chloro-2-[(4-chlorobenzyl)thio]-1-(4-isopropoxyphenyl)-1H-benzimidazole hydrochloride

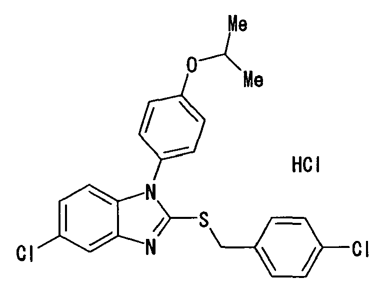

By the method in the same manner as in Example 64, the title compound (41 mg, yield 63%) was obtained as a white powder from the compound (60 mg, 0.14 mmol) of Example 46.
¹H-NMR (300MHz, CDCl₃) δ:1.42 (6H, d, J = 6.0 Hz), 4.61-4.69 (1H, m), 5.10 (2H, s), 7.06-7.11 (3H, m), 7.22 (2H, d, J = 8.7 Hz), 7.30 (2H, d, J = 9.3 Hz), 7.38 (1H, dd, J = 1.8, 9.0 Hz), 7.49 (2H, d, J = 8.4 Hz), 8.23 (1H, s).

### Example 67

### 5-Chloro-2-[(4-chlorobenzyl)thio]-1-[4-(2,2,2-trifluoroethoxy)phenyl]-1H-benzimidazole hydrochloride

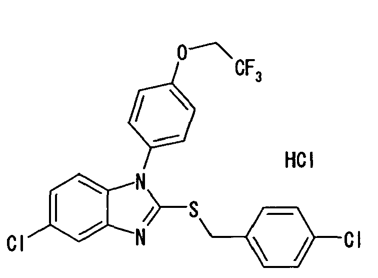

By the method in the same manner as in Example 45, 5-chloro-2-[(4-chlorobenzyl)thio]-1-[4-(2,2,2-trifluoroethoxy)phenyl]-1H-benzimidazole (100 mg) was obtained as a colorless oil from the compound (150 mg, 0.37 mmol) of Example 44 and 2,2,2-trifluoroethane iodide (784 mg, 3.74 mmol).
By the method in the same manner as in Example 64, the title compound (50 mg, 26%) was obtained as a white powder from the obtained 5-chloro-2-[(4-chlorobenzyl)thio]-1-[4-(2,2,2-trifluoroethoxy)phenyl]-1H-benzimidazole.
¹H-NMR (300MHz, CDCl₃) δ:4.46 (2H, t, J = 7.8 Hz), 5.08 (2H, s), 7.05 (1H, d, J = 9.0 Hz), 7.18 (2H, d, J = 8.7 Hz), 7.26-7.40 (5H, m), 7.46 (2H, d, J = 8.4 Hz), 8.20 (1H, s).

### Example 68

### N-(4-Chlorobenzyl)-6-chloro-1-(4-ethoxyphenyl)-1H-benzimidazol-2-amine hydrochloride

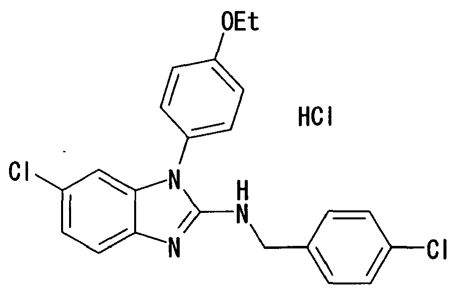

To a solution of the compound (160 mg, 0.39 mmol) of Example 47 in ethyl acetate (5 mL) was added a 4N hydrogen chloride-ethyl acetate (0.5 mL), and the mixture was concentrated under reduced pressure. To the residue was added diisopropyl ether, and the obtained white solid was collected by filtration to give the title compound (171 mg, yield 98%) as a white powder.
¹H-NMR (300MHz, CDCl₃) δ:1.48 (3H, t, J = 7.0 Hz), 4.11 (2H, q, J = 7.0 Hz), 4.83 (2H, d, J = 6.0 Hz), 6.86 (1H, d, J = 1.8 Hz), 6.95-7.05 (1H, br), 7.09 (2H, d, J = 8.7 Hz), 7.24-7.29 (5H, m), 7.42 (2H, d, J = 8.1 Hz), 7.64 (1H, d, J = 8.7 Hz).
melting point: 173-175°C
Elemental analysis:
Calculated (%) for C₂₂H₁₉N₃OCl₂·HCl·0.2H₂O:C, 58.41; H, 4.55; N, 9.29.
Found (%):C, 58.45; H, 4.68; N, 8.99.

### Example 69

### N-(4-Bromobenzyl)-6-chloro-1-(4-ethoxyphenyl)-1H-benzimidazol-2-amine hydrochloride

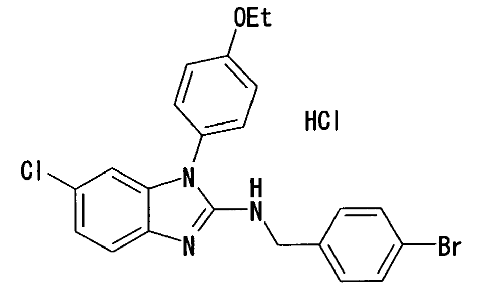

By the reaction in the same manner as in Example 68, the title compound (181 mg, yield 99%) was obtained as a white powder from the compound (170 mg, 0.37 mmol) of Example 50.
¹H-NMR (300MHz, CDCl₃) δ:1.48 (3H, t, J = 7.0 Hz), 4.11 (2H, q, J = 7.0 Hz), 4.85 (2H, d, J = 6.3 Hz), 6.86 (1H, d, J = 1.8 Hz), 6.90-7.00 (1H, br), 7.08 (2H, d, J = 8.7 Hz), 7.25-7.33 (7H, m), 7.64 (1H, d, J = 8.7 Hz).
melting point: 135-138°C
Elemental analysis:
Calculated (%) for C₂₂H₁₉N₃OBrCl·HCl·0.4H₂O:C, 52.80; H, 4.18; N, 8.39.
Found (%) :C, 52.51; H, 4.25; N, 8.14.

### Example 70

### N-(4-tert-Butylbenzyl)-6-chloro-1-(4-ethoxyphenyl)-1H-benzimidazol-2-amine hydrochloride

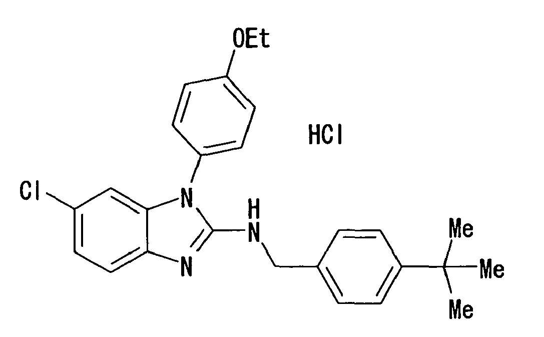

By the reaction in the same manner as in Example 68, the title compound (183 mg, yield 95%) was obtained as a white powder from the compound (180 mg, 0.41 mmol) of Example 51.
¹H-NMR (300MHz, CDCl₃) δ:1.28 (9H, s), 1.48 (3H, t, J = 7. 1 Hz), 4.11 (2H, q, J = 7.1 Hz), 4.87 (2H, d, J = 6.0 Hz), 6.40-6.50 (1H, br), 6.86 (1H, d, J = 1.8 Hz), 7.08 (2H, d, J = 8.7 Hz), 7.26-7.35 (7H, m), 7.71 (1H, d, J = 8.4 Hz).
melting point: 188-189°C
Elemental analysis:
Calculated (%) for C₂₆H₂₈N₃OCl·HCl:C, 66.38; H, 6.21; N, 8.93. Found (%) :C, 66.24; H, 6.18; N, 8.83.

### Example 71

### N-[1-(4-Bromophenyl)ethyl]-6-chloro-1-(4-ethoxyphenyl)-1H-benzimidazol-2-amine

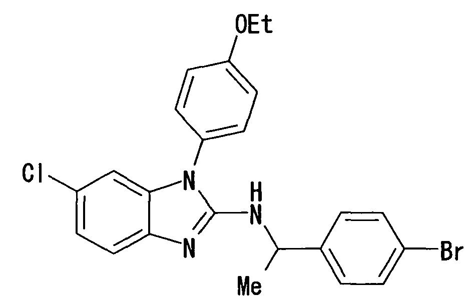

A mixture of the compound (100 mg, 0.33 mmol) of Reference Example 27 and 4-bromo-α-methylbenzylamine (0.5 mL) was stirred at 140°C for 40 hr. After cooling, to the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure. The residue was purified by silica gel chromatography (eluent; ethyl acetate/hexane=1/3) to give the title compound (57 mg, yield 37%) as a white solid.
¹H-NMR (300MHz, CDCl₃) δ:1.48 (3H, t, J = 7.0 Hz), 1.56 (3H, d, J = 7.8 Hz), 4.11 (2H, q, J = 7.0 Hz), 4.30 (1H, d, J = 7.8 Hz), 5.23-5.30 (1H, m), 6.84 (1H, d, J = 1.8 Hz), 7.05-7.09 (3H, m), 7.20-7.30 (4H, m), 7.36 (1H, d, J = 8.7 Hz), 7.40 (2H, d, J = 9.0 Hz).

### Example 72

### N-(4-tert-Butylbenzyl)-6-chloro-1-(4-methoxyphenyl)-1H-benzimidazol-2-amine

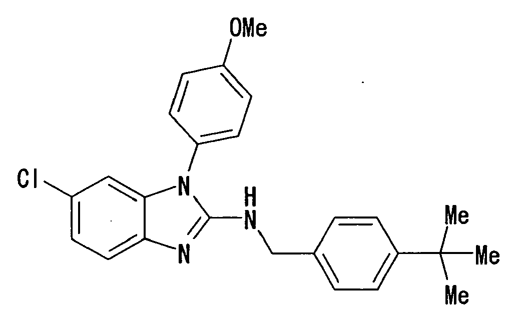

By the reaction in the same manner as in Example 50, the title compound (539 mg, yield 75%) was obtained as a white powder from the compound (500 mg, 1.71 mmol) of Reference Example 59 and 4-tert-butylbenzylamine (1.5 mL).
¹H-NMR (300MHz, CDCl₃) δ:1.30 (9H, s), 3.87 (3H, s), 4.37 (1H, t, J = 5.7 Hz), 4.68 (2H, d, J = 5.7 Hz), 6.86 (1H, d, J = 2.1 Hz), 7.03-7.11 (3H, m), 7.26-7.43 (7H, m).
melting point: 208-209°C
Elemental analysis:
Calculated (%) for C₂₅H₂₆N₃OCl:C, 71.50; H, 6.24; N, 10.01. Found (%):C, 71.33; H, 6.38; N, 9.84.

### Example 73

### N-(4-tert-Butylbenzyl)-6-chloro-1-(4-propoxyphenyl)-1H-benzimidazol-2-amine

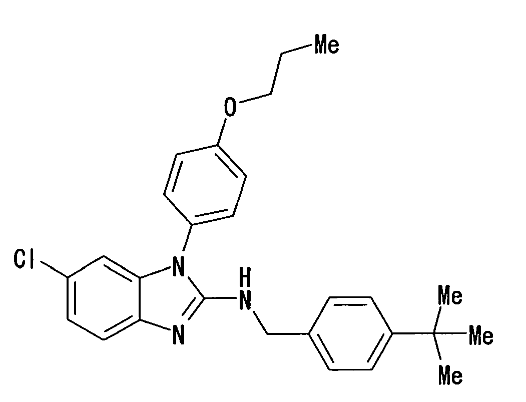

By the reaction in the same manner as in Example 50, the title compound (11 mg, yield 8%) was obtained as white crystals from the compound (100 mg, 0.31 mmol) of Reference Example 63 and 4-tert-butylbenzylamine (0.1 mL).
¹H-NMR (300MHz, CDCl₃) δ:1.07 (3H, t, J = 7.4 Hz), 1.30 (9H, s), 1.81-1.88 (2H, m), 3.97 (2H, t, J = 6.5 Hz), 4.36 (1H, t, J = 5.1 Hz), 4.67 (2H, d, J = 5.4 Hz), 6.86 (1H, d, J = 2.1 Hz), 7.03-7.11 (3H, m), 7.26-7.43 (7H, m).
melting point: 98-100°C
Elemental analysis:
Calculated (%) for C₂₇H₃₀N₃OCl·0.5Et₂O:C, 71.81; H, 7.27; N, 8.66. Found (%) :C, 71.54; H, 6.91; N, 8.82.

### Example 74

### 6-Chloro-N-(4-chlorobenzyl)-1-(4-propoxyphenyl)-1H-benzimidazol-2-amine

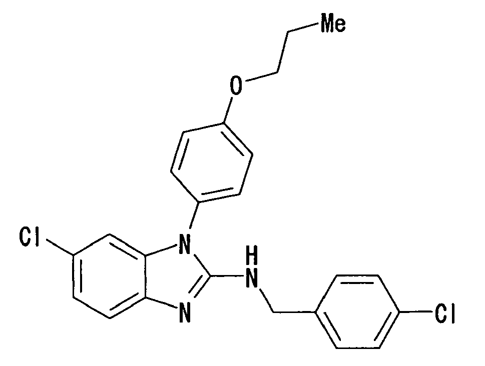

By the reaction in the same manner as in Example 50, the title compound (23 mg, yield 17%) was obtained as white crystals from the compound (100 mg, 0.31 mmol) of Reference Example 63 and p-chlorobenzylamine (0.1 mL).
¹H-NMR (300MHz, CDCl₃) δ:1.07 (3H, t, J = 7.4 Hz), 1.82-1.89 (2H, m), 3.98 (2H, t, J = 6.5 Hz), 4.41 (1H, t, J = 5.7 Hz), 4.68 (2H, d, J = 5.7 Hz), 6.87 (1H, d, J = 2.1 Hz), 7.04-7.11 (3H, m), 7.26-7.31 (6H, m), 7.40 (1H, d, J = 8.4 Hz).
melting point: 130-131°C
Elemental analysis:
Calculated (%) for C₂₃H₂₁N₃OCl₂:C, 64.80; H, 4.96; N, 9.86.
Found (%):C, 64.54; H, 4.89; N, 9.79.

### Example 75

### 1-(4-Butoxyphenyl)-N-(4-tert-butylbenzyl)-6-chloro-1H-benzimidazol-2-amine

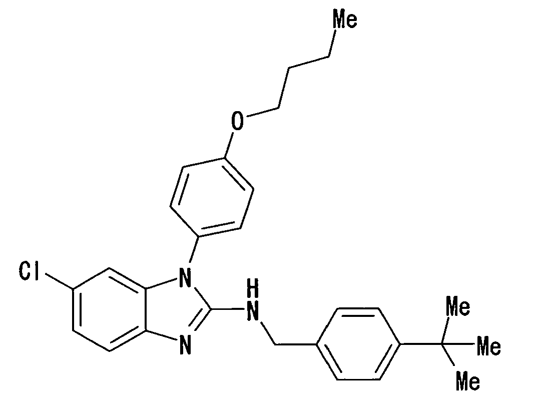

By the reaction in the same manner as in Example 50, the title compound (315 mg, yield 46%) was obtained as a pale-yellow amorphous solid from the compound (500 mg, 1.49 mmol) of Reference Example 67 and 4-tert-butylbenzylamine (0.5 mL).
¹H-NMR (300MHz, CDCl₃) δ:1.00 (3H, t, J = 7.4 Hz), 1.39 (9H, s), 1.46-1.58 (2H, m), 1.76-1.85 (2H, m), 4.01 (2H, t, J = 6.3 Hz), 4.37 (1H, t, J = 5.4 Hz), 4.67 (2H, d, J = 5.4 Hz), 6.86 (1H, d, J = 1.8 Hz), 7.02-7.11 (3H, m), 7.26-7.42 (7H, m).
Elemental analysis:
Calculated (%) for C₂₈H₃₂N₃OCl:C, 72.79; H, 6.98; N, 9.09.
Found (%):C, 72.47; H, 6.98; N, 8.99.

### Example 76

### 1-(4-Butoxyphenyl)-6-chloro-N-(4-chlorobenzyl)-1H-benzimidazol-2-amine

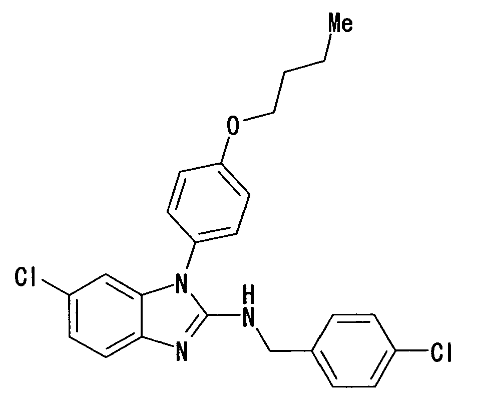

By the reaction in the same manner as in Example 50, the title compound (42 mg, yield 32%) was obtained as white crystals from the compound (100 mg, 0.30 mmol) of Reference Example 67 and p-chlorobenzylamine (0.3 mL).
¹H-NMR (300MHz, CDCl₃) δ:1.00 (3H, t, J = 7.4 Hz), 1.46-1.59 (2H, m), 1.77-1.86 (2H, m), 4.02 (2H, t, J = 6.5 Hz), 4.40 (1H, t, J = 5.6 Hz), 4.68 (2H, d, J = 6.0 Hz), 6.87 (1H, d, J = 1.8 Hz), 7.04 (3H, m), 7.26-7.33 (6H, m), 7.40 (1H, d, J = 8.4 Hz).
melting point: 122-123°C
Elemental analysis:
Calculated (%) for C₂₄H₂₃N₃OCl₂:C, 65.46; H, 5.26; N, 9.54.
Found (%):C, 65.35; H, 5.17; N, 9.40.

### Example 77

### N-(4-Bromobenzyl)-1-(4-butoxyphenyl)-6-chloro-1H-benzimidazol-2-amine

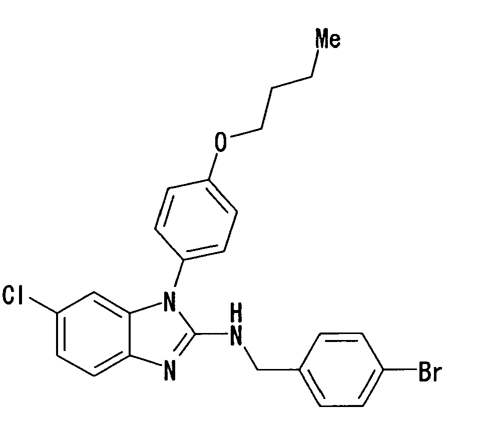

By the reaction in the same manner as in Example 50, the title compound (63 mg, yield 43%) was obtained as white crystals from the compound (100 mg, 0.30 mmol) of Reference Example 67 and p-bromobenzylamine (0.3 mL).
¹H-NMR (300MHz, CDCl₃) δ:1.00 (3H, t, J = 7.4 Hz), 1.46-1.59 (2H, m), 1.77-1.86 (2H, m), 4.02 (2H, t, J = 6.5 Hz), 4.40 (1H, t, J = 6.0 Hz), 4.66 (2H, d, J = 6.0 Hz), 6.87 (1H, d, J = 2.1 Hz), 7.04-7.11 (3H, m), 7.21-7.30 (4H, m), 7.35-7.46 (3H, m).
melting point: 123-124°C
Elemental analysis:
Calculated (%) for C₂₄H₂₃N₃OBrCl:C, 59.46; H, 4.78; N, 8.67. Found value (%):C, 59.38; H, 4.58; N, 8.61.

### Example 78

### N-(4-tert-Butylbenzyl)-6-chloro-1-[4-(2;2,2-trifluoroethoxy)phenyl]-1H-benzimidazol-2-amine

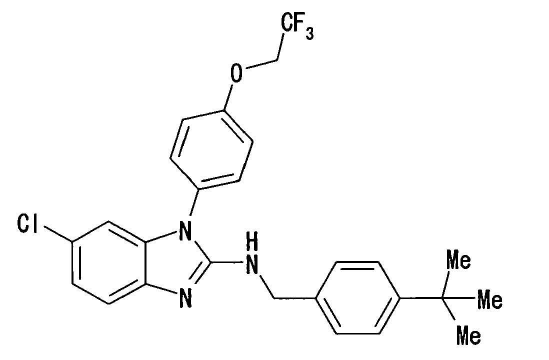

By the reaction in the same manner as in Example 50, the title compound (52 mg, yield 38%) was obtained as white crystals from the compound (100 mg, 0.28 mmol) of Reference Example 73 and 4-tert-butylbenzylamine (0.2 mL).
¹H-NMR (300MHz, CDCl₃) δ:1.30 (9H, s), 4.33 (1H, t, J = 5.3 Hz), 4.42 (2H, q, J = 8.0 Hz), 4.67 (2H, d, J = 5.7 Hz), 6.86 (1H, d, J = 2.1 Hz), 7.09-7.15 (3H, m), 7.26-7.29 (3H, m), 7.35-7.44 (4H, m).
melting point: 151-152°C
Elemental analysis:
Calculated (%) for C₂₆H₂₅N₃OClF₃:C, 64.00; H, 5.16; N, 8.61. Found (%):C, 63.94; H, 5.12; N, 8.63.

### Example 79

### 6-Chloro-N-(4-chlorobenzyl)-1-[4-(2,2,2-trifluoroethoxy)phenyl]-1H-benzimidazol-2-amine

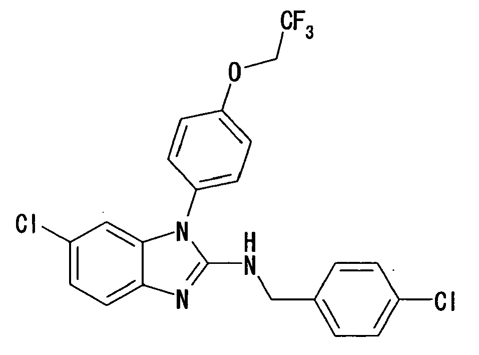

By the reaction in the same manner as in Example 50, the title compound (15 mg, yield 11%) was obtained as white crystals from the compound (100 mg, 0.28 mmol) of Reference Example 73 and p-chlorobenzylamine (0.1 mL).
¹H-NMR (300MHz, CDCl₃) δ: 4.35-4.47 (3H, m), 4.68 (2H, d, J = 6.0 Hz), 6.87 (1H, d, J = 2.1 Hz), 7.10-7.17 (3H, m), 7.26-7.29 (4H, m), 7.32-7.43 (3H, m).
melting point: 150-151°C
Elemental analysis:
Calculated (%) for C₂₂H₁₆N₃OCl₂F₃·0.5Et₂O:C, 57.27; H, 4.20; N, 8.35.
Found (%) :C, 57.52; H, 4.07; N, 8.54.

### Example 80

### N-(4-tert-Butylbenzyl)-6-chloro-1-[4-(trifluoromethoxy)phenyl]-1H-benzimidazol-2-amine

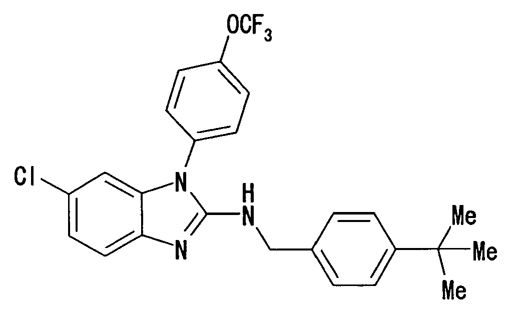

By the reaction in the same manner as in Example 50, the title compound (31 mg, yield 38%) was obtained as white crystals from the compound (60 mg, 0.17 mmol) of Reference Example 76 and 4-tert-butylbenzylamine (0.2 mL).
¹H-NMR (300MHz, CDCl₃) δ: 1.30 (9H, s), 4.37 (1H, t, J = 5.4 Hz), 4.69 (2H, d, J = 5.4 Hz), 6.90 (1H, d, J = 1.8 Hz), 7.13 (1H, dd, J = 1.8, 8.7 Hz), 7.26-7.30 (3H, m), 7.36-7.49 (6H, m).
melting point: 181-182°C
Elemental analysis:
Calculated (%) for C₂₅H₂₃N₃OClF₃:C, 63.36; H, 4.89; N, 8.87. Found (%):C, 63.44; H, 4.83; N, 8.74.

### Example 81

### N-(4-tert-Butylbenzyl)-5-chloro-1-(4-ethoxyphenyl)-1H-benzimidazol-2-amine

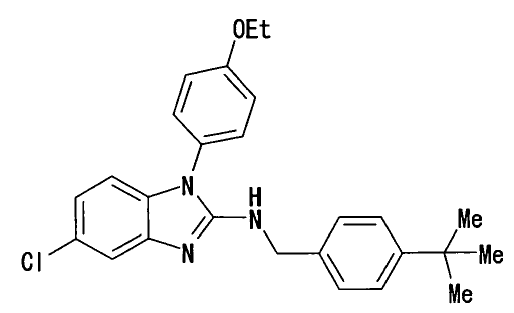

By the reaction in the same manner as in Example 50, the title compound (391 mg, yield 55%) was obtained as white crystals from the compound (500 mg, 1.63 mmol) of Reference Example 79 and 4-tert-butylbenzylamine (1 mL).
¹H-NMR (300MHz, CDCl₃) δ: 1.30 (9H, s), 1.46 (3H, t, J = 7.0 Hz), 4.08 (2H, q, J = 7.0 Hz), 4.39 (1H, t, J = 5.4 Hz), 4.68 (2H, d, J = 5.4 Hz), 6.77 (1H, d, J = 8.1 Hz), 6.95 (1H, dd, J = 1.8, 8.4 Hz), 7.03 (2H, d, J = 9.0 Hz), 7.26-7.33 (4H, m), 7.35 (2H, d, J = 8.1 Hz), 7.49 (1H, d, J = 2.1 Hz).
melting point: 211-212°C
Elemental analysis:
Calculated (%) for C₂₆H₂₈N₃OCl:C, 71.96; H, 6.50; N, 9.68.
Found (%):C, 71.87; H, 6.49; N, 9.50.

### Example 82

### 5-Chloro-N-(4-chlorobenzyl)-1-(4-ethoxyphenyl)-1H-benzimidazol-2-amine

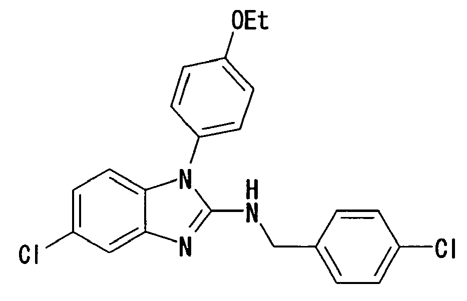

By the reaction in the same manner as in Example 50, the title compound (83 mg, yield 61%) was obtained as white crystals from the compound (100 mg, 0.33 mmol) of Reference Example 79 and p-chlorobenzylamine (0.5 mL).
¹H-NMR (300MHz, CDCl₃) δ: 1.46 (3H, t, J = 7.0 Hz), 4.08 (2H, q, J = 7.0 Hz), 4.43 (1H, t, J = 5.7 Hz), 4.68 (2H, d, J = 5.7 Hz), 6.78 (1H, d, J = 8.4 Hz), 6.96 (1H, dd, J = 1.8, 8.4 Hz), 7.04 (2H, d, J = 8.7 Hz), 7.26-7.30 (6H, m), 7.48 (1H, d, J = 1.8 Hz).
melting point: 109-110°C
Elemental analysis:
Calculated (%) for C₂₂H₁₉N₃OCl₂:C, 64.09; H, 4.64; N, 10.19. Found (%):C, 64.03; H, 4.70; N, 10.12.

### Example 83

### N-(4-tert-Butylbenzyl)-5-chloro-1-(4-propoxyphenyl)-1H-benzimidazol-2-amine

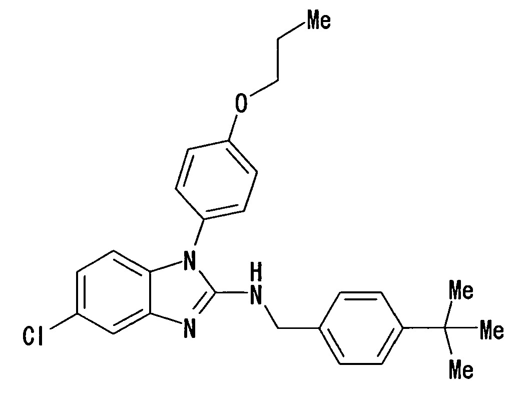

By the reaction in the same manner as in Example 50, the title compound (46 mg, yield 33%) was obtained as white crystals from the compound (100 mg, 0.31 mmol) of Reference Example 82 and 4-tert-butylbenzylamine (0.1 mL).
¹H-NMR (300MHz, CDCl₃) δ: 1.06 (3H, t, J = 7.4 Hz), 1.30 (9H, s), 1.79-1.88 (2H, m), 3.97 (2H, t, J = 6.5 Hz), 4.39 (1H, t, J = 5.4 Hz), 4.68 (2H, d, J = 5.4 Hz), 6.77 (1H, d, J = 8.4 Hz), 6.95 (1H, dd, J = 2.1, 9.3 Hz), 7.04 (2H, d, J = 9.0 Hz), 7.26-7.31 (4H, m), 7.35 (2H, d, J = 8.4 Hz), 7.49 (1H, d, J = 1.8 Hz).
melting point: 190-191°C
Elemental analysis:
Calculated (%) for C₂₇H₃₀N₃OCl:C, 72.39; H, 6.75; N, 9.38. Found (%):C, 72.31; H, 6.76; N, 9.22.

### Example 84

### N-(4-Bromobenzyl)-5-chloro-1-(4-propoxyphenyl)-1H-benzimidazol-2-amine

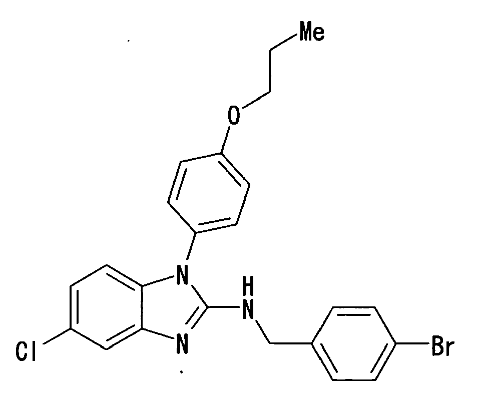

By the reaction in the same manner as in Example 50, the title compound (17 mg, yield 12%) was obtained as white crystals from the compound (100 mg, 0.31 mmol) of Reference Example 82 and p-bromobenzylamine (0.1 mL).
¹H-NMR (300MHz, CDCl₃) δ: 1.07 (3H, t, J = 7.4 Hz), 1.79-1.91 (2H, m), 3.97 (2H, t, J = 6.6 Hz), 4.43 (1H, t, J = 6.0 Hz), 4.66 (2H, d, J = 6.0 Hz), 6.79 (1H, d, J = 8.4 Hz), 6.96 (1H, d, J = 8.4 Hz), 7.05 (2H, d, J = 8.7 Hz), 7.21-7.33 (4H, m), 7.43-7.48 (3H, m).
melting point: 112-113°C
Elemental analysis:
Calculated (%) for C₂₃H₂₁N₃OBrCl:C, 58.68; H, 4.50; N, 8.93. Found (%):C, 58.57; H, 4.30; N, 8.84.

### Example 85

### 5-Chloro-N-(4-chlorobenzyl)-1-(4-propoxyphenyl)-1H-benzimidazol-2-amine

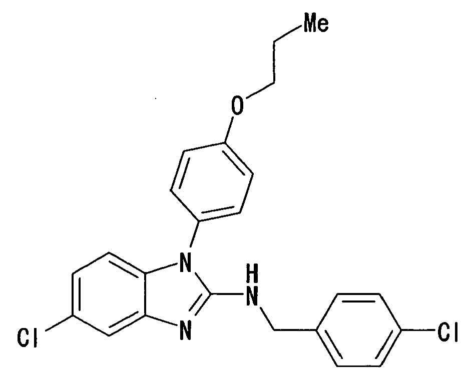

By the reaction in the same manner as in Example 50, the title compound (42 mg, yield 31%) was obtained as white crystals from the compound (100 mg, 0.31 mmol) of Reference Example 82 and p-chlorobenzylamine (0.1 mL).
¹H-NMR (300MHz, CDCl₃) δ: 1.07 (3H, t, J = 7.4 Hz), 1.80-1.90 (2H, m), 3.97 (2H, t, J = 6.5 Hz), 4.40-4.50 (1H, br), 4.68 (2H, d, J = 6.0 Hz), 6.78 (1H, d, J = 8.1 Hz), 6.96 (1H, dd, J = 1.8, 8.4 Hz), 7.05 (2H, d, J = 8.4 Hz), 7.27-7.29 (6H, m), 7.48 (1H, d, J = 2.1 Hz).
melting point: 122-123°C
Elemental analysis:
Calculated (%) for C₂₃H₂₁N₃OCl₂·0.3H₂O:C, 63.98; H, 5.04; N, 9.73. Found (%):C, 64.26; H, 4.87; N, 9.30.

### Example 86

### 1-(4-Butoxyphenyl)-N-(4-tert-butylbenzyl)-5-chloro-1H-benzimidazol-2-amine

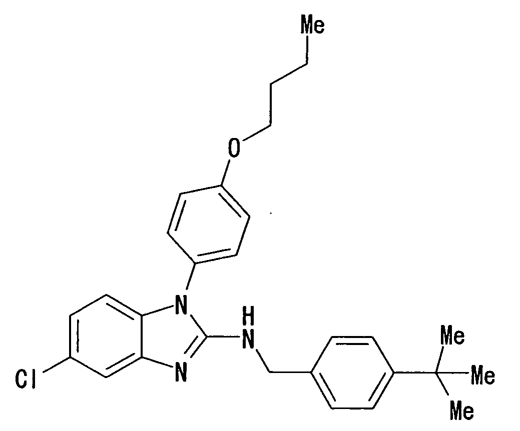

By the reaction in the same manner as in Example 50, the title compound (268 mg, yield 39%) was obtained as white crystals from the compound (500 mg, 1.49 mmol) of Reference Example 84 and 4-tert-butylbenzylamine (0.5 mL).
¹H-NMR (300MHz, CDCl₃) δ: 0.99 (3H, t, J = 7.4 Hz), 1.30 (9H, s), 1.45-1.55 (2H, m), 1.76-1.85 (2H, m), 4.01 (2H, t, J = 6.5 Hz), 4.39 (1H, t, J = 5.6 Hz), 4.68 (2H, d, J = 5.7 Hz), 6.78 (1H, d, J = 8.4 Hz), 6.95 (1H, dd, J = 1.8, 8.4 Hz), 7.05 (2H, d, J = 8.7 Hz), 7.26-7.30 (4H, m), 7.35 (2H, d, J = 8.4 Hz), 7.49 (1H, d, J = 1.8 Hz).
melting point: 158-159°C
Elemental analysis:
Calculated (%) for C₂₈H₃₂N₃OCl:C, 72.79; H, 6.98; N, 9.09.
Found (%):C, 72.69; H, 6.90; N, 8.83.

### Example 87

### 1-(4-Butoxyphenyl)-5-chloro-N-(4-chlorobenzyl)-1H-benzimidazol-2-amine

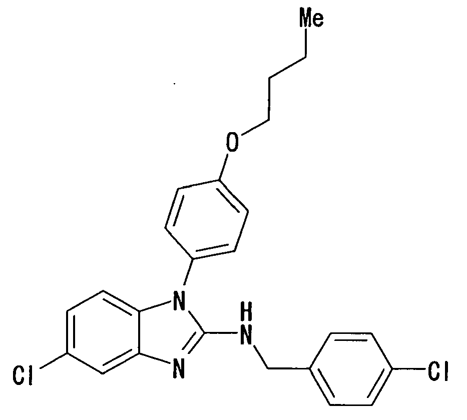

By the reaction in the same manner as in Example 50, the title compound (75 mg, yield 57%) was obtained as white crystals from the compound (100 mg, 0.30 mmol) of Reference Example 84 and p-chlorobenzylamine (0.3 mL).
¹H-NMR (300MHz, CDCl₃) δ: 1.00 (3H, t, J = 7.4 Hz), 1.46-1.57 (2H, m), 1.76-1.85 (2H, m), 4.01 (2H, t, J = 6.5 Hz), 4.42 (1H, t, J = 5.7 Hz), 4.68 (2H, d, J = 5.7 Hz), 6.79 (1H, d, J = 8.4 Hz), 6.96 (1H, dd, J = 2.1, 8.4 Hz), 7.05 (2H, d, J = 9.0 Hz), 7.26-7.31 (6H, m), 7.47 (1H, d, J = 2.1 Hz).
melting point: 100-101°C
Elemental analysis:
Calculated (%) for C₂₄H₂₃N₃OCl₂:C, 65.46; H, 5.26; N, 9.54.
Found (%):C, 65.35; H, 5.19; N, 9.41.

### Example 88

### N-(4-Bromobenzyl)-1-(4-butoxyphenyl)-5-chloro-1H-benzimidazol-2-amine

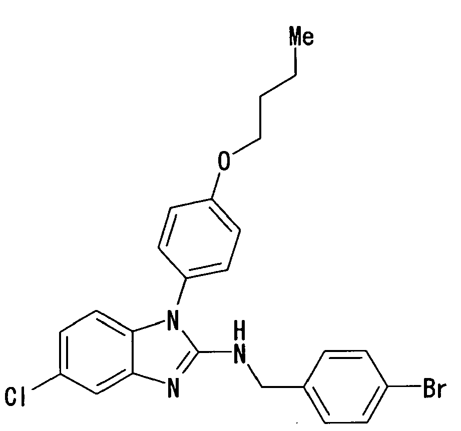

By the reaction in the same manner as in Example 50, the title compound (29 mg, yield 20%) was obtained as white crystals from the compound (100 mg, 0.30 mmol) of Reference Example 84 and p-bromobenzylamine (0.5 mL).
¹H-NMR (300MHz, CDCl₃) δ: 1.00 (3H, t, J = 7.4 Hz), 1.46-1.58 (2H, m), 1.76-1.86 (2H, m), 4.01 (2H, t, J = 6.5 Hz), 4.42 (1H, t, J = 6.0 Hz), 4.66 (2H, d, J = 6.0 Hz), 6.78 (1H, d, J = 8.4 Hz), 6.96 (1H, dd, J = 1.8, 8.4 Hz), 7.05 (2H, d, J = 9.0 Hz), 7.21-7.29 (4H, m), 7.43-7.48 (3H, m).
melting point: 95-96°C
Elemental analysis:
Calculated (%) for C₂₄H₂₃N₃OBrCl:C, 59.46; H, 4.78; N, 8.67. Found (%):C, 59.37; H, 4.56; N, 8.60.

### Example 89

### N-(4-tert-Butylbenzyl)-5-chloro-1-[4-(2,2,2-trifluoroethoxy)phenyl]-1H-benzimidazol-2-amine

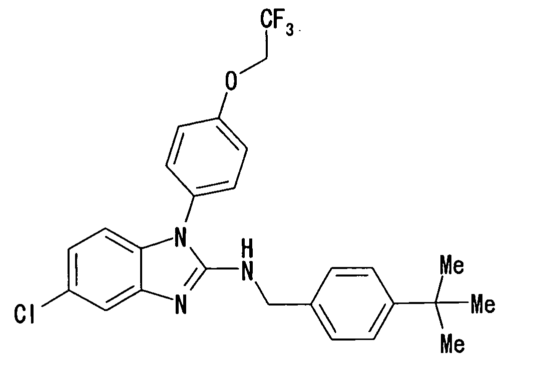

By the reaction in the same manner as in Example 50, the title compound (60 mg, yield 44%) was obtained as white crystals from the compound (100 mg, 0.28 mmol) of Reference Example 87 and 4-tert-butylbenzylamine (0.1 mL).
¹H-NMR (300MHz, CDCl₃) δ: 1.30 (9H, s), 4.34-4.46 (3H, m), 4.68 (2H, d, J = 5.7 Hz), 6.77 (1H, d, J = 8.4 Hz), 6.97 (1H, dd, J = 1.8, 8.4 Hz), 7.12 (2H, dd, J = 2.1, 6.9 Hz), 7.26-7.29 (3H, m), 7.35-7.38 (3H, m), 7.50 (1H, d, J = 1.8 Hz).
melting point: 190-191°C
Elemental analysis:
Calculated (%) for C₂₆H₂₅N₃OClF₃:C, 64.00; H, 5.16; N, 8.61. Found (%):C, 63.80; H, 5.21; N, 8.47.

### Example 90

### N-(4-Bromobenzyl)-5-chloro-1-[4-(2,2,2-trifluoroethoxy)phenyl]-1H-benzimidazol-2-amine

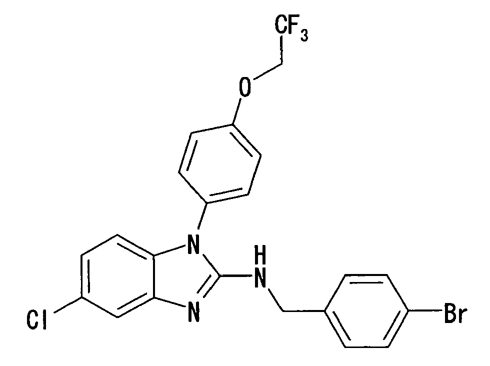

By the reaction in the same manner as in Example 50, the title compound (55 mg, yield 38%) was obtained as white crystals from the compound (100 mg, 0.28 mmol) of Reference Example 87 and p-bromobenzylamine (0.1 mL).
¹H-NMR (300MHz, CDCl₃) δ: 4.38-4.46 (3H, m), 4.66 (2H, d, J = 5.7 Hz), 6.78 (1H, d, J = 8.4 Hz), 6.97 (1H, dd, J = 1.8, 8.4 Hz), 7.13 (2H, d, J = 8.7 Hz), 7.21-7.26 (2H, m), 7.35 (2H, d, J = 8.7 Hz), 7.43-7.49 (3H, m).
melting point: 146-147°C
Elemental analysis:
Calculated (%) for C₂₂H₁₆N₃OBrClF₃:C, 51.74; H, 3.16; N, 8.23. Found (%):C, 51.70; H, 3.21; N, 8.11.

### Example 91

### 5-Chloro-N-(4-chlorobenzyl)-1-[4-(2,2,2-trifluoroethoxy)phenyl]-1H-benzimidazol-2-amine

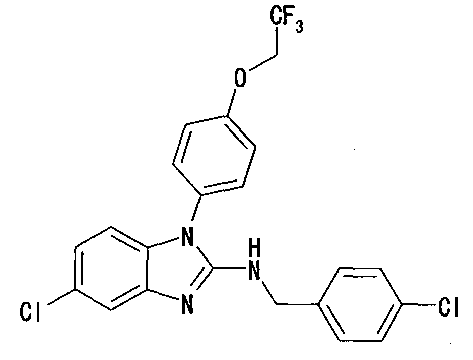

By the reaction in the same manner as in Example 50, the title compound (48 mg, yield 37%) was obtained as white crystals from the compound (100 mg, 0.28 mmol) of Reference Example 87 and p-chlorobenzylamine (0.1 mL).
¹H-NMR (300MHz, CDCl₃) δ: 4.38-4.46 (3H, m), 4.68 (2H, d, J = 6.0 Hz), 6.78 (1H, d, J = 8.4 Hz), 6.97 (1H, dd, J = 1.5, 8.4 Hz), 7.12 (2H, d, J = 8.7 Hz), 7.26-7.39 (6H, m), 7.49 (1H, d, J = 1.5 Hz).
melting point: 138-139°C
Elemental analysis:
Calculated (%) for C₂₂H₁₆N₃OCl₂F₃·0.5Et₂O:C, 57.27; H, 4.20; N, 8.35.
Found (%):C, 57.59; H, 3.94; N, 8.38.

### Example 92

### N-(4-tert-Butylbenzyl)-5,6-dichloro-1-(4-methoxyphenyl)-1H-benzimidazol-2-amine

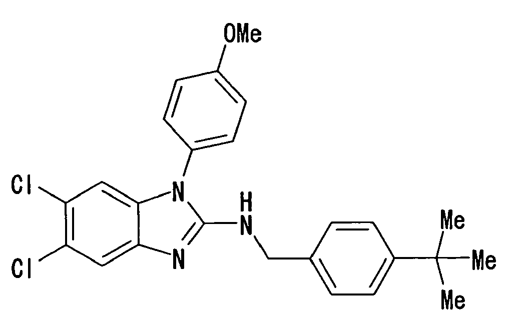

By the reaction in the same manner as in Example 50, the title compound (904 mg, yield 93%) was obtained as white crystals from the compound (700 mg, 2.14 mmol) of Reference Example 91 and 4-tert-butylbenzylamine (2 mL).
¹H-NMR (300MHz, CDCl₃) δ: 1.30 (9H, s), 3.87 (3H, s), 4.42 (1H, t, J = 5.4 Hz), 4.67 (2H, d, J = 5.4 Hz), 6.93 (1H, s), 7.05 (2H, d, J = 8.7 Hz), 7.26-7.37 (6H, m), 7.56 (1H, s).
melting point: 191-192°C

### Example 93

### N-(4-tert-Butylbenzyl)-5,6-dichloro-1-(4-ethoxyphenyl)-1H-benzimidazol-2-amine

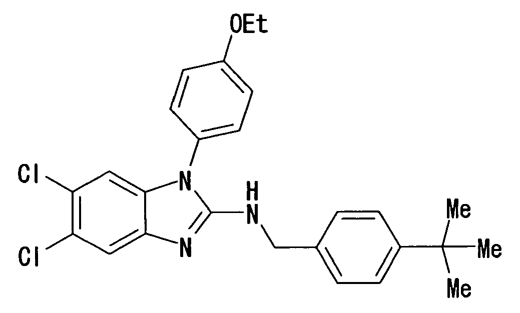

By the reaction in the same manner as in Example 50, the title compound (215 mg, yield 78%) was obtained as white crystals from the compound (200 mg, 0.59 mmol) of Reference Example 96 and 4-tert-butylbenzylamine (0.3 mL).
¹H-NMR (300MHz, CDCl₃) δ: 1.30 (9H, s), 1.46 (3H, t, J = 7.0 Hz), 4.08 (2H, q, J = 7.0 Hz), 4.43 (1H, t, J = 5.4 Hz), 4.66 (2H, d, J = 5.4 Hz), 6.93 (1H, s), 7.04 (2H, d, J = 8.7 Hz), 7.25-7.30 (4H, m), 7.35 (2H, d, J = 8.7 Hz), 7.56 (1H, s). melting point: 186-187°C
Elemental analysis:
Calculated (%) for C₂₆H₂₇N₃OCl₂:C, 66.67; H, 5.81; N, 8.97.
Found (%):C, 66.65; H, 5.72; N, 8.98.

### Example 94

### 5,6-Dichloro-N-(4-chlorobenzyl)-1-(4-ethoxyphenyl)-1H-benzimidazol-2-amine

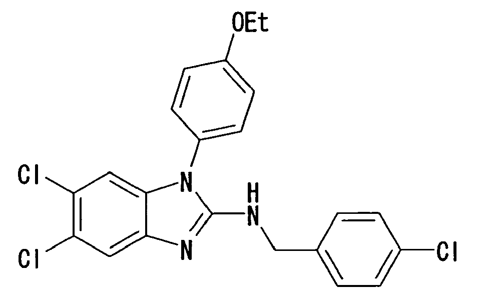

By the reaction in the same manner as in Example 50, the title compound (96 mg, yield 74%) was obtained as white crystals from the compound (100 mg, 0.29 mmol) of Reference Example 96 and p-chlorobenzylamine (0.2 mL).
¹H-NMR (300MHz, CDCl₃) δ: 1.47 (3H, t, J = 7.0 Hz), 4.09 (2H, q, J = 7.0 Hz), 4.46 (1H, t, J = 6.0 Hz), 4.67 (2H, d, J = 6.0 Hz), 6.91 (1H, s), 7.05 (2H, d, J = 8.7 Hz), 7.26-7.31 (6H, m), 7.55 (1H, s).
melting point: 141-142°C
Elemental analysis:
Calculated (%) for C₂₂H₁₈N₃OCl₃:C, 59.15; H, 4.06; N, 9.41.
Found (%):C, 59.44; H, 4.29; N, 9.04.

### Example 95

### N-(4-Bromobenzyl)-5,6-dichloro-1-(4-ethoxyphenyl)-1H-benzimidazol-2-amine

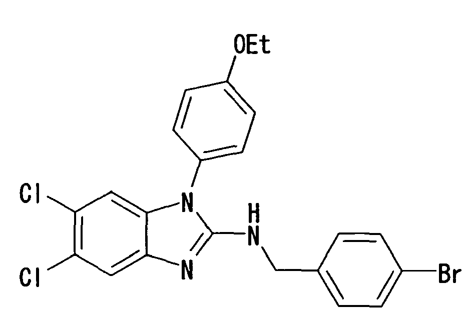

By the reaction in the same manner as in Example 50, the title compound (97 mg, yield 68%) was obtained as white crystals from the compound (100 mg, 0.29 mmol) of Reference Example 96 and p-bromobenzylamine (0.2 mL).
¹H-NMR (300MHz, CDCl₃) δ: 1.47 (3H, t, J = 7.0 Hz), 4.09 (2H, q, J = 7.0 Hz), 4.46 (1H, t, J = 6.0 Hz), 4.65 (2H, d, J = 6.0 Hz), 6.94 (1H, s), 7.05 (2H, d, J = 9.0 Hz), 7.17-7.29 (4H, m), 7.44 (2H, d, J = 9.0 Hz), 7.55 (1H, s).
melting point: 92-93°C
Elemental analysis:
Calculated (%) for C₂₂H₁₈N₃OBrCl₂·0.2Et₂O:C, 54.12; H, 3.98; N, 8.30.
Found (%):C, 54.29; H, 3.93; N, 8.30.

### Example 96

### N-(4-tert-Butylbenzyl)-5,6-dichloro-1-(4-propoxyphenyl)-1H-benzimidazol-2-amine

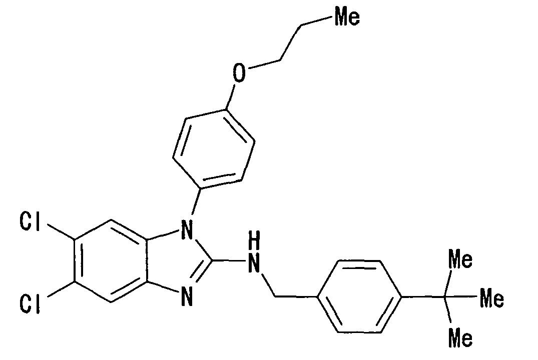

By the reaction in the same manner as in Example 50, the title compound (81 mg, yield 61%) was obtained as white crystals from the compound (100 mg, 0.28 mmol) of Reference Example 100 and 4-tert-butylbenzylamine (0.1 mL).
¹H-NMR (300MHz, CDCl₃) δ: 1.07 (3H, t, J = 7.5 Hz), 1.30 (9H, s), 1.79-1.91 (2H, m), 3.97 (2H, t, J = 6.5 Hz), 4.44 (1H, t, J = 5.4 Hz), 4.66 (2H, d, J = 5.4 Hz), 6.93 (1H, s), 7.04 (2H, d, J = 8.4 Hz), 7.25-7.30 (4H, m), 7.35 (2H, d, J = 8.4 Hz), 7.56 (1H, s).
melting point: 122-123°C
Elemental analysis:
Calculated (%) for C₂₇H₂₉N₃OCl₂:C, 67.22; H, 6.06; N, 8.71.
Found (%):C, 67.20; H, 6.04; N, 8.64.

### Example 97

### N-(4-Bromobenzyl)-5,6-dichloro-1-(4-propoxyphenyl)-1H-benzimidazol-2-amine

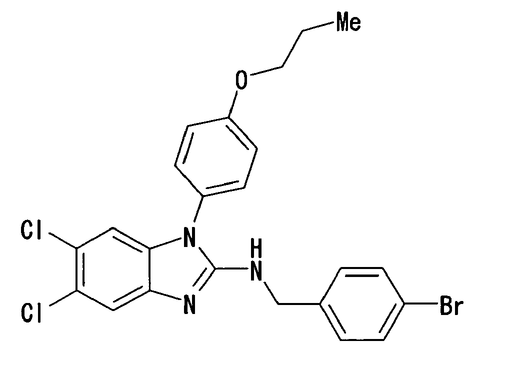

By the reaction in the same manner as in Example 50, the title compound (47 mg, yield 33%) was obtained as white crystals from the compound (100 mg, 0.28 mmol) of Reference Example 100 and p-bromobenzylamine (0.1 mL).
¹H-NMR (300MHz, CDCl₃) δ: 1.07 (3H, t, J = 7.4 Hz), 1.82-1.89 (2H, m), 3.98 (2H, t, J = 6.6 Hz), 4.47 (1H, t, J = 6.0 Hz), 4.65 (2H, d, J = 6.0 Hz), 6.94 (1H, s), 7.07 (2H, d, J = 8.4 Hz), 7.20-7.28 (4H, m), 7.44 (2H, d, J = 8.4 Hz), 7.55 (1H, s).
melting point: 106-107°C
Elemental analysis:
Calculated (%) for C₂₃H₂₀N₃OBrCl₂·0.5Et₂O:C, 55.37; H, 4.65; N, 7.75.
Found (%):C, 55.79; H, 4.47; N, 7.90.

### Example 98

### 5,6-Dichloro-N-(4-chlorobenzyl)-1-(4-propoxyphenyl)-1H-benzimidazol-2-amine

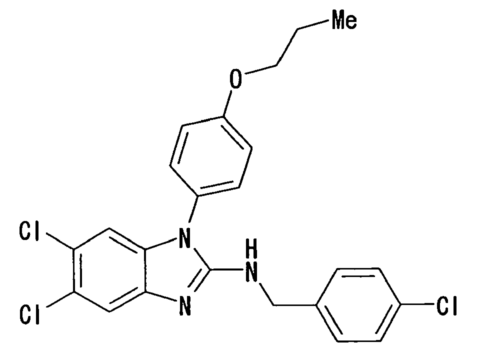

By the reaction in the same manner as in Example 50, the title compound (83 mg, yield 64%) was obtained as white crystals from the compound (100 mg, 0.28 mmol) of Reference Example 100 and p-chlorobenzylamine (0.1 mL).
¹H-NMR (300MHz, CDCl₃) δ: 1.07 (3H, t, J = 7.4 Hz), 1.80-1.91 (2H, m), 3.98 (2H, t, J = 6.5 Hz), 4.47 (1H, t, J = 5.6 Hz), 4.67 (2H, d, J = 5.7 Hz), 6.94 (1H, s), 7.06 (2H, d, J = 8.4 Hz), 7.26-7.31 (6H, m), 7.55 (1H, s). melting point: 166-167°C
Elemental analysis:
Calculated (%) for C₂₃H₂₀N₃OCl₃·0.5Et₂O:C, 60.31; H, 5.06; N, 8.44.
Found (%):C, 60.79; H, 4.81; N, 8.65.

### Example 99

### 1-(4-Butoxyphenyl)-N-(4-tert-butylbenzyl)-5,6-dichloro-1H-benzimidazol-2-amine

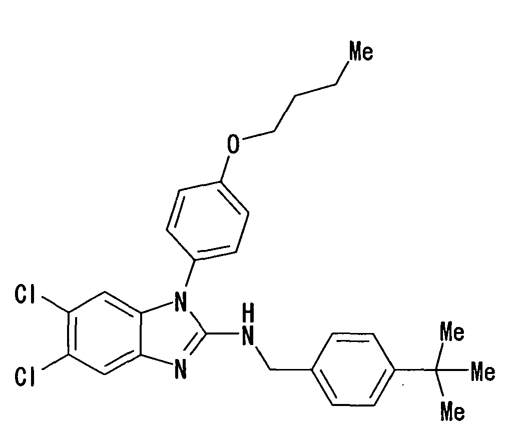

By the reaction in the same manner as in Example 50, the title compound (478 mg, yield 71%) was obtained as a pale-brown amorphous solid from the compound (500 mg, 1.35 mmol) of Reference Example 103 and 4-tert-butylbenzylamine (1 mL).
¹H-NMR (300MHz, CDCl₃) δ: 1.00 (3H, t, J = 7.4 Hz), 1.30 (9H, s), 1.46-1.55 (2H, m), 1.76-1.85 (2H, m), 4.01 (2H, t, J = 6.3 Hz), 4.43 (1H, t, J = 5.4 Hz), 4.66 (2H, d, J = 5.4 Hz), 6.93 (1H, s), 7.04 (2H, d, J = 8.7 Hz), 7.26-7.28 (4H, m), 7.35 (2H, d, J = 8.1 Hz), 7.56 (1H, s).
Elemental analysis:
Calculated (%) for C₂₈H₃₁N₃OCl₂:C, 67.74; H, 6.29; N, 8.46.
Found (%):C, 67.47; H, 6.31; N, 8.34.

### Example 100

### 1-(4-Butoxyphenyl)-5,6-dichloro-N-(4-bromobenzyl)-1H-benzimidazol-2-amine

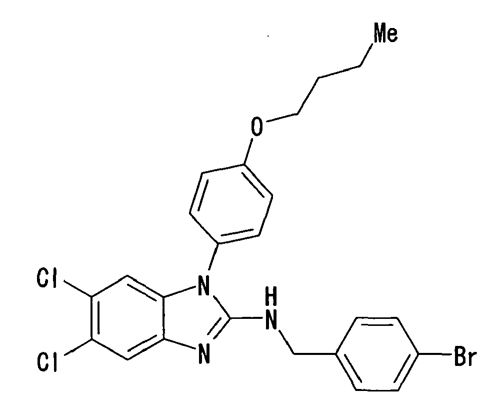

A mixture of the compound (100 mg, 0.27 mmol) of Reference Example 103, p-bromobenzylamine hydrochloride (602 mg, 2.71 mmol) and isopropylethylamine (350 mg, 2.71 mmol) in DMF (1 mL) was stirred at 120°C for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/3) and recrystallized from diethyl ether to give the title compound (22 mg, yield 16%) as pale-brown crystals.
¹H-NMR (300MHz, CDCl₃) δ: 1.00 (3H, t, J = 7.4 Hz), 1.46-1.59 (2H, m), 1.77-1.86 (2H, m), 4.02 (2H, t, J = 6.6 Hz), 4.46 (1H, t, J = 5.9 Hz), 4.65 (2H, d, J = 5.9 Hz), 6.94 (1H, s), 7.06 (2H, d, J = 9.0 Hz), 7.17-7.28 (4H, m), 7.45 (2H, d, J = 8.4 Hz), 7.55 (1H, s). melting point: 161-162°C
Elemental analysis:
Calculated (%) for C₂₄H₂₂N₃OBrCl₂:C, 55.51; H, 4.27; N, 8.09. Found (%):C, 55.73; H, 4.25; N, 7.99.

### Example 101

### 1-(4-Butoxyphenyl)-5,6-dichloro-N-(4-chlorobenzyl)-1H-benzimidazol-2-amine

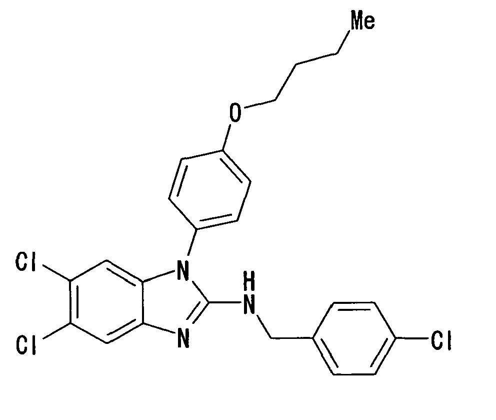

By the reaction in the same manner as in Example 50, the title compound (24 mg, yield 19%) was obtained as white crystals from the compound (100 mg, 0.27 mmol) of Reference Example 103 and p-chlorobenzylamine (0.5 mL).
¹H-NMR (300MHz, CDCl₃) δ: 1.00 (3H, t, J = 7.4 Hz), 1.46-1.58 (2H, m), 1.77-1.86 (2H, m), 4.02 (2H, t, J = 6.5 Hz), 4.46 (1H, t, J = 5.4 Hz), 4.67 (2H, d, J = 5.4 Hz), 6.94 (1H, s), 7.06 (2H, d, J = 8.7 Hz), 7.25-7.31 (6H, m), 7.55 (1H, s). melting point: 162-163°C
Elemental analysis:
Calculated (%) for C₂₄H₂₂N₃OCl₃:C, 60.71; H, 4.67; N, 8.85.
Found (%):C, 60.63; H, 4.57; N, 8.72.

### Example 102

### N-(4-tert-Butylbenzyl)-5,6-dichloro-1-[4-(2,2,2-trifluoroethoxy)phenyl]-1H-benzimidazol-2-amine

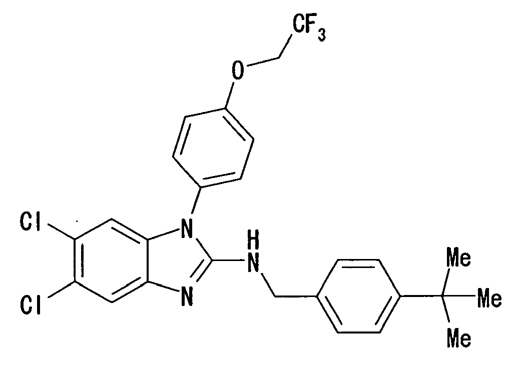

By the reaction in the same manner as in Example 50, the title compound (73 mg, yield 55%) was obtained as white crystals from the compound (100 mg, 0.25 mmol) of Reference Example 107 and 4-tert-butylbenzylamine (0.1 mL).
¹H-NMR (300MHz, CDCl₃) δ: 1.30 (9H, s), 4.38-4.46 (3H, m), 4.66 (2H, d, J = 5.7 Hz), 6.93 (1H, s), 7.12 (2H, d, J = 9.0 Hz), 7.26-7.28 (3H, m), 7.33-7.38 (3H, m), 7.57 (1H, s). melting point: 136-137°C
Elemental analysis:
Calculated (%) for C₂₆H₂₄N₃OCl₂F₃:C, 59.78; H, 4.63; N, 8.04. Found (%):C, 60.04; H, 4.89; N, 7.74.

### Example 103

### N-(4-Bromobenzyl)-5,6-dichloro-1-[4-(2,2,2-trifluoroethoxy)phenyl]-1H-benzimidazol-2-amine

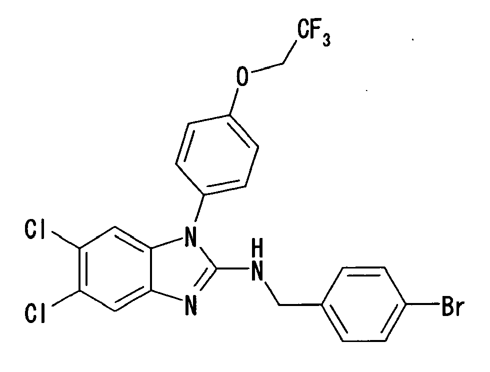

By the reaction in the same manner as in Example 50, the title compound (86 mg, yield 63%) was obtained as white crystals from the compound (100 mg, 0.25 mmol) of Reference Example 107 and p-bromobenzylamine (0.1 mL).
¹H-NMR (300MHz, CDCl₃) δ: 4.39-4.47 (3H, m), 4.65 (2H, d, J = 5.7 Hz), 6.94 (1H, s), 7.13-7.26 (4H, m), 7.35 (2H, d, J = 8.4 Hz), 7.45 (2H, d, J = 8.4 Hz), 7.56 (1H, s). melting point: 118-120°C
Elemental analysis:
Calculated (%) for C₂₂H₁₅N₃OBrCl₂F₃·0.3Et₂O:C, 49.11; H, 3.19; N, 7.40.
Found (%):C, 49.34; H, 3.15; N, 7.43.

### Example 104

### 5,6-Dichloro-N-(4-chlorobenzyl)-1-[4-(2,2,2-trifluoroethoxy)phenyl]-1H-benzimidazol-2-amine

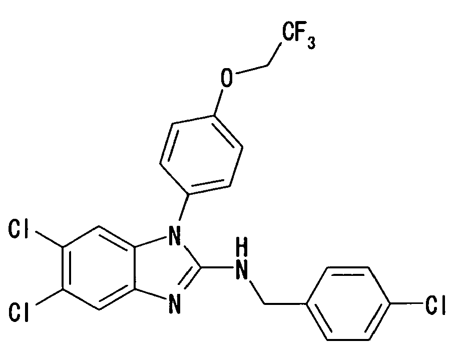

By the reaction in the same manner as in Example 50, the title compound (92 mg, yield 73%) was obtained as white crystals from the compound (100 mg, 0.25 mmol) of Reference Example 107 and p-chlorobenzylamine (0.1 mL).
¹H-NMR (300MHz, CDCl₃) δ: 4.39-4.47 (3H, m), 4.67 (2H, d, J = 5.7 Hz), 6.94 (1H, s), 7.14 (2H, d, J = 8.4 Hz), 7.26-7.38 (6H, m), 7.56 (1H, s). melting point: 138-139°C
Elemental analysis:
Calculated (%) for C₂₂H₁₅N₃OCl₃F₃:C, 52.77; H, 3.02; N, 8.39. Found (%) :C, 52.70; H, 3.26; N, 8.21.

### Example 105

### N-(4-tert-Butylbenzyl)-5,6-dichloro-1-[4-(trifluoromethoxy)phenyl]-1H-benzimidazol-2-amine

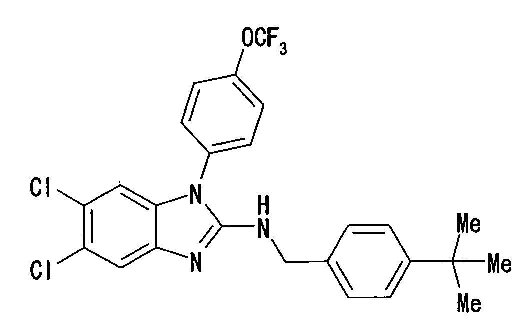

By the reaction in the same manner as in Example 50, the title compound (43 mg, yield 32%) was obtained as white crystals from the compound (100 mg, 0.26 mmol) of Reference Example 109 and 4-tert-butylbenzylamine (0.1 mL).
¹H-NMR (300MHz, CDCl₃) δ: 1.29 (9H, s), 4.40-4.43 (1H, br), 4.67 (2H, d, J = 5.7 Hz), 6.98 (1H, s), 7.12 (2H, d, J = 9.0 Hz), 7.26-7.30 (3H, m), 7.34-7.39 (3H, m), 7.60 (1H, s).
melting point: 158-159°C
Elemental analysis:
Calculated (%) for C₂₅H₂₂N₃OCl₂F₃:C, 59.07; H, 4.36; N, 8.27. Found (%):C, 59.13; H, 4.30; N, 8.27.

### Example 106

### N-(4-tert-Butylbenzyl)-6-chloro-1-[4-(1,1,2,2-tetrafluoroethoxy)phenyl]-1H-benzimidazol-2-amine hydrochloride

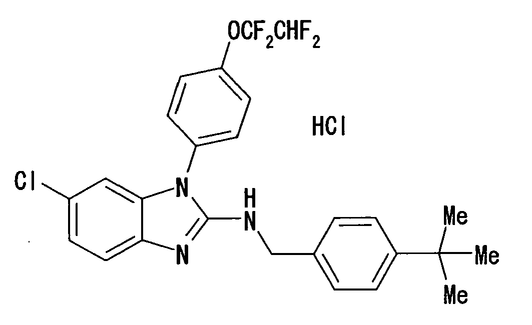

By the reaction in the same manner as in Reference Example 26, 6-chloro-1-[4-(1,1,2,2-tetrafluoroethoxy)phenyl]-1,3-dihydro-2H-benzimidazol-2-one (50 mg) was obtained as a pale-brown powder from 4-chloro-N²-[4-(1,1,2,2-tetrafluoroethoxy)phenyl]benzene-1,2-diamine (330 mg) obtained in the production process of Reference Example 111.

By the reaction in the same manner as in Reference Example 27, 2,6-dichloro-1-[4-(1,1,2,2-tetrafluoroethoxy)phenyl]-1H-benzimidazole (70 mg) was obtained as a colorless oil from 6-chloro-1-[4-(1,1,2,2-tetrafluoroethoxy)phenyl]-1,3-dihydro-2H-benzimidazol-2-one (50 mg, 0.14 mmol).

A mixture of 2,6-dichloro-1-[4-(1,1,2,2-tetrafluoroethoxy)phenyl]-1H-benzimidazole (70 mg, 0.18 mmol) and 4-tert-butylbenzylamine (0.1 mL) was stirred at 120°C for 17 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/3) to give N-(4-tert-butylbenzyl)-6-chloro-1-[4-(1,1,2,2-tetrafluoroethoxy)phenyl]-1H-benzimidazol-2-amine (60 mg) as a pale-brown oil.

To a solution of the oil in ethyl acetate (5 mL) was added a 4N hydrogen chloride-ethyl acetate solution (0.5 mL), and the mixture was concentrated under reduced pressure. The obtained solid was recrystallized from ether-hexane to give the title compound (20 mg, yield 21%) as a white powder.
¹H-NMR (300MHz, CDCl₃) δ: 1.31 (9H, s), 4.83 (2H, brs), 6.00(1H, tt, J = 2.4, 53.0 Hz), 6.85 (1H, d, J = 1.5 Hz), 7.26-7.32 (6H, m), 7.37-7.45 (4H, m), 7.67 (1H, d, J = 8.7 Hz), 14.80-15.40 (1H, br). melting point: 130-133°C
Elemental analysis:
Calculated (%) for C₂₆H₂₄N₃OClF₄·HCl:C, 57.58; H, 4.65; N, 7.75. Found (%):C, 57.31; H, 4.93; N, 7.50.

### Example 107

### Ethyl 2-[(4-chlorobenzyl)thio]-1-(4-ethoxyphenyl)-1H-benzimidazole-5-carboxylate

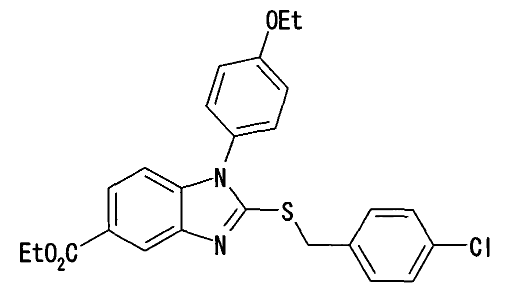

A solution of the compound (900 mg, 2.34 mmol) of Reference Example 114, p-chlorobenzyl chloride (451 mg, 2.80 mmol) and potassium carbonate (483 mg, 3.50 mmol) in DMF (20 mL) was stirred at room temperature for 18 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) to give the title compound (850 mg) as a white solid.

The obtained solid was recrystallized from ether-hexane to give the title compound (729 mg, yield 67%) as white crystals.
¹H-NMR (300MHz, CDCl₃) δ:1.40-1.58 (6H, m), 4.09 (2H, q, J = 7.0 Hz), 4.41 (2H, q, J = 7.1 Hz), 4.57 (2H, s), 7.01-7.10 (3H, m), 7.24-7.28 (4H, m), 7.36 (2H, d, J = 8.4 Hz), 7.91 (1H, dd, J = 1.5, 8.4 Hz), 8.45 (1H, d, J = 1.5 Hz). melting point: 133-134°C
Elemental analysis:
Calculated (%) for C₂₅H₂₃N₂O₃SCl:C, 64.30; H, 4.96; N, 6.00. Found (%) :C, 64.25; H, 4.76; N, 5.87.

### Example 108

### 5,6-Dichloro-2-[(4-chlorobenzyl)thio]-1-(4-methoxyphenyl)-1H-benzimidazole

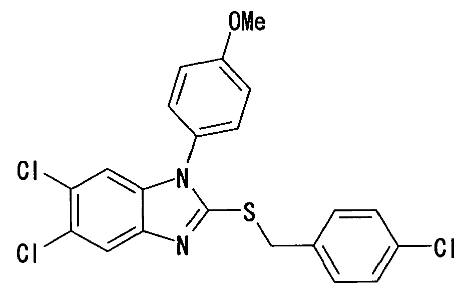

By the reaction in the same manner as in Example 8, the title compound (1.30 g, yield 63%) was obtained as white crystals from the compound (1.50 g, 4.61 mmol) of Reference Example 92 and p-chlorobenzyl chloride (817 mg, 5.07 mmol).
¹H-NMR (300MHz, CDCl₃) δ:3.88 (3H, s), 4.53 (2H, s), 7.03 (2H, d, J = 9.0 Hz), 7.15 (1H, s), 7.22-7.27 (4H, m), 7.34 (2H, d, J = 8.4 Hz), 7.79 (1H, s). melting point: 141-142°C
Elemental analysis:
Calculated (%) for C₂₁H₁₅N₂OSCl₃:C, 56.08; H, 3.36; N, 6.23; Cl, 23.65.
Found (%):C, 56.12; H, 3.37; N, 6.19; C1, 23.45.

### Example 109

### 4-{5,6-Dichloro-2-[(4-chlorobenzyl)thio]-1H-benzimidazol-1-yl}phenol

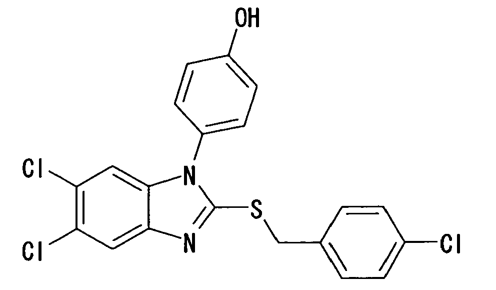

By the reaction in the same manner as in Example 44, the title compound (660 mg, yield 56 %) was obtained as a white powder from the compound (1.20 g, 2.67 mmol) of Example 108.
¹H-NMR (300MHz, CDCl₃) δ:4.62 (2H, s), 7.04 (2H, d, J = 9.0 Hz), 7.14-7.16 (3H, m), 7.25 (2H, d, J = 8.4 Hz), 7.26-7.36 (2H, m), 7.78 (1H, d, J = 2.7 Hz), 9.33 (1H, br s).

### Example 110

### 5,6-Dichloro-2-[(4-chlorobenzyl)thio]-1-(4-isopropoxyphenyl)-1H-benzimidazole

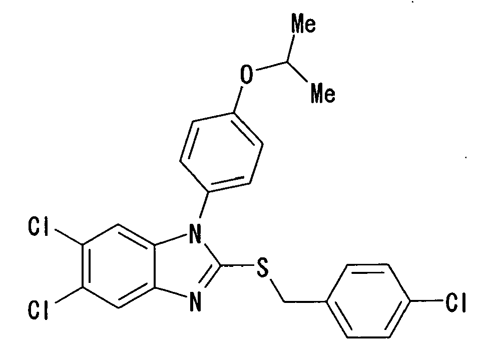

By the reaction in the same manner as in Example 10, the title compound (70 mg, yield 64%) was obtained as white crystals from the compound (100 mg, 0.23 mmol) of Example 109 and isopropyl bromide (282 mg, 2.29 mmol).
¹H-NMR (300MHz, CDCl₃) δ:1.39 (6H, d, J = 6.0 Hz), 4.53 (2H, s), 4.56-4.64 (1H, m), 6.99 (2H, d, J = 9.0 Hz), 7.16-7.27 (5H, m), 7.34 (2H, d, J = 8.4 Hz), 7.79 (1H, s). melting point: 149-150°C
Elemental analysis:
Calculated (%) for C₂₃H₁₉N₂OSCl₃:C, 57.81; H, 4.01; N, 5.86. Found (%):C, 57.74; H, 3.90; N, 5.75.

### Example 111

### 5,6-Dichloro-2-[(4-chlorobenzyl)thio]-1-(4-ethoxyphenyl)-1H-benzimidazole

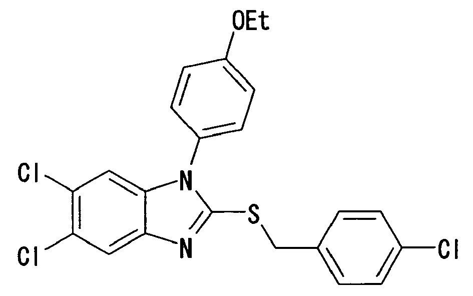

By the reaction in the same manner as in Example 10, the title compound (57 mg, yield 53%) was obtained as white crystals from the compound (100 mg, 0.23 mmol) of Example 109 and ethyl iodide (358 mg, 2.29 mmol).
¹H-NMR (300MHz, CDCl₃) δ:1.46 (3H, t, J = 7.0 Hz), 4.09 (2H, q, J = 7.0 Hz), 4.53 (2H, s), 6.99-7.06 (2H, m), 7.15 (1H, s), 7.20-7.27 (4H, m), 7.33 (2H, d, J = 8.4 Hz), 7.79 (1H, s).
melting point: 131-132°C
Elemental analysis:
Calculated (%) for C₂₂H₁₇N₂OSCl₃:C, 56.97; H, 3.69; N, 6.04. Found (%):C, 56.99; H, 3.73; N, 6.10.

### Example 112

### 5,6-Dichloro-2-[(4-chlorobenzyl)thio]-1-(4-propoxyphenyl)-1H-benzimidazole

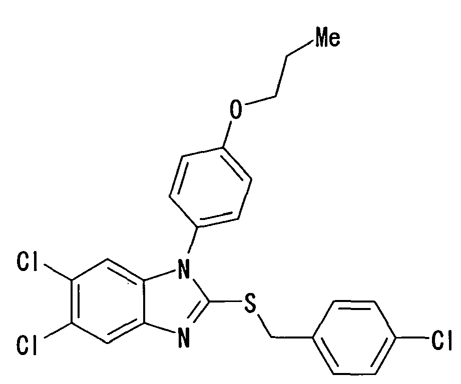

By the reaction in the same manner as in Example 10, the title compound (67 mg, yield 61%) was obtained as white crystals from the compound (100 mg, 0.23 mmol) of Example 109 and n-propyl iodide (390 mg, 2.29 mmol).
¹H-NMR (300MHz, CDCl₃) δ:1.07 (3H, t, J = 7.4 Hz), 1.80-1.91 (2H, m), 3.98 (2H, t, J = 6.5 Hz), 4.53 (2H, s), 7.02 (2H, d, J = 8.7 Hz), 7.15 (1H, s), 7.20-7.27 (4H, m), 7.34 (2H, d, J = 8.7 Hz), 7.79 (1H, s). melting point: 119-120°C
Elemental analysis:
Calculated (%) for C₂₃H₁₉N₂OSCl₃:C, 57.81; H, 4.01; N, 5.86. Found (%) :C, 57.88; H, 3.99; N, 5.87.

### Example 113

### 1-(4-Butoxyphenyl)-5,6-dichloro-2-[(4-chlorobenzyl)thio]-1H-benzimidazole

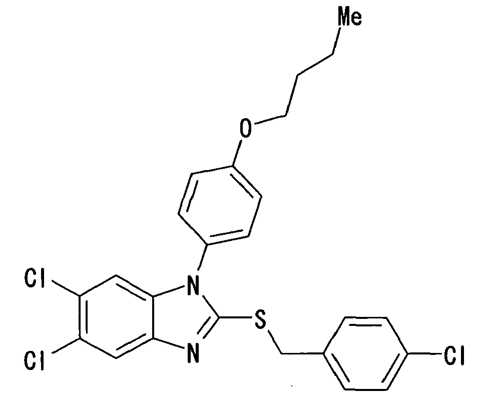

By the reaction in the same manner as in Example 10, the title compound (37 mg, yield 33%) was obtained as white crystals from the compound (100 mg, 0.23 mmol) of Example 109 and n-butyl iodide (422 mg, 2.29 mmol).
¹H-NMR (300MHz, CDCl₃) δ:1.00 (3H, t, J = 7.4 Hz), 1.96-1.58 (2H, m), 1.78-1.86 (2H, m), 4.02 (2H, t, J = 6.5 Hz), 4.53 (2H, s), 7.01 (2H, d, J = 9.0 Hz), 7.15 (1H, s), 7.20-7.27 (4H, m), 7.34 (2H, dd, J = 2.1, 8.7 Hz), 7.79 (1H, s). melting point: 99-100°C
Elemental analysis:
Calculated (%) for C₂₉H₂₁N₂OSCl₃:C, 58.61; H, 4.30; N, 5.70. Found (%):C, 58.69; H, 4.30; N, 5.71.

### Example 114

### tert-Butyl (4-{6-chloro-2-[(4-chlorobenzyl)thio]-1H-benzimidazol-1-yl}phenyl)carbamate

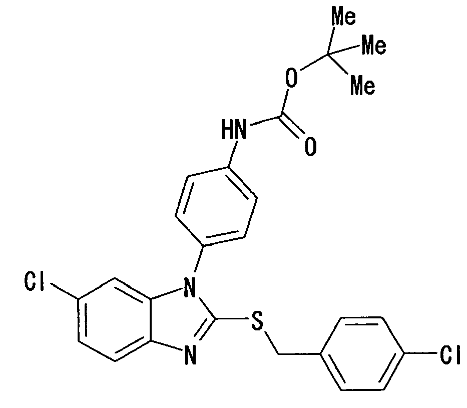

A mixed solution of the compound (150 mg, 0.35 mmol) of Example 42, diphenylphosphoryl azide (99 mg, 0.36 mmol) and triethylamine (35 mg, 0.35 mmol) in tert-butanol (5 mL) was stirred at 120°C for 16 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/3) to give the title compound (50 mg) as a colorless oil.

The obtained oil was recrystallized from ether-hexane to give the title compound (44 mg, yield 25%) as white crystals.
¹H-NMR (300MHz, CDCl₃) δ:1.54 (9H, s), 4.53 (2H, s), 6.63 (1H, s), 7.07 (1H, d, J = 1.8 Hz), 7.19-7.35 (7H, m), 7.54 (2H, d, J = 8.7 Hz), 7.61 (1H, d, J = 8.7 Hz). melting point: 163-164°C
Elemental analysis:
Calculated (%) for C₂₅H₂₃N₃O₂SCl₂:C, 60.00; H, 4.63; N, 8.40. Found (%) :C, 59.95; H, 4.67; N, 8.38.

### Example 115

### (4-{6-Chloro-2-[(4-chlorobenzyl)thio]-1H-benzimidazol-1-yl}phenyl)methanol

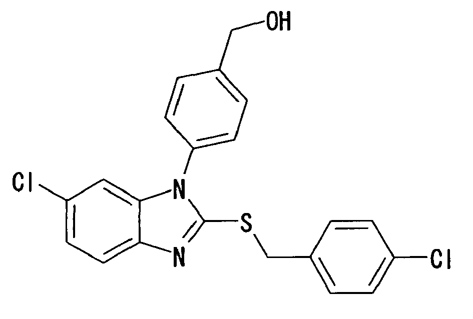

The compound (300 mg, 0.66 mmol) of Example 19 was added to a suspension of lithium aluminiumhydride (25 mg, 0.66 mmol) in THF (20 mL) under ice-cooling, and the mixture was stirred for 1 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/2) and recrystallized from ether-hexane to give the title compound (232 mg, yield 85%) as white crystals.
¹H-NMR (300MHz, CDCl₃) δ:1.86 (1H, t, J = 5.9 Hz), 4.55 (2H, s), 4.81 (2H, d, J = 5.7 Hz), 7.09 (1H, d, J = 2.1 Hz), 7.21-7.26 (3H, m), 7.32-7.37 (4H, m), 7.55 (2H, d, J = 8.4 Hz), 7.63 (1H, d, J = 8.4 Hz). melting point: 121-122°C
Elemental analysis:
Calculated (%) for C₂₁H₁₆N₂OSCl₂:C, 60.73; H, 3.88; N, 6.74. Found (%) :C, 60.81; H, 3.89; N, 6.66.

### Example 116

### 6-Chloro-2-[(4-chlorobenzyl)thio]-1-[4-(methoxymethyl)phenyl]-1H-benzimidazole

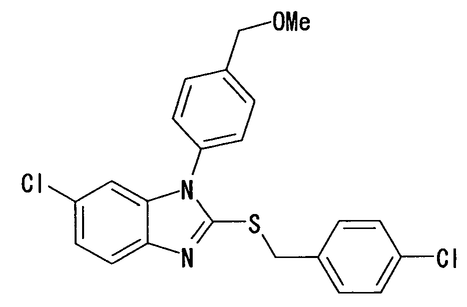

To a solution of the compound (80 mg, 0.19 mmol) of Example 115 in DMF (4 mL) was added 60% sodium hydride (12 mg, 0.29 mmol) in oil under ice-cooling, and the mixture was stirred for 30 min. To the mixture was added methyl iodide (120 µL, 1.93 mmol), and the mixture was stirred at room temperature for 18 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) and recrystallized from ether-hexane to give the title compound (31 mg, yield 38%) as white crystals.
¹H-NMR (300MHz, CDCl₃) δ:3.47 (3H, s), 4.54 (4H, s), 7.09 (1H, d, J = 1.8 Hz), 7.20-7.26 (3H, m), 7.34 (4H, d, J = 9.3 Hz), 7.52 (2H, d, J = 8.4 Hz), 7.63 (1H, d, J = 8.7 Hz). melting point: 97-98°C
Elemental analysis:
Calculated (%) for C₂₂H₁₈N₂OSCl₂·0.1H₂O:C, 61.29; H, 4.25; N, 6.50.
Found (%) :C, 61.18; H, 4.25; N, 6.50.

### Example 117

### 6-Chloro-2-[(4-chlorobenzyl)thio]-1-[4-(ethoxymethyl)phenyl]-1H-benzimidazole

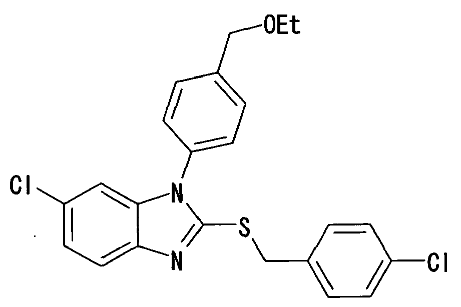

By the reaction in the same manner as in Example 116, the title compound (24 mg, yield 28%) was obtained as white crystals from the compound (80 mg, 0.19 mmol) of Example 115 and ethyl iodide (154 µL, 1.93 mmol).
¹H-NMR (300MHz, CDCl₃) δ:1.30 (3H, t, J = 7.0 Hz), 3.62 (2H, q, J = 7.0 Hz), 4.54 (2H, s), 4.59 (2H, s), 7.09 (1H, d, J = 1.8 Hz), 7.20-7.26 (3H, m), 7.33 (4H, d, J = 7.5 Hz), 7.52 (2H, d, J = 8.4 Hz), 7.63 (1H, d, J = 8.7 Hz). melting point: 95-96°C
Elemental analysis:
Calculated (%) for C₂₃H₂₀N₂OSCl₂:C, 62.30; H, 4.55; N, 6.32. Found (%):C, 62.00; H, 4.53; N, 6.25.

### Example 118

### 6-Chloro-2-[(4-chlorobenzyl)thio]-1-[4-(phenoxymethyl)phenyl]-1H-benzimidazole

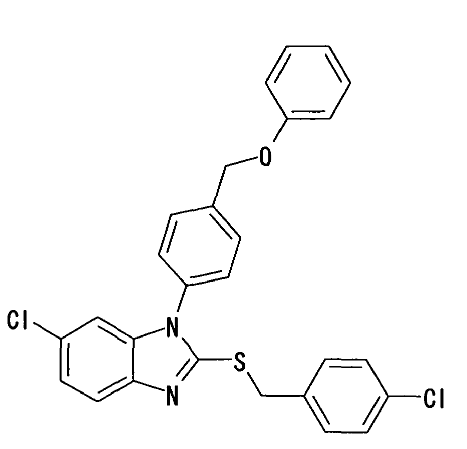

To a solution of the compound (100 mg, 0.24 mmol) of Example 115, phenol (25 mg, 0.26 mmol) and triphenylphosphine (69 mg, 0.26 mmol) in THF (10 mL) was added diethyl azodicarboxylate (46 mg, 0.26 mmol), and the mixture was stirred at room temperature for 24 hr. To the reaction mixture was added saturated aqueous sodium hydrogencarbonate, and the mixture was extracted twice with ethyl acetate. The organic layers were combined, washed with saturated brine, dried over magnesium sulfate and concentrated under reduced pressure.

The residue was purified by silica gel chromatography (eluent; ethyl acetate/hexane=1/5) and recrystallized from ether-hexane to give the title compound (44 mg, yield 38%) as white crystals.
¹H-NMR (300MHz, CDCl₃) δ: 4.55 (2H, s), 5.15 (2H, s), 6.98-7.03 (3H, m), 7.12 (1H, d, J = 1.8 Hz), 7.21-7.41 (9H, m), 7.61-7.65 (3H, m).
melting point: 163-164°C

### Example 119

### 1-[4-(Benzyloxy)phenyl]-6-chloro-2-[(4-chlorobenzyl)thio]-1H-benzimidazole

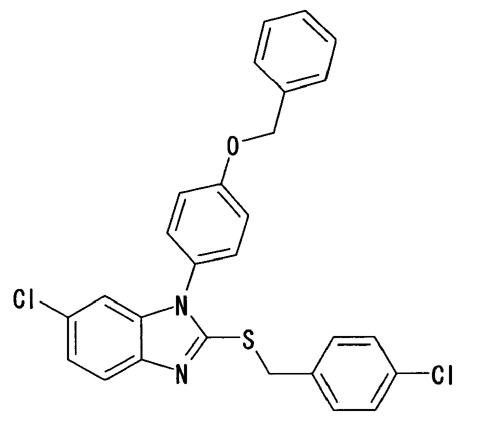

By the reaction in the same manner as in Example 10, the title compound (76 mg, yield 62%) was obtained as white crystals from the compound (100 mg, 0.23 mmol) of Example 9 and benzyl bromide (85 mg, 0.50 mmol).
¹H-NMR (300MHz, CDCl₃) δ: 4.53 (2H, s), 5.12 (2H, s), 7.05-7.13 (3H, m), 7.18-7.26 (5H, m), 7.31-7.48 (7H, m), 7.62 (1H, d, J = 8.4 Hz). melting point: 193-194°C
Elemental analysis:
Calculated (%) for C₂₇H₂₀N₂OSCl₂:C, 65.99; H, 4.10; N, 5.70. Found (%):C, 65.85; H, 4.18; N, 5.67.

### Example 120

### 6-Chloro-2-[(4-chlorobenzyl)thio]-1-[4-(2,2,2-trifluoroethoxy)phenyl]-1H-benzimidazole hydrochloride

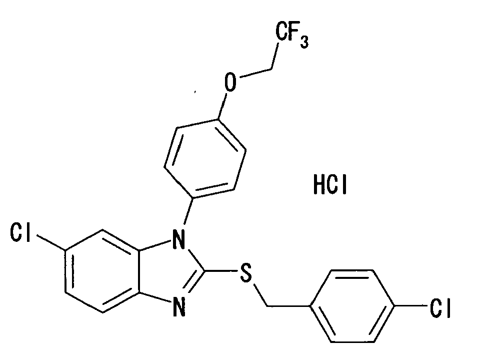

By the reaction in the same manner as in Example 10, 6-chloro-2-[(4-chlorobenzyl)thio]-1-[4-(2,2,2-trifluoroethoxy)phenyl]-1H-benzimidazole (80 mg) was obtained as a colorless oil from the compound (100 mg, 0.23 mmol) of Example 9 and 2,2,2-trifluoroethyl iodide (523 mg, 2.50 mmol).

To a solution of the obtained 6-chloro-2-[(4-chlorobenzyl)thio]-1-[4-(2,2,2-trifluoroethoxy)phenyl]-1H-benzimidazole (80 mg) in diethyl ether (10 mL) was added a 4N hydrogen chloride-ethyl acetate solution (0.5 mL), and the mixture was concentrated under reduced pressure concentration. The residue was recrystallized from diethyl ether to give the title compound (55 mg, yield 42%) as white crystals.
¹H-NMR (300MHz, CDCl₃) δ: 4.46 (2H, q, J = 7.9 Hz), 5.09 (2H, s), 7.12 (1H, d, J = 1.8 Hz), 7.19 (2H, d, J = 9.0 Hz), 7.26-7.32 (4H, m), 7.45-7.50 (3H, m), 8.12 (1H, d, J = 8.7 Hz).
melting point: 106-107°C
Elemental analysis:
Calculated (%) for C₂₂H₁₅N₂OSCl₃F₃·0.9HCl·0.2H₂O:C, 50.84; H, 3.12; N, 5.39; C1, 19.78.
Found (%):C, 50.90; H, 3.24; N, 5.39; C1, 19.85.

### Example 121

### 5-Chloro-2-[(4-chlorobenzyl)thio]-1-(4-propoxyphenyl)-1H-benzimidazole hydrochloride

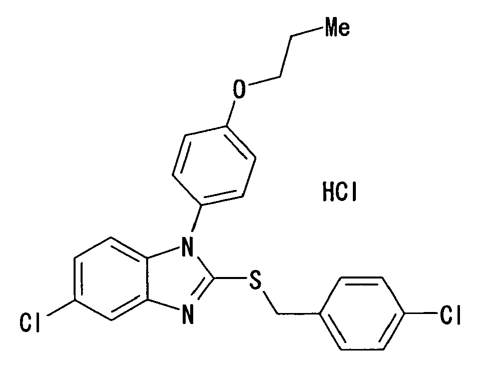

By the reaction in the same manner as in Example 120, the title compound (103 mg, yield 58%) was obtained as white crystals from the compound (150 mg, 0.37 mmol) of Example 44 and n-propyl iodide (635 mg, 3.74 mmol).
¹H-NMR (300MHz, CDCl₃) δ: 1.08 (3H, t, J = 7.4 Hz), 1.81-1.93 (2H, m), 4.01 (2H, t, J = 6.5 Hz), 5.09 (2H, s), 7.05-7.10 (3H, m), 7.20-7.29 (4H, m), 7.37 (1H, dd, J = 1.8, 8.7 Hz), 7.47 (2H, d, J = 8.7 Hz), 8.21 (1H, d, J = 1.2 Hz). melting point: 104-112°C
Elemental analysis:
Calculated (%) for C₂₃H₂₀N₂OSCl₃F₃·0.9HCl·0.2H₂O:C, 57.58; H, 4.43; N, 5.84; C1, 21.43.
Found (%):C, 57.51; H, 4.42; N, 5.82; Cl, 21.15.

### Example 122

### 1-(4-Butoxyphenyl)-5-chloro-2-[(4-chlorobenzyl)thio]-1H-benzimidazole hydrochloride

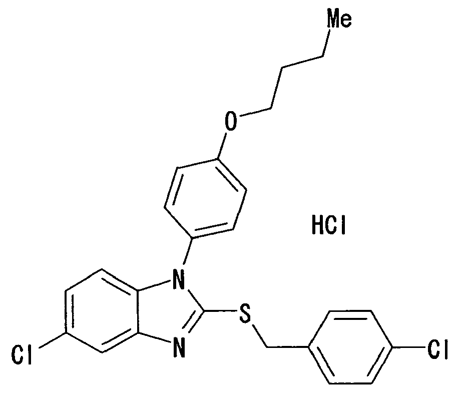

By the reaction in the same manner as in Example 120, the title compound (52 mg, yield 28%) was obtained as white crystals from the compound (150 mg, 0.37 mmol) of Example 44 and n-butyl iodide (688 mg, 3.74 mmol).
¹H-NMR (300MHz, CDCl₃) δ: 1.01(3H, t, J = 7.4 Hz), 1.47-1.59 (2H, m), 1.78-1.87 (2H, m), 4.04 (2H, t, J = 6.5 Hz), 5.08 (2H, s), 7.05-7.10 (3H, m), 7.20-7.29 (4H, m), 7.36 (1H, dd, J = 1.8, 9.0 Hz), 7.47 (2H, d, J = 8.4 Hz), 8.21 (1H, d, J = 1.5 Hz). melting point: 83-89°C
Elemental analysis:
Calculated (%) for C₂₄H₂₂N₂OSCl₂·0.9HCl·0.9H₂O:C, 56.92; H, 4.92; N, 5.53.
Found (%):C, 56.90; H, 4.87; N, 5.53.

### Example 123

### 5-Chloro-2-[(4-chlorobenzyl)thio]-1-[4-(cyclopropylmethoxy)phenyl]-1H-benzimidazole hydrochloride

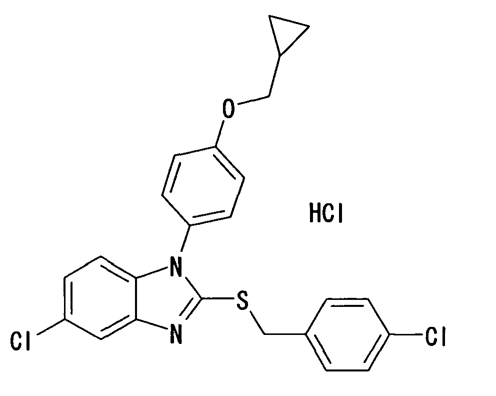

By the reaction in the same manner as in Example 120, the title compound (131 mg, yield 72%) was obtained as white crystals from the compound (150 mg, 0.37 mmol) of Example 44 and (bromomethyl)cyclopropane (504 mg, 3.74 mmol).
¹H-NMR (300MHz, CDCl₃) δ: 0.37-0.42 (2H, m), 0.68-0.74 (2H, m), 1.28-1.36 (1H, m), 3.89 (2H, t, J = 6.9 Hz), 5.08 (2H, s), 7.08 (3H, t, J = 8.4 Hz), 7.20-7.29 (4H, m), 7.37 (1H, dd, J = 1.5, 9.0 Hz), 7.47 (2H, d, J = 8.4 Hz), 8.22 (1H, s). melting point: 118-126°C
Elemental analysis:
Calculated (%) for C₂₄H₂₀N₂OSCl₂·0.9HCl·0.2H₂O:C, 58.61; H, 4.32; N, 5.69; C1, 20.90.
Found (%):C, 58.51; H, 4.33; N, 5.68; Cl, 20.84.

### Example 124

### 6-Chloro-2-[(4-chlorobenzyl)thio]-1-[4-(4,5-dihydro-1,3-oxazol-2-yl)phenyl]-1H-benzimidazole

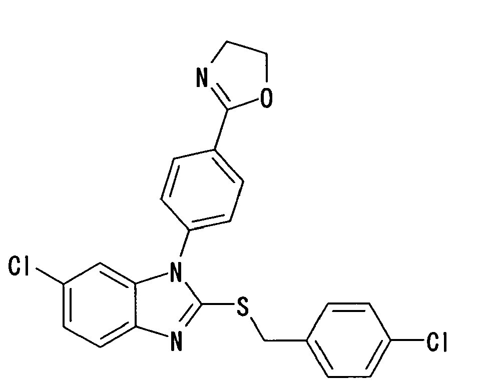

A mixture of the compound (50 mg, 0.11 mmol) of Example 43 and thionyl chloride (1 mL) was stirred at room temperature for 24 hr. To the reaction mixture was added saturated aqueous sodium hydrogencarbonate, and the mixture was extracted with ethyl acetate. The organic layers were combined, washed with saturated brine, dried over magnesium sulfate and concentrated under reduced pressure.

The residue was purified by silica gel chromatography (eluent; ethyl acetate/hexane=1/3 to 1/2) to give 4-{6-chloro-2-[(4-chlorobenzyl)thio]-1H-benzimidazol-1-yl}-N-(2-chloroethyl)benzamide (40 mg) as a white solid.

To a solution of the obtained 4-{6-chloro-2-[(4-chlorobenzyl)thio]-1H-benzimidazol-1-yl}-N-(2-chloroethyl)benzamide (40 mg, 0.081 mmol) in THF (5 mL) was added sodium hydride (10 mg, 0.24 mmol), and the mixture was stirred at room temperature for 3 hr. To the reaction mixture was added saturated aqueous sodium hydrogencarbonate, and the mixture was extracted with ethyl acetate. The organic layer was washed with saturated brine, dried over magnesium sulfate and concentrated under reduced pressure.

The residue was purified by silica gel chromatography (eluent; ethyl acetate/hexane=1/2) and recrystallized from ether-hexane to give the title compound (20 mg, yield 54%) as white crystals.
¹H-NMR (300MHz, CDCI₃) δ: 4.13 (2H, t, J = 9.5 Hz), 4.84 (2H, t, J = 9.4 Hz), 4.54 (2H, s), 7.12 (1H, d, J = 1.8 Hz), 7.22-7.26 (3H, m), 7.33 (2H, d, J = 8.7 Hz), 7.41 (2H, d, J = 8.4 Hz), 7.63 (1H, d, J = 8.4 Hz), 8.12 (2H, d, J = 8.7 Hz). melting point: 143-144°C
Elemental analysis:
Calculated (%) for C₂₃H₁₇N₃OSCl₂·0.1H₂O:C, 60.56; H, 3.80; N, 9.21.
Found (%):C, 60.35; H, 3.86; N, 9.06.

### Example 125

### 6-Chloro-2-[(4-chlorobenzyl)thio]-1-(6-methoxypyridin-3-yl)-1H-benzimidazole hydrochloride

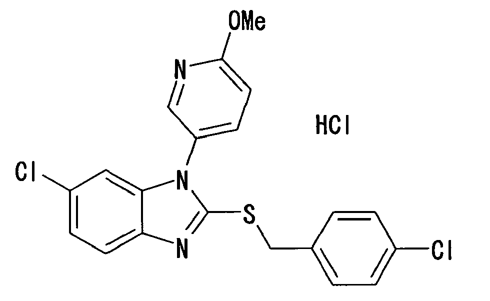

By the reaction in the same manner as in Example 1, 6-chloro-2-[(4-chlorobenzyl)thio]-1-(6-methoxypyridin-3-yl)-1H-benzimidazole (120 mg) was obtained as a colorless oil from the compound (100 mg, 0.34 mmol) of Reference Example 117 and p-chlorobenzyl chloride (66 mg, 0.41 mmol).
¹H-NMR (300MHz, CDCl₃) δ:4.01 (3H, s), 4.53 (2H, s), 6.89 (1H, d, J = 8.7 Hz), 7.04 (1H, d, J = 2.1 Hz), 7.21-7.26 (3H, m), 7.31-7.35 (2H, m), 7.51 (1H, dd, J = 2.7, 8.7 Hz), 7.62 (1H, d, J = 8.7 Hz), 8.18 (1H, d, J = 2.1 Hz).

To a solution of the obtained 6-chloro-2-[(4-chlorobenzyl)thio]-1-(6-methoxypyridin-3-yl)-1H-benzimidazole (120 mg) in diethyl ether (5 mL) was added a 4N hydrogen chloride-ethyl acetate solution (0.5 mL), and the mixture was concentrated under reduced pressure to give the title compound (92 mg, yield 55%) as a white powder.
melting point: 80-86°C
Elemental analysis:
Calculated (%) for C₂₀H₁₅N₃OSCl₂·HCl·0.7H₂O:C, 51.51; H, 3.76; N, 9.00.
Found (%) :C, 51.71; H, 3.60; N, 9.13.

### Example 126

### 6-Chloro-1-(6-methoxypyridin-3-yl)-2-({[6-(trifluoromethyl)pyridin-3-yl]methyl}thio)-1H-benzimidazole hydrochloride

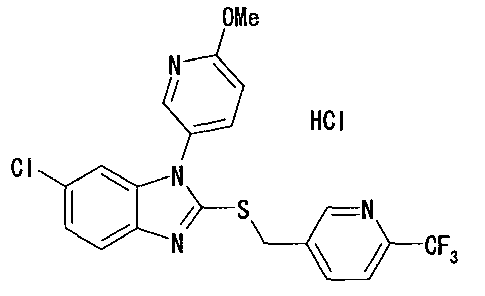

By the reaction in the same manner as in Example 1, 6-chloro-1-(6-methoxypyridin-3-yl)-2-({[6-(trifluoromethyl)pyridin-3-yl]methyl}thio)-1H-benzimidazole (120 mg) was obtained as a colorless oil from the compound (100 mg, 0.34 mmol) of Reference Example 117 and 3-(chloromethyl)-6-(trifluoromethyl)pyridine (80 mg, 0.41 mmol).
¹H-NMR (300MHz, CDCl₃) δ:4.02 (3H, s), 4.61 (2H, s), 6.92 (1H, d, J = 8.7 Hz), 7.05 (1H, d, J = 1.8 Hz), 7.23 (1H, dd, J = 2.1, 8.4 Hz), 7.53 (1H, dd, J = 1.8, 9.0 Hz), 7.61 (2H, d, J = 8.4 Hz), 7.99 (1H, dd, J = 1.8, 8.1 Hz), 8.18 (1H, d, J = 2.4 Hz), 8.82 (1H, d, J = 1.8 Hz).

To a solution of the obtained 6-chloro-1-(6-methoxypyridin-3-yl)-2-({[6-(trifluoromethyl)pyridin-3-yl]methyl}thio)-1H-benzimidazole (120 mg) in diethyl ether (5 mL) was added a 4N hydrogen chloride-ethyl acetate solution (0.5 mL), and the mixture was concentrated under reduced pressure to give the title compound (93 mg, yield 49%) as a white powder.
melting point: 69-76°C
Elemental analysis:
Calculated (%) for C₂₀H₁₄N₄OSClF₃·0.9HCl:C, 48.28; H, 3.17; N, 11.85.
Found (%):C, 48.68; H, 3.16; N, 11.57.

### Example 127

### 6-Chloro-1-(4-ethoxyphenyl)-2-[(4-isopropylbenzyl)thio]-1H-benzimidazole hydrochloride

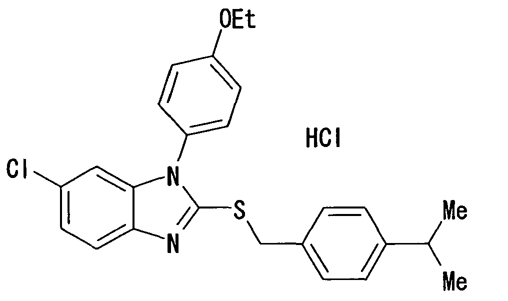

By the reaction in the same manner as in Example 8, 6-chloro-1-(4-ethoxyphenyl)-2-[(4-isopropylbenzyl)thio]-1H-benzimidazole (130 mg) was obtained as a colorless oil from the compound (150 mg, 0.49 mmol) of Reference Example 6 and 4-isopropylbenzyl chloride (100 mg, 0.59 mmol).

To a solution of the obtained 6-chloro-1-(4-ethoxyphenyl)-2-[(4-isopropylbenzyl)thio]-1H-benzimidazole (130 mg) in diethyl ether (5 mL) was added a 4N hydrogen chloride-ethyl acetate solution (0.3 mL), and the mixture was concentrated under reduced pressure. The residue was further recrystallized from diethyl ether-hexane to give the title compound (67 mg, yield 31%) as white crystals.
¹H-NMR (300MHz, CDCl₃) δ:1.20 (6H, d, J = 6.9 Hz), 1.48 (3H, t, J = 7.0 Hz), 2.82-2.91 (1H, m), 4.11 (2H, q, J = 7.0 Hz), 5.06 (2H, s), 7.06 (2H, d, J = 9.0 Hz), 7.13-7.26 (5H, m), 7.36 (2H, d, J = 8.4 Hz), 7.46 (1H, dd, J = 2.1, 8.7 Hz), 8.14 (1H, d, J = 8.7 Hz). melting point: 96-100°C
Elemental analysis:
Calculated (%) for C₂₅H₂₅N₂OSCl·HCl:C, 63.42; H, 5.54; N, 5.92. Found (%):C, 63.59; H, 5.52; N, 5.78.

### Example 128

### 2-(4-{6-Chloro-2-[(4-chlorobenzyl)thio]-1H-benzimidazol-1-yl}phenyl)propan-2-ol

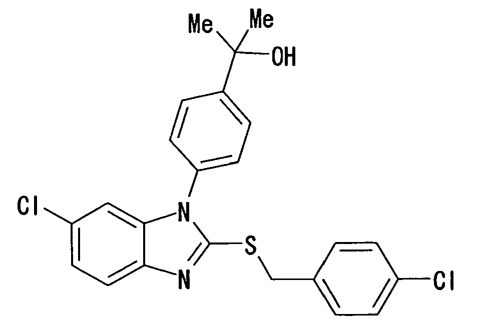

To a solution of the compound (300 mg, 0.66 mmol) of Example 19 in THF (20 mL) was added a 3.0 M diethyl ether solution (0.66 mL, 1.98 mmol) of methylmagnesium bromide under ice-cooling, and the mixture was stirred at 0°C for 2 hr and at room temperature for 3 hr. To the reaction mixture was added saturated aqueous sodium hydrogencarbonate, and the mixture was extracted with ethyl acetate. The organic layer was washed with saturated brine, dried over magnesium sulfate and concentrated under reduced pressure.

The residue was purified by silica gel chromatography (eluent; ethyl acetate/hexane=2/5) and recrystallized from ether-hexane to give the title compound (54 mg, yield 18%) as a white amorphous solid.
¹H-NMR (300MHz, CDCl₃) δ:1.49 (6H, s), 4.58 (2H, s), 5.19 (1H, s), 7.10 (1H, d, J = 1.5 Hz), 7.26 (1H, dd, J = 1.2, 8.7 Hz), 7.35-7.50 (6H, m), 7.68 (3H, t, J = 7.8 Hz).
Elemental analysis:
Calculated (%) for C₂₃H₂₀N₂OSCl₂:C, 62.30; H, 4.55; N, 6.32. Found (%):C, 62.29; H, 4.52; N, 6.06.

### Example 129

### 2-[4-({[6-Chloro-1-(4-ethoxyphenyl)-1H-benzimidazol-2-yl]thio}methyl)phenyl]propan-2-ol

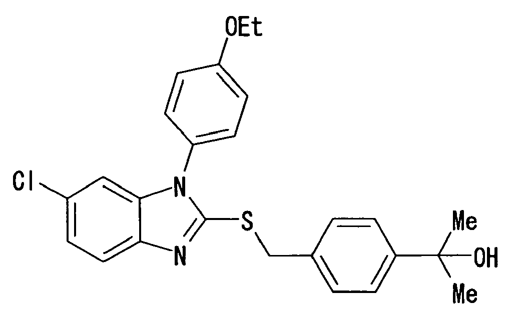

To a solution of the compound (200 mg, 0.44 mmol) of Example 37 in THF (15 mL) was a 3.0 M diethyl ether solution (0.44 mL, 1.32 mmol) of methylmagnesium bromide under ice-cooling, and the mixture was stirred at 0°C for 2 hr and at room temperature for 3 hr. To the reaction mixture was added saturated aqueous sodium hydrogencarbonate, and the mixture was extracted with ethyl acetate. The organic layer was washed with saturated brine, dried over magnesium sulfate and concentrated under reduced pressure.

The residue was purified by silica gel chromatography (eluent; ethyl acetate/hexane=2/5) to give the title compound (91 mg, yield 47%) as a white amorphous solid.
¹H-NMR (300MHz, CDCl₃) δ:1.34-1.38 (9H, m), 4.11 (2H, q, J = 6.9 Hz), 4.54 (2H, s), 4.96 (1H, s), 7.07-7.13 (3H, m), 7.25 (1H, dd, J = 1.8, 8.4 Hz), 7.33-7.41 (6H, m), 7.66 (1H, d, J = 8.4 Hz).
Elemental analysis:
Calculated (%) for C₂₅H₂₅N₂OSCl:C, 66.29; H, 5.56; N, 6.18. Found (%):C, 66.00; H, 5.63; N, 6.05.

### Example 130

### 6-Chloro-2-[(4-chlorobenzyl)thio]-1-(pyridin-2-yl)-1H-benzimidazole

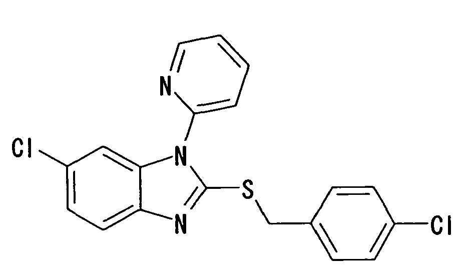

By the reaction in the same manner as in Example 8, the title compound (74 mg, yield 83%) was obtained as white crystals from the compound (60 mg, 0.23 mmol) of Reference Example 118 and p-chlorobenzyl chloride (41 mg, 0.25 mmol).
¹H-NMR (300MHz, CDCI₃) δ:4.58 (2H, s), 7.24-7.27 (3H, m), 7.35-7.41 (3H, m), 7.48-7.54 (2H, m), 7.63 (1H, d, J = 8.7 Hz), 7.93 (1H, dt, J = 1.5, 7.8 Hz), 8.65-8.67 (1H, m). melting point: 107-108°C
Elemental analysis:
Calculated (%) for C₁₉H₁₃N₃OSCl₂:C, 59.07; H, 3.39; N, 10.88. Found (%):C, 59.24; H, 3.26; N, 10.86.

### Example 131

### 5,6-Dichloro-2-[(4-chlorobenzyl)thio]-1-[4-(2,2,2-trifluoroethoxy)phenyl]-1H-benzimidazole

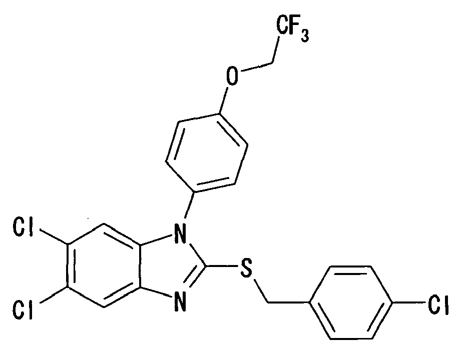

By the reaction in the same manner as in Example 10, 5,6-dichloro-2-[(4-chlorobenzyl)thio]-1-[4-(2,2,2-trifluoroethoxy)phenyl]-1H-benzimidazole (80 mg) was obtained as a colorless oil from the compound (100 mg, 0.23 mmol) of Example 109 and 2,2,2-trifluoroethyl iodide (481 mg, 2.29 mmol). Recrystallized from diethyl ether gave the title compound (66 mg, yield 52%) as white crystals.
¹H-NMR (300MHz, CDCl₃) δ: 4.42 (2H, q, J = 7.9 Hz), 4.53 (2H, s), 7.02-7.14 (3H, m), 7.23-7.35 (6H, m), 7.80 (1H, s).
melting point: 90-92°C
Elemental analysis:
Calculated (%) for C₂₂H₁₄N₂OSCl₃F₃:C, 51.03; H, 2.72; N, 5.41. Found (%):C, 51.12; H, 2.74; N, 5.45.

### Example 132

### 6-Chloro-2-[(4-chlorobenzyl)thio]-1-[4-(1,3-dioxolan-2-yl)phenyl]-1H-benzimidazole

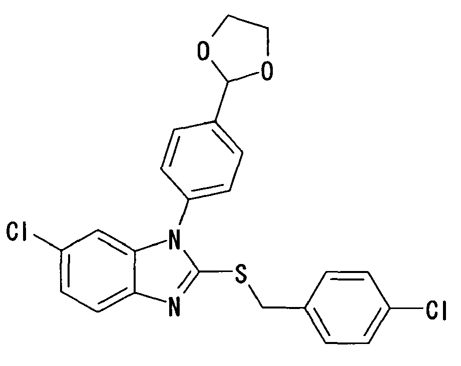

A mixture of the compound (500 mg, 1.20 mmol) of Example 115 and manganese dioxide (2.5 g) in chloroform (30 mL) was stirred at room temperature for 80 hr. The reaction mixture was filtrated, and the filtrate was concentrated and recrystallized from diethyl ether to give 4-{6-chloro-2-[(4-chlorobenzyl)thio]-1H-benzimidazol-1-yl}benzaldehyde (346 mg, 70%) as white crystals.

A solution of the obtained 4-{6-chloro-2-[(4-chlorobenzyl)thio]-1H-benzimidazol-1-yl}benzaldehyde (100 mg, 0.24 mmol), ethylene glycol (0.2 mL, 2.40 mmol) and p-toluenesulfonic acid monohydrate (50 mg, 0.26 mmol) in toluene (5 mL) was heated under reflux for 3 hr. After cooling, saturated aqueous sodium hydrogencarbonate was added, and the mixture was extracted with ethyl acetate. The organic layer was washed with saturated brine, dried over magnesium sulfate and concentrated under reduced pressure.

The residue was purified by silica gel chromatography (eluent; ethyl acetate/hexane=1/3) and recrystallized from diethyl ether-hexane to give the title compound (62 mg, yield 56) as white crystals.
¹H-NMR (300MHz, CDCl₃) δ:4.04-4.20 (4H, m), 4.54 (2H, s), 5.89 (1H, s), 7.10 (1H, d, J = 2.1 Hz), 7.21-7.28 (3H, m), 7.32-7.40 (4H, m), 7.61-7.68 (3H, m). melting point: 129-130°C
Elemental analysis:
Calculated (%) for C₂₃H₁₈N₂O₂SCl₂:C, 60.40; H, 3.97; N, 6.12. Found (%):C, 60.61; H, 4.04; N, 6.16.

### Example 133

### 1-[4-(Benzyloxy)pheriyl]-5-chloro-2-[(4-chlorobenzyl)thio]-1H-benzimidazole

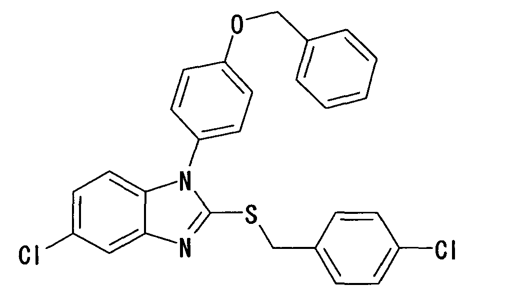

By the reaction in the same manner as in Example 10, the title compound (100 mg, yield 81%) was obtained as white crystals from the compound (100 mg, 0.25 mmol) of Example 44 and benzyl bromide (51 mg, 0.30 mmol).
¹H-NMR (300MHz, CDCl₃) δ: 4.54 (2H, s), 5.12 (2H, s), 6.98 (1H, d, J = 8.4 Hz), 7.04-7.19 (3H, m), 7.23-7.28 (5H, m), 7.33-7.48 (6H, m), 7.70 (1H, d, J = 2.1 Hz). melting point: 137-138°C
Elemental analysis:
Calculated (%) for C₂₇H₂₀N₂OSCl₂:C, 65.99; H, 4.10; N, 5.70. Found (%):C, 65.84; H, 4.04; N, 5.64.

### Example 134

### 4-{2-[(4-tert-Butylbenzyl)amino]-5,6-dichloro-1H-benzimidazol-1-yl}phenol

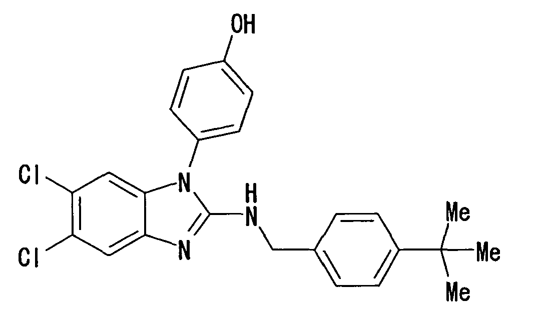

By the reaction in the same manner as in Example 44, the title compound (65 mg, yield 8 %) was obtained as a white powder from the compound (800 mg, 1.76 mmol) of Example 92.
¹H-NMR (300MHz, CDCl₃) δ:1.32 (9H, s), 3.40 (1H, brs), 4.50 (2H, brs), 6.93-7.00 (3H, m), 7.18-7.36 (7H, m), 7.52 (1H, d, J = 1.8 Hz).

### Example 135

### 6-Chloro-2-[(4-chlorobenzyl)thio]-1-[4-(1,1,2,2-tetrafluoroethoxy)phenyl]-1H-benzimidazole

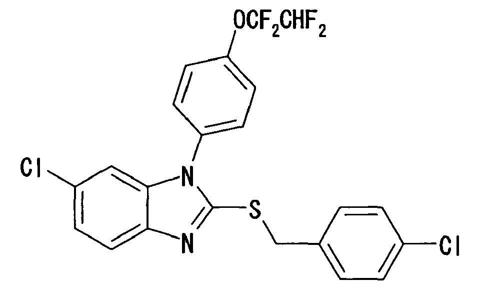

By the reaction in the same manner as in Example 8, the title compound (6 mg, yield 15%) was obtained as white crystals from the compound (30 mg, 0.080 mmol) of Reference Example 111 and p-chlorobenzyl chloride (15 mg, 0.096 mmol).
¹H-NMR (300MHz, CDCl₃) δ: 4.55 (2H, s), 5.96 (1H, tt, J = 2.7, 53.0 Hz), 7.10 (1H, d, J = 2.1 Hz), 7.17-7.27 (4H, m), 7.33-7.39 (5H, m), 7.64 (1H, d, J = 8.7 Hz). melting point: 139-140°C
Elemental analysis:
Calculated (%) for C₂₂H₁₄N₂OSCl₂F₄:C, 52.71; H, 2.81; N, 5.59. Found (%):C, 52.35; H, 2.96; N, 5.12.

### Example 136

### 6-Chloro-2-[(4-chlorobenzyl)thio]-1-[4-(1,3-oxazol-5-yl)phenyl]-1H-benzimidazole

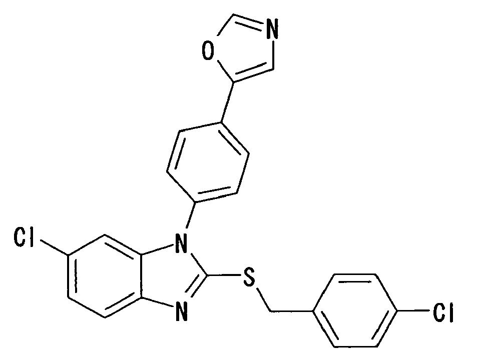

A mixed solution of 4-{6-chloro-2-[(4-chlorobenzyl)thio]-1H-benzimidazol-1-yl}benzaldehyde (150 mg, 0.36 mmol) obtained in the production process of Example 132, tosylmethyl isocyanide (71 mg, 0.36 mmol) and potassium carbonate (50 mg, 0.36 mmol) in methanol (20 mL) was heated under reflux for 2 hr. After cooling, water was added, and the mixture was extracted with ethyl acetate. The organic layer was washed with saturated brine, dried over magnesium sulfate and concentrated under reduced pressure.

The residue was purified by silica gel chromatography (eluent; ethyl acetate/hexane=1/3) to give the title compound (115 mg, yield 71%) as white crystals.
¹H-NMR (300MHz, CDCI₃) δ:4.56 (2H, s), 7.13 (1H, d, J = 1.5 Hz), 7.23-7.27 (3H, m), 7.34 (2H, d, J = 8.4 Hz), 7.42-7.46 (3H, m), 7.64 (1H, d, J = 8.7 Hz), 7.83 (2H, d, J = 9.0 Hz), 7.98 (1H, s). melting point: 130-131°C
Elemental analysis:
Calculated (%) for C₂₃H₁₅N₃OSCl₂:C, 61.07; H, 3.34; N, 9.29. Found (%):C, 61.00; H, 3.27; N, 9.12.

### Example 137

### 2-[(4-Bromobenzyl)thio]-7-chloro-1-(4-ethoxyphenyl)-1H-benzimidazole

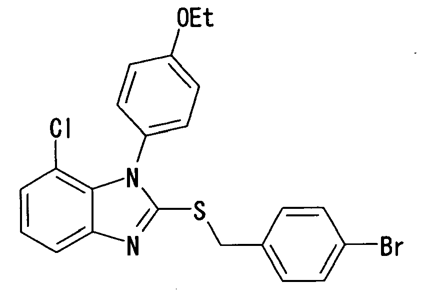

By the reaction in the same manner as in Example 8, the title compound (89 mg, yield 57%) was obtained as white crystals from the compound (100 mg, 0.33 mmol) of Reference Example 121 and p-bromobenzyl bromide (98 mg, 0.39 mmol).
¹H-NMR (300MHz, CDCl₃) δ: 1.46 (3H, t, J = 7.0 Hz), 4.09 (2H, q, J = 7.0 Hz), 4.51 (2H, s), 6.95 (2H, d, J = 9.0 Hz), 7.11-7.29 (6H, m), 7.40 (2H, d, J = 8.7 Hz), 7.62 (1H, dd, J = 1.8, 7.5 Hz). melting point: 143-144°C
Elemental analysis:
Calculated (%) for C₂₂H₁₈N₂OSBrCl:C, 55.77; H, 3.83; N, 5.91. Found (%):C, 55.80; H, 3.84; N, 5.89.

### Example 138

### 7-Chloro-2-[(4-chlorobenzyl)thio]-1-(4-ethoxyphenyl)-1H-benzimidazole

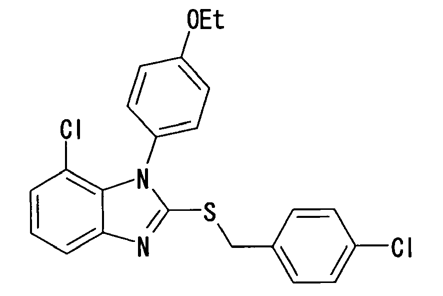

By the reaction in the same manner as in Example 8, the title compound (111 mg, yield 78%) was obtained as white crystals from the compound (100 mg, 0.33 mmol) of Reference Example 121 and p-chlorobenzyl chloride (63 mg, 0.39 mmol).
¹H-NMR (300MHz, CDCl₃) δ: 1.45 (3H, t, J = 7.0 Hz), 4.09 (2H, q, J = 7.0 Hz), 4.52 (2H, s), 6.95 (2H, d, J = 9.0 Hz), 7.09-7.26 (6H, m), 7.34 (2H, d, J = 8.4 Hz), 7.63 (1H, dd, J = 1.8, 7.5 Hz).
melting point: 130-131°C
Elemental analysis:
Calculated (%) for C₂₂H₁₈N₂OSCl₂:C, 61.54; H, 4.23; N, 6.52. Found (%):C, 61.47; H, 4.01; N, 6.26.

### Example 139

### 2-[(4-tert-Butylbenzyl)thio]-7-chloro-1-(4-ethoxyphenyl)-1H-benzimidazole

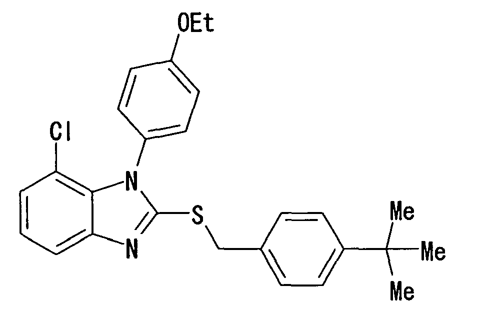

By the reaction in the same manner as in Example 8, the title compound (103 mg, yield 69%) was obtained as white crystals from the compound (100 mg, 0.33 mmol) of Reference Example 121 and 4-tert-butylbenzyl chloride (72 mg, 0.39 mmol). ¹H-NMR (300MHz, CDCl₃) δ: 1.28 (9H, s), 1.45 (3H, t, J = 7.0 Hz), 4.07 (2H, q, J = 7.0 Hz), 4.55 (2H, s), 6.94 (2H, d, J = 8.7 Hz), 7.08-7.33 (8H, m), 7.64 (1H, dd, J = 1.8, 7.5 Hz).
melting point: 175-176°C
Elemental analysis:
Calculated (%) for C₂₆H₂₇N₂OSCl:C, 69.24; H, 6.03; N, 6.21.
Found (%) :C, 69.11; H, 6.02; N, 6.10.

### Example 140

### 5-Chloro-2-[(4-chlorobenzyl)thio]-1-[4-(trifluoromethoxy)phenyl]-1H-benzimidazole hydrochloride

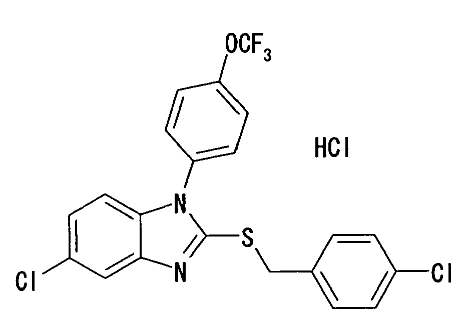

By the reaction in the same manner as in Example 8, 5-chloro-2-[(4-chlorobenzyl)thio]-1-[4-(trifluoromethoxy)phenyl]-1H-benzimidazole (20 mg) was obtained as a colorless oil from the compound (30 mg, 0.087 mmol) of Reference Example 122 and p-chlorobenzyl chloride (17 mg, 0.10 mmol).

To a solution of the obtained 5-chloro-2-[(4-chlorobenzyl)thio]-1-[4-(trifluoromethoxy)phenyl]-1H-benzimidazole (20 mg) in diethyl ether (5 mL) was added a 4N hydrogen chloride-ethyl acetate solution (0.5 mL), and the mixture was concentrated under reduced pressure. The residue was recrystallized from diethyl ether to give the title compound (8 mg, yield 18%) as white crystals.
¹H-NMR (300MHz, CDCl₃) δ: 5.06 (2H, s), 7.08 (1H, d, J = 8.7 Hz), 7.17-7.50 (9H, m), 8.18 (1H, d, J = 1.8 Hz).
melting point: 96-100°C
Elemental analysis:
Calculated (%) for C₂₁H₁₃N₂OSCl₂F₃·HCl:C, 49.87; H, 2.79; N, 5.54. Found (%):C, 50.01; H, 2.78; N, 5.57.

### Example 141

### 6-Chloro-2-[(4-chlorobenzyl)thio]-1-[4-(trifluoromethoxy)phenyl]-1H-benzimidazole

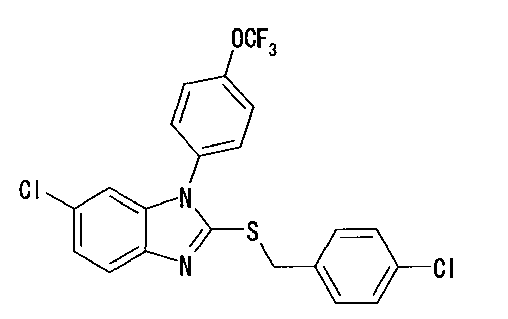

By the reaction in the same manner as in Example 8, the title compound (61 mg, yield 45%) was obtained as white crystals from the compound (100 mg, 0.29 mmol) of Reference Example 74 and p-chlorobenzyl chloride (56 mg, 0.35 mmol).
¹H-NMR (300MHz, CDCI₃) δ: 4.55 (2H, s), 7.09 (1H, d, J = 2.4 Hz), 7.20-7.29 (3H, m), 7.33 (2H, d, J = 8.4 Hz), 7.40 (4H, s), 7.63 (1H, d, J = 8.4 Hz).
melting point: 88-89°C
Elemental analysis:
Calculated (%) for C₂₁H₁₃N₂OSCl₂F₃:C, 52.14; H, 3.04; N, 5.79. Found (%) :C, 51.94; H, 2.77; N, 5.38.

### Example 142

### 5,6-Dichloro-2-[(4-chlorobenzyl)thio]-1-[4-(trifluoromethoxy)phenyl]-1H-benzimidazole

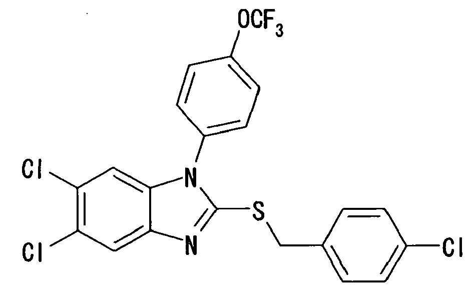

By the reaction in the same manner as in Example 8, the title compound (91 mg, yield 69%) was obtained as white crystals from the compound (100 mg, 0.26 mmol) of Reference Example 110 and p-chlorobenzyl chloride (51 mg, 0.32 mmol).
¹H-NMR (300MHz, CDCI₃) δ: 4.54 (2H, s), 7.19 (1H, s), 7.25-7.28 (3H, m), 7.33 (1H, s), 7.40 (4H, s), 7.81 (1H, s).
melting point: 111-112°C
Elemental analysis:
Calculated (%) for C₂₁H₁₂N₂OSCl₃F₃:C, 50.07; H, 2.40; N, 5.56.
Found (%):C, 50.16; H, 2.45; N, 5.58.

| **Formulation Example 1** (production of capsule) | |
|---|---|
| 1) compound of Example 10 | 30 mg |
| 2) microcrystalline cellulose | 10 mg |
| 3) lactose | 19 mg |
| 4) magnesium stearate | 1 mg |
| Total | 60 mg |

The above-mentioned 1), 2), 3) and 4) are mixed and filled in a gelatin capsule.

| **Formulation Example 2** (production of tablet) | |
|---|---|
| 1) compound of Example 10 | 30 g |
| 2) lactose | 50 g |
| 3) corn starch | 15 g |
| 4) carboxymethylcellulose calcium | 44 g |
| 5) magnesium stearate | 1 g |
| 1000 tablets total | 140 g |

The total amount of the above-mentioned 1), 2) and 3) and 30 g of 4) are kneaded with water, vacuum dried and granulated. The granulated powder is mixed with 14 g of 4) and 1 g of 5) and tableted with a tableting machine. In this way, 1000 tablets containing 30 mg of the compound of Example 10 per tablet are obtained.

### Experimental Example 1

### Evaluation of GPR40 antagonist activity with changes in intracellular calcium concentration as index

An assay system of GPR40 antagonist was established as follows. In this assay system, as the GPR40 agonist, 3-(4-((6-benzyloxy-2',6'-dimethyl-biphenyl-3-yl)methoxy)phenyl)propanoic acid (compound described in Example 340 of WO2004/041266) was used.

First, NFAT-TA was cut out from pNFAT-TA-Luc (manufactured by BD Bioscience Clontech) with HindIII and NheI, the recovered fragment was inserted into pGL3 basic neo digested with HindIII and NheI to give plasmid:pGL3basic neo NFAT-TA-Luc.

This plasmid was used for "the CHO cell line (CHO-hGPR40 No.104), which was made to express human GPR40" described in WO2004/041266, and a cell line CHO(dhfr-)/GPR40-NFAT-Luc, wherein a reporter gene was stably expressed by a method known *per se,* was prepared.

This cell line was diluted with a medium (nucleic acid-free MEM (Minimum Essential Medium)-α medium added with dialyzed FBS (Fetal Bovine Serum) to a final concentration of 10%) in such a manner that 2×10⁴ cells/100 µl would be contained, dispensed to a white walled 96 well plate (manufactured by Coster) by 100 µl per well, and cultured overnight in a CO₂ incubator. Then the medium was removed by suction from the 96 well plate, and a 10 mM test compound-dimethyl sulfoxide (manufactured by Wako Pure Chemical Industries, Ltd.) solution diluted with a medium (nucleic acid-free MEM-α medium added with bovine serum albumin (BSA) free of fatty acid to a final concentration of 0.1%) to each concentration was added by 50 µl per well. Further, 3-(4-((6-benzyloxy-2',6'-dimethyl-biphenyl-3-yl)methoxy)phenyl)propanoic acid diluted with a medium (nucleic acid-free MEM-α medium added with BSA free of fatty acid to a final concentration of 0.1%) to a final concentration of 40 nM was added to the 96 well plate by 50 µl per well.

After culture in a CO₂ incubator for 4 hr, the culture medium was removed from the 96 well plate. Then, PicaGene (manufactured by TOYO INK MFG. CO., LTD.) was added to the 96 well plate, and the luciferase activity was determined using PerkinElmer ARVO (trade name, manufactured by PerkinElmer), based on which changes in the intracellular calcium concentration were evaluated. After assay of luciferase activity, nonlinear regression analysis was performed using PRISM 3 (trade name, manufactured by GraphPad) and IC₅₀ value was calculated with changes in the intracellular calcium concentration as an index.

As a result, the IC₅₀ value of the compounds described in Example 7, Example 8, Example 10, Example 12, Example 36, Example 46, Example 50, Example 51 and Example 67 was less than 100 nM. In other words, it has been confirmed that the compound of the present invention is a superior GPR40 antagonist.

### Experimental Example 2

### Evaluation of insulin secretion suppressive activity using rat pancreatic islet

The effect of the compound of the present invention on the insulin secretion from the rat pancreatic islet, which is induced by palmitic acid stimulation, was evaluated.

Male SD rats (7-week-old, Clea Japan, Inc.) were anesthetized with Nembutal (50 mg/kg, i.p.) and subjected to laparotomy. While confirming and following the course of the common bile duct to the peripheral thereof, and the duct was ligated at the liver side immediately from the opening of the duodenum. Then, the common bile duct was detached from the surrounding tissue on the duodenum side from the hepatic duct bifurcation and a thread was placed thereon. An indwelling needle (22G, manufactured by TERUMO CORPORATION) was inserted into the common bile duct at a position on the liver side from this part and fixed by ligation with the thread placed earlier thereon. 10 ml of 150 mg/ml collagenase (manufactured by Wako Pure Chemical Industries, Ltd., for cell dispersion)-Hank's Balanced Salt Solution (HBSS, manufactured by Invitrogen)-1% Bovine Serum Albumin (BSA, manufactured by Wako Pure Chemical Industries, Ltd.)-0.01 mg/ml DnaseI (manufactured by Roche Molecular Biochemicals) - 2.5% N-2-Hydroxyethylpiperazine-N'-2-Ethane Sulfonic Acid (HEPES, manufactured by Invitrogen) was injected from the common bile duct. The pancreas was removed from the rat, placed in a 50 ml centrifugation tube and shaken in a thermostatic tank at 37°C for 20 min.

The tube was vigorously shaken up and down with a hand for 30 sec, a HBSS-10% Fetal Bovine Serum (FBS)-0.01 mg/ml DnaseI - 2.5% HEPES solution (30 ml) was added and the mixture was ice-cooled.

The obtained pancreatic tissue solution was filtered through a polypropylene mesh (manufactured by Osaka Rikou, 750 micron screen) to remove undigested tissues and the filtrate was centrifuged (1200 rpm, 30 sec). The sediment was dispersed in HBSS-1% BSA -0.01 mg/ml DnaseI - 2.5% HEPES, stood in ice for 5 min and the supernatant was discarded. The sediment was dispersed again in HBSS-1% BSA -0.01 mg/ml DnaseI - 2.5% HEPES, stood in ice for 5 min and the supernatant was discarded. The sediment was dispersed again in HBSS-1% BSA -0.01 mg/ml DnaseI - 2.5% HEPES and the dispersion was centrifuged (1200 rpm, 3 min).

The isolated pancreatic islet was obtained from the sediment using the Ficoll-Conray specific gravity gradient. First, Ficoll-Conray solution A [containing 8.3% Ficoll PM400 (trade name, manufactured by Amersham Biosciences) and Conray 400 (trade name, sodium iotalamate (66.8 w/v%), manufactured by Daiichi Pharmaceutical Co., Ltd.) corresponding to 11.1% based on sodium iotalamate, 1 mg/ml glucose, 100 µg/ml streptomycin, 100 unit/ml penicillin-G and 0.002% phenol red], solution D (the aforementioned solution A added with an equivalent amount of distilled water), solution C (the aforementioned solution D added with equivalent amount of solution A), solution B (the aforementioned solution C added with equivalent amount of solution A) were prepared.

Ficoll-Con ray solution A (7 ml) was added to the above-mentioned sediment, and transferred to a 15 ml centrifugation tube by pipetting. Solution B (2 ml), solution C (2 ml) and solution D (2 ml) were gently overlayered in this order in the tube.

The thus-obtained tube was centrifuged at 2000 rpm for 10 min, and the pancreatic islet gathered in the solution C - solution D boundary layer and solution B - solution C boundary layer was recovered. The recovered pancreatic islet was suspended in HBSS-1% BSA -0.01 mg/ml DnaseI - 2.5% HEPES and centrifuged at 1200 rpm for 3 min. This operation was repeated twice to wash the pancreatic islet. The pancreatic islet after washing was cultured overnight in RPMI1640 medium (containing 5.5 mM glucose)-10% FBS-1 mM HEPES in a 5% CO₂ incubator at 37°C.

Then, pancreatic islet having the same size was collected under a microscope, centrifuged at 1200 rpm for 3 min and suspended in 10 ml of 1 mM glucose-KRBH-BSA (116 mM NaCl-4.7 mM KCl-1.17 mM KH₂PO₄-1.17 mM MgSO₄-25 mM NaHCO₃-2.52 mM CaCl₂-24 mM HEPES-0.2% BSA). The operation of suspending again in 10 ml of 1 mM glucose-KRBH-BSA and centrifuging at 1200 rpm for 3 min was repeated twice. The obtained suspension was transferred into a 3.5 cm ϕ schale and stood still in a 5% CO₂ incubator at 37°C.

The thus-obtained pancreatic islet was placed in each well of a 48 well plate by 10 islets and 1 mM glucose-KRBH-BSA was added to 132 µl.

Then, the test compound was suspended in 1 mM glucose-KRBH-BSA (test compound addition group) or 1 mM glucose-KRBH-BSA (test compound non-addition group) to a 20-fold final concentration and 20 µl thereof was added to each well.

The thus-obtained 48 well plate was stood still in a 5% CO₂ incubator at 37°C for 15 min and 48 µl of an aqueous palmitic acid (manufactured by CHEM SERVICE)-BSA (6:1) solution (palmitic acid addition group) or 10% BSA aqueous solution (palmitic acid non-addition group) was added to each well. Then, 200 µl of 31 mM glucose-KRBH-BSA (16 mM glucose addition group) or 1 mM glucose-KRBH-BSA (1 mM glucose addition group) was added to each well.

The thus-obtained 48 well plate was stood still in a 5% CO₂ incubator at 37°C for 30 min. The supernatant (150 µl) was taken from the obtained mixture, filtered through a 0.45 µm filter, and the insulin concentration of the supernatant was measured by ELISA. The pancreatic islet in the rest of the supernatant was disrupted by ultrasonication, and the DNA concentration of the supernatant was measured using a PicoGreen dsDNA Quantitation Kit (manufactured by Molecular Probes). The insulin amount per unit DNA was calculated from the thus-obtained insulin concentration and the DNA concentration, and taken as the insulin secretion amount (pg/ng DNA). The results are shown in [Table 1].

**[Table 1] Insulin secretion promotion suppressive action by the compound of the present invention**

| Glucose (mM) | compound of Example 10 (µM) | palmitic acid (mM) | insulin secretion amount (pg/ng DNA) |
|---|---|---|---|
| 1 | 0 | 0 | 41±8 |
| 16 | 0 | 0 | 307±92 |
| 16 | 10 | 0 | 353±122 |
| 16 | 0 | 1 | 930±261 |
| 16 | 10 | 1 | 369±141 |

| | | | |
|---|---|---|---|
| each group: mean±SD (n=4) | | | |

As is clear from Table 1, the compound of the present invention decreased the insulin secretion amount from pancreatic islet, which was increased by the addition of palmitic acid. In other words, it has been clarified that the compound of the present invention has an insulin secretion suppressive action.

### Experimental Example 3

### Evaluation method of GPR40 antagonist with changes in intracellular calcium concentration as index

In the same manner as in Experimental Example 1, the IC₅₀ value of the compound of the present invention was calculated.

As a result, the IC₅₀ value of the compounds described in Example 33, Example 38, Example 47, Example 65, Example 66, Example 68, Example 69, Example 70, Example 71, Example 72, Example 73, Example 74, Example 75, Example 76, Example 78, Example 80, Example 81, Example 82, Example 83, Example 86, Example 89, Example 92, Example 93, Example 94, Example 95, Example 96, Example 97, Example 98, Example 99, Example 100, Example 101, Example 102, Example 103, Example 105, Example 106, Example 108, Example 110, Example 111, Example 112, Example 113, Example 121, Example 122, Example 131, Example 132 and Example 135 was less than 100 nM. In other words, it has been confirmed that the compound of the present invention is a superior GPR40 antagonist.

### Industrial Applicability

A fused imidazole compound represented by the formula (I) or a salt thereof or a prodrug thereof has a superior GPR40 receptor function regulating action, and useful as an agent for the prophylaxis or treatment of obesity, hyperinsulinemia and the like.

This application is based on patent application No. 2004-296963 filed in Japan, the content of which is hereby incorporated by reference.

## Claims

1. A GPR40 receptor function regulator comprising a fused imidazole compound represented by the formula: 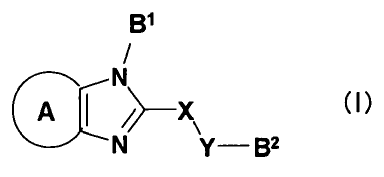 wherein
ring A is an optionally substituted ring,
B¹ and B² are each independently an optionally substituted cyclic group,
X is an optionally substituted C₁₋₃ alkylene group, -O-, -NR^{X}-or -S(O)n^{X}- wherein R^{X} is a hydrogen atom or a substituent, and
n^{X} is an integer of 0 to 2, and
Y is a bond, an optionally substituted C₁₋₃ alkylene group, -O-, -NR^{Y}- or -S(O)n^{Y}- wherein R^{Y} is a hydrogen atom or a substituent, and n^{Y} is an integer of 0 to 2,
or a salt thereof, or a prodrug thereof.

2. The regulator of claim 1, which is a regulator of a physiological function involving a GPR40 receptor or an agent for the prophylaxis or treatment of a pathology or disease involving GPR40 receptor.

3. The regulator of claim 1, which is an insulin secretion regulator, a pancreas protector or an insulin sensitizer.

4. The regulator of claim 1, which is an agent for the prophylaxis or treatment of diabetes, diabetic neuropathy, diabetic nephropathy, retinopathy, obesity, metabolic syndrome, insulin resistance, impaired glucose tolerance, hyperinsulinemia, hypertension, hyperlipidemia, arteriosclerosis, cardiac failure, cardiac infarction, thrombotic disease, deficits in memory and learning, depression and mania, visual disorder, appestat disorder, lipotoxicity, pancreatic fatigue, immune disease, inflammatory disease or cancer.

5. A method of regulating a GPR40 receptor function, which comprises administering an effective amount of the fused imidazole compound or a salt thereof or a prodrug thereof of claim 1 to a mammal.

6. Use of the fused imidazole compound or a salt thereof or a prodrug thereof of claim 1 for the production of a GPR40 receptor function regulator.

7. A compound represented by the formula: 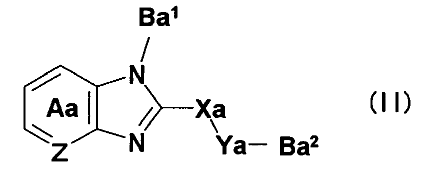 wherein
ring Aa is a benzene ring substituted by substituent(s) other than a nitro group and a diethylsulfamoyl group, or an optionally substituted pyridine ring,
Z is CH or N,
Ba¹ is an optionally substituted 5-membered aromatic group or an optionally substituted 6-membered cyclic group,
Ba² is an optionally substituted 5- or 6-membered aromatic group,
Xa is -O-, -NRa- wherein Ra is a hydrogen atom or an optionally substituted C₁₋₆ alkyl group, or -S-, and
Ya is an optionally substituted C₁₋₃ alkylene group,
provided that Xa-Ya should not be -NHCO-, and Ba¹ should not be a substituted triazinyl group,
or a salt thereof,
with the proviso that 2-(benzylthio)-5-chloro-1-phenyl-1H-benzimidazole and 2-(2-chlorobenzylthio)-5-chloro-1-phenyl-1H-benzimidazole are excluded.

8. The compound of claim 7, wherein ring Aa is a benzene ring substituted by 1 to 3 halogen atoms.

9. The compound of claim 7, wherein Ba¹ is an optionally substituted phenyl group.

10. The compound of claim 7, wherein Ba¹ is a phenyl group having a substituent at the para-position.

11. The compound of claim 10, wherein the substituent is selected from an optionally substituted C₁₋₆ alkyl group, an optionally substituted C₁₋₆ alkoxy group, a C₁₋₆ alkoxy-carbonyl group and a C₁₋₆ alkyl-carbonyl group.

12. The compound of claim 7, wherein Ba² is an optionally substituted phenyl group or an optionally substituted pyridyl group.

13. The compound of claim 7, wherein Ba² is a phenyl group having a substituent at the para-position or a 3-pyridyl group having a substituent at the 6-position.

14. The compound of claim 13, wherein the substituent is selected from a halogen atom and an optionally halogenated C₁₋₆ alkyl group.

15. The compound of claim 7, wherein Xa is -S- or -NH-.

16. The compound of claim 7, wherein Ya is a methylene group.

17. The compound of claim 7, which is
6-chloro-2-[(4-chlorobenzyl)thio]-1-(4-ethoxyphenyl)-1H-benzimidazole,
6-chloro-1-(4-ethoxyphenyl)-2-({[6-(trifluoromethyl)pyridin-3-yl]methyl}thio)-1H-benzimidazole,
2-[(4-tert-butylbenzyl)thio]-6-chloro-1-(4-ethoxyphenyl)-1H-benzimidazole,
2-[(4-chlorobenzyl)thio]-1-(4-methoxyphenyl)-1H-imidazo[4,5-b]pyridine,
5-chloro-2-[(4-chlorobenzyl)thio]-1-[4-(2,2,2-trifluoroethoxy)phenyl]-1H-benzimidazole,
1-(4-butoxyphenyl)-N-(4-tert-butylbenzyl)-5,6-dichloro-1H-benzimidazol-2-amine, or
N-(4-tert-butylbenzyl)-5,6-dichloro-1-[4-(trifluoromethoxy)phenyl]-1H-benzimidazol-2-amine.

18. A prodrug of the compound of claim 7.

19. A pharmaceutical agent comprising the compound of claim 7 or a prodrug thereof.

20. A method of antagonizing a GPR40 receptor, which comprises administering an effective amount of a non-peptidic nitrogen-containing heterocyclic compound to a mammal.
